# EUROPÄISCHE PATENTSCHRIFT

(11) **EP 2 483 268 B1**
(45) Veröffentlichungstag und Bekanntmachung des Hinweises auf die Patenterteilung: **29.06.2016**
(21) Anmeldenummer: 10768174.4
(22) Anmeldetag: 06.09.2010
(51) Int. Cl.: C07D 413/04, C07D 413/14, A61K 31/4178, A61K 31/44, A61P 35/00, A61P 37/00, A61P 29/00, A61P 11/06, C07D 401/04, C07D 401/14, C07D 409/14, C07D 471/04

(54) **PYRIDINYL-IMIDAZOLONDERIVATE ZUR HEMMUNG VON PI3-KINASEN**
PYRIDINYL-IMIDAZOLONE DERIVATIVES FOR INHIBITING PI3 KINASES
DÉRIVÉS DE PYRIDINYL-IMIDAZOLONE POUR L'INHIBITION DES KINASES PI3

(30) Priorität: 28.09.2009 DE 102009043260
(43) Veröffentlichungstag der Anmeldung: 08.08.2012
(73) Patentinhaber: Merck Patent GmbH, 64293 Darmstadt (DE)
(72) Erfinder: EMDE, Ulrich, 64291 Darmstadt (DE); BUCHSTALLER, Hans-Peter, 64347 Griesheim (DE); KLEIN, Markus, 64291 Darmstadt (DE); ESDAR, Christina, 55128 Mainz (DE); BOMKE, Joerg, 69123 Heidelberg (DE)
(86) Internationale Anmeldenummer: PCT/EP2010/005459
(87) Internationale Veröffentlichungsnummer: WO 2011/035855

(56) Entgegenhaltungen:
- WO-A1-2005/044130
- WO-A1-2007/021877
- WO-A1-2009/114552

## Beschreibung

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und deren Verwendung zur Modulation, insbesondere zur Hemmung der Aktivität oder Funktion der Phosphoinositid-3'-OH-Kinase-Familie (nachstehend PI3-Kinasen), zweckmäßigerweise PI3Kα, PI3Kδ, PI3Kβ und/oder PI3Kγ. Die vorliegende Erfindung betrifft zweckmäßigerweise die Verwendung von Pyridinyl-imidazolonderivaten bei der Behandlung von einer oder mehreren Krankheitszuständen, ausgewählt aus: Autoimmunstörungen, Entzündungskrankheiten, Herz-Kreislauf-Erkrankungen, neurodegenerativen Krankheiten, Allergie, Asthma, Pankreatitis, Multiorganversagen, Nierenkrankheiten, Blutplättchenaggregation, Krebs, Spermienbeweglichkeit, Transplantationsabstoßung, Transplantatabstoßung und Lungenverletzungen.

Zellmembranen stellen einen großen Speicher für sekundäre Botenstoffe bereit, die bei einer Reihe von Signaltransduktionswegen in Anspruch genommen werden können. Bezüglich der Funktion und Regulation von Effektorenzymen bei Phospholipid-Signalwegen erzeugen diese Enzyme sekundäre Botenstoffe aus den Membran-Phospholipid-Pools. PI3-Kinasen der Klasse I (beispielsweise PI3Kα) sind Kinase-Enzyme mit doppelter Spezifität, d. h. sie zeigen sowohl Lipidkinase-Aktivität (Phosphorylierung von Phosphoinositiden) als auch Proteinkinaseaktivität, von der gezeigt wurde, dass sie Protein als Substrat phosphorylieren kann, einschließlich der Autophosphorylierung als intramolekularer regulatorischer Mechanismus. Diese Enzyme der Phospholipid-Signalwirkung werden durch verschiedene extrazelluläre Signale, wie Wachstumsfaktoren, Mitogene, Integrine (Zell-Zell-Wechselwirkungen), Hormone, Cytokine, Viren, und Neurotransmitter, wie in Schema I nachstehend beschrieben, und auch durch die intrazelluläre Regulation durch andere Signalwirkungsmoleküle (Cross-Talk, wobei das ursprüngliche Signal einige parallele Wege aktivieren kann, die in einem zweiten Schritt durch intrazelluläre Signalwirkungsereignisse Signale auf PI3Ks übertragen), wie beispielsweise kleine GTPasen, Kinasen, oder Phosphatasen, aktiviert. Die intrazelluläre Regulation kann auch aufgrund einer aberranten oder fehlenden Expression zellulärer Onkogene oder Tumorsuppressoren auftreten. Die intrazellulären Inositol-Phopholipid (Phosphoinositide)-Signalwege starten mit der Aktivierung von Signalwirkungsmolekülen (extrazellulären Liganden, Stimuli, Rezeptordimerisierung, Transaktivierung durch einen heterologen Rezeptor (beispielsweise Rezeptortyrosinkinase) und mit der Rekrutierung und Aktivierung von PI3K, einschließlich der Beteiligung von G-Protein-verknüpftem Transmembran-Rezeptor, der in die Plasmamembran integriert ist.

PI3K wandelt das Membran-Phospholipid PI(4,5)P₂ in PI(3,4,5)P₃ um, das als sekundärer Botenstoff arbeitet. PI und PI(4)P sind ebenfalls Substrate von PI3K und können zu PI3P bzw. PI(3,4)P₂ phosphoryliert bzw. umgewandelt werden. Zudem können diese Phosphoinositide durch 5-spezifische und 3'-spezifische Phosphatasen in andere Phosphoinositide umgewandelt werden, so dass die PI3K-Enzymaktivität entweder direkt oder indirekt zur Erzeugung von zwei 3-Phosphoinositid-Subtypen führt, die als sekundäre Botenstoffe in intrazellulären Signaltransduktionswegen arbeiten (Trends Biochem. Sci. 22(7) S. 267-72 (1997) von Vanhaesebroeck et al; Chem. Rev. 101(8) S. 2365-80 (2001) von Leslie et al (2001); Annu. Rev. Cell. Dev. Biol. 17p, 615-75 (2001) von Katso et al. und Cell. Mol. Life Sci. 59(5) S. 761-79 (2002) von Toker et al.). Mehrfache PI3K-lsoformen, die durch ihre katalytischen Untereinheiten, ihre Regulation durch entsprechende regulatorische Untereinheiten, Expressionsmuster und signalspezifische Funktionen (p110α, β, δ und γ) kategorisiert werden, führen diese Enzymreaktion aus (Exp. Cell. Res. 25 (1) S. 239-54 (1999) von Vanhaesebroeck und Katso et al., 2001, siehe oben).

Die nah verwandten Isoformen p110α und β werden ubiquitär exprimiert, wohingegen δ und γ spezifischer im hämatopoetischen Zellsystem, in den glatten Muskelzellen, Myozyten und Endothelzellen exprimiert werden (Trends Biochem. Sci. 22(7) S. 267-72 (1997) von Vanhaesebroeck et al.). Ihre Expression kann auch in einer induzierbaren Weise je nach dem zellulären Gewebetyp und Stimuli sowie entsprechend der jeweiligen Krankheit reguliert werden. Die Induzierbarkeit der Proteinexpression umfasst die Proteinsynthese sowie die Proteinstabilisierung, die zum Teil durch Assoziierung mit regulatorischen Untereinheiten reguliert wird.

Bisher wurden acht Säugetier-PI3Ks identifiziert, die auf der Basis der Sequenzhomologie, Struktur, Bindungspartner, Aktivierungsmodus, und Substratvorliebe in 3 Hauptklassen (I, II und III) unterteilt werden. In vitro können PI3Ks der Klasse I Phosphatidylinositol (PI), Phosphatidylinositol-4-phosphat (PI4P) und Phosphatidylinositol-4,5-bisphosphat (PI(4,5)P₂) phosphorylieren, so dass man Phosphatidylinositol-3-phosphat (PI3P), Phosphatidylinositol-3,4-bisphosphat (PI(3,4)P₂, bzw. Phosphatidylinositol-3,4,5-trisphosphat (PI(3,4,5)P₃ erhält. PI3Ks der Klasse II phosphorylieren PI und Phosphatidylinositol-4-phosphat. PI3Ks der Klasse III können nur PI phosphorylieren (Vanhaesebroeck et al., 1997, siehe oben; Vanhaesebroeck et al., 1999, siehe oben und Leslie et al, 2001, siehe oben).

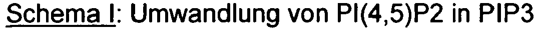

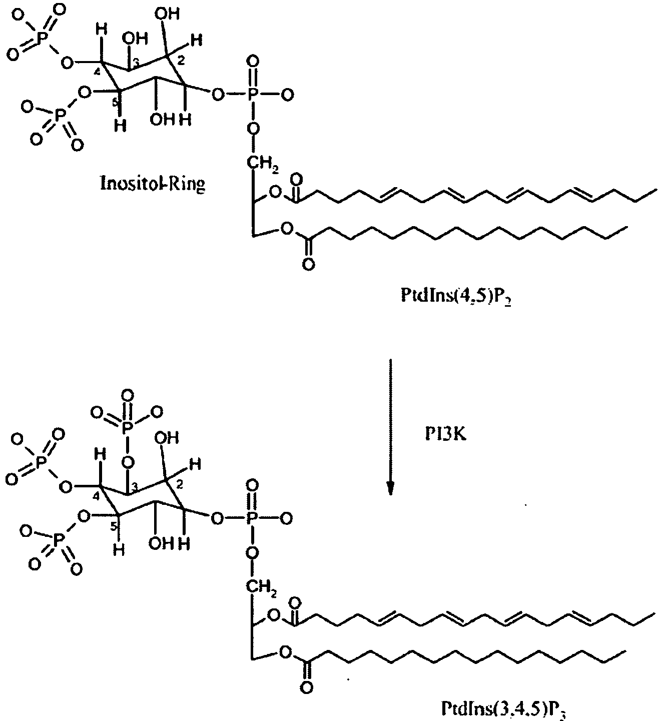

Wie in Schema I oben veranschaulicht, phosphorylieren die Phosphoinositid-3-Kinasen (PI3Ks) das Hydroxyl des dritten Kohlenstoffatoms am Inositol-Ring. Die Phosphorylierung der Phosphoinositide, die Ptdlns in 3,4,5-Triphosphat (Ptdlns(3,4,5)P₃), Ptdlns(3,4)P₂ und Ptdlns(3)P überführt, ergibt sekundäre Botenstoffe für verschiedene Signaltransduktionswege, wie sie u. a. für Zellproliferation, Zelldifferenzierung, Zellwachstum, Zellgröße, Zellüberleben, Apoptose, Adhäsion, Zellbeweglichkeit, Zellwanderung, Chemotaxis, Invasion, Cytoskelett-Umlagerung, Zellformänderungen, Vesikel-Trafficking und Stoffwechselweg essentiell sind (Katso et al, 2001, siehe oben and Mol. Med. Today 6(9) S. 347-57 (2000) von Stein). G-Protein-gekoppelte Rezeptoren vermitteln die Phosphoinositid-3'-OH-Kinase-Aktivierung über kleine GTPasen, wie Gβγ und Ras, und folglich spielt die PI3K-Signalwirkung eine zentrale Rolle bei der Entwicklung und Koordination der Zellpolarität und der dynamischen Organisierung des Cytoskeletts - die zusammen die Antriebsquelle zur Zellbewegung bereitstellen.

Chemotaxis - die gerichtete Bewegung von Zellen in Richtung eines Konzentrationsgradienten chemischer Lockstoffe, die auch als Chemokine bezeichnet werden, ist auch an vielen wichtigen Krankheiten beteiligt, wie Entzündung/ Autoimmunität, Neurodegeneration, Antiogenese, Invasion/Metastase und Wundheilung (Immunol. Today 21(6) S. 260-4 (2000) von Wyman et al.; Science 287(5455) S. 1049-53 (2000) von Hirsch et al.; FASEB J. 15(11) S. 2019-21 (2001) von Hirsch et al. und Nat. Immunol. 2(2) S. 108-15 (2001) von Gerard et al.).

Fortschritte mit genetischen Ansätzen und pharmakologischen Werkzeugen haben Einblicke in die Signal- und Molekülwege vermittelt, die die Chemotaxis in Reaktion auf durch chemische Lockstoffe aktivierte G-Protein-gekoppelte Sensoren vermitteln. PI3-Kinase, die zur Erzeugung dieser phosphorylierten Signalwirkungsprodukte verantwortlich ist, wurde ursprünglich als Aktivität identifiziert, die mit viralen Onkoproteinen und Wachstumsfaktor-Tyrosinkinasen assoziiert ist, die Phosphatidylinositol (PI) und seine phosphorylierten Derivate am 3'-Hydroxyl des Inositol-Rings phosphorylieren (Panayotou et al., Trends Cell Biol. 2 S. 358-60 (1992)). Neuere biochemische Untersuchungen haben jedoch ergeben, dass PI3-Kinasen der Klasse I (beispielsweise die Klasse IB Isoform PI3Kγ) doppelt spezifische Kinase-Enzyme sind, was bedeutet, dass sie sowohl Lipidkinase- als auch Proteinkinase-Aktivität aufweisen, von der gezeigt wurde, dass sie zur Phosphorylierung anderer Proteine als Substrate sowie zur Autophosphorylierung als intramolekularer regulatorischer Mechanismus fähig ist.

PI3-Kinase-Aktivierung ist daher wahrscheinlich an verschiedenen Zellantworten beteiligt, einschließlich Zellwachstum, Differenzierung und Apoptose (Parker et al., Current Biology, 5 S. 577-99 (1995); Yao et al., Science, 267 S. 2003-05 (1995)). PI3-Kinasen scheinen an einer Reihe von Aspekten der Leukocyten-Aktivierung beteiligt zu sein. Eine p85-assoziierte PI3-Kinase-Aktivität ist Befunden zufolge physikalisch mit der Cytoplasma-Domäne von CD28 assoziiert, was ein wichtiges co-stimulatorisches Molekül für die Aktivierung von T-Zellen durch Antigen ist (Pages et al., Nature, 369 S. 327-29 (1994); Rudd, Immunity 4 S. 527-34 (1996)). Die Aktivierung von T-Zellen durch CD28 senkt die Schwelle zur Aktivierung durch Antigen und steigert die Höhe und die Dauer der proliferativen Reaktion. Diese Wirkungen gehen mit Anstiegen der Transkription einer Reihe von Genen einher, wie u. a. Interleukin-2 (IL2), einem wichtigen T-Zell-Wachstumsfaktor (Fraser et al., Science 251 S. 313-16 (1991)). Wird CD28 so mutiert, dass es nicht länger mit PI3-Kinase interagieren kann, versagt die Initiation der IL-2-Produktion, was eine entscheidende Rolle für PI3-Kinase bei der T-Zell-Aktivierung nahe legt. PI3Kγ wurde als Mittler der G-β-γ-abhängigen Regulation der JNK-Aktivität identifiziert, und G-β-γ sind Untereinheiten für heterotrimere G-Proteine (Lopez-Ilasaca et al, J. Biol. Chem. 273(5) S. 2505-8 (1998)). Zelluläre Prozesse, bei denen PI3Ks eine wesentliche Rolle spielen, umfassen die Suppression der Apoptose, Reorganisation des Aktinskeletts, Herzmyocyten-Wachstum, Glycogensynthasestimulation durch Insulin, TNFα-vermitteltes Neutrophilen-Priming und Superoxid-Erzeugung und die Leukozytenwanderung und Adhäsion an Endothelzellen.

Von Laffargue et al., Immunity 16(3) S. 441-51 (2002) wurde beschrieben, dass PI3Kγ Entzündungssignale über verschiedene G(i)-gekoppelte Rezeptoren weiterleitet, und dass es für die Mastzellenfunktion, Stimuli im Zusammenhang mit Leukozyten, sowie Immunologie entscheidend ist, einschließlich Cytokinen, Chemokinen, Adenosinen, Antikörpern, Integrinen, Aggregationsfaktoren, Wachstumsfaktoren, Viren oder Hormonen (J. Cell. Sci. 114(Pt 16) S. 2903-10 (2001) von Lawlor et al.; Laffargue et al., 2002, siehe oben und Curr. Opinion Cell Biol. 14(2) S. 203-13 (2002) von Stephens et al.).

Spezifische Inhibitoren gegen einzelne Mitglieder einer Familie von Enzymen liefern unschätzbare Werkzeuge für die Entschlüsselung der Funktionen jedes Enzyms. Zwei Verbindungen, LY294002 und Wortmannin (siehe nachstehend), wurden weithin als PI3-Kinase-lnhibitoren verwendet. Diese Verbindungen sind nicht-spezifische PI3K-lnhibitoren, da sie nicht zwischen den vier Mitgliedern der PI3-Kinasen der Klasse I unterscheiden. Die IC₅₀-Werte von Wortmannin gegen jede der verschiedenen PI3-Kinasen der Klasse I liegen beispielsweise im Bereich von 1 bis 10 nM. Entsprechend sind die IC₅₀-Werte für LY294002 gegen jede dieser PI3-Kinasen etwa 15 bis 20 µM (Fruman et al., Ann. Rev. Biochem., 67, S. 481-507 (1998)), zudem hat es IC₅₀-Werte von 5 - 10 µM auf CK2-Proteinkinase und eine geringfügige inhibitorische Aktivität auf Phospholipasen. Wortmannin ist ein Pilz-Metabolit, der die PI3K-Aktivität durch kovalente Bindung an die katalytische Domäne dieses Enzyms irreversibel hemmt. Die Hemmung der PI3K-Aktivität durch Wortmanin eliminiert die anschließende Zellreaktion auf den extrazellulären Faktor. Neutrophile reagieren beispielsweise auf das Chemokin fMet-Leu-Phe (fMLP) durch Stimulation von PI3K und Synthese von Ptdlns (3, 4, 5)P₃. Diese Synthese korreliert mit der Aktivierung des respiratorischen Bursts, der an der Zerstörung der Neutrophilen eindringender Mikroorganismen beteiligt ist. Die Behandlung der Neutrophilen mit Wortmannin verhindert die fMLP-induzierte respiratorische Burst-Reaktion (Thelen et al., Proc. Natl. Acad. Sci. USA, 91, p. 4960-64 (1994)). Tatsächlich zeigen diese Experimente mit Wortmannin, sowie ein anderer experimenteller Beweis, dass die PI3K-Aktivität in Zellen der hämatopoetischen Linie, insbesondere Neutrophilen, Monocyten und andere Arten von Leukozyten, an vielen der Nicht-Gedächtnis-Immunreaktion beteiligt sind, die mit akuter und chronischer Entzündung einhergehen.

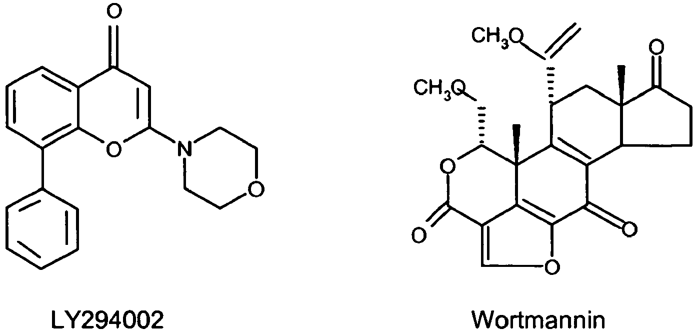

Auf der Basis von Untersuchungen mit Wortmannin gibt es einen Beweis, dass die PI3-Kinasefunktion auch für einige Aspekte der Leukozyten-Signalwirkung durch G-Protein-gekoppelte Rezeptoren notwendig ist (Thelen et al., 1994, siehe oben). Darüber hinaus wurde gezeigt, dass Wortmannin und LY294002 die Neutrophilenwanderung und die Freisetzung von Superoxid blockieren. Die Carboxygenase-hemmenden Benzofuran-Derivate sind von John M. Janusz et al., in J. Med. Chem. 1998; Vol. 41, No. 18. offenbart.

Es ist mittlerweile gut verstanden, dass die Deregulation von Onkogenen und Tumorsuppressorgenen zur Bildung maligner Tumore beiträgt, beispielsweise über den Anstieg von Zellwachstum und Proliferation oder ein gesteigertes Zellüberleben. Es ist nun auch bekannt, dass Signalwege, die durch die PI3K-Familie vermittelt werden, eine zentrale Rolle bei einer Reihe von Zellprozessen spielen, wie u. a. bei der Proliferation, und dem Überleben, und die Deregulation dieser Wege ist ein ursächlicher Faktor bei einem breiten Spektrum von Krebserkrankungen beim Menschen und anderen Krankheiten (Katso et al., Annual Rev. Cell Dev. Biol, 2001, 17: 615-617 und Foster et al, J. Cell Science. 2003, U6: 3037-3040).

PI3K der Klasse I ist ein Heterodimer, das aus einer katalytischen p110-Untereinheit und einer regulatorischen Untereinheit besteht, und die Familie ist auf der Basis der regulatorischen Partner und der Regulationsmechanismen weiter in Enzyme der Klasse la und Klasse Ib unterteilt. Die Enzyme der Klasse la bestehen aus drei verschiedenen katalytischen Untereinheiten (p110 α, p110β, und p110δ), die mit fünf verschiedenen regulatorischen Untereinheiten (p85α, p55α, p50α, p85β und p55γ) dimerisieren, wobei alle katalytischen Untereinheiten mit allen regulatorischen Untereinheiten interagieren können, so dass verschiedene Heterodimere erhalten werden. Die PI3K der Klasse la werden im Allgemeinen in Reaktion auf die Wachstumsfaktor-Stimulation von Rezeptor-Tyrosinkinasen über die Interaktion der regulatorischen SH2-Domänen-Untereinheit mit spezifischen Phosphotyrosin-Resten des aktivierten Rezeptors oder Adapterproteinen, wie IRS-1, aktiviert. Kleine GTPasen (beispielsweise ras) sind ebenfalls an der Aktivierung von PI3K zusammen mit der Rezeptor-Tyrosinkinaseaktivierung beteiligt. Sowohl p110α als auch p110β werden konstitutiv bei allen Zelltypen beteiligt, wohingegen die p110δ-Expression stärker auf Leukozytenpopulationen und einige Epithelzellen eingeschränkt ist. Das einzige Enzym der Klasse Ib besteht dagegen aus einer katalytischen p110γ-Untereinheit, die mit einer regulatorischen p101-Untereinheit interagiert. Das Enzym der Klasse Ib wird darüber hinaus durch G-Protein-gekoppelte Rezeptor-(GPCR)-Systeme aktiviert, und seine Expression scheint auf Leukocyten beschränkt zu sein.

Mittlerweile zeigt ein deutlicher Beweis, dass PI3K-Enzyme der Klasse la zur Tumorigenese bei einer großen Vielzahl von menschlichen Krebserkrankungen beitragen, und zwar entweder direkt oder indirekt (Vivanco and Sawyers, Nature Reviews Cancer, 2002, 2, 489-501). Die p110α-Untereinheit ist beispielsweise in einigen Tumoren amplifiziert, wie beispielsweise in Ovartumoren (Shayesteh, et al., Nature Genetics, 1999, 21: 99-102) und Cervix (Ma et al, Oncogene, 2000, 19: 2739-2744). Neuerdings wurden aktivierende Mutationen in p110α (PIK3CA-Gen) mit verschiedenen anderen Tumoren in Verbindung gebracht, wie beispielsweise Kolon- und Brust- und Lungentumoren (Samuels, et al., Science, 2004, 304, 554). Tumor-verwandte Mutationen bei p85α wurden ebenfalls bei Krebserkrankungen identifiziert, wie Ovar- und Kolon-Krebs (Philp et al., Cancer Research, 2001, 61, 7426-7429). Neben direkten Effekten ist die Aktivierung der PI3K der Klasse I wahrscheinlich an tumorigenen Ereignissen beteiligt, die stromaufwärts von Signalwegen auftreten, beispielsweise mittels ligandenabhängiger oder ligandenunabhängiger Aktivierung von Rezeptor Tyrosin-Kinasen, GPCR-Systemen oder Integrinen (Vara et al., Cancer Treatment Reviews, 2004, 30, 193-204). Beispiele für solche stromaufwärts gelegenen Signalwege umfassen die Überexpression der Rezeptor-Tyrosin-Kinase Erb2 bei einer Reihe von Tumoren, die zur Aktivierung von PI3K-vermittelten Wegen (Harari et al., Oncogene, 2000, Jj), 6102-6114) und Überexpression des Onkogens Ras führen (Kauffmann-Zeh et al., Nature, 1997, 385, 544-548). Zudem können PI3Ks der Klasse la indirekt zur Tumorigenese beitragen, die durch verschiedene stromabwärts gelegene Signalereignisse verursacht werden. Der Funktionsverlust der PTEN-Tumorsuppressor-Phosphatase, die die Umwandlung von PI(3,4,5,)P₃ zurück zu PI(4,5)P₂ katalysiert, ist beispielsweise mit einem sehr breiten Bereich an Tumoren über die Deregulation der PI3K-vermittelten Produktion von PI(3,4,5)P₃ assoziiert (Simpson and Parsons, Exp. Cell Res., 2001, 264, 29-41). Zudem trägt die Steigerung der Wirkungen anderer PI3K-vermittelter Signalereignisse wahrscheinlich zu einer Reihe von Krebserkrankungen bei, beispielsweise durch Aktivierung von AKT (Nicholson and Andeson, Cellular Signaling, 2002, 14, 381-395).

Neben einer Rolle bei der Vermittlung der Proliferations- und Überlebenssignalwirkung in Tumorzellen gibt es einen guten Beweis, dass PI3K-Enzyme der Klasse I auch zur Tumorigenese beitragen, und zwar über ihre Funktion bei tumorassoziierten Stromazellen. Die PI3K-Signalwirkung spielt bekanntlich eine wichtige Rolle bei der Vermittlung angiogener Ereignisse in Endothelzellen in Reaktion auf proangiogene Faktoren wie VEGF (abid et al., Arterioscler. Thromb. Vasc. Biol., 2004, 24, 294-300). Da die PI3K-Enzyme der Klasse I auch an Beweglichkeit und Wanderung beteiligt sind (Sawyer, Expert Opinion investing. Drugs, 2004, 13, 1-19), wird angenommen, dass die PI3K-Inhibitoren einen therapeutischen Nutzen über die Hemmung von Tumorzellinvasion und Metastase bereitstellen.

Die Synthese von kleinen Verbindungen, die die Signaltransduktion der PI3-Kinasen spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.
Es wurde gefunden, dass die erfindungsgemäßen Verbindungen Inhibitoren der Phosphoinositid-3-Kinasen (PI3Ks) sind.
Die erfindungsgemäßen Verbindungen inhibieren Proteinkinasen, insbesondere PI3K, mTOR and DNA-PK. Darüber hinaus aktivieren sie die Foxo3A
Translocation.
Laut Literatur spielen DNA-PK und mTOR ebenso wie die PI3K's eine wichtige Rolle in biochemischen Regulationsmechnismen und damit z.B. bei der Entstehung und Ausbreitung von Krebserkrankungen.
So sind andere heterocyclische DNA-PK Inhibitoren in WO 2009/130469 beschrieben.
mTOR-Kinase-Inhibitoren zur Krebsbekämpfung sind in WO 2010/062571 A1 beschrieben.

Wird das Phosphoinositid-3-Kinase-(PI3K)-Enzym durch eine erfindungsgemäße Verbindung gehemmt, kann PI3K nicht seine enzymatischen, biologischen und/oder pharmakologischen Wirkungen ausüben. Die erfindungsgemäßen Verbindungen eignen sich daher zur Behandlung von Autoimmun-Erkrankungen, Entzündungserkrankungen, Herz-Kreislauf-Erkrankungen, neurodegenerativen Erkrankungen, Allergie, Asthma, Pankreatitis, Multiorganversagen, Nierenerkrankungen, Blutplättchenaggregation, Krebs, Spermienbeweglichkeit, Transplantationsabstoßung, Transplantat-Abstoßung und Lungenverletzungen. Die Verbindungen der Formel I eignen sich insbesondere als Arzneimittel für die Behandlung von Autoimmun-Krankheiten, Entzündungserkrankungen, Herz-Kreislauf-Erkrankungen, neurodegenerativen Erkrankungen, Allergie, Asthma, Pankreatitis, Multiorganversagen, Nierenerkrankungen, Blutplättchenaggregation, Krebs, Spermienbeweglichkeit, Transplantationsabstoßung, Transplantatabstoßung und Lungenverletzungen.
Die Verbindungen der Formel I eignen sich insbesondere auch für die Behandlung von Krebs zusammen mit einer Strahlentherapie.

Gemäß einer Ausführungsform der vorliegenden Erfindung sind die Verbindungen der Formel (I) Inhibitoren von einer oder mehreren Phosphatoinositid-3-Kinasen (PI3Ks), zweckmäßigerweise Phosphatoinositid-3-kinase γ (PI3Kγ), Phosphatoinositid-3-kinase α (PI3Kα), Phosphatoinositid-3-kinase β (PI3Kβ), und/oder Phosphatoinositid-3-kinase δ (PI3K δ).

Die Verbindungen der Formel (I) eignen sich zur Modulation, insbesondere zur Hemmung der Aktivität von Phosphatoinositid-3-kinasen (PI3K), zweckmäßigerweise Phosphatoinositid-3-kinase (PI3Kδ). Daher eignen sich die erfindungsgemäßen Verbindungen auch zur Behandlung von Störungen, die durch PI3Ks vermittelt werden. Die Behandlung beinhaltet die Modulation - insbesondere die Hemmung oder die Abwärtsregulation - von Phosphatoinositid-3-kinasen.

Vorzugsweise werden die erfindungsgemäßen Verbindungen zur Herstellung eines Arzneimittels zur Behandlung einer Störung verwendet, die aus multipler Sklerose, Psoriasis, rheumatoider Arthritis, systemischem Lupus erythematodes, entzündlicher Darmerkrankung, Lungenentzündung, Thrombose oder Gehirninfektion bzw. -entzündung, wie Meningitis oder Encephalitis, Alzheimer-Krankheit, Chorea Huntington, ZNS-Trauma, Schlaganfall, oder ischämischen Zuständen, Herzkreislauf-Erkrankungen, wie Atherosklerose, Herzhypertrophie, Fehlfunktion der Herzmyocyten, erhöhtem Blutdruck oder Vasokonstriktion ausgewählt ist.

Vorzugsweise eignen sich die Verbindungen der Formel I zur Verwendung zur Behandlung von Autoimmunkrankheiten oder Entzündungskrankheiten, wie multipler Sklerose, Psoriasis, rheumatoider Arthritis, systemischem Lupus erythematodes, entzündlicher Darmerkrankung, Lungenentzündung, Thrombose oder Gehirninfektion bzw. -entzündung, wie Meningitis oder Encephalitis.

Vorzugsweise eignen sich die Verbindungen der Formel I zur Verwendung zur Behandlung von neurodegenerativen Krankheiten, wie u. a. multipler Sklerose, Alzheimer-Krankheit, Chorea Huntington, ZNS-Trauma, Schlaganfall oder ischämischen Zuständen.

Vorzugsweise eignen sich die Verbindungen der Formel I zur Verwendung zur Behandlung von Herz-Kreislauf-Erkrankungen, wie Atherosklerose, Herzhypertrophie, Fehlfunktion der Herzmyocyten, erhöhtem Blutdruck oder Vasokonstriktion.

Vorzugsweise eignen sich die Verbindungen der Formel I zur Verwendung zur Behandlung von chronischer obstruktiver Lungenkrankheit, Fibrose nach anaphylaktischem Schock, Psoriasis, allergischen Erkrankungen, Asthma, Schlaganfall, ischämischen Zuständen, Ischämie-Reperfusion, Blutplättchen-Aggregation bzw. Aktivierung, Skelettmuskel-Atrophie bzw. -Hypertrophie, Leukozyten-Rekrutierung bei Krebsgewebe, Angiogenese, Invasions-Metastase, insbesondere Melanom, Karposi-Sarkom, akuten und chronischen Bakterien- und Virusinfektionen, Sepsis, Transplantationsabstoßung, Transplantatabstoßung, Glomerulosklerose, Glomerulomephritis, progressiver Nierenfibrose, Endothel- und Epithel-Verletzungen in der Lunge und Atemwegsentzündung in der Lunge.

Da die pharmazeutisch aktiven Verbindungen der vorliegenden Erfindung als PI3-Kinase-lnhibitoren aktiv sind, insbesondere die Verbindungen, die pI3Kα entweder selektiv oder zusammen mit einer oder mehreren von PI3Kδ, PI3Kβ und/oder PI3Kγ hemmen, weisen sie bei der Behandlung von Krebs therapeutischen Nutzen auf.

Die Erfindung betrifft vorzugsweise Verbindungen der Formel I zur Verwendung zur Behandlung von Krebs in einem Säugetier, einschließlich Mensch, wobei der Krebs ausgewählt ist aus: Gehirn (Gliomen), Glioblastomen, Leukämien, Bannayan-Zonana-Syndrom, Cowden-Krankheit, Lhermitte-Duclos-Krankheit, Brust-, entzündlichem Brust-Krebs, Wilm's Tumor, Ewings's Sarkom, Rhabdomyosarkom, Ependymom, Medulloblastom, Kolon, Kopf und Hals, Niere, Lunge, Leber, Melanom, Ovar, Pankreas, Prostata, Sarkom, Osteosarkom, Riesenzelltumor von Knochen und Thyroid.

Die Erfindung betrifft vorzugsweise Verbindungen der Formel I zur Verwendung zur Behandlung von Krebs in einem Säugetier, einschließlich Mensch, wobei der Krebs ausgewählt ist aus: lymphoblastischer T-Zell-Leukämie, chronischer myelogener Leukämie, chronischer Lymphocyten-Leukämie, Hairy-Cell-Leukämie, akuter lymphoblastischer Leukämie, akuter myelogener Leukämie, chronischer Neutrophilen-Leukämie, akuter lymphoblastischer T-Zell-Leukämie, Plasmacytom, immunoblastischer Großzell-Leukämie, Mantelzell-Leukämie, multiplem Myelom, Megakaryoblasten-Leukämie, multiplem Myelom, akuter Megakaryocyten-Leukämie, Promyelocyten-Leukämie und Erythroleukämie.

Die Erfindung betrifft vorzugsweise Verbindungen der Formel I zur Verwendung zum Behandeln von Krebs in einem Säugetier, einschließlich Mensch, wobei der Krebs ausgewählt ist aus malignem Lymphom, Hodgkins-Lymphom, Non-Hodgkins-Lymphom, lymphoblastischem T-Zell-Lymphom, Burkitt's Lymphom und Follikel-Lymphom.

Die Erfindung betrifft vorzugsweise ein Verfahren zum Behandeln von Krebs in einem Säugetier, einschließlich Mensch, wobei der Krebs ausgewählt ist aus: Neuroblastom, Blasenkrebs, urothelialem Krebs, Lungenkrebs, Vulva-Krebs, Cervix-Krebs, Endometrium-Krebs, Nierenkrebs, Mesotheliom, Ösophagus-Krebs, Speicheldrüsenkrebs, hepatozellulärem Krebs, Darmkrebs, nasopharyngealem Krebs, bukkalem Krebs, Mundkrebs, GIST (gastrointestinalem Stromatumor) und Hodenkrebs.

Weiterhin können die Verbindungen der Formel I verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Weiterhin können die Verbindungen der Formel I zur Isolierung und zur Untersuchung der Aktivität oder Expression von PI3-Kinase verwendet werden. Außerdem eigenen sie sich insbesondere zur Verwendung in diagnostischen Verfahren zu Erkrankungen im Zusammenhang mit unregulierter oder gestörter PI3-Kinase-Aktivität.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer-(HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J.).

### STAND DER TECHNIK

Andere heterocyclische DNA-PK Inhibitoren sind in WO 2009/130469 beschrieben.

mTOR-Kinase-Inhibitoren zur Krebsbekämpfung sind in WO 2010/062571 A1 beschrieben.

Pyridopyrimidinderivate sind als PI3K-lnhibitoren zur Behandlung verschiedenster Krankheiten in WO 2009/039140 beschrieben.

Andere Imidazol(on)-derivate sind offenbart in: WO 2008/094556, WO 2005/105790, WO 2004/026859, WO 2003/035638 und WO 9638421.

Andere Imidazolverbindungen zur Krebsbekämpfung sind in WO 2007/021877 A1 und in WO 2009/114552 beschrieben. Pyrazolverbindungen zur Krebsbekämpfung sind in WO 2005/044130 beschrieben.

### ZUSAMMENFASSUNG DER ERFINDUNG

Die Erfindung betrifft Verbindungen der Formel I 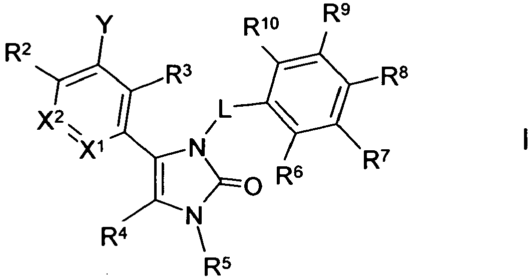 worin
- X¹: CR¹ oder N,
- X²: CR¹ oder N,
wobei X¹ oder X² N
und das jeweils andere CR¹ bedeutet,
- Y: Het oder Ar,
- L: [C(R^{a})₂]ₘ,
- R¹ R², R³, R⁴: H,
- R⁵: H oder A',
- R⁶, R⁹, R¹⁰: H,
- R⁷: H oder A"',
- R⁸: H, A", Het¹, CN, Hal, NHHet¹ oder NA'Het¹,
- A: unverzweigtes oder verzweigtes Alkyl mit 1-10 C-Atomen, worin 1-7 H-Atome durch F und/oder Cl ersetzt sein können, oder
cyclisches Alkyl mit 3-7 C-Atomen,
- A': unverzweigtes oder verzweigtes Alkyl mit 1-6 C-Atomen, worin 1-5 H-Atome durch F und/oder Cl ersetzt sein können,
- A": unverzweigtes oder verzweigtes Alkyl mit 1-10 C-Atomen, worin 1-7 H-Atome durch OH, CN, NH₂, CONH₂, COOH, COOA, NHCOA, -C=H, F, Cl und/oder Br ersetzt sein können, und/oder worin eine oder zwei nicht-benachbarte CH- und/oder CH₂-Gruppen durch O, NH, NA', S, SO, SO₂ und/oder CH=CH-Gruppen ersetzt sein können,
- A'": unverzweigtes oder verzweigtes Alkyl mit 1-10 C-Atomen, worin 1-7 H-Atome durch F und/oder Cl ersetzt sein können, und/oder worin eine oder zwei nicht-benachbarte CH- und/oder CH₂-Gruppen durch O, NH und/oder NA' ersetzt sein können,
- R^{a}: H oder A',
- Het: Pyridinyl, Pyrimidinyl, Furyl, Thienyl, Oxazolyl, Oxadiazolyl, Imidazolyl, Pyrrolyl, Isoxazolyl, Benzodioxolyl, Dihydrobenzofuranyl, Dihydrobenzodioxinyl, Benzofuranyl, Benzodioxanyl, Indolyl, Chinolinyl, Isochinolinyl, Benzimidazolyl, Benzothiadiazolyl, Indazolyl, Dihydrobenzimidazolyl, Dihydroindolyl, Imidazo-pyridinyl, Pyrrolo-pyridinyl, Pyrazolo-pyridinyl, 2,3-Dihydro-pyrrolo-pyridinyl oder Tetrahydro-naphthyridinyl,
wobei die Reste auch ein-, zwei- oder dreifach durch A, (CH₂)ₙNH₂, (CH₂)ₙNHA, (CH₂)ₙNA₂, NHCOA, (CH₂)ₙCONH₂, (CH₂)ₙCONHA, (CH₂)ₙCONA₂, CN, =O, Hal, (CH₂)ₙOH, (CH₂)ₙOA, COOH und/oder COOA substituiert sein können,
- Ar: unsubstituiertes oder ein-, zwei- oder dreifach durch (CH₂)ₙOH und/oder (CH₂)ₙOA substituiertes Phenyl,
- Het¹: Piperidinyl, Pyrrolidinyl, Morpholinyl, Piperazinyl, Oxazolidinyl, Tetrahydrothienyl, Tetrahydropyranyl oder Thiomorpholinyl, wobei die Reste auch ein- oder zweifach durch =O, NH₂, Pyridylmethyl und/oder A substituiert sein können,
- Hal: F, Cl, Br oder I,
- m: 0 oder 1,
- n: 0, 1, 2, 3 oder 4,
bedeuten,
sowie ihre pharmazeutisch verwendbaren Salze, Tautomere und Stereoisomere, einschließlich deren Mischungen in allen Verhältnissen,

Unter Verbindungen der Formel I versteht man auch die Hydrate und Solvate dieser Verbindungen.
Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.
Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I sowie ihrer pharmazeutisch verwendbaren Salze, Tautomeren und Stereoisomeren, dadurch gekennzeichnet, daß man in einer Suzuki-Reaktion eine Verbindung der Formel I,
worin Y Hal bedeutet,
in eine Verbindung der Formel I umwandelt,
worin Y Het oder Ar bedeutet,
und/oder
eine Base oder Säure der Formel I in eines ihrer Salze umwandelt.

Vor- und nachstehend haben die Reste X¹, X², L, Y, R², R³, R⁴, R⁵, R⁶, R⁷, R⁸, R⁹, R¹⁰ die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2-, 3- oder 4-Methylpentyl, 1,1- , 1,2- , 1,3- , 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl. Cyclisches Alkyl (Cycloalkyl) bedeutet vorzugsweise Cyclopropyl, Cyclobutyl, Cylopentyl, Cyclohexyl oder Cycloheptyl.

A' bedeutet vorzugsweise Alkyl, ist unverzweigt (linear) oder verzweigt, und hat 1, 2, 3, 4, 5 oder 6 C-Atome.

A" bedeutet Alkyl, ist unverzweigt (linear) oder verzweigt, und hat 1, 2, 3, 4, 5, 6, 7, 8, 9 oder 10 C-Atome, worin 1-7 H-Atome durch OH, CN, NH₂, CONH₂, COOH, COOA, NHCOA, -C=H, F, Cl und/oder Br ersetzt sein können, und/oder worin eine oder zwei nicht-benachbarte CH- und/oder CH₂-Gruppen durch O, NH, NA', S, SO, SO₂ und/oder CH=CH-Gruppen ersetzt sein können.

A'" bedeutet Alkyl, ist unverzweigt (linear) oder verzweigt, und hat 1, 2, 3, 4, 5, 6, 7, 8, 9 oder 10 C-Atome, worin 1-7 H-Atome durch F und/oder Cl ersetzt sein können,
und/oder worin eine oder zwei nicht-benachbarte CH- und/oder CH₂-Gruppen durch O, NH und/oder NA' ersetzt sein können.

R¹, R², R³, R⁴ bedeuten vorzugsweise H.
R⁶, R⁹, R¹⁰ bedeuten vorzugsweise H.
R⁷ bedeutet vorzugsweise H oder A"'.
R⁸ bedeutet vorzugsweise H, A", Het¹, CN, Hal, NHHet¹ oder NA'Het¹.

Ar bedeutet z.B. Phenyl, o-, m- oder p-Hydroxyphenyl, o-, m- oder p-Methoxyphenyl, o-, m- oder p-Ethoxyphenyl.
Ar bedeutet besonders bevorzugt unsubstituiertes oder ein-, zwei- oder dreifach durch (CH₂)ₙOH und/oder (CH₂)ₙOA substituiertes Phenyl.

Het bedeutet besonders bevorzugt Pyridinyl, Pyrimidinyl, Furyl, Thienyl, Oxazolyl, Oxadiazolyl, Imidazolyl, Pyrrolyl, Isoxazolyl, Benzodioxolyl, Dihydrobenzofuranyl, Dihydrobenzodioxinyl, Benzofuranyl, Benzodioxanyl, Indolyl, Chinolinyl, Isochinolinyl, Benzimidazolyl, Benzothiadiazolyl, Indazolyl, Dihydrobenzimidazolyl, Dihydroindolyl, Imidazo-pyridinyl, Pyrrolo-pyridinyl, Pyrazolo-pyridinyl, 2,3-Dihydro-pyrrolo-pyridinyl oder Tetrahydro-naphthyridinyl,
wobei die Reste auch ein-, zwei- oder dreifach durch A, (CH₂)ₙNH₂, (CH₂)ₙNHA, (CH₂)ₙNA₂, NHCOA, (CH₂)ₙCONH₂, (CH₂)ₙCONHA, (CH₂)nCONA2, CN, =O, Hal, (CH₂)ₙOH, (CH₂)ₙOA, COOH und/oder COOA substituiert sein können.

Het¹ bedeutet vorzugsweise Piperidinyl, Pyrrolidinyl, Morpholinyl, Piperazinyl, Oxazolidinyl, Tetrahydrothienyl, Tetrahydropyranyl oder Thiomorpholinyl, wobei die Reste auch ein- oder zweifach durch =O, NH₂, Pyridylmethyl und/oder A' substituiert sein können.

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind. Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis li ausgedrückt werden, die der Formel I entsprechen und worin die nicht näher bezeichneten Reste die bei der Formel I angegebene Bedeutung haben, worin jedoch
- in Ia R¹, R², R³, R⁴: H bedeuten;
- in Ib R⁶, R⁹, R¹⁰: H bedeuten;
- in Ic R⁷: H oder A"',
bedeutet;
- in Id R⁸: H, A", Het¹, CN, Hal, NHHet¹ oder NA'Het¹,
bedeutet;
- in le Het: einen ein- oder zweikernigen ungesättigten oder aromatischen Heterocyclus mit 1 bis 4 N-, O- und/oder S-Atomen, der unsubstituiert oder ein-, zwei- oder dreifach durch A, (CH₂)ₙNH₂, (CH₂)ₙNHA, (CH₂)ₙNA₂, NHCOA, (CH₂)ₙCONH₂, (CH₂)ₙCONHA, (CH₂)ₙCONA₂, CN, =O, Hal, (CH₂)ₙOH, (CH₂)ₙOA, COOH und/oder COOA substituiert sein kann,
bedeutet;
- in If Ar: unsubstituiertes oder ein-, zwei- oder dreifach durch (CH₂)ₙOH und/oder (CH₂)ₙOA substituiertes Phenyl,
bedeutet;
- in Ig Het: Pyridinyl, Pyrimidinyl, Furyl, Thienyl, Oxazolyl, Oxadiazolyl, Imidazolyl, Pyrrolyl, Isoxazolyl, Benzodioxolyl, Dihydrobenzofuranyl, Dihydrobenzodioxinyl, Benzofuranyl, Benzodioxanyl, Indolyl, Chinolinyl, Isochinolinyl, Benzimidazolyl, Benzothiadiazolyl, Indazolyl, Dihydrobenzimidazolyl, Dihydroindolyl, Imidazo-pyridinyl, Pyrrolo-pyridinyl, Pyrazolo-pyridinyl, 2,3-Dihydro-pyrrolo-pyridinyl oder Tetrahydro-naphthyridinyl, wobei die Reste auch ein-, zwei- oder dreifach durch A, (CH₂)ₙNH₂, (CH₂)ₙNHA, (CH₂)ₙNA₂, NHCOA, (CH₂)ₙCONH₂, (CH₂)ₙCONHA, (CH₂)ₙCONA₂, CN, =O, Hal, (CH₂)ₙOH, (CH₂)ₙOA, COOH und/oder COOA substituiert sein können,
bedeutet;
- in Ih Het¹: Piperidinyl, Pyrrolidinyl, Morpholinyl, Piperazinyl, Oxazolidinyl, Tetrahydrothienyl, Tetrahydropyranyl oder Thiomorpholinyl, wobei die Reste auch ein- oder zweifach durch =O, NH₂, Pyridylmethyl und/oder A substituiert sein können,
bedeutet;
- in li X¹: CR¹ oder N,
- X²: CR¹ oder N,
wobei X¹ oder X² N
und das jeweils andere CR¹ bedeutet,
- Y: Het oder Ar,
- L: [C(R^{a})₂]ₘ,
- R¹, R², R³, R⁴: H,
- R⁵: H oder A',
- R⁶, R⁹, R¹⁰: H,
- R⁷: H oder A'",
- R⁸: H, A", Het¹, CN, Hal, NHHet¹ oder NA'Het¹,
- A: unverzweigtes oder verzweigtes Alkyl mit 1-10 C-Atomen, worin 1-7 H-Atome durch F und/oder Cl ersetzt sein können, oder
cyclisches Alkyl mit 3-7 C-Atomen,
- A': unverzweigtes oder verzweigtes Alkyl mit 1-6 C-Atomen, worin 1-5 H-Atome durch F und/oder Cl ersetzt sein können,
- A": unverzweigtes oder verzweigtes Alkyl mit 1-10 C-Atomen, worin 1-7 H-Atome durch OH, CN, NH₂, CONH₂, COOH, COOA, NHCOA, -C=H, F, Cl und/oder Br ersetzt sein können, und/oder worin eine oder zwei nicht-benachbarte CH- und/oder CH₂-Gruppen durch O, NH, NA', S, SO, SO₂ und/oder CH=CH-Gruppen ersetzt sein können,
- A'": unverzweigtes oder verzweigtes Alkyl mit 1-10 C-Atomen, worin 1-7 H-Atome durch F und/oder Cl ersetzt sein können, und/oder worin eine oder zwei nicht-benachbarte CH- und/oder CH₂-Gruppen durch O, NH und/oder NA' ersetzt sein können,
- R^{a}: H oder A',
- Het: Pyridinyl, Pyrimidinyl, Furyl, Thienyl, Oxazolyl, Oxadiazolyl, Imidazolyl, Pyrrolyl, Isoxazolyl, Benzodioxolyl, Dihydrobenzofuranyl, Dihydrobenzodioxinyl, Benzofuranyl, Benzodioxanyl, Indolyl, Chinolinyl, Isochinolinyl, Benzimidazolyl, Benzothiadiazolyl, Indazolyl, Dihydrobenzimidazolyl, Dihydroindolyl, Imidazo-pyridinyl, Pyrrolo-pyridinyl, Pyrazolopyridinyl, 2,3-Dihydro-pyrrolo-pyridinyl oder Tetrahydronaphthyridinyl,
wobei die Reste auch ein-, zwei- oder dreifach durch A, (CH₂)ₙNH₂, (CH₂)ₙNHA, (CH₂)ₙNA₂, NHCOA, (CH₂)ₙCONH₂, (CH₂)ₙCONHA, (CH₂)ₙCONA₂, CN, =O, Hal, (CH₂)ₙOH, (CH₂)ₙOA, COOH und/oder COOA substituiert sein können,
- Ar: unsubstituiertes oder ein-, zwei- oder dreifach durch (CH₂)ₙOH und/oder (CH₂)ₙOA substituiertes Phenyl,
- Het¹: Piperidinyl, Pyrrolidinyl, Morpholinyl, Piperazinyl, Oxazolidinyl, Tetrahydrothienyl, Tetrahydropyranyl oder Thiomorpholinyl, wobei die Reste auch ein- oder zweifach durch =O, NH₂, Pyridylmethyl und/oder A substituiert sein können,
- Hal: F, Cl, Br oder I,
- m: 0 oder 1,
- n: 0, 1, 2, 3 oder 4,
bedeuten;
sowie ihre pharmazeutisch verwendbaren Salze, Tautomere und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorzugsweise erhalten werden, indem man in einer Suzuki-Reaktion eine Verbindung der Formel I,
worin Y Hal bedeutet,
in eine Verbindung der Formel I umwandelt,
worin Y Het oder Ar bedeutet.

Vorzugsweise wird hierzu eine Verbindung der Formel I,
worin Y Hal bedeutet,
mit einer Verbindung der Formel II

X-Y II,

worin
X vorzugsweise 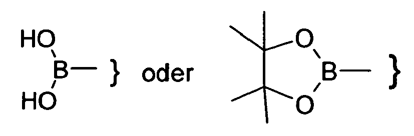 bedeutet,
und Y die in Anspruch 1 angegebene Bedeutung hat,
umgesetzt.

Die Umsetzung erfolgt unter Standardbedingungen einer Suzuki-Kopplung. Die Reaktionszeit liegt je nach den angewendeten Bedingungen zwischen einigen Minuten und 14 Tagen, die Reaktionstemperatur zwischen etwa -30° und 140°, normalerweise zwischen 0° und 100°, insbesondere zwischen etwa 60° und etwa 90°.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist Dimethoxyethan oder Dioxan.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, lodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Besonders bevorzugt sind Hydrochlorid, Dihydrochlorid, Hydrobromid, Maleat, Mesylat, Phosphat, Sulfat und Succinat.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre pharmazeutisch verwendbaren Salze und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie deren Salze davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre pharmazeutisch verwendbaren Salze und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Salze und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze und Stereoisomere, einschließlich deren Mischungen in allen Verhältnissen,
und einer wirksamen Menge eines weiteren Arzneimittelwirkstoffs gelöst oder in lyophilisierter Form vorliegt.

### VERWENDUNG

Die vorliegenden Verbindungen eignen sich als pharmazeutische Wirkstoffe für Säugetiere, insbesondere für den Menschen, bei der Behandlung von Krankheiten.

Die vorliegende Erfindung umfasst die Verbindungen der Formel I zur Verwendung bei der Behandlung oder Vorbeugung von Autoimmun-Erkrankungen, Entzündungserkrankungen, Herz-Kreislauf-Erkrankungen, neurodegenerativen Erkrankungen, Allergie, Asthma, Pankreatitis, Multiorganversagen, Nierenerkrankungen, Blutplättchenaggregation, Krebs, Spermienbeweglichkeit, Transplantationsabstoßung, Transplantat-Abstoßung und Lungenverletzungen.

Vorzugsweise werden die erfindungsgemäßen Verbindungen zur Herstellung eines Arzneimittels zur Behandlung einer Störung verwendet, die aus multipler Sklerose, Psoriasis, rheumatoider Arthritis, systemischem Lupus erythematodes, entzündlicher Darmerkrankung, Lungenentzündung, Thrombose oder Gehirninfektion bzw. -entzündung, wie Meningitis oder Encephalitis, Alzheimer-Krankheit, Chorea Huntington, ZNS-Trauma, Schlaganfall, oder ischämischen Zuständen, Herzkreislauf-Erkrankungen, wie Atherosklerose, Herzhypertrophie, Fehlfunktion der Herzmyocyten, erhöhtem Blutdruck oder Vasokonstriktion ausgewählt ist.

Die vorliegende Erfindung umfasst die Verwendung der Verbindungen der Formel I und/oder ihre physiologisch unbedenklichen Salze zur Herstellung eines Arzneimittels zur Behandlung oder Vorbeugung von Autoimmunkrankheiten oder Entzündungskrankheiten, wie multipler Sklerose, Psoriasis, rheumatoider Arthritis, systemischem Lupus erythematodes, entzündlicher Darmerkrankung, Lungenentzündung, Thrombose oder Gehirninfektion bzw. - entzündung, wie Meningitis oder Encephalitis.

Die vorliegende Erfindung umfasst die Verwendung der Verbindungen der Formel I und/oder ihre physiologisch unbedenklichen Salze zur Herstellung eines Arzneimittels zur Behandlung oder Vorbeugung von neurodegenerativen Krankheiten, wie u. a. multipler Sklerose, Alzheimer-Krankheit, Chorea Huntington, ZNS-Trauma, Schlaganfall oder ischämischen Zuständen.

Die vorliegende Erfindung umfasst die Verwendung der Verbindungen der Formel I und/oder ihre physiologisch unbedenklichen Salze zur Herstellung eines Arzneimittels zur Behandlung oder Vorbeugung von Herz-Kreislauf-Erkrankungen, wie Atherosklerose, Herzhypertrophie, Fehlfunktion der Herzmyocyten, erhöhtem Blutdruck oder Vasokonstriktion.

Die vorliegende Erfindung umfasst die Verwendung der Verbindungen der Formel I und/oder ihre physiologisch unbedenklichen Salze zur Herstellung eines Arzneimittels zur Behandlung oder Vorbeugung von chronischer obstruktiver Lungenkrankheit, Fibrose nach anaphylaktischem Schock, Psoriasis, allergischen Erkrankungen, Asthma, Schlaganfall, ischämischen Zuständen, Ischämie-Reperfusion, Blutplättchen-Aggregation bzw. Aktivierung, Skelettmuskel-Atrophie bzw. -Hypertrophie, Leukozyten-Rekrutierung bei Krebsgewebe, Angiogenese, Invasions-Metastase, insbesondere Melanom, Karposi-Sarkom, akuten und chronischen Bakterien- und Virusinfektionen, Sepsis, Transplantationsabstoßung, Transplantatabstoßung, Glomerulosklerose, Glomerulomephritis, progressiver Nierenfibrose, Endothel- und Epithel-Verletzungen in der Lunge und Atemwegsentzündung in der Lunge.

Die vorliegende Erfindung umfasst die Verwendung der Verbindungen der Formel I und/oder ihre physiologisch unbedenklichen Salze zur Herstellung eines Arzneimittels zur Behandlung oder Vorbeugung von Krebs in einem Säugetier, einschließlich Mensch, wobei der Krebs ausgewählt ist aus: Gehirn (Gliomen), Glioblastomen, Leukämien, Bannayan-Zonana-Syndrom, Cowden-Krankheit, Lhermitte-Duclos-Krankheit, Brust-, entzündlichem Brust-Krebs, Wilm's Tumor, Ewings's Sarkom, Rhabdomyosarkom, Ependymom, Medulloblastom, Kolon, Kopf und Hals, Niere, Lunge, Leber, Melanom, Ovar, Pankreas, Prostata, Sarkom, Osteosarkom, Riesenzelltumor von Knochen und Thyroid.

Die vorliegende Erfindung umfasst die Verwendung der Verbindungen der Formel I und/oder ihre physiologisch unbedenklichen Salze zur Herstellung eines Arzneimittels zur Behandlung oder Vorbeugung von Krebs in einem Säugetier, einschließlich Mensch, wobei der Krebs ausgewählt ist aus: lymphoblastischer T-Zell-Leukämie, chronischer myelogener Leukämie, chronischer Lymphocyten-Leukämie, Hairy-Cell-Leukämie, akuter lymphoblastischer Leukämie, akuter myelogener Leukämie, chronischer Neutrophilen-Leukämie, akuter lymphoblastischer T-Zell-Leukämie, Plasmacytom, immunoblastischer Großzell-Leukämie, Mantelzell-Leukämie, multiplem Myelom, Megakaryoblasten-Leukämie, multiplem Myelom, akuter Megakaryocyten-Leukämie, Promyelocyten-Leukämie und Erythroleukämie.

Die vorliegende Erfindung umfasst die Verwendung der Verbindungen der Formel I und/oder ihre physiologisch unbedenklichen Salze zur Herstellung eines Arzneimittels zur Behandlung oder Vorbeugung von Krebs in einem Säugetier, einschließlich Mensch, wobei der Krebs ausgewählt ist aus malignem Lymphom, Hodgkins-Lymphom, Non-Hodgkins-Lymphom, lymphoblastischem T-Zell-Lymphom, Burkitt's Lymphom und Follikel-Lymphom.
Die Erfindung betrifft vorzugsweise ein Verfahren zum Behandeln von Krebs in einem Säugetier, einschließlich Mensch, wobei der Krebs ausgewählt ist aus: Neuroblastom, Blasenkrebs, urothelialem Krebs, Lungenkrebs, Vulva-Krebs, Cervix-Krebs, Endometrium-Krebs, Nierenkrebs, Mesotheliom, Ösophagus-Krebs, Speicheldrüsenkrebs, hepatozellulärem Krebs, Darmkrebs, nasopharyngealem Krebs, bukkalem Krebs, Mundkrebs, GIST (gastrointestinalem Stromatumor) und Hodenkrebs.

Weiterhin können die Verbindungen der Formel I verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Ebenfalls umfasst ist die Verwendung der Verbindungen der Formel I und/oder ihre physiologisch unbedenklichen Salze zur Herstellung eines Arzneimittels bei einem Säugetier, wobei man eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Die offenbarten Verbindungen der Formel I können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die hier definierte Antikrebsbehandlung kann als alleinige Therapie angewendet werden oder zusätzlich zu der erfindungsgemäßen Verbindung herkömmliche Operation oder Strahlungstherapie oder Chemotherapie umfassen. Eine derartige Chemotherapie kann eine oder mehrere der folgenden Kategorien von Antitumormitteln umfassen:
(i) antiproliferative/antineoplastische/DNA schädigende Mittel und Kombinationen davon, wie in der medizinischen Onkologie verwendet, wie Alkylierungsmittel (zum Beispiel Cisplatin, Carboplatin, Cyclophosphamid, Nitrogen Mustard, Melphalan, Chlorambucil, Busulphan und Nitrosoharnstoffe); Antimetaboliten (z.B. Antifolate, wie Fluorpyrimidine, wie 5-Fluoruracil und Tegafur, Raltitrexed, Methotrexat, Cytosinarabinosid, Hydroxyharnstoff und Gemcitabin); Antitumor-Antibiotika (z.B. Anthracycline, wie Adriamycin, Bleomycin, Doxorubicin, Daunomycin, Epirubicin, Idarubicin, Mitomycin-C, Dactinomycin und Mithramycin); antimitotische Mittel (zum Beispiel Vinca-Alkaloide, wie Vincristin, Vinblastin, Vindesin und Vinorelbin, und Taxoide, wie Taxol und Taxoter); Topoisomerase-Inhibitoren (zum Beispiel Epipodophyllotoxine, wie Etoposid und Teniposid, Amsacrin, Topotecan, Irinotecan und Camptothecin) und zelldifferenzierende Mittel (zum Beispiel all-trans-Retinsäure, 13-cis-Retinsäure und Fenretinid);
(ii) zytostatische Mittel, wie Anti-Östrogene (z.B. Tamoxifen, Toremifen, Raloxifen, Droloxifen und lodoxyfen), den Östrogenrezeptor nach unten regulierende Mittel (zum Beispiel Fulvestrant), Anti-Androgene (z.B. Bicalutamid, Flutamid, Nilutamid und Cyproteronacetat), LHRH-Antagonisten oder LHRH-Agonisten (zum Beispiel Goserelin, Leuprorelin und Buserelin), Progesterone (zum Beispiel Megestrolacetat), Aromatase-Inhibitoren (zum Beispiel Anastrozol, Letrozol, Vorazol und Exemestan) und Inhibitoren der 5α-Reduktase, wie Finasterid;
(iii) Mittel, die die Invasion von Krebszellen hemmen (zum Beispiel Metalloproteinase-Inhibitoren, wie Marimastat und Inhibitoren der Urokinase-Plasminogenaktivator-Rezeptor-Funktion);
(iv) Inhibitoren der Wachstumsfaktor-Funktion, zum Beispiel umfassen solche Inhibitoren Wachstumsfaktor-Antikörper, Wachstumsfaktor-Rezeptor-Antikörper (zum Beispiel den Anti-erbb2-Antikörper Trastuzumab [Herceptin™] und den Anti-erbb1-Antikörper Cetuximab [C225]), Farnesyltransferase-Inhibitoren, Tyrosinkinase-Inhibitoren und Serin / Threonin-Kinase-Inhibitoren, zum Beispiel Inhibitoren der epidermalen Wachstumsfaktor-Familie (zum Beispiel Inhibitoren der Tyrosinkinasen der EGFR-Familie, wie N-(3-Chlor-4-fluorphenyl)-7-methoxy-6-(3-morpholinopropoxy)-chinazolin-4-amin (Gefitinib, AZD1839), N-(3-Ethinylphenyl)-6,7-bis(2-methoxyethoxy)chinazolin-4-amin (Erlotinib, OSI-774) und 6-Acrylamido-N-(3-chlor-4-fluorphenyl)-7-(3-morpholinopropoxy)chinazolin-4-amin (Cl 1033)), zum Beispiel Inhibitoren der von Plättchen abstammenden Wachstumsfaktor-Familie und zum Beispiel Inhibitoren der Hepatozytenwachstumsfaktor-Familie;
(v) antiangiogene Mittel, wie solche, die die Wirkungen des vaskulären endothelialen Wachstumsfaktors hemmen (zum Beispiel der Antikörper gegen den vaskulären Endothelzell-Wachstumsfaktor Bevacizumab [Avastin™], Verbindungen, wie die in den veröffentlichten internationalen Patentanmeldungen WO 97/22596, WO 97/30035, WO 97/32856 und WO 98/13354 offenbarten) und Verbindungen, die durch andere Mechanismen wirken (zum Beispiel Linomid, Inhibitoren der Integrin-αvß3-Funktion und Angiostatin);
(vi) gefäßschädigende Mittel, wie Combretastatin A4 und in den internationalen Patentanmeldungen WO 99/02166, WO 00/40529, WO 00/41669, WO 01/92224, WO 02/04434 und WO 02/08213 offenbarte Verbindungen;
(vii) Antisense-Therapien, zum Beispiel diejenigen, die gegen die vorstehend aufgelisteten Ziele gerichtet sind, wie ISIS 2503, ein anti-Ras-Antisense;
(viii) Genetherapieansätze, einschließlich beispielsweise Ansätze zum Ersetzen von veränderten Genen, wie verändertem p53 oder verändertem BRCA1 oder BRCA2, GDEPT- (gene-directed enzyme pro-drug-Therapie-) Ansätze, die diejenigen, die Cytosindesaminase, Thymidinkinase oder ein bakterielles Nitroreduktase-Enzym verwenden, sowie Ansätze zur Erhöhung der Patiententoleranz gegenüber Chemotherapie oder Strahlungstherapie, wie Multi-Drug-Resistence-Gen-Therapie; und
(ix) Immuntherapieansätze, einschließlich beispielsweise Ex-vivo- und Invivo-Ansätzen zur Erhöhung der Immunogenität von Patiententumorzellen, wie Transfektion mit Cytokinen, wie Interleukin 2, Interleukin 4 oder Granulozyten-Makrophagen-Kolonie-stimulierendem Faktor, Ansätze zur Verringerung der T-Zell-Anergie, Ansätze unter Verwendung transfizierter Immunzellen, wie mit Cytokin transfizierter dendritischer Zellen, Ansätze unter Verwendung mit Cytokin transfizierter Tumorzelllinien und Ansätze unter Verwendung anti-idiotypischer Antikörper.

Bevorzugt aber nicht ausschliesslich werden die Arzneimittel der nachstehenden Tabelle 1 mit den Verbindungen der Formel I kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | BBR-3464 (Hoffmann-La Roche) |
| | Ormiplatin | SM-11355 (Sumitomo) |
| | Iproplatin | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyhamstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder Mitoxantron | Gimatecan (Sigma- Tau) |
| | Irinotecan (CPT-11) | Diflomotecan (Beaufour-Ipsen) |
| | 7-Ethyl-10-hydroxycamptothecin | TAS-103 (Taiho) |
| | Topotecan | Elsamitrucin (Spectrum) |
| | Dexrazoxanet (TopoTarget) | J-107088 (Merck & Co) |
| | Pixantron (Novuspharrna) | BNP-1350 (BioNumerik) |
| | Rebeccamycin-Analogon (Exelixis) | CKD-602 (Chong Kun Dang) |
| | BBR-3576 (Novuspharrna) | KW-2170 (Kyowa Hakko) |
| | | |
| Antitumor-Antibiotika | Dactinomycin (Actinomycin D) | Amonafid |
| | Doxorubicin (Adriamycin) | Azonafid |
| | Deoxyrubicin | Anthrapyrazol |
| | Valrubicin | Oxantrazol |
| | Daunorubicin (Daunomycin) | Losoxantron |
| | Epirubicin | Bleomycinsulfat (Blenoxan) |
| | Therarubicin | Bleomycinsäure |
| | Idarubicin | Bleomycin A |
| | Rubidazon | Bleomycin B |
| | Plicamycinp | Mitomycin C |
| | Porfiromycin | MEN-10755 (Menarini) |
| | Cyanomorpholinodoxorubicin | GPX-100 (Gem |
| | Mitoxantron (Novantron) | Pharmaceuticals) |
| | | |
| Antimitotische Mittel | Paclitaxel | SB 408075 (GlaxoSmithKline) |
| | Docetaxel | E7010 (Abbott) |
| | Colchicin | PG-TXL (Cell Therapeutics) |
| | Vinblastin | IDN 5109 (Bayer) |
| | Vincristin | A 105972 (Abbott) |
| | Vinorelbin | A 204197 (Abbott) |
| | Vindesin | LU 223651 (BASF) |
| | Dolastatin 10 (NCl) | D 24851 (ASTA Medica) |
| | Rhizoxin (Fujisawa) | ER-86526 (Eisai) |
| | Mivobulin (Warner-Lambert) | Combretastatin A4 (BMS) |
| | Cemadotin (BASF) | Isohomohalichondrin-B |
| | RPR 109881A (Aventis) | (PharmaMar) |
| | TXD 258 (Aventis) | ZD 6126 (AstraZeneca) |
| | Epothilon B (Novartis) | PEG-Paclitaxel (Enzon) |
| | T 900607 (Tularik) | AZ10992 (Asahi) |
| | T 138067 (Tularik) | !DN-5109 (Indena) |
| | Cryptophycin 52 (Eli Lilly) | AVLB (Prescient NeuroPharma) |
| | Vinflunin (Fabre) | Azaepothilon B (BMS) |
| | Auristatin PE (Teikoku Hormone) | BNP- 7787 (BioNumerik) |
| | BMS 247550 (BMS) | CA-4-Prodrug (OXiGENE) |
| | BMS 184476 (BMS) | Dolastatin-10 (NrH) |
| | BMS 188797 (BMS) | CA-4 (OXiGENE) |
| | Taxoprexin (Protarga) | |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylat synthase-Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter International) |
| | Glufosfamid (Baxter International) | |
| | Albumin + 32P (Isotope Solutions) | Apaziquon (Spectrum Pharmaceuticals) |
| | Thymectacin (NewBiotics) | |
| | Edotreotid (Novartis) | O6-Benzylquanin (Paligent) |
| | | |
| Farnesyltrans-ferase-Inhibitoren | Arglabin (NuOncology Labs) | Tipifarnib (Johnson & Johnson) |
| | lonafarnib (Schering-Plough) | Perillylalkohol (DOR BioPharma) |
| | BAY-43-9006 (Bayer) | |
| | | |
| Pumpen-Inhibitoren | CBT-1 (CBA Pharma) | Zosuquidar-Trihydrochlorid (Eli Lilly) |
| | Tariquidar (Xenova) | |
| | MS-209 (Schering AG) | Biricodar-Dicitrat (Vertex) |
| | | |
| Histonacetyltrans-ferase-Inhibitoren | Tacedinalin (Pfizer) | Pivaloyloxymethylbutyrat (Titan) |
| | SAHA (Aton Pharma) | |
| | MS-275 (Scherinq AG) | Depsipeptid (Fujisawa) |
| | | |
| Metalloproteinase-Inhibitoren Ribonucleosidreduktase-Inhibitoren | Neovastat (Aeterna Laboratories) | CMT -3 (CollaGenex) |
| | Marimastat (British Biotech) | BMS-275291 (Celltech) |
| | Galliummaltolat (Titan) | Tezacitabin (Aventis) |
| | Triapin (Vion) | Didox (Molecules for Health) |
| | | |
| TNF-alpha-Agonisten / Antagonisten | Virulizin (Lorus Therapeutics) | Revimid (Celgene) |
| | CDC-394 (Celgene) | |
| | | |
| Endothelin-A-Rezeptor-Antaqonisten | Atrasentan (Abbot) | YM-598 (Yamanouchi) |
| | ZD-4054 (AstraZeneca) | |
| | | |
| Retinsäurerezeptor-Agonisten | Fenretinid (Johnson & Johnson) | Alitretinoin (Ligand) |
| | LGD-1550 (Liqand) | |
| | | |
| Immunmodulatoren | Interferon | Dexosom-Therapie (Anosys) |
| | Oncophage (Antigenics) | Pentrix (Australian Cancer Technology) |
| | GMK (Progenics) | |
| | Adenokarzinom-Impfstoff (Biomira) | JSF-154 (Tragen) |
| | | Krebsimpfstoff (Intercell) |
| | CTP-37 (AVI BioPharma) | Norelin (Biostar) |
| | JRX-2 (Immuno-Rx) | BLP-25 (Biomira) |
| | PEP-005 (Peplin Biotech) | MGV (Progenics) |
| | Synchrovax-Impfstoffe | !3-Alethin (Dovetail) |
| | (CTL Immuno) | CLL-Thera (Vasogen) |
| | Melanom-Impfstoff (CTL lmmuno) | |
| | p21-RAS-lmpfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol |
| | Toremofin | (EntreMed) |
| | Dexamethason | Arzoxifen (Eli Lilly) |
| | | |
| Photodynamische Mittel | Talaporfin (Light Sciences) | Pd-Bacteriopheophorbid |
| | Theralux (Theratechnologies) | (Yeda) |
| | Motexafin-Gadolinium | Lutetium-Texaphyrin |
| | (Pharmacyclics) | (Pharmacyclics) Hypericin |
| | | |
| Tyrosinkinase-Inhibitoren | Imatinib (Novartis) | Kahalid F (PharmaMar) |
| | Leflunomid (Sugen/Pharmacia) | CEP- 701 (Cephalon) |
| | ZDI839 (AstraZeneca) | CEP-751 (Cephalon) |
| | Erlotinib (Oncogene Science) | MLN518 (Millenium) |
| | Canertjnib (Pfizer) | PKC412 (Novartis) |
| | Squalamin (Genaera) | Phenoxodiol O |
| | SU5416 (Pharmacia) | Trastuzumab (Genentech) |
| | SU6668 (Pharmacia) | C225 (ImClone) |
| | ZD4190 (AstraZeneca) | rhu-Mab (Genentech) |
| | ZD6474 (AstraZeneca) | MDX-H210 (Medarex) |
| | Vatalanib (Novartis) | 2C4 (Genentech) |
| | PKI166 (Novartis) | MDX-447 (Medarex) |
| | GW2016 (GlaxoSmithKline) | ABX-EGF (Abgenix) |
| | EKB-509 (Wyeth) | IMC-1C11 (ImClone) |
| | EKB-569 (Wyeth) | |
| Verschiedene Mittel | SR-27897 (CCK-A-Inhibitor, Sanofi-Synthelabo) | BCX-1777 (PNP-Inhibitor, BioCryst) |
| | Tocladesin (cyclisches-AMP-Agonist, Ribapharm) | Ranpimase (Ribonuclease-Stimulans, Alfacell) |
| | Alvocidib (CDK-Inhibitor, Aventis) | Galarubicin (RNA-Synthese-Inhibitor, Dong-A) |
| | CV-247 (COX-2-lnhibitor, Ivy Medical) | |
| | | Tirapazamin (Reduktionsmittel, SRI International) |
| | P54 (COX-2-lnhibitor, Phytopharm) | |
| | | N-Acetylcystein (Reduktionsmittel, Zambon) |
| | CapCell™ (CYP450-Stimulans, Bavarian Nordic) | |
| | | R-Flurbiprofen (NF-kappaB-Inhibitor, Encore) |
| | GCS-IOO (gal3-Antagonist, GlycoGenesys) | |
| | | 3CPA (NF-kappaB-lnhibitor, Active Biotech) |
| | G17DT-lmmunogen (Gastrin-Inhibitor, Aphton) | |
| | | Seocalcitol (Vitamin-D-Rezeptor-Agonist, Leo) |
| | Efaproxiral (Oxygenator, Allos Therapeutics) | |
| | | 131-I-TM-601 (DNA-Antagonist, TransMolecular) |
| | PI-88 (Heparanase-Inhibitor, Progen) | |
| | | Eflornithin (ODC-Inhibitor, ILEX Oncology) |
| | Tesmilifen (Histamin-Antagonist, YM BioSciences) | |
| | | Minodronsäure (Osteoclasten-Inhibitor, Yamanouchi) |
| | Histamin (Histamin-H2- | |
| | Rezeptor- Agonist, Maxim) | Indisulam (p53-Stimulans, Eisai) |
| | Tiazofurin (IMPDH-Inhibitor, Ribapharm) | Aplidin (PPT-Inhibitor, PharmaMar) |
| | Cilengitid (Integrin-Antagonist, Merck KGaA) | Rituximab (CD20-Antikörper, Genentech) |
| | SR-31747 (IL-1-Antagonist, Sanofi-Synthelabo) | Gemtuzumab (CD33-Antikörper, Wyeth Ayerst) |
| | CCI-779 (mTOR-Kinase-lnhibitor, Wyeth) | PG2 (Hämatopoese-Verstärker, Pharmagenesis) |
| | Exisulind (PDE-V-Inhibitor, Cell Pathways) | Immunol™ (Triclosan-Oralspülung, Endo) |
| | CP-461 (PDE-V-Inhibitor, Cell Pathways) | Triacetyluridin (Uridin-Prodrug, Wellstat) |
| | AG-2037 (GART-Inhibitor, Pfizer) | SN-4071 (Sarkom-Mittel, Signature BioScience) |
| | WX-UK1 (Plasminogenaktivator-Inhibitor, Wilex) | |
| | | TransMID-107™ (Immunotoxin, KS Biomedix) |
| | PBI-1402 (PMN-Stimulans, ProMetic LifeSciences) | |
| | | PCK-3145 (Apoptose-Förderer, Procyon) |
| | Bortezomib (Proteasom-Inhibitor, Millennium) | |
| | | Doranidazol (Apoptose-Förderer, Pola) |
| | SRL-172 (T-Zell-Stimulans, SR Pharma) | |
| | | CHS-828 (cytotoxisches Mittel, Leo) |
| | TLK-286 (Glutathion-S-Transferase-Inhibitor, Telik) | |
| | | trans-Retinsäure (Differentiator, NIH) |
| | PT-100 (Wachstumsfaktor-Agonist, Point Therapeutics) | |
| | | MX6 (Apoptose-Förderer, MAXIA) |
| | Midostaurin (PKC-Inhibitor, Novartis) | |
| | | Apomin (Apoptose-Förderer, ILEX Oncology) |
| | Bryostatin-1 (PKC-Stimulans, GPC Biotech) | |
| | | Urocidin (Apoptose-Förderer, Bioniche) |
| | CDA-II (Apoptose-Förderer, Everlife) | |
| | | Ro-31-7453 (Apoptose-Förderer, La Roche) |
| | SDX-101 (Apoptose-Förderer, Salmedix) | |
| | | Brostallicin (Apoptose-Förderer, Pharmacia) |
| | Ceflatonin (Apoptose-Förderer, ChemGenex) | |
| | | |

Eine derartige gemeinsame Behandlung kann mithilfe gleichzeitiger, aufeinander folgender oder getrennter Dosierung der einzelnen Komponenten der Behandlung erzielt werden. Solche Kombinationsprodukte setzen die erfindungsgemäßen Verbindungen ein.

### ASSAYS

Die in den Beispielen beschriebenen Verbindungen der Formel I wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

Massenspektrometrie (MS): El (Elektronenstoß-lonisation) M⁺
FAB (Fast Atom Bombardment) (M+H)⁺
ESI (Electrospray lonization) (M+H)⁺

APCI-MS (atmospheric pressure chemical ionization - mass spectrometry) (M+H)⁺.

### Abkürzungen:

M - mol/L
min. - Minute/n
h - Stunde/n
THF - Tetrahydrofuran
Me - Methyl
MTBE - *tert*.-Butylmethylether
DMF - N,N-Dimethylformamid
EtOAc - Ethylacetat
HOAc - Essigsäure
PE - Petrolether
Et₂O - Diethylether
NBS - N-Bromsuccinimid
MeOH - Methanol
EtOH - Ethanol
TFA - Trifluoressigsäure
Tf - Triflat (-SO₂-CF₃)
TMS - Trimethylsilyl
konz. HCl - konzentrierte Salzsäure
Cy - Cyclohexyl
Dppf - 1,1'-Bis(diphenylphosphino)ferrocen

**Allgemeine experimentelle Bedingungen:** Sämtliche Arbeiten mit luft- oder feuchtigkeitsempfindlichen Substanzen werden unter einer Argon- oder Stickstoffatmosphäre durchgeführt. Alle kommerziell erhältlichen Reagenzien und Lösungsmittel werden, falls nicht anders angegeben, ohne weitere Reinigung eingesetzt.

**Dünnschichtchromatographie (DC):** Merck DC-Festplatten Kieselgel 60 F-254 (Glas oder Aluminium). Die Detektion erfolgt im UV, mit I₂ und/oder mit 5% ethanolischer Phosphmolybdat-Lösung mit nachfolgendem Erhitzen mittels Heizluftgebläse.

Säulenchromatographie: Stationäre Phase Merck Kieselgel 60, 63-200 µm oder Merck Kieselgel 60, 40-63 µm.

**Mikrowelle (MW):** Emrys™ Optimizer EXP von der Firma Personal Chemistry

**Schmelzpunkte** (Smp.): Die Schmelzpunktbestimmung erfolgt mittels einer Apparatur der Firma Büchi, Melting Point B-5459. Alle angegebenen Schmelzpunkte sind nicht korrigiert.

**Kernresonanzspetroskopie** (**NMR**)**:** ¹H- und ¹³C-NMR Spektrenaufnahme erfolgt an 300, 400 und 500 MHz NMR-Geräten der Firma Bruker. Die chemischen Verschiebungen δ sind in ppm, die Kopplungskonstanten in Hz angegeben.

**RP-HPLC mit UV- und MS-Detektion (LC-MS):**
t_{R} - Retentonszeit; TIC - Total Ion Count, [MH]⁺ als m/z Werte; Gerät - Aglient 1100 Series (DAD und MS Detektor) mit ELS-Detekor Sedex 75 der Firma ERC; lonenquelle - Electrospray (positiver Modus); Scan - 100-1000 m/z; Fragmentationvoltage - 60 V; Gas-Temperatur - 300°C; DAD - 220 nm;
Flussrate - 2.4 mL/min, ein Splitter reduziert die Flussrate nach dem DAD für die MS-Detektion auf 0,75mL/min.; Säule - Chromolith Speed ROD RP-18e 50-4.6; Lösungsmittel - LiChrosolv (Merck KGaA); mobile Phase A - H₂O (0.01% TFA); mobile Phase B - Acetonitril (0.01% TFA); Gradient - in 2.6 min von 96% A zu 100% B; danach für 0.7 min 100% B.

Die generelle Route zur Herstellung der erfindungsgemäßen Verbindungen ist im nachfolgenden Schema dargestellt:

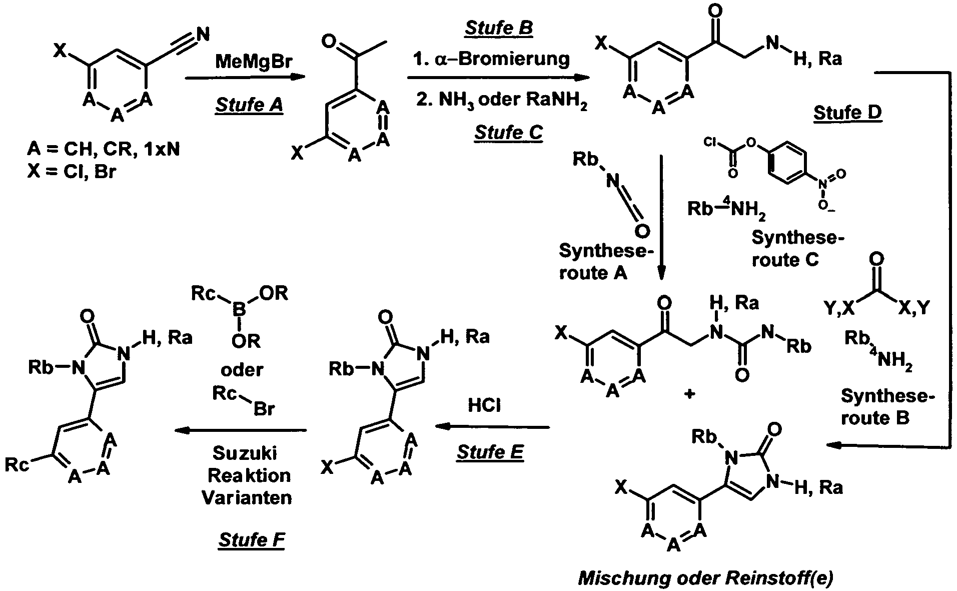

Die heteroaromatischen Nitrile, die als Startmaterialen Verwendung finden, können durch literaturbekannte Prozeduren selbsthergestellt oder bei kommerziellen Anbietern eingekauft werden.

Die Reaktionsführung der Stufen D und F kann über verschiedene Syntheserouten bzw. Reaktionsvarianten laufen, die einem Fachmann bekannt sind.

Der Substituent Ra kann auch durch eine alternative Synthesestrategie eingeführt werden:

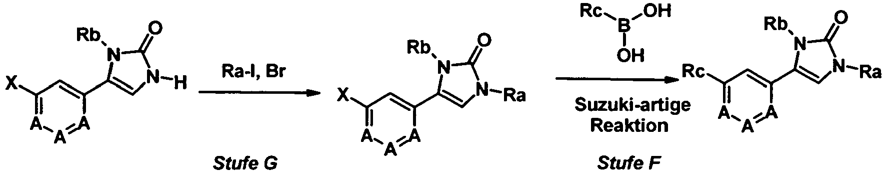

Die C-C-Verknüpfung in Stufe F kann auch durch eine inverse Reaktionsführung der Suzuki Reaktion erreicht werden:

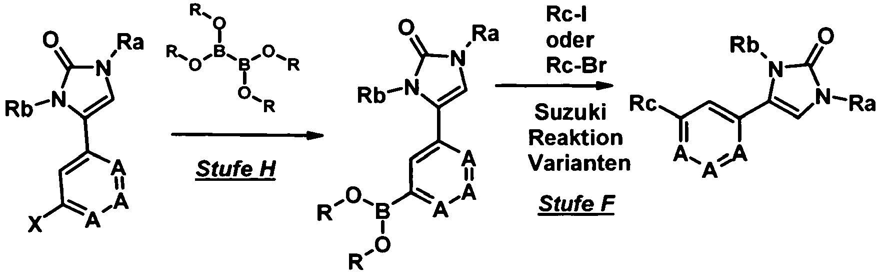

Mit Hilfe der beschriebenen Reaktionspfade ist es möglich alle möglichen Substituenten und/oder Substituentenkombinationen in die Grundstruktur einzuführen. Es ist aber auch möglich, durch weitere Syntheseoperationen strukturelle Veränderungen bzw. Erweiterungen vorzunehmen. Als ein nicht limitierendes Beispiel sei das folgende Beispiel beschrieben (Stufe I):

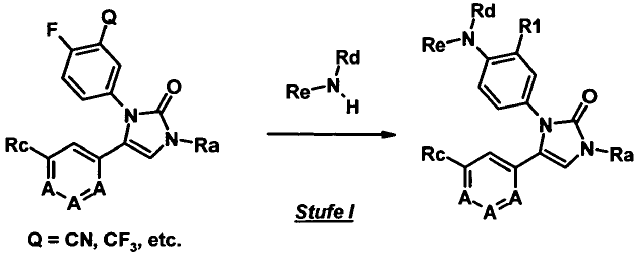

### Beispiele

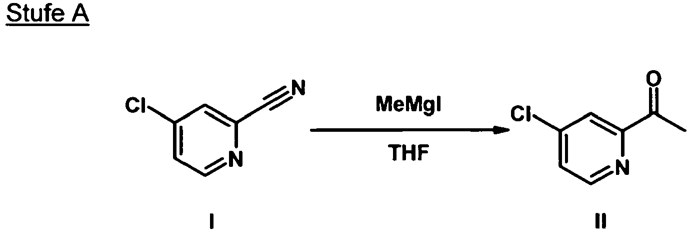

In einem 2L Dreihalskolben bestückt mit Rührer und Thermometer wird in einer N₂-Schutzgasatmosphäre Nitril I (60 g, 0.42 mol) in THF (1000 mL) gelöst und mittels Eisbad auf 0°C gekühlt. Kommerziell erhältliches MeMgI (200 mL einer 3 M Lösung in THF, 0.6 mmol) wird langsam über ca. 45 min. zugeben. Es bildet sich zunächst eine klare, dunkle Lösung. Die Zutropfrate der MeMgl Addition wird so einjustiert, dass die Lösungstemperatur im Reaktionsgefäß zwischen 0-10°C liegt. Nachdem das Grignard Reagent komplett zugegeben wurde erhält man eine grüne Suspension, die für weitere 2h bei 0°C gerührt wird. Danach wird die Reaktionsmischung in Eiswasser (1500 mL) gegeben. 2 M HCl wird zugeführt, bis die Reaktionsmischung einen ca. pH2. aufweist Die Mischung wird weitere 15 min. gerührt und dann mehrmals extrahiert (Zugabe von EtOAc und Wasser). Die vereinigten organischen Phasen werden mit wässriger gesättigter Kochsalzlösung gewaschen und mit Na₂SO₄ getrocknet. Sämtliche Lösungsmittel werden durch Destillation unter reduzierten Druck am Rotationsverdampfer entfernt. Man erhält als Rohprodukt 66 g gelbbraunes Öl. Das Rohprodukt wird mittels Säulenchromatographie (800 g Si60, MTBE) aufgereinigt. Die geeigneten Fraktionen (charakterisiert durch DC-Analytik) werden vereinigt. Nach entfernen der Lösungsmittel erhält man Keton **II** (51 g, 0.32 mol, 76% Ausbeute) als klares dunkles Öl. Analytische Daten Keton **II:** DC: R_{f} = 0.57 (Si60, PE/EtOAc 8:1); LC-MS: t_{R} = 1.829 min (UV = 220 nm), t_{R} = 1.842 min. (TIC, mit [M+H]⁺ = 156); ¹H NMR (300 MHz, CDCl₃) δ 8.58 (d, 1H), 8.03 (dd, *J* = 2.1, 0.4, 1 H), 7.47 (dd, *J =* 5.2, 2.1, 1H), 2.71 (d, *J =* 3.2, 3H).
Synthesiert nach J.Y.Woo, I.W.Bin, R.J.Keol, S.M.Ja, K.W.Bae, C.E.Chil, Bioorg. Med. Chem. 2004, 12, 5909-5915.

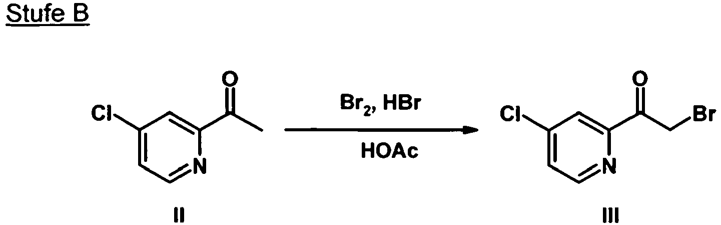

Das Pyridinketon **II** (7.78 g, 50 mmol) wird in konz. HOAc (100 mL) gelöst und HBr (11 mL einer 32% HBr Lösung in konz. HOAc, 60 mmol) zugegeben. Während der Zugabe entsteht ein nebelartiger Niederschlag. Brom (2.6 mL, 50 mmol) wird zugetropft. Der Niederschlag löst sich während der Br₂-Addition auf. Die Reaktionsmischung wird 15 min. bei 75°C gerührt. Nach abkühlen auf Raumtemperatur bildet sich ein Niederschlag aus. Die Suspension wird in gesättigte wässrige NaHCO₃ Lösung (300 mL) gegeben und die daraus resultierende Mischung mit EtOAc (3 x 150 mL) extrahiert. Die vereinigten organischen Phasen werden mit gesättigter wässriger Kochsalzlösung (50 mL) gewaschen und mit Na₂SO₄ getrocknet. Anschließend werden die Lösungsmittel unter reduzierten Druck am Rotationsverdampfer entfernt. Als Rohprodukt wird ein oranges Öl (13.4 g) erhalten. Das Rohprodukt wird säulenchromatographisch aufgereinigt (200 g Si60, Gradient CH₂Cl₂/PE 1:3 zu CH₂Cl₂/PE 1:1) und die geeigneten Fraktionen (charakterisiert durch DC-Analytik) vereinigt. Nach entfernen der Lösungsmittel erhält man Ketobromid **III** (8.3 g, 35.4 mol, 71% Ausbeute) als blasses gelbes Öl, das bei längerer Lagerung kristallisiert. Analytische Daten Ketobromid **III:** DC: R_{f} = 0.66 (Si60, CH₂Cl₂/PE 1:1); Smp.: 72°C; LC-MS: breiter Peak - t_{R} = 2.213 min (UV = 220 nm), t_{R} = 2.194 min. (TIC, mit [M]⁺ = 234); ¹H NMR (400 MHz, CDCl₃) δ 8.59 (d, *J =* 5.2, 1 H), 8.09 (d, *J =* 2.0, 1H), 7.52 (dd, *J* = 5.2, 2.1, 1 H), 4.81 (s, 2H). 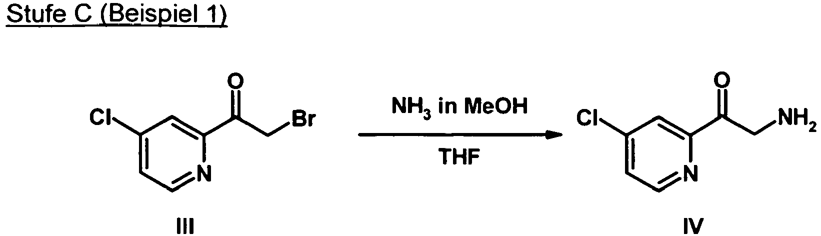 Das Ketobromid **III** (10.6 g, 45.0 mmol) wird in THF (100 mL) gelöst und mittels Eisbad gekühlt. Eine möglichst frische Ammoniaklösung (25.7 mL einer kommerziell erhältlichen 7 M Ammoniak Lösung in MeOH, 180 mmol) wird schnell zugegeben. Die blassgelbe Reaktionsmischung wird für 1 h gerührt. Während dieser Zeit lässt man die Temperatur der Reaktionslösung auf Raumtemperatur ansteigen. Die Farbe der Reaktionslösung veränderte während der Ammoniak Lösungszugabe zunächst schnell in ein tiefes Rosa um dann nach ca. 1 h eine blassrosa Suspension zu ergeben. Diethylether (50 mL) wird zugegeben und der ausfallende Feststoff durch Filtration gesammelt, mit Et₂O gewaschen und schließlich an der Luft getrocknet. So erhält man in Form von schwach rosa gefärbten Kristallen das primäre Amin **IV** (5.75 g, 33.7 mmol, 75% yield) als Rohprodukt. Amin **IV** zersetzt sich recht schnell unter normal üblichen Lagerbedingungen (Luft, Raumtemperatur). Deshalb wird das Rohprodukt von Amin **IV** nicht weiter aufgereinigt sondern möglichst schnell in eine Folgereaktion verbraucht (siehe auch Stufe D). Analytische Daten Amin **IV** (Rohprodukt): Smp.: 184°C (direkt nach der Herstellung gemessen); RP-HPLC: t_{R} = 0.67 min (UV = 220 nm) + kleinerer Peak von Verunreinigungen; LC-MS: breiter Peak - t_{R} = 0.514 min (220 nm), t_{R} = 0.591 min. (TIC, mit [MH]⁺ = 171).

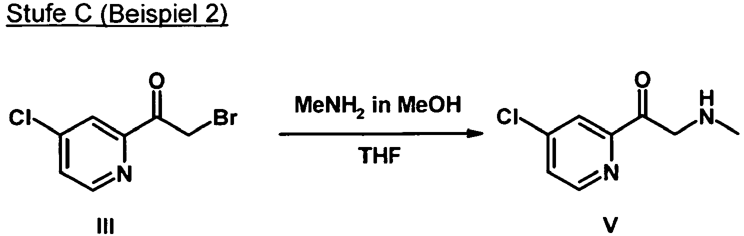

Das Ketobromid **III** (600 mg, ∼75% RP-HPLC Reinheit, 1.92 mmol) wird in THF (5 mL) gelöst. Die Reaktionsmischung wird in eisgekühltes THF (8 mL) gegeben. Eine frische Methylamin Lösung (1.0 mL einer kommerziell erhältlichen 40% Methylamin Lösung in MeOH, 9.8 mmol) wird schnell zugetropft. Die orange-gelbe Reaktionsmischung wird für 40 min. gerührt. Während dieser Zeit lässt man die Temperatur der Reaktionslösung auf Raumtemperatur ansteigen. Diethylether (4 mL) wird zugegeben. Der ausgefallene Feststoff wird abfiltriert, mit Et₂O gewaschen und an der Luft getrocknet. Man erhält Amin **V** (340 mg) als Rohprodukt. Amin **V** zersetzt sich schnell unter üblichen Lagerbedingungen (Luft, Raumtemperatur) und wird deshalb möglichst schnell weiter umgesetzt. Analytische Daten Amin V (Rohprodukt): LC-MS: t_{R} = 0.646 min (UV = 220 nm), t_{R} = 0.647 min. (TIC, mit [MH]⁺ = 185).

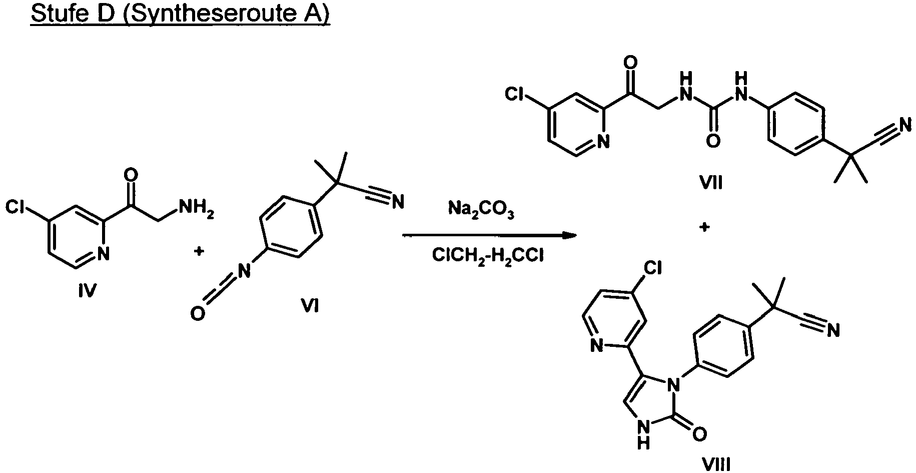

Natriumcarbonat (5.09 g, 106 mmol), Amin **IV** (Rohprodukt; 3.03 g, ∼90% RP-HPLC Reinheit, ∼16.0 mmol), und Isocyanat **VI** (2.40 g, 12.9 mmol) werden in 1,2-Dichloroethan (20 mL) suspendiert. Die orange Suspension wird bei 70°C für 1 h gerührt. Per RP-HPLC Analytik wirdt eine ∼20:80 Ratio der Reaktionsmischung der neuformierten Produkte Harnstoff **VII** und Imidazolon **VIII** ermittelt. Die Reaktionsmischung wird mit einer CH₂Cl₂/MeOH (1:1) Lösung verdünnt und filtriert um überschüssiges Na₂CO₃. zu entfernen. Das Filtrat wird gesammelt. Der Feststoff wird mit zusätzlichen CH₂Cl₂/MeOH (1:1) nachgewaschen und die Filtrate vereinigt. Unter reduzierten Druck werden am Rotationsverdampfer die Lösungsmittel entfernt. Man erhält als Rohprodukt eine Mischung aus Harnstoff VII und Imidazolon **VIII** (6.62 g, als eine ∼20:80 Rohmischung **VII/VIII** - RP-HPLC Analytik) als gelbes zähes Öl (siehe Stufe E für die weitere Verarbeitung der Rohmischung). Analytische Daten Rohmischung Harnstoff **VII** und Imidazolon **VIII:** RP-HPLC: a) Harnstoff VII - t_{R} = 2.61 min (UV = 220 nm) und b) Imidazolon **VIII** - t_{R} = 2.37 min (UV = 220 nm); LC-MS: a) Harnstoff **VII** - t_{R} = 2.149 min (UV = 220 nm), t_{R} = 2.141 min. (TIC, mit [MH]⁺ = 357) und b) Imidazolon VIII - t_{R} = 1.878 min (UV = 220 nm), t_{R} = 1.870 min. (TIC, mit [MH]⁺ = 340).

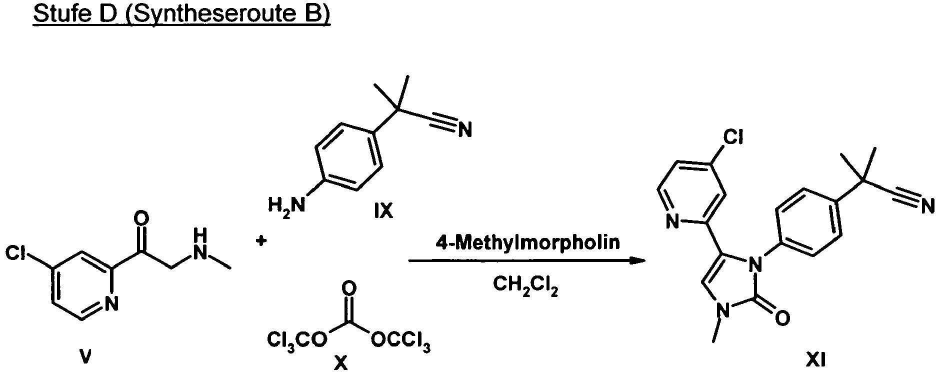

Unter einer N₂-Schutzgasatmosphäre wird Amin **V** (Rohprodukt; 695 mg, 3.76 mmol) und Nitril **IX** (414 mg, 2.58 mmol) in Dichlormethan (16 mL) suspendiert. 4-Methylmorpholin (0.6 mL, 5.46 mmol) wird zugegeben. Bei Raumtemperatur wird in Dichlormethan (3 mL) gelöstes Bis(trichlormethyl)-carbonat **X** (460 mg, 1.55 mmol) über ca. 15 min. zugetropft. Die Reaktionslösung wird bei Raumtemperatur weitere 1.5 h gerührt. Die Reaktionsmischung wird mit Wasser (30 mL) versetzt und für 10 min. kräftig gerührt. Die organische Phase wird separiert, zweimal mit Wasser gewaschen und mit Na₂SO₄ getrocknet. Unter reduziertem Druck werden am Rotationsverdampfer die Lösungsmittel entfernt. 730 mg Rohprodukt werden erhalten. Das Rohprodukt wird mittels Säulenchromatographie aufgereinigt (80 g Si60, CH₂Cl₂/5% MeOH) und die geeigneten Fraktionen vereinigt. Nach Entfernen der Lösungsmittel erhält man Imidazolon **XI** (279 mg, 0.79 mmol, 51% Ausbeute) als gelben Feststoff (siehe Stufe E für die weitere Verarbeitung der Rohmischung). Analytische Daten Imidazolon **XI**: DC: R_{f} = 0.31 (Si60, CH₂Cl₂/4% MeOH/1% NH₃); Smp.: 167°C; LC-MS: t_{R} = 2.056 min (UV = 220 nm), t_{R} = 2.063 min. (TIC, mit [MH]⁺ = 353); ¹H NMR (400 MHz, DMSO) δ 8.40 (d, J = 5.3, 1H), 7.63 - 7.55 (m, 2H), 7.51 (s, 1H), 7.39 - 7.29 (m, 3H), 7.15 (d, J = 1.8, 1H), 3.35 (s, 3H), 1.74 (s, 6H).

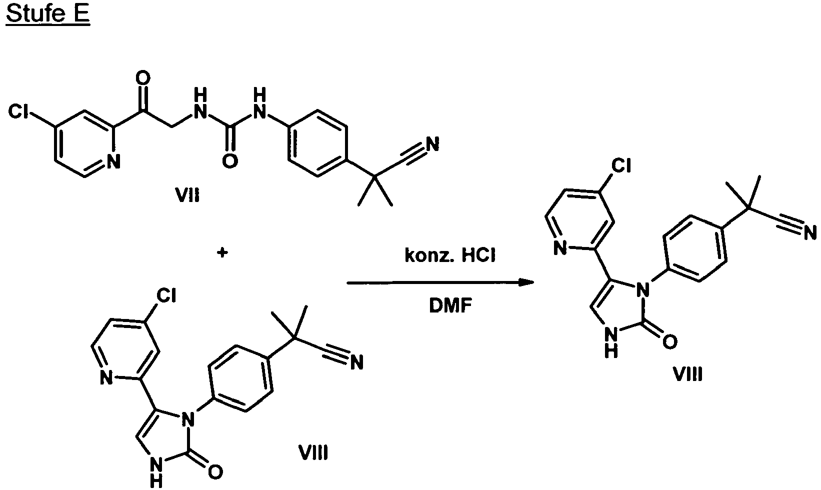

Eine Rohmischung aus Harnstoff VII und Imidazolon **VIII** (4.60 g, als eine ∼20:80 Rohmischung **VII/VIII -** RP-HPLC Analytik) wird in wenig CH₂Cl₂ gelöst. Konz. HCl (2.15 mL einer 37% Salzsäure, 25.8 mmol) und DMF (15 mL) werden zugegeben. Sofort wird eine klare, rauchende Lösung erhalten. Die Reaktionsmischung wird auf 90°C erhitzt. Die Farbe der Reaktionsmischung ändert sich in Rot. Die rote Lösung wird für ca. 30 min. bei 100°C gerührt. Nach Abkühlen auf Raumtemperatur wird die Reaktionslösung in eine 1:1 Mischung aus gesättigter wässriger NaHCO₃ Lösung (50 mL) und Wasser (50 mL) gegeben. Die Mischung wird mit CH₂Cl₂ (3 x 80 mL) extrahiert, mit Na₂SO₄ getrocknet und anschließend die Lösungsmittel unter reduzierten Druck am Rotationsverdampfer entfernt. 5.6 g eines rotrangen, öligen Rückstands werden als Rohprodukt erhalten. Das Rohprodukt wird mittels Säulenchromatographie aufgereinigt (110 g Si60, Gradient CH₂Cl₂/3% MeOH/1% NH₃ zu CH₂Cl₂/7% MeOH/1%NH₃) und die geeigneten Fraktionen (charakterisiert durch DC-Analytik) gesammelt. Nach Entfernen der Lösungsmittel wird 2-Propanol zugegeben. Der ausgefallene Feststoff wird abfiltriert und an der Luft getrocknet. Imidazolon **VIII** (782 mg, 2.31 mmol, 18% Ausbeute über drei Stufen) als beigen Feststoff. Analytische Daten Imidazolon **VIII:** DC: R_{f} = 0.25 (Si60, CH₂Cl₂/5% MeOH/1% NH₃); Smp.: 251 °C; LC-MS: t_{R} = 1.878 min (220 nm), t_{R} = 1.870 min. (TIC, mit [MH]⁺ = 340); ¹H NMR (400 MHz, DMSO) δ 10.88 (s, 1 H), 8.25 (d, *J* = 5.4, 1 H), 7.55 - 7.52 (m, 1 H), 7.52 - 7.50 (m, 1 H), 7.33 (s, 1 H), 7.31 (d, *J =* 1.6, 1 H), 7.26 (dd, *J =* 5.3, 2.0, 1 H), 7.24 - 7.22 (m, 1H), 7.21 - 7.19 (m, 1H), 1.70 (s, 6H).

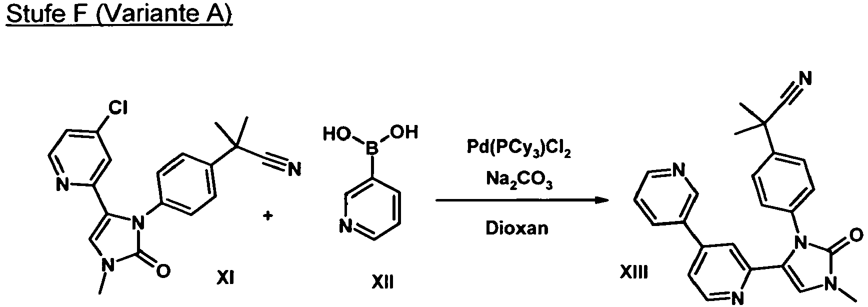

In einem Mikrowellensynthesegefäß wird Imidazolon XI (141.1 mg, 0.40 mmol), Boronsäure **XII** (90.2 mg, 0.44 mmol) und Dichlorobis(tricyclohexyl-phosphin)palladium (II) (23.6 mg, 0.03 mmol) gegeben. Wässriges Natriumcarbonat (0.6 mL einer 2 N Na₂CO₃ Lösung, entgast, dann unter N₂, 1.2 mmol) und Dioxan (3 mL, entgast, dann unter N₂) werden zugegeben. Die Mischung wird in einem Synthesemikrowellenofen (Personal Chemistry 300W, 140°C, 45 min.) erhitzt. Die Reaktionsmischung wird in eine Wasser/Dichlormethan Mischung (20 mL/20 mL) gegeben. Die Phasen werden getrennt und die wässrige Phase dreimal mit Dichlormethan (je 20 mL) extrahiert. Die vereinigten organischen Phasen werden mit Na₂SO₄ getrocknet. Anschließend werden unter reduzierten Druck am Rotationsverdampfer die Lösungsmittel entfernt und 200 mg Rohprodukt erhalten. Das Rohprodukt wird mittels präparativer LC-MS (Säule: Chromolith prep RP-18e, 100-25; Injektionsvolumen: 1300µL; mobile Phase A: Wasser + 0,1% Ameisensäure, mobile Phase B: Acetonitril + 0,1% Ameisensäure, Gradient: 0.0 min 20%B [Fluss 0.0 min bis 1.0 min.: 25ml/min], 1,0 min 20% B [Fluss ab 1.0 min.: 50ml/min], 2.2 min 20%B, 7.5 min 40%B, 7.6 min 100%B, 9.0 min 100%B) aufgereinigt. Die geeigneten Fraktionen (detektiert mittels analytischer LC-MS) werden vereinigt und mit gesättigter, wässriger Natriumhydrogencarbonat Lösung (60 mL) gewaschen. Die wässrige Lösung wird dreimal mit Dichlormethan (je 80 mL) extrahiert. Die vereinigten organischen Phasen werden mit gesättigter, wässriger Kochsalzlösung (20 mL) gewaschen und mit Na₂SO₄ getrocknet. Nach Entfernen der Lösungsmittel unter reduzierten Druck wird Imidazolon **XIII** (119 mg, 0.30 mmol, 75% Ausbeute) in Form farbloser Kristalle erhalten. Analytische Daten Imidazolon **XIII** ("A10"): DC: R_{f} = 0.17 (Si60, CH₂Cl₂/3% MeOH/1% NH₃); Smp.: 180°C; LC-MS: t_{R} = 1.693 min (UV = 220 nm), t_{R} = 1.716 min. (TIC, mit [MH]⁺ = 396); ¹H NMR (400 MHz, DMSO) δ 8.64 (dd, *J* = 4.8, 1.5, 1H), 8.62 - 8.61 (m, 1H), 8.47 (dd, *J* = 5.2, 0.6, 1H), 7.89 (ddd, *J* = 8.0, 2.3, 1.7, 1 H), 7.63 - 7.59 (m, 2H), 7.52 (dd, *J* = 5.2, 1.7, 1 H), 7.50 - 7.46 (m, 2H), 7.34 - 7.30 (m, 2H), 7.18 (d, *J =* 0.9, 1 H), 3.32 (s, 3H), 1.74 (s, 6H); ¹³C NMR (101 MHz, DMSO) δ 152.27, 149.70, 149.49, 146.95, 143.78, 139.87, 135.32, 133.54, 132.09, 127.31, 125.19, 123.97, 123.42, 121.07, 118.38, 116.94, 115.46, 36.01, 29.78, 27.78.

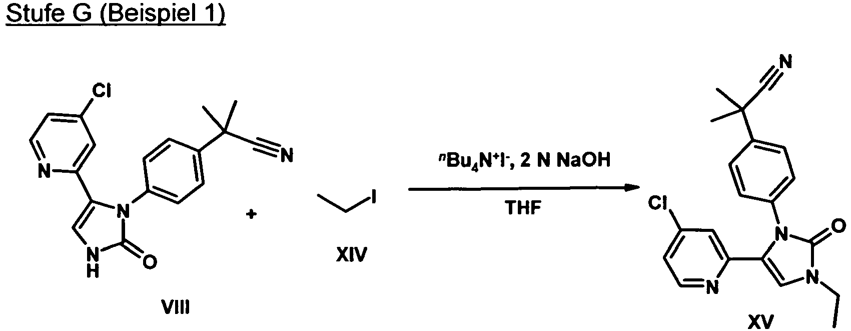

Imidazolon VIII (780 mg, 2.3 mmol) wird inTHF (8 mL) suspendiert. Tetra-n-butylammoniumiodid (85.9 mg, 0.23 mmol) und NaOH (4.65 mL einer 2 M NaOH Lösung, 0.009 mol) werden zugegeben. Die Suspension wird zur Lösung. Ethyliodid **XIV** (558 µL, 6.91 mmol) wird zugetropft, der Reaktionskolben wird verschlossen und die innere Kolbentemperatur auf 45°C erhöht. Nach 3 h Rühren zeigt ein analytisches LC-MS als Reaktionskontrolle einen guten Reaktionsumsatz. Die Reaktionslösung wir bei Raumtemperatur über Nacht gerührt. Die Reaktionsmischung wir in eine Wasser/Dichlormethan Mischung (30 mL/50 mL) gegeben. Die Phasen werden separiert und die wässrige Phase dreimal mit Dichlormethan (je 40 mL) extrahiert. Die vereinigten organischen Phasen werden mit Na₂SO₄ getrocknet und die Lösungsmittel bei reduzierten Druck am Rotationsverdampfer entfernt. Es werden 1.33 g orange farbiges Öl als Rohprodukt erhalten. Das Rohprodukt wird mittels Säulenchromatographie aufgereinigt (60 g Si60, Gradient CH₂Cl₂/2% MeOH/1% NH₃ zu CH₂Cl₂/3%MeOH/1%NH₃) und die geeigneten Fraktionen (charakterisiert mittels DC-Analytik) gesammelt. Nach Entfernen der Lösungsmittel wird Imidazolon **XV** (838 mg, 2.28 mmol, 99% Ausbeute) als gelbes Öl erhalten. Analytische Daten Imidazolon **XV**: DC: R_{f} = 0.35 (Si60, CH₂Cl₂/4% MeOH/1% NH₃); LC-MS: t_{R} = 2.125 min (UV = 220 nm), t_{R} = 2.132 min. (TIC, mit [MH]⁺ = 367); ¹H NMR (400 MHz, DMSO) δ 8.27 (d, *J =* 5.4, 1 H), 7.54 (d, *J =* 2.4, 2H), 7.53 - 7.51 (m, 1 H), 7.27 (d, *J* = 2.0, 1 H), 7.25 (t, *J* = 2.1, 1 H), 7.23 (d, *J* = 2.0, 1 H), 7.22 (d, *J* = 1.5, 1 H), 3.70 (q, *J* = 7.2, 2H), 1.70 (s, 6H), 1.29 (dd, *J =* 8.3, 6.2, 3H).

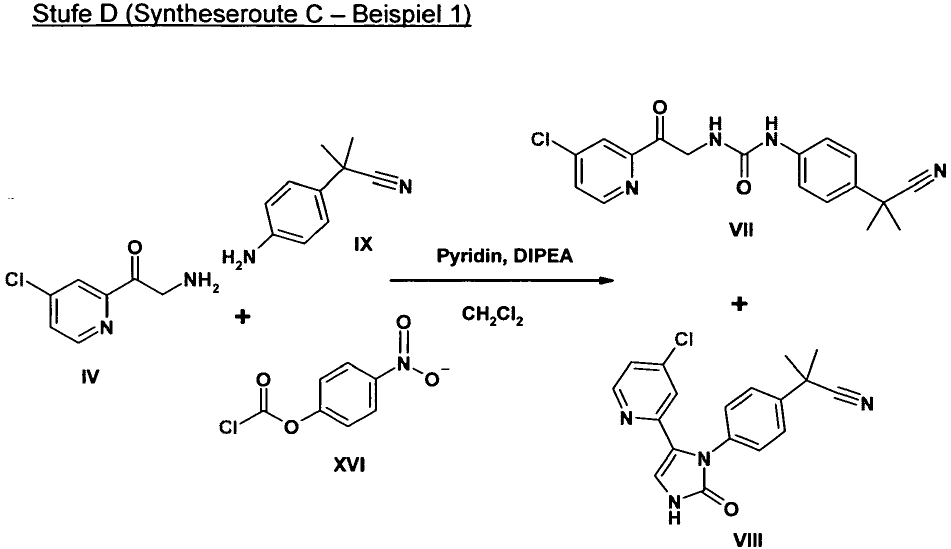

Anillin **IX** (8.42 g, 49.4 mmol), und Formiat **XVI** (9.95 g, 49.4 mmol) werden in trockenen CH₂Cl₂ (150 mL) suspendiert. In die dicke, nicht rührbare Suspension wird unter Eisbadkühlung (exotherme Reaktion!) Pyridin (6.43 mL, 79.7 mmol) langsam zugetropft. Die klare orange Reaktionslösung wird für 1 h bei Raumtemperatur gerührt. Mittels LC-MS Analytik wird überprüft, ob das Reaktionsprodukt entstanden ist. Amin IV (8.47 g, ∼95% RP-HPLC Reinheit, 50.2 mmol) wird zugegeben. Danach wird DIPEA (13.3 mL, 129 mmol) langsam (über ∼10 min.) zugetropft. Beim Eintropfen ist eine Gelbfärbung zu beobachten (Bildung von Nitrophenol). Die orange-braune Lösung mit Bodensatz wird für 15 min. bei Raumtemperatur gerührt. Die Reaktionsmischung wird auf gesättigte wässrige NaHCO₃-Lösung (200 mL) gegeben und für 20 min. gerührt. Die organische Phase wird separiert und die wässrige Phase mit CH₂Cl₂ (2 x 150 mL) extrahiert. Die vereinigten organischen Phasen werden mit Na₂SO₄ getrocknet. Unter reduzierten Druck werden am Rotationsverdampfer die Lösungsmittel entfernt. Man erhält als feuchtes Rohprodukt eine Mischung aus Harnstoff **VII** und Imidazolon **VIII** (27.7 g), das direkt weiter umgesetzt wird. Analytische Daten Rohmischung Harnstoff **VII** und Imidazolon **VIII**: siehe Stufe D (Syntheseroute A).

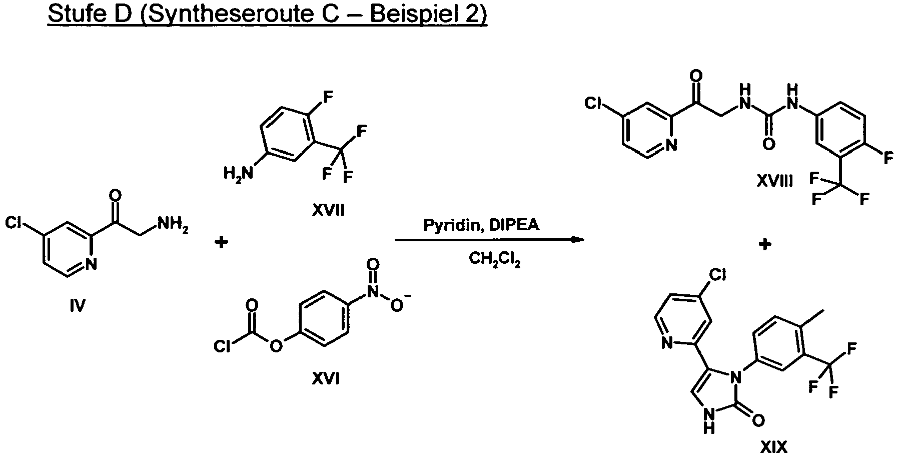

Anillin XVII (4.78 g, 26.7 mmol), und Formiat **XVI** (5.40 g, 26.7 mmol) werden in trockenen CH₂Cl₂ (80 mL) suspendiert. In die helle Suspension wird unter Eisbadkühlung Pyridin (2.37 mL, 29.4 mmol) zugetropft. Die klare, gelb-beige Reaktionslösung wird für 2h bei Raumtemperatur gerührt. Mittels LC-MS Analytik wird überprüft, ob das Reaktionsprodukt entstanden ist. Amin **IV** (4.67 g, 27.4 mmol) und DIPEA (4.99 mL, 29.4 mmol) werden zugegeben. Man erhält eine orange Suspension, die sich innerhalb einer 1 h Rühren hell beige färbt. Über Nacht wird weiter bei Raumtemperatur gerührt. Die Reaktionssuspension wird auf eine Mischung aus Wasser (120 mL)/ CH₂Cl₂ (100 mL) gegeben. Die wässrige Phase mit CH₂Cl₂ (3 x 100 mL) extrahiert. Die vereinigten organischen Phasen werden mit Na₂SO₄ getrocknet. Unter reduzierten Druck werden am Rotationsverdampfer die Lösungsmittel entfernt. Man erhält als Rohprodukt eine Mischung aus Harnstoff XVIII und Imidazolon **XIX** (15.9 g), das direkt weiter umgesetzt wird. Analytische Daten Rohmischung Harnstoff **XVIII** und Imidazolon **XIX:** LC-MS: a) Harnstoff **XVII -** t_{R} = 2.299 min (UV = 220 nm), t_{R} = 2.292 min. (TIC, mit [MH]⁺ = 376) und b) Imidazolon **XIX -** t_{R} = 2.027 min (UV = 220 nm), t_{R} = 2.020 min. (TIC, mit [MH]⁺ = 358).

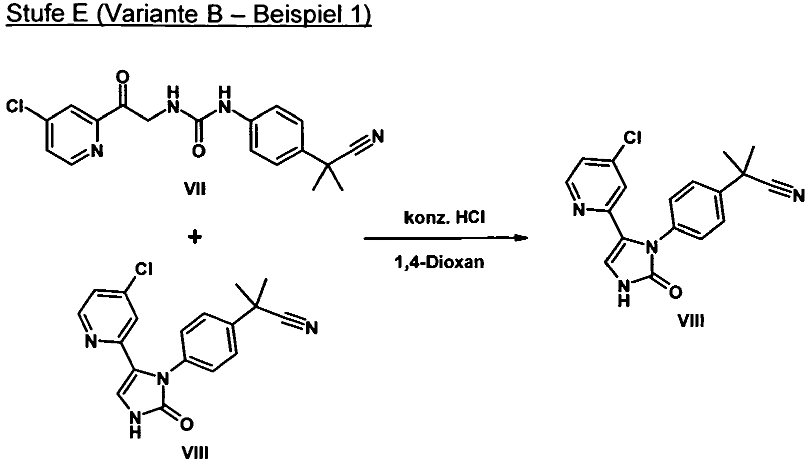

Eine Rohmischung aus Harnstoff **VII** und Imidazolon **VIII** (27.4 g; siehe auch Stufe D - Syntheseroute C Beispiel 1) wird in 1,4-Dioxan (100 mL) gelöst und auf 80°C erhitzt. Zu der orangefarbenen Lösung wird konz. HCl (5 mL einer 37% Salzsäure, 60.4 mmol) zugegeben. Die Reaktionsmischung wird bei 100°C für 1 h gerührt. Danach wird bei Raumtemperatur über Nacht gerührt. Die Reaktionslösung wird auf gesättigte wässrige NaHCO₃ Lösung (150 mL) gegeben. Die wässrige Phase wird mit CH₂Cl₂ (2 x 100 mL) extrahiert, die organischen Phasen vereinigt und mit Na₂SO₄ getrocknet. Anschließend werden die Lösungsmittel unter reduzierten Druck am Rotationsverdampfer entfernt. Das erhaltene Rohprodukt wird in EtOAc (150 mL) kurz ausgekocht, eingekühlt und abfiltriert. Der erhaltene Niederschlag wird mit Et₂O gewaschen und and der Luft getrocknet. Imidazolon **VIII** (3.5 g, 10.3 mmol, 21% Ausbeute über zwei Stufen) wird als beiger Feststoff erhalten. Analytische Daten Imidazolon **VIII:** siehe Stufe D (Syntheseroute A).

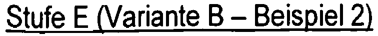

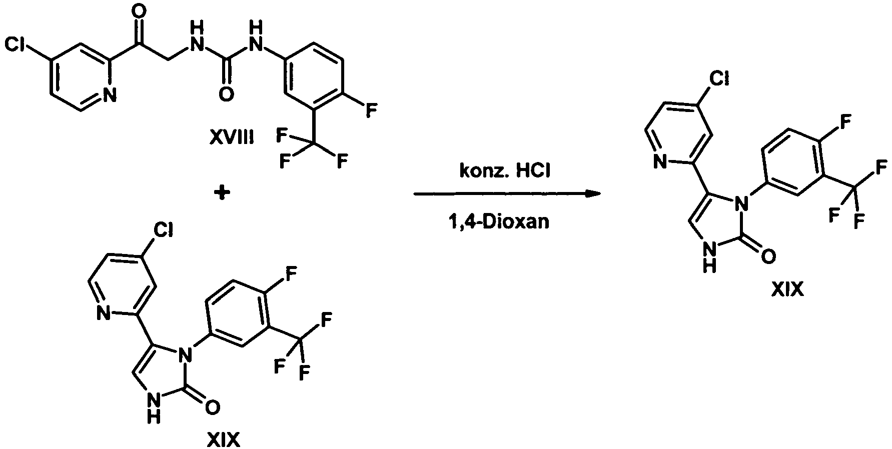

Eine Rohmischung aus Harnstoff **XVIII** und Imidazolon **XIX** (15.9 g; siehe auch Stufe D - Syntheseroute C Beispiel 2) wird in 1,4-Dioxan (200 mL) gelöst und auf 100°C erhitzt. Zur Lösung wird konz. HCl (4.42 mL einer 37% Salzsäure, 53.4 mmol) zugegeben. Die Reaktionsmischung trübt sich ein; wird nach 5 min. Rühren wieder klar. Die Reaktionslösung wird weitere 15 min. bei Raumtemperatur gerührt. Danach wird am Rotationsverdampfer ein Teil des 1,4-Dioxans entfernt. Die Reaktionslösung wird auf gesättigte wässrige NaHCO₃ Lösung (200 mL) gegeben. Die wässrige Phase wird mit CH₂Cl₂ (4 x 120 mL) extrahiert, die organischen Phasen vereinigt und mit Na₂SO₄ getrocknet. Anschließend werden die Lösungsmittel unter reduzierten Druck am Rotationsverdampfer entfernt. Das erhaltene orange Rohprodukt (13.3 g) wird mittels Säulenchromatographie aufgereinigt (150 g Si60, Gradient CH₂Cl₂/2% MeOH/1% NH₃ zu CH₂Cl₂/10%MeOH/1%NH₃) und die geeigneten Fraktionen (charakterisiert mittels DC-Analytik) gesammelt. Nach entfernen der Lösungsmittel und trocknen im Vakuum wird Imidazolon **XIX** (2.06 g, 5.76 mmol, 21% Ausbeute über zwei Stufen) als beiger Feststoff erhalten. Analytische Daten Imidazolon **XIX:** DC: R_{f} = 0.20 (Si60, CH₂Cl₂/5% MeOH/1% NH₃); LC-MS: t_{R} = 2.107 min (UV = 220 nm), t_{R} = 2.102 min. (TIC, mit [MH]⁺ = 358); ¹H NMR (400 MHz, DMSO) δ 10.99 (s, 1H), 8.18 (d, J = 5.4 Hz, 1H), 7.65 - 7.45 (m, 5H), 7.25 (dd, J = 5.4, 1.9 Hz, 1 H).

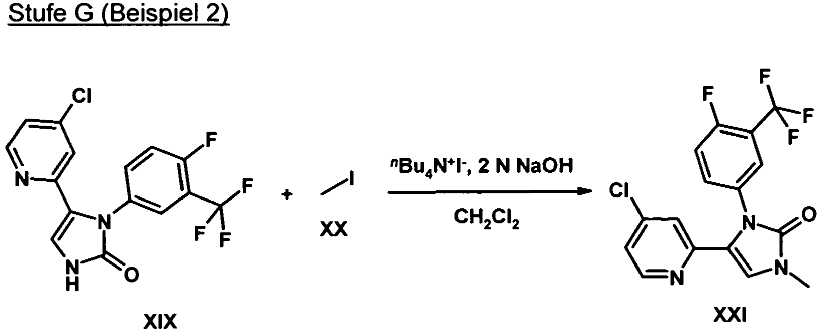

Imidazolon **XIX** (2.06 g, 5.76 mmol) wird in CH₂Cl₂ (8 mL) suspendiert. Tetra-n-butylammoniumiodid (214.9 mg, 0.576 mmol) und Methyliodid **XX** (466 µL, 7.49 mmol) werden zugegeben. Anschließend wird NaOH (8.64 mL einer 2 M NaOH Lösung, 0.017 mol) zugefügt, der Reaktionskolben wird verschlossen und die innere Kolbentemperatur auf ca. 50°C erhöht. Nach 2 h Rühren zeigt ein analytisches LC-MS als Reaktionskontrolle einen vollständigen Reaktionsumsatz. Die Reaktionsmischung wir in eine Wasser/ CH₂Cl₂ Mischung (90 mU80 mL) gegeben. Die Phasen werden separiert und die wässrige Phase mit CH₂Cl₂, versetzt mit 4%MeOH (4 x 40 mL), extrahiert. Die vereinigten organischen Phasen werden mit Na₂SO₄ getrocknet und die Lösungsmittel bei reduzierten Druck am Rotationsverdampfer entfernt. Als Rohprodukt werden 2.5 g oranges Öl erhalten. Das Rohprodukt wird mittels Chromatographie auf einer Flashchromatographieanlage (CombiFlash R_{f}, Teledyne Isco) aufgereinigt aufgereinigt (80 g Si60, Eluent A: CH₂Cl₂, Eluent B: EtOH/CH₂Cl₂ 1:1, Gradient: Eluent B von 0→10% über 20 min.) und die geeigneten Fraktionen gesammelt. Nach Entfernen der Lösungsmittel wird Metyhlimidazolon **XXI** (1.88 g, 5.09 mmol, 88% Ausbeute) als beige-gelber Feststoff erhalten. Analytische Daten Metyhlimidazolon XXI: DC: R_{f} = 0.34 (Si60, CH₂Cl₂/6% MeOH/1% NH₃); LC-MS: t_{R} = 2.200 min (UV = 220 nm), t_{R} = 2.199 min. (TIC, mit [MH]⁺ = 372); 1H NMR (400 MHz, DMSO) δ 8.20 (dd, J = 5.4, 0.5 Hz, 1 H), 7.62 - 7.49 (m, 5H), 7.26 (dd, J = 5.4, 2.0 Hz, 1 H), 3.30 (s, 3H).

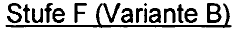

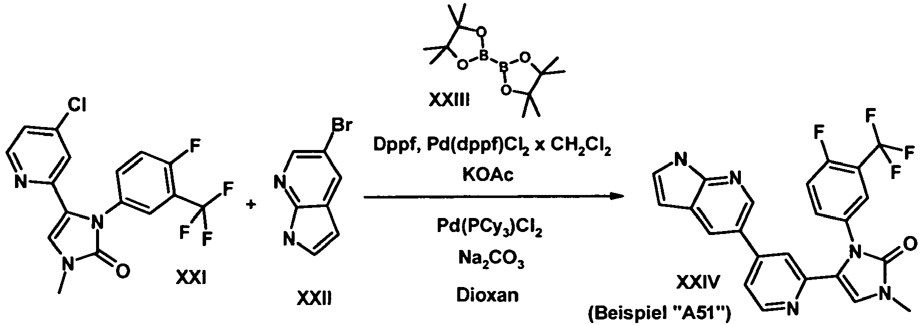

In einem 100 mL Kolben werden Azaindol XXII (1.01 g, 5.14 mmol), Diboronsäureester **XXIII** (1.31 g, 5.14 mol), Kaliumacetat (1.553 g, 15.82 mmol), 1,1'-Bis(diphenylphosphino)-ferrocen (65.77 mg, 0.119 mmol, abgekürzte Bezeichnung: Dppf) und 1,1'-Bis(diphenylphosphino)ferrocen-palladium(II)dichlorid Dichloromethan Komplex (96.84 mg, 0.119 mmol, abgekürzte Bezeichnung: Pd(dppf)Cl₂ × CH₂Cl₂) in 1,4-Dioxan (20 mL, entgast) vorgelegt. Die braune Suspension wird bei 130°C Ölbadtemperatur refluxiert. Nach 3 h Rühren zeigt ein analytisches LC-MS als Reaktionskontrolle einen vollständigen Reaktionsumsatz an. Imidazolon **XXI** (1.47 g, 3.96 mmol), Dichlorobis(tricyclohexylphosphin)palladium (II) (87.58 mg, 0.119 mmol, abgekürzte Bezeichnung: Pd(Cy₃)Cl₂) und wässriges Natriumcarbonat (5.93 mL einer 2 N Na₂CO₃ Lösung, entgast, dann unter N₂, 11.86 mmol) werden zu der Suspension gegeben. Die Reaktionslösung wird bei 125°C Ölbadtemperatur über Nacht refluxiert. Ein analytisches LC-MS als Reaktionskontrolle zeigt vollständigen Reaktionsumsatz an. Die Reaktionsmischung wird in eine Wasser/Dichlormethan Mischung (80 mU90 mL) gegeben. Die Phasen werden getrennt und die wässrige Phase viermal mit Dichlormethan, versetzt mit 10% MeOH (je 60 mL), extrahiert. Die vereinigten organischen Phasen werden mit Na₂SO₄ getrocknet. Anschließend werden unter reduzierten Druck am Rotationsverdampfer die Lösungsmittel entfernt und 6 g rotbraunes Rohprodukt erhalten. Das Rohprodukt wird mittels Säulenchromatographie aufgereinigt (120 g Si60, Gradient CH₂Cl₂/6% MeOH/1% NH₃ zu CH₂Cl₂/7%MeOH/1%NH₃) und die geeigneten Fraktionen (charakterisiert mittels DC-Analytik) gesammelt. Nach entfernen der Lösungsmittel wird das erhaltene Produkt in 2-Propanol (ca. 100 mL) ausgekocht, abfiltriert und im Vakuum getrocknet. Man erhält Pyridinimidazolon **XXIV** (1.24 g, 2.74 mmol, 69% Ausbeute) als beigefarbenen Feststoff. Analytische Daten Pyridinimidazolon **XXIV** ("A51"): DC: R_{f} = 0.24 (Si60, CH₂Cl₂/3% MeOH/1% NH₃); Smp.: 229°C; LC-MS: t_{R} = 1.948 min (UV = 220 nm), t_{R} = 1.936 min. (TIC, mit [MH]⁺ = 454); ¹H NMR (500 MHz, DMSO) δ 11.86 (s, 1 H), 8.58 (d, J = 2.1 Hz, 1 H), 8.30 (dd, J = 9.3, 3.7 Hz, 2H), 7.72 (dd, J = 17.9, 3.8 Hz, 2H), 7.62 - 7.52 (m, 5H), 6.54 - 6.47 (m, 1 H), 3.32 (s, 3H).

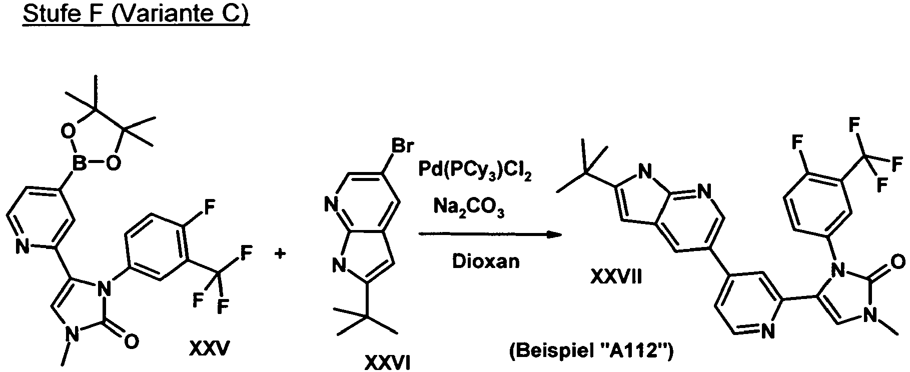

In einem Mikrowellensynthesegefäß werden Boronsäureester **XXV** (694.9 mg, 1.50 mmol), Azaindolbromid **XXVI** (379.7 mg, 1.50 mol), Pd(Cy₃)Cl₂ (33.2 mg, 0.045 mmol) und wässriges Natriumcarbonat (2.25 mL einer 2 N Na₂CO₃ Lösung, entgast, dann unter N₂, 4.50 mmol) in 1,4-Dioxan (12 mL, entgast) suspendiert. Die braune Suspension wird in einem Synthesemikrowellenofen (Personal Chemistry 300W, 140°C, 36 min.) erhitzt. Ein analytisches LC-MS als Reaktionskontrolle zeigt einen vollständigen Reaktionsumsatz an. Die Reaktionsmischung wird in eine Wasser/Dichlormethan Mischung (50 mL70 mL) gegeben. Die Phasen werden getrennt und die wässrige Phase mit Dichlormethan (3 x 40 mL), extrahiert. Die vereinigten organischen Phasen werden mit Na₂SO₄ getrocknet, unter reduzierten Druck am Rotationsverdampfer die Lösungsmittel entfernt und 1.5 g rotbraunes Rohprodukt erhalten. Das Rohprodukt wird mittels Chromatographie auf einer Flashchromatographieanlage (CombiFlash R_{f}, Teledyne Isco) aufgereinigt (40 g Si60, Eluent A: CH₂Cl₂, Eluent B: EtOH/CH₂Cl₂ 1:1, Gradient: Eluent B von 0→18.5% über 20 min.) und die geeigneten Fraktionen gesammelt. Nach Entfernen der Lösungsmittel wird Pyridinimidazolon **XXVII** (573 mg, 1.13 mmol, 75% Ausbeute) als beigefarbener Feststoff erhalten. Analytische Daten Pyridinimidazolon **XXVII** ("A112"): DC: R_{f} = 0.35 (Si60, CH₂Cl₂/5% EtO); LC-MS: t_{R} = 2.378 min (UV = 220 nm), t_{R} = 2.363 min. (TIC, mit [MH]⁺ = 510); ¹H NMR (400 MHz, DMSO) δ 11.75 (s, 1 H), 8.48 (d, J = 2.2 Hz, 1 H), 8.31 (d, J = 5.2 Hz, 1 H), 8.14 (d, J = 2.1 Hz, 1 H), 7.73 - 7.66 (m, 2H), 7.54 (ddd, J = 7.0, 5.9, 1.5 Hz, 4H), 6.20 (d, J = 2.1 Hz, 1 H), 3.33 (s, 3H), 1.38 (s, 9H).

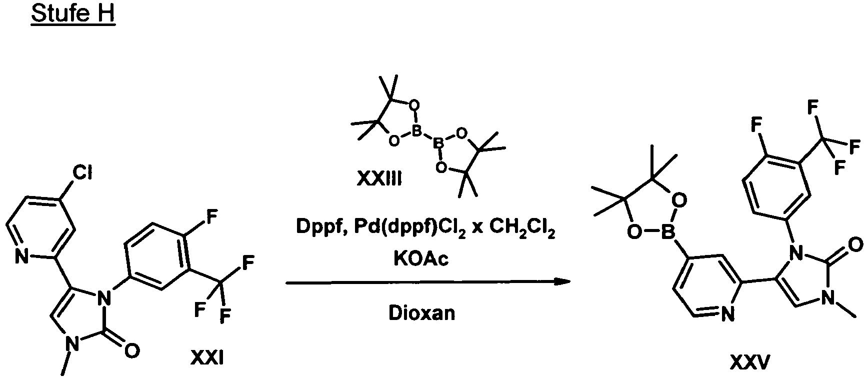

In einem Mikrowellensynthesegefäß werden Imidazolon **XXI** (1.835 g, 4.937 mmol), Diboronsäureester XXIII (1.25 g, 4.937 mol), Kaliumacetat (1.454 g, 14.81 mmol), Dppf (82.4 mg, 0.148 mmol) und Pd(dppf)Cl₂ x CH₂Cl₂ Komplex (120.9 mg, 0.148 mmol) in 1,4-Dioxan (40 mL, entgast) vorgelegt. Die braune Suspension wird in einem Synthesemikrowellenofen (Personal Chemistry 300W, 140°C, 36 min.) erhitzt. Ein analytisches LC-MS als Reaktionskontrolle zeigt einen vollständigen Reaktionsumsatz an. Die Reaktionsmischung wird in eine Wasser/Dichlormethan Mischung (60 mL/40 mL) gegeben. Die Phasen werden getrennt und die wässrige Phase dreimal mit Dichlormethan, versetzt mit 10% MeOH (je 30 mL), extrahiert. Die vereinigten organischen Phasen werden mit Na₂SO₄ getrocknet. Anschließend werden unter reduzierten Druck am Rotationsverdampfer die Lösungsmittel entfernt und Boronsäureester **XXV** (3.25g) als rotbraunes Rohprodukt erhalten. Das Rohprodukt wird ohne weitere Aufreinigung umgesetzt (siehe Stufe F, Variante C). Analytische Daten Boronsäureester **XXV:** LC-MS: t_{R} = 1.615 min (UV = 220 nm), t_{R} = 1.613 min. (TIC, mit [MH]⁺ = 382, Boronsäurepeak [MH]⁺).

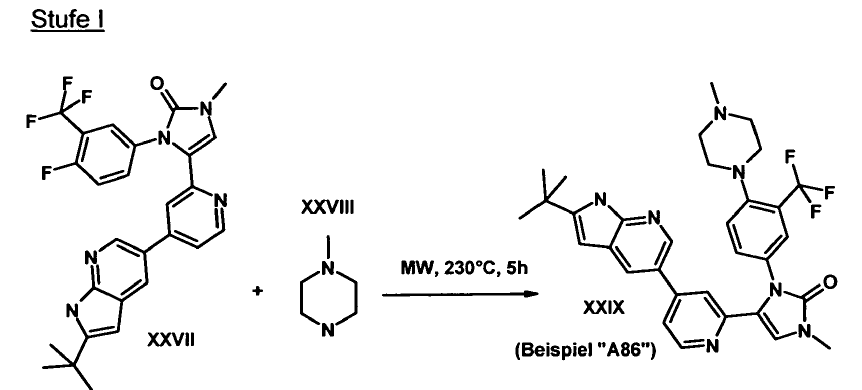

In einem Mikrowellensynthesegefäß werden Pyridinimidazolon **XXVII** (150 mg, 0.294 mmol) mit N-Methylpiperazin **XXVIII** (0.688 mL, 6.183 mol) vermischt. Die Reaktionslösung wird in einem Synthesemikrowellenofen (Personal Chemistry 300W, 230°C, 5h) erhitzt. Ein analytisches LC-MS als Reaktionskontrolle zeigt einen vollständigen Reaktionsumsatz an. Überschüssiges N-Methylpiperazin wird unter reduzierten Druck am Rotationsverdampfer die Lösungsmittel entfernt und ein braunes Öl (450 mg) erhalten. Das Rohprodukt wird mittels präparativer LC-MS (Säule: Chromolith prep RP-18e, 100-25; Injektionsvolumen: 1300µL; mobile Phase A: Wasser + 0,1% Ameisensäure, mobile Phase B: Acetonitril + 0,1% Ameisensäure, Gradient: 0.0 min 20%B [Fluss 0.0 min bis 1.0 min.: 25ml/min], 1,0 min 20% B [Fluss ab 1.0 min.: 50ml/min], 2.2 min 20%B, 7.5 min 40%B, 7.6 min 100%B, 9.0 min 100%B) aufgereinigt. Die geeigneten Fraktionen (detektiert mittels analytischer LC-MS) werden vereinigt und mit gesättigter, wässriger Natriumhydrogencarbonat Lösung (60 mL) gewaschen. Die wässrige Lösung wird mit Dichlormethan (3 x 40 mL) extrahiert. Die vereinigten organischen Phasen werden mit gesättigter, wässriger Kochsalzlösung (20 mL) gewaschen und mit Na₂SO₄ getrocknet. Nach Entfernen der Lösungsmittel unter reduzierten Druck wird Pyridinimidazolon **XXIX** (21 mg, 0.036 mmol, 12% Ausbeute) als hellgelber Feststoff erhalten. Analytische Daten Pyridinimidazolon **XXIX** ("A86"): DC: R_{f} = 0.34 (Si60, CH₂Cl₂/10% MeOH/1% NH₃); LC-MS: t_{R} = 1.762 min (UV = 220 nm), t_{R} = 1.755 min. (TIC, mit [MH]⁺ = 590); ¹H NMR (400 MHz, DMSO) δ 11.72 (s, 1H), 8.40 (d, J = 5.3 Hz, 1H), 8.29 (d, J = 2.2 Hz, 1H), 7.85 (d, J = 2.1 Hz, 1 H), 7.63 - 7.59 (m, 2H), 7.54 - 7.45 (m, 3H), 7.19 (d, J = 0.9 Hz, 1 H), 6.14 (d, J = 2.1 Hz, 1 H), 3.33 (s, 3H), 2.90 (t, J = 4.6 Hz, 4H), 2.24 (s, 3H), 1.37 (s, 9H).

Analog werden die nachstehenden Verbindungen hergestellt:

### 2-Methyl-2-{4-[3-methyl-2-oxo-5-(4-chinolin-3-yl-pyridin-2-yl)-2,3-dihydro-imidazol-1-yl]-phenyl}-propionitril ("A1")

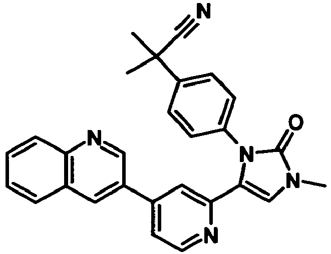

Analytische Daten "A1 ": DC: R_{f} = 0.22 (Si60, CH₂Cl₂/2% MeOH/10% NH₃); Smp.: 228°C; LC-MS: t_{R} = 2.059 min (UV = 220 nm), t_{R} = 2.056 min. (TIC, mit [MH]⁺ = 446); ¹H NMR (400 MHz, CDCl₃) δ [ppm] 8.78 (d, *J* = 2.3, 1 H), 8.61 (dd, *J =* 5.1, 0.7, 1 H), 8.11 (d, *J* = 8.4, 1 H), 8.01 (d, *J* = 2.1, 1 H), 7.84 (d, *J* = 8.1, 1H), 7.76 (ddd, *J* = 8.4, 6.9, 1.4, 1 H), 7.63 - 7.56 (m, 3H), 7.44 - 7.38 (m, 3H), 7.03 (s, 1H), 7.00 (dd, *J =* 1.6, 0.7, 1H), 3.44 (s, 3H), 1.75 (s, 6H); ¹³C NMR (101 MHz, CDCl₃) δ [ppm] 153.56, 150.54, 149.06, 148.47, 148.24, 145.44, 140.88, 135.72, 133.67, 130.47, 129.43, 128.20, 127.79, 127.57, 126.06, 124.24, 122.82, 119.56, 118.67, 115.04, 37.14, 30.83, 29.27.

### 2-Methyl-2-{4-[3-methyl-2-oxo-5-(5-chinolin-3-yl-pyridin-3-yl)-2,3-dihydro-imidazol-1-yl]-phenyl}-propionitril ("A2")

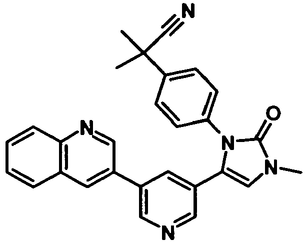

Analytische Daten "A2": LC-MS: t_{R} = 1.950 min (UV = 220 nm), t_{R} = 1.952 min. (TIC, mit [MH]⁺ = 446); ¹H NMR (500 MHz, CDCl₃) δ [ppm] 8.84 (d, *J* = 2.3, 1 H), 8.80 (d, *J =* 2.1, 1 H), 8.47 (d, *J =* 2.0, 1 H), 8.15 - 8.12 (m, 1H), 8.09 (d, *J =* 2.1, 1 H), 7.86 (d, *J =* 7.9, 1 H), 7.76 (ddd, *J =* 8.4, 6.9, 1.3, 1 H), 7.61 (dd, *J* = 11.5, 4.4, 2H), 7.53 (ddd, *J* = 14.2, 5.5, 2.0, 3H), 7.34 - 7.28 (m, 2H), 3.44 (s, 3H), 1.73 (s, 6H).

### 2-(4-{5-[4-(3-Methoxy-phenyl)-pyridin-2-yl]-3-methyl-2-oxo-2,3-dihydroimidazol-1-yl}-phenyl)-2-methyl-propionitril ("A3")

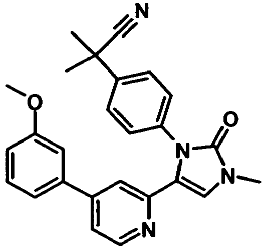

Analytische Daten "A3": DC: R_{f} = 0.29 (Si60, CH₂Cl₂/4% MeOH/1% NH₃); LC-MS: t_{R} = 2.140 min (UV = 220 nm), t_{R} = 2.164 min. (TIC, mit [MH]⁺ = 425); ¹H NMR

(300 MHz, DMSO-d₆) δ [ppm] 8.51 - 8.47 (m, 1H), 7.69 - 7.63 (m, 2H), 7.51 (dd, *J* = 5.2, 1.7, 1 H), 7.49 (s, 1 H), 7.41 - 7.35 (m, 3H), 7.13 (d, *J* = 1.0, 1 H), 7.10 - 6.99 (m, 3H), 3.85 (s, 3H), 3.37 (s, 3H), 1.78 (s, 6H);
¹³C NMR (75 MHz, DMSO) δ [ppm] 159.75, 152.81, 149.85, 148.59, 146.94, 140.36, 138.40, 135.85, 130.15, 127.78, 125.69, 124.44, 121.72, 118.91, 118.60, 117.42, 115.72, 114.46, 112.40, 66.31, 55.19, 36.47, 30.25, 28.21.

### 5-(6-Methoxy-[3,4']bipyridinyl-2'-yl)-1-(4-piperazin-1-yl-3-trifluormethyl-phenyl)-1,3-dihydro-imidazol-2-on ("A4")

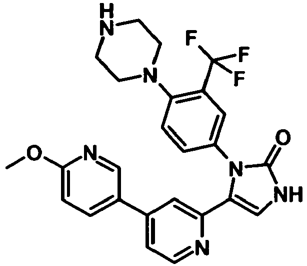

Analytische Daten "A4": DC: R_{f} = 0.25 (Si60, CH₂Cl₂/10% MeOH/1% NH₃); LC-MS: t_{R} = 1.495 min (UV = 220 nm), t_{R} = 1.490 min. (TIC, mit [MH]⁺ = 497); ¹H NMR (500 MHz, DMSO-d₆) δ [ppm] 8.40 (d, *J* = 2.4, 1 H), 8.35 (d, *J* = 5.3, 1 H), 8.00 (dd, *J* = 8.7, 2.6, 1 H), 7.55 - 7.39 (m, 5H), 7.36 (s, 1 H), 6.92 (d, *J* = 8.6, 1 H), 3.90 (s, 3H), 2.82 (d, *J* = 4.2, 8H).

### 1-(4-Piperazin-1-yl-3-trifluormethyl-phenyl)-5-(4-chinolin-3-yl-pyridin-2-yl)-1,3-dihydro-imidazol-2-on ("A5")

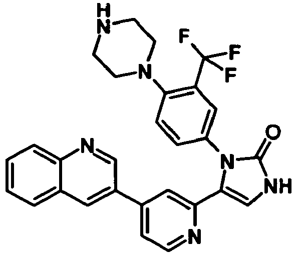

Analytische Daten "A5": DC: R_{f} = 0.23 (Si60, CH₂Cl₂/10% MeOH/1% NH₃); Smp.: 155°C (schäumt); LC-MS: t_{R} = 1.449 min (UV = 220 nm), t_{R} = 1.503 min. (TIC, mit [MH]⁺ = 517); ¹H NMR (400 MHz, DMSO-d₆) δ [ppm] 9.23 (d, *J* = 2.3, 1H), 8.75 (d, *J =* 2.1, 1H), 8.49 (dd, *J =* 5.2, 0.5, 1 H), 8.16 - 8.07 (m, 3H), 7.89 (ddd, *J* = 8.4, 6.9, 1.4, 1 H), 7.80 (s, 1 H), 7.78 - 7.73 (m, 2H), 7.58 (dd, *J* = 9.8, 5.5, 2H), 7.51 - 7.47 (m, 2H), 2.86 (d, *J* = 5.8, 8H);
¹³C NMR (101 MHz, DMSO) δ [ppm] 154.04, 151.46, 150.10, 149.71, 149.19, 148.04, 144.99, 134.31, 133.37, 132.45, 130.94, 130.26, 129.23, 129.00, 127.84, 127.72, 126.11, 125.52, 124.93, 123.38, 119.51, 118.52, 112.91, 54.82, 46.31.

### 2-{4-[5-(2'-Methoxy-[4,4']bipyridinyl-2-yl)-3-methyl-2-oxo-2,3-dihydro-imidazol-1-yl]-phenyl}-2-methyl-propionitril ("A6")

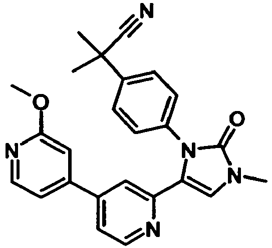

Analytische Daten "A6": DC: R_{f} = 0.38 (Si60, CH₂Cl₂/8% MeOH/1% NH₃); Smp.: 138°C; LC-MS: t_{R} = 2.029 min (UV = 220 nm), t_{R} = 2.035 min. (TIC, mit [MH]⁺ = 426); ¹H NMR (300 MHz, DMSO-d₆) δ [ppm] 8.48 (d, *J =* 5.3, 1 H), 8.22 (d, *J* = 5.4, 1 H), 7.62 - 7.58 (m, 2H), 7.52 (dd, *J* = 5.2, 1.7, 1 H), 7.33-7.28 (m, 2H), 7.26-7.22 (m, 1H), 7.16 (d, *J =* 0.8, 1H), 7.06 (dd, *J =* 5.4, 1.5, 1 H), 6.82 (d, J = 0.8, 1 H), 3.88 (s, 3H), 3.31 (s, 3H), 1.73 (s, 6H);
¹³C NMR (75 MHz, DMSO) δ [ppm] 164.36, 152.79, 150.12, 148.89, 147.79, 144.30, 140.40, 135.80, 127.80, 125.69, 125.45, 124.43, 121.46, 119.72, 118.75, 116.92, 114.43, 107.66, 53.27, 36.47, 30.27, 28.18.

### 2-Methyl-2-(4-{3-methyl-5-[4-(2-methyl-3H-imidazo[4,5-b]pyridin-6-yl)-pyridin-2-yl]-2-oxo-2,3-dihydro-imidazol-1-yl}-phenyl)-propionitril ("A7")

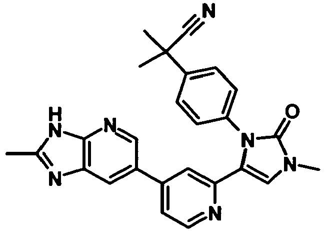

Analytische Daten "A7": DC: R_{f} = 0.41 (Si60, CH₂Cl₂/8% MeOH/1% NH₃); Smp.: 288°C; LC-MS: t_{R} = 1.502 min (UV = 220 nm), t_{R} = 1.496 min. (TIC, mit [MH]⁺ = 450); ¹H NMR (400 MHz, DMSO-d₆) δ [ppm] 8.42 (d, *J* = 5.2, 1H), 8.23 (s, 1 H), 8.05 (d, *J =* 1.7, 1 H), 7.61 (d, *J =* 8.6, 2H), 7.55 (dd, *J =* 5.2, 1.7, 1 H), 7.47 (s, 1 H), 7.32 (d, *J =* 8.6, 2H), 7.26 (s, breit, 1 H), 3.32 (s, 3H), 2.54 (s, 3H), 1.75 (s, 6H);
¹³C NMR (101 MHz, DMSO-d₆) δ [ppm] 152.29, 149.29, 148.15, 145.18, 141.37, 140.73, 139.79, 135.39, 127.23, 125.12, 123.96, 121.25, 118.55, 117.13, 115.26, 112.94, 36.08, 29.77, 27.86, 14.64.

### 2-{4-[5-(6-Amino-[3,4']bipyridinyl-2'-yl)-3-methyl-2-oxo-2,3-dihydro-imidazol-1-yl]-phenyl}-2-methyl-propionitril ("A8")

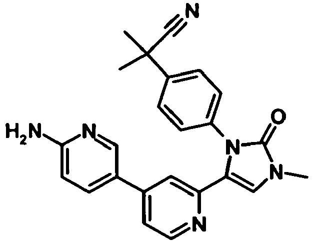

Analytische Daten "A8": DC: R_{f} = 0.21 (Si60, CH₂Cl₂/5% MeOH/1% NH₃); Smp.: 252°C; LC-MS: t_{R} = 1.306 min (UV = 220 nm), t_{R} = 1.325 min. (TIC, mit [MH]⁺ = 411); ¹H NMR (400 MHz, DMSO-d₆) δ [ppm] 8.33 (dd, J = 5.3, 0.6, 1 H), 8.07 - 8.05 (m, 1 H), 7.63 - 7.58 (m, 2H), 7.44 - 7.38 (m, 2H), 7.35 (dd, *J* = 5.3, 1.8, 1 H), 7.33 - 7.28 (m, 2H), 6.99 (d, *J* = 1.0, 1 H), 6.48 - 6.44 (m, 1 H), 6.35 (s, 2H), 3.30 (s, 3H), 1.74 (s, 6H);
¹³C NMR (101 MHz, DMSO-d₆) δ [ppm] 159.88, 152.27, 149.18, 147.94, 145.89, 144.58, 139.82, 135.39, 134.23, 127.30, 125.15, 123.96, 121.38, 119.60, 116.58, 115.10, 114.93, 107.30, 36.04, 29.74, 27.83.

### 2-{4-[5-(6-Methoxy-[3,4']bipyridinyl-2'-yl)-3-methyl-2-oxo-2,3-dihydro-imidazol-1-yl]-phenyl}-2-methyl-propionitril ("A9")

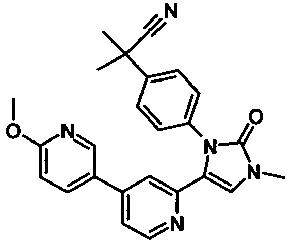

Analytische Daten "A9": DC: R_{f} = 0.27 (Si60, CH₂Cl₂/5% MeOH/1% NH₃); Smp.: 187°C; LC-MS: t_{R} = 2.071 min (UV = 220 nm), t_{R} = 2.083 min. (TIC, mit [MH]⁺ = 426); ¹H NMR (400 MHz, DMSO-d₆) δ [ppm] 8.42 (d, *J* = 5.2, 1H), 8.22 (d, *J* = 2.2, 1H), 7.83 (dd, J = 8.7, 2.6, 1H), 7.63 - 7.58 (m, 2H), 7.48 - 7.43 (m, 2H), 7.34 - 7.29 (m, 2H), 7.10 (d, *J* = 0.9, 1 H), 6.89 (d, *J* = 8.9, 1 H), 3.88 (s, 3H), 3.31 (s, 3H), 1.74 (s, 6H);
¹³C NMR (101 MHz, DMSO-d₆) δ [ppm] 163.61, 152.27, 149.38, 148.13, 144.62, 143.55, 139.91, 136.61, 135.38, 127.37, 125.59, 125.22, 123.98, 121.16, 117.68, 116.22, 115.23, 110.28, 52.89, 36.01, 29.77, 27.75.

### 2-[4-(5-[3,4']Bipyridinyl-2'-yl-3-methyl-2-oxo-2,3-dihydro-imidazol-1-yl)-phenyl]-2-methyl-propionitril ("A10")

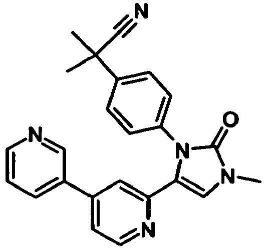

Analytische Daten ("A10"): DC: R_{f} = 0.17 (Si60, CH₂Cl₂/3% MeOH/1% NH₃); Smp.: 180°C; LC-MS: t_{R} = 1.693 min (UV = 220 nm), t_{R} = 1.716 min. (TIC, mit [MH]⁺ = 396); ¹H NMR (400 MHz, DMSO-d₆) δ [ppm] 8.64 (dd, *J* = 4.8, 1.5, 1 H), 8.62 - 8.61 (m, 1 H), 8.47 (dd, *J* = 5.2, 0.6, 1 H), 7.89 (ddd, *J* = 8.0, 2.3, 1.7, 1 H), 7.63 - 7.59 (m, 2H), 7.52 (dd, *J* = 5.2, 1.7, 1 H), 7.50 - 7.46 (m, 2H), 7.34 - 7.30 (m, 2H), 7.18 (d, *J* = 0.9, 1 H), 3.32 (s, 3H), 1.74 (s, 6H);
¹³C NMR (101 MHz, DMSO-d₆) δ [ppm] 152.27, 149.70, 149.49, 146.95, 143.78, 139.87, 135.32, 133.54, 132.09, 127.31, 125.19, 123.97, 123.42, 121.07, 118.38, 116.94, 115.46, 36.01, 29.78, 27.78.

### 2-[4-(5-[4,4']Bipyridinyl-2-yl-3-methyl-2-oxo-2,3-dihydro-imidazol-1-yl)-phenyl]-2-methyl-propionitril ("A11")

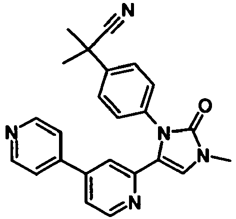

Analytische Daten "A11": DC: R_{f} = 0.16 (Si60, CH₂Cl₂/3% MeOH/1% NH₃); Smp.: 226°C; LC-MS: t_{R} = 1.576 min (UV = 220 nm), t_{R} = 1.578 min. (TIC, mit [MH]⁺ = 396); ¹H NMR (400 MHz, DMSO-d₆) δ [ppm] 8.66 (dd, *J* = 4.5, 1.6, 2H), 8.52 - 8.48 (m, 1 H), 7.62 - 7.58 (m, 2H), 7.56 (dd, *J* = 5.2, 1.7, 1 H), 7.50 - 7.47 (m, 3H), 7.34 - 7.29 (m, 2H), 7.26 (d, *J* = 0.8, 1 H), 3.32 (s, 3H), 1.72 (s, 6H);
¹³C NMR (101 MHz, DMSO-d₆) δ [ppm] 152.29, 149.92, 149.66, 148.51, 144.00, 143.68, 139.78, 135.33, 127.26, 125.22, 124.05, 120.98, 120.42, 118.27, 116.88, 115.63, 35.90, 29.79, 27.68.

### 2-Methyl-2-(4-{3-methyl-2-oxo-5-[4-(1H-pyrrolo[2,3-b]pyridin-5-yl)-pyridin-2-yl]-2,3-dihydro-imidazol-1-yl}-phenyl)-propionitril ("A12")

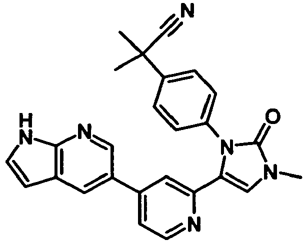

Analytische Daten "A12": DC: R_{f} = 0.13 (Si60, CH₂Cl₂/3% MeOH/1% NH₃); Smp.: 248°C; LC-MS: t_{R} = 1.799 min (UV = 220 nm), t_{R} = 1.805 min. (TIC, mit [MH]⁺ = 435); ¹H NMR (400 MHz, DMSO-d₆) δ [ppm] 11.87 (s, 1 H), 8.43 (d, *J* = 5.2, 1 H), 8.24 (d, *J* = 2.2, 1 H), 8.04 (d, *J* = 2.2, 1 H), 7.66 - 7.61 (m, 2H), 7.56 - 7.51 (m, 2H), 7.45 (s, 1 H), 7.37 - 7.32 (m, 2H), 7.16 (d, *J* = 0.9, 1 H), 6.49 (d, *J* = 2.5, 1 H), 3.32 (s, 3H), 1.75 (s, 6H);
¹³C NMR (101 MHz, DMSO-d₆) δ [ppm]152.30, 149.31, 148.26, 148.05, 145.71, 140.60, 139.88, 135.38, 127.34, 127.09, 125.55, 125.19, 124.29, 123.98, 121.32, 119.04, 118.34, 116.88, 115.17, 99.83, 36.09, 29.77, 27.86.

### 2-(4-{5-[4-(4-Methoxy-phenyl)-pyridin-2-yl]-3-methyl-2-oxo-2,3-dihydroimidazol-1-yl}-phenyl)-2-methyl-propionitril ("A13")

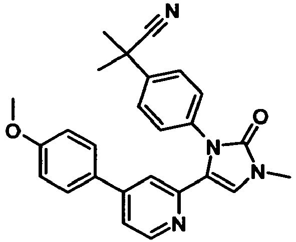

Analytische Daten "A13": DC: R_{f} = 0.18 (Si60, CH₂Cl₂/3% MeOH/1% NH₃); Smp.: 219°C; LC-MS: t_{R} = 2.118 min (UV = 220 nm), t_{R} = 2.150 min. (TIC, mit [MH]⁺ = 425); ¹H NMR (400 MHz, DMSO-d₆) δ [ppm] 8.39 (dd, *J =* 5.2, 0.5, 1H), 7.63 - 7.59 (m, 2H), 7.42 - 7.36 (m, 4H), 7.34 - 7.30 (m, 2H), 7.04 (d, *J* = 1.0, 1 H), 7.00 - 6.96 (m, 2H), 3.79 (s, 3H), 3.31 (s, 3H), 1.73 (s, 6H);
¹³C NMR (101 MHz, DMSO-d₆) δ [ppm] 159.74, 152.29, 149.30, 148.02, 146.10, 139.85, 135.41, 128.52, 127.33, 127.22, 125.27, 124.07, 121.33, 117.70, 116.25, 115.04, 113.97, 54.74, 35.90, 29.75, 27.70.

### 2-(4-{5-[5-(4-Methoxy-phenyl)-pyridin-3-yl]-3-methyl-2-oxo-2,3-dihydroimidazol-1-yl}-phenyl)-2-methyl-propionitril ("A14")

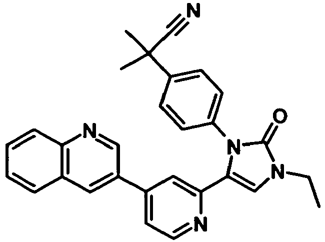

Analytische Daten "A14": DC: R_{f} = 0.36 (Si60, CH₂Cl₂/4% MeOH/1% NH₃); Smp.: 200°C; LC-MS: t_{R} = 2.160 min (UV = 220 nm), t_{R} = 2.159 min. (TIC, mit [MH]⁺ = 460); ¹H NMR (400 MHz, DMSO-d₆) δ [ppm] 9.01 (d, *J* = 2.3, 1H), 8.65 (d, *J* = 2.2, 1 H), 8.49 (d, J = 5.2, 1 H), 8.09 - 8.03 (m, 2H), 7.84 (ddd, *J* = 8.4, 6.9, 1.4, 1 H), 7.72 - 7.67 (m, 2H), 7.61 - 7.55 (m, 4H), 7.35 - 7.31 (m, 2H), 3.74 (q, *J* = 7.2, 2H), 1.72 (s, 6H), 1.33 (t, *J* = 7.2, 3H);
¹³C NMR (101 MHz, DMSO-d₆) δ [ppm] 151.81, 149.38, 148.56, 148.07, 147.03, 143.92, 139.55, 135.39, 133.31, 129.98, 129.26, 128.23, 128.06, 127.10, 126.87, 126.70, 125.01, 124.00, 121.33, 118.58, 117.40, 114.04, 37.59, 36.00, 27.79, 13.94.

### 2-{4-[5-(6-Methoxy-[3,4']bipyridinyl-2'-yl)-2-oxo-2,3-dihydro-imidazol-1-yl]-phenyl}-2-methyl-propionitril ("A15")

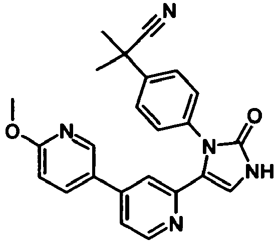

Analytische Daten "A15": DC: R_{f} = 0.18 (Si60, CH₂Cl₂/5% MeOH/1% NH₃); Smp.: 277°C; LC-MS: t_{R} = 1.968 min (UV = 220 nm), t_{R} = 1.963 min. (TIC, mit [MH]⁺ = 412); ¹H NMR (400 MHz, DMSO-d₆) δ [ppm] 10.80 (s, breit, 1H), 8.39 (d, *J =* 5.2, 1 H), 8.28 (d, *J* = 2.6, 1 H), 7.88 (dd, *J* = 8.7, 2.6, 1 H), 7.59 - 7.55 (m, 2H), 7.46 (dd, *J* = 5.2, 1.7, 1 H), 7.31 - 7.26 (m, 3H), 7.22 (d, *J* = 0.8, 1 H), 6.90 (d, *J =* 8.7, 1 H), 3.89 (s, 3H), 1.73 (s, 6H);
¹³C NMR (101 MHz, DMSO-d₆) δ [ppm] 163.61, 153.07, 149.24, 148.50, 144.69, 143.59, 139.50, 136.70, 135.40, 127.24, 125.65, 125.03, 124.03, 122.69, 117.64, 116.36, 111.41, 110.28, 52.89, 35.97, 27.76.

### 1-[4-(2-Amino-1,1-dimethyl-ethyl)-phenyl]-5-(4-chinolin-3-yl-pyridin-2-yl)-1,3-dihydro-imidazol-2-on ("A16")

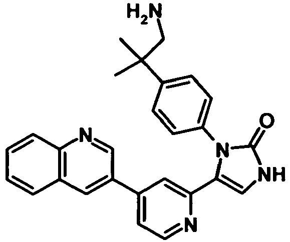

Analytische Daten "A16": DC: R_{f} = 0.11 (Si60, CH₂Cl₂/8% MeOH/1% NH₃); Smp.: 244-247°C; LC-MS: t_{R} = 1.494 min (UV = 220 nm), t_{R} = 1.489 min. (TIC, mit [MH]⁺ = 436); ¹H NMR (400 MHz, DMSO-d₆) δ [ppm] 8.91 (d, *J =* 2.3, 1 H), 8.60 (d, *J* = 2.1, 1 H), 8.50 (dd, *J* = 5.2, 0.6, 1 H), 8.07 - 8.01 (m, 2H), 7.82 (ddd, *J* = 8.4, 6.9, 1.5, 1 H), 7.71 - 7.64 (m, 2H), 7.43 - 7.38 (m, 3H), 7.31 (s, 1 H), 7.21 - 7.18 (m, 2H), 3.34 (s, breit, 3H), 2.68 (s, 2H), 1.25 (s, 6H);
¹³C NMR (101 MHz, DMSO-d₆) δ [ppm] 153.23, 149.42, 148.84, 148.02, 147.00, 146.52, 143.73, 133.32, 133.23, 129.93, 129.30, 128.23, 128.05, 126.84, 126.68, 126.47, 125.88, 122.90, 118.47, 117.22, 111.26, 53.40, 25.58.

### 2-Methyl-2-{4-[2-oxo-5-(4-chinolin-3-yl-pyridin-2-yl)-2,3-dihydro-imidazol-1-yl]-phenyl}-propionitril ("A17")

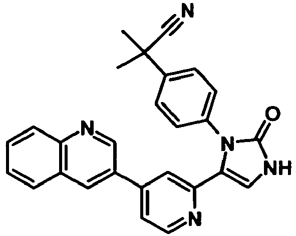

Analytische Daten "A17": DC: R_{f} = 0.26 (Si60, CH₂Cl₂/7% MeOH/1% NH₃); Smp.: 271 °C; LC-MS: t_{R} = 1.949 min (UV = 220 nm), t_{R} = 1.952 min. (TIC, mit [MH]⁺ = 432); ¹H NMR (400 MHz, DMSO-d₆) δ [ppm] 10.85 (s, 1 H), 9.06 (d, *J* = 2.3, 1 H), 8.69 (d, *J* = 2.2, 1 H), 8.47 (d, *J* = 5.2, 1 H), 8.07 (t, *J* = 9.2, 2H), 7.84 (ddd, *J* = 8.4, 6.9, 1.5, 1 H), 7.72 - 7.67 (m, 2H), 7.64 (s, 1 H), 7.59 - 7.56 (m, 2H), 7.38 (s, 1 H), 7.32 - 7.28 (m, 2H), 1.72 (s, 6H);
¹³C NMR (101 MHz, DMSO-d₆) δ [ppm] 153.12, 149.30, 148.87, 148.16, 147.03, 143.92, 139.26, 135.43, 133.36, 129.97, 129.31, 128.24, 128.08, 127.03, 126.87, 126.73, 124.90, 124.05, 122.64, 118.53, 117.49, 111.75, 35.96, 27.80.

### 2-Methyl-2-(4-{3-methyl-2-oxo-5-[4-(3-trifluormethyl-1H-pyrrolo[2,3-b]pyridin-5-yl)-pyridin-2-yl]-2,3-dihydro-imidazol-1-yl}-phenyl)-propionitril ("A18")

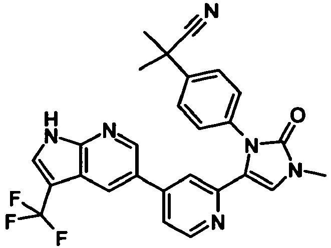

Analytische Daten "A18": DC: R_{f} = 0.26 (Si60, CH₂Cl₂/5% MeOH/1% NH₃); Smp.: 159°C; LC-MS: t_{R} = 2.048 min (UV = 220 nm), t_{R} = 2.047 min. (TIC, mit [MH]⁺= 503); ¹H NMR (400 MHz, DMSO-d₆) δ [ppm] 12.74 (s, 1H), 8.43 (d, *J* = 5.2, 1 H), 8.40 (d, *J =* 2.1, 1 H), 8.25 (s, 2H), 7.59 (dd, *J =* 5.1, 3.5, 3H), 7.48 (s, 1 H), 7.36 - 7.30 (m, 3H), 3.33 (s, 3H), 1.72 (s, 6H);
¹³C NMR (101 MHz, DMSO-d₆) δ [ppm] 152.33, 149.30, 148.29, 147.85, 144.91, 142.66, 139.63, 135.42, 128.24, 127.09, 126.26, 125.01, 124.78, 124.39, 123.91, 122.14, 121.24, 118.76, 117.42, 115.37, 114.83, 103.19, 102.83, 36.01, 29.75, 27.80.

### 1-Methyl-3-(4-piperazin-1-yl-3-trifluormethyl-phenyl)-4-(4-chinolin-3-yl-pyridin-2-yl)-1,3-dihydro-imidazol-2-on ("A19")

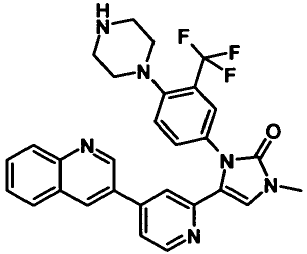

Analytische Daten "A19": DC: R_{f} = 0.12 (Si60, CH₂Cl₂/6% MeOH/1% NH₃); LC-MS: t_{R} = 1.526 min (UV = 220 nm), t_{R} = 1.534 min. (TIC, mit [MH]⁺ = 531); ¹H NMR (400 MHz, DMSO-d₆) δ [ppm] 9.12 (d, *J =* 2.3, 1H), 8.64 (d, *J* = 2.1, 1 H), 8.46 (d, *J =* 5.2, 1 H), 8.08 (d, *J =* 8.4, 1 H), 8.02 (d, *J =* 7.6, 1 H), 7.84 (ddd, *J* = 8.4, 7.0, 1.4, 1 H), 7.73 - 7.67 (m, 2H), 7.62 (s, 1 H), 7.58 - 7.44 (m, 4H), 3.33 (s, breit, 4H + Wasser), 2.80 (d, *J* = 3.7, 8H);
¹³C NMR (101 MHz, DMSO-d₆) δ [ppm] 152.28, 150.82, 149.26, 148.37, 148.10, 147.06, 143.98, 133.26, 132.31, 131.62, 129.97, 129.22, 128.25, 127.98, 126.86, 126.71, 125.28, 124.07, 120.93, 118.60, 117.41, 115.64, 53.89, 45.34, 29.82.

### 4-(6-Methoxy-[3,4']bipyridinyl-2'-yl)-1-methyl-3-(4-piperazin-1-yl-3-trifluormethyl-phenyl)-1,3-dihydro-imidazol-2-on ("A20")

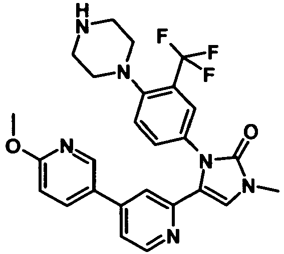

Analytische Daten "A20": DC: R_{f} = 0.12 (Si60, CH₂Cl₂/6% MeOH/1% NH₃); LC-MS: t_{R} = 1.512 min (UV = 220 nm), t_{R} = 1.569 min. (TIC, mit [MH]⁺ = 511); ¹H NMR (400 MHz, DMSO-d₆) δ [ppm] 8.42 (dd, *J* = 5.2, 0.5, 1H), 8.38 - 8.36 (m, 1 H), 8.00 (dd, *J =* 8.7, 2.6, 1 H), 7.59 (dd, *J =* 9.6, 5.5, 2H), 7.54 (s, 1 H), 7.53 - 7.49 (m, 2H), 7.36 (d, *J* = 0.9, 1 H), 6.95 (dd, *J* = 8.7, 0.4, 1 H), 3.94 (s, 3H), 3.36 (s, 3H), 3.32 (s, breit, 1 H + Wasser), 2.88 (s, 8H);
¹³C NMR (101 MHz, DMSO-d₆) δ [ppm] 163.65, 152.25, 150.87, 149.17, 148.10, 144.65, 143.73, 136.72, 132.38, 131.72, 125.57, 125.36, 124.25, 120.98, 117.76, 116.39, 115.40, 110.26, 53.79, 52.86, 45.33, 29.79.

### 2-{4-[3-Methyl-2-oxo-5-(4-chinolin-3-yl-pyridin-2-yl)-2,3-dihydro-imidazol-1-yl]-phenyl}-isobutyramid ("A21")

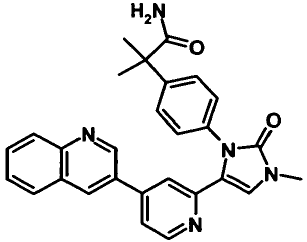

Analytische Daten "A21": DC: R_{f} = 0.25 (Si60, CH₂Cl₂/2% [MeOH + 10% NH₃]); Smp.: 259°C; LC-MS: t_{R} = 1.729 min (UV = 220 nm), t_{R} = 1.724 min. (TIC, mit [MH]⁺ = 464); ¹H NMR (400 MHz, DMSO-d₆) δ [ppm] 8.89 (d, *J* = 2.3, 1 H), 8.60 (d, *J =* 2.1, 1H), 8.51 (d, *J* = 5.2, 1 H), 8.06 (d, *J* = 8.5, 2H), 7.83 (ddd, J = 8.3, 6.9, 1.4, 1 H), 7.71 - 7.65 (m, 2H), 7.46 - 7.41 (m, 3H), 7.38 (d, *J* = 0.9, 1 H), 7.24 - 7.20 (m, 2H), 7.03 (s, 1 H), 6.95 (s, 1 H), 3.32 (s, 3H), 1.47 (s, 6H);
¹³C NMR (101 MHz, DMSO-d₆) δ [ppm] 177.05, 152.42, 149.51, 148.54, 148.00, 146.98, 144.84, 143.82, 133.93, 133.31, 129.98, 129.27, 128.18, 128.13, 126.87, 126.70, 126.53, 125.65, 121.32, 118.58, 117.25, 115.22, 54.36, 45.33, 29.77, 26.28.

### 2-Methyl-2-{4-[3-methyl-2-oxo-5-(4-chinolin-3-yl-pyridin-2-yl)-2,3-dihydro-imidazol-1-yl]-phenyl}-propionsäure ("A22")

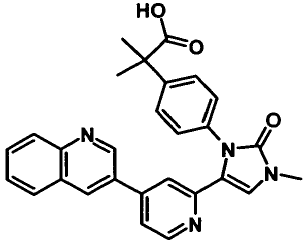

Analytische Daten "A22": DC: R_{f} = 0.31 (Si60, CH₂Cl₂/5% MeOH); LC-MS: t_{R} = 1.889 min (UV = 220 nm), t_{R} = 1.889 min. (TIC, mit [MH]⁺ = 465); ¹H NMR (300 MHz, DMSO-d₆) δ [ppm] 12.42 (s, 1 H), 8.99 (d, *J* = 2.3, 1 H), 8.60 (d, *J* = 2.1, 1 H), 8.50 (d, *J* = 5.1, 1 H), 8.05 (dd, *J* = 7.3, 5.3, 2H), 7.87 - 7.79 (m, 1 H), 7.73 - 7.65 (m, 2H), 7.50 - 7.39 (m, 4H), 7.23 (d, *J* = 8.6, 2H), 3.32 (s, 3H), 1.50 (s, 6H);
¹³C NMR (75 MHz, DMSO-d₆) δ [ppm] 177.33, 152.92, 149.96, 149.08, 148.54, 147.52, 144.05, 134.64, 133.77, 130.44, 129.76, 128.70, 128.59, 127.32, 127.03, 126.02, 121.77, 119.09, 117.82, 115.83, 45.68, 30.26, 26.49.

### 2-Methyl-2-(4-{3-methyl-2-oxo-5-[4-(1H-pyrrolo[2,3-b]pyridin-4-yl)-pyridin-2-yl]-2,3-dihydro-imidazol-1-yl}-phenyl)-propionitril ("A23")

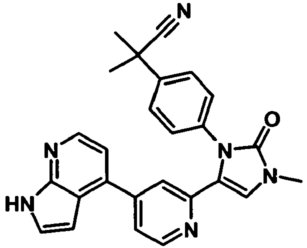

Analytische Daten "A23": DC: R_{f} = 0.27 (Si60, CH₂Cl₂/5% MeOH/1% NH₃); LC-MS: t_{R} = 1.727 min (UV = 220 nm), t_{R} = 1.725 min. (TIC, mit [MH]⁺ = 435); ¹H NMR (300 MHz, DMSO-d₆) δ [ppm] 11.91 (s, 1H), 8.56 (dd, *J=* 5.1, 0.6, 1 H), 8.25 (d, *J* = 4.9, 1 H), 7.65 - 7.59 (m, 2H), 7.55 - 7.50 (m, 2H), 7.44 (s, 1H), 7.38 - 7.31 (m, 2H), 7.19 (d, *J* = 0.7, 1 H), 6.96 (d, *J* = 4.9, 1 H), 6.18 (dd, *J* = 3.5, 1.8, 1 H), 3.32 (s, 3H), 1.70 (s, 6H);
¹³C NMR (75 MHz, DMSO-d₆) δ [ppm] 152.88, 150.10, 149.09, 148.72, 145.92, 142.71, 140.28, 136.82, 135.80, 127.62, 127.26, 125.87, 124.54, 121.66, 120.39, 118.93, 116.65, 116.05, 113.82, 98.37, 36.42, 30.26, 28.20.

### 2-(4-{3-Ethyl-2-oxo-5-[4-(1H-pyrrolo[2,3-b]pyridin-5-yl)-pyridin-2-yl]-2,3-dihydro-imidazol-1-yl}-phenyl)-2-methyl-propionitril ("A24")

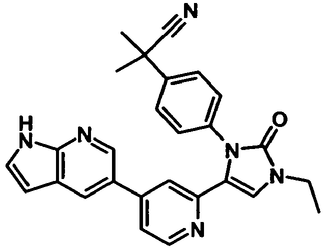

Analytische Daten "A24": DC: R_{f} = 0.26 (Si60, CH₂Cl₂/5% MeOH/1% NH₃); LC-MS: t_{R} = 1.843 min (UV = 220 nm), t_{R} = 1.840 min. (TIC, mit [MH]⁺ = 449); ¹H NMR (300 MHz, DMSO) δ 11.72 (s, 1 H), 8.28 (d, *J =* 5.2, 1 H), 8.12 (d, *J =* 2.2, 1 H), 7.92 (d, *J* = 2.1, 1H), 7.51 - 7.45 (m, 2H), 7.40 (dd, J = 6.5, 5.0, 3H), 7.23 - 7.17 (m, 2H), 7.07 (d, *J =* 0.9, 1 H), 6.36 (dd, *J* = 3.4, 1.7, 1 H), 3.59 (q, *J* = 7.2, 2H), 1.60 (s, 6H), 1.18 (t, *J* = 7.2, 3H); ¹³C NMR (75 MHz, DMSO) δ 152.29, 149.76, 148.75, 148.60, 146.23, 141.11, 140.29, 135.85, 127.79, 127.56, 126.07, 125.62, 124.79, 124.48, 122.02, 119.55, 118.83, 117.41, 114.17, 100.34, 38.05, 36.56, 28.34, 14.41.

### 1-Methyl-3-(4-piperazin-1-yl-3-trifluormethyl-phenyl)-4-[4-(1 H-pyrrolo[2,3-b]pyridin-5-yl)-pyridin-2-yl]-1,3-dihydro-imidazol-2-on ("A25")

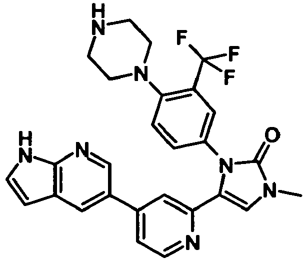

Analytische Daten "A25": DC: R_{f} = 0.20 (Si60, CH₂Cl₂/10% MeOH/1% NH₃); Smp.: 163°C; LC-MS: t_{R} = 1.443 min (UV = 220 nm), t_{R} = 1.432 min. (TIC, mit [MH]⁺ = 520); ¹H NMR (300 MHz, DMSO-d₆) δ [ppm] 11.86 (s, 1H), 8.41 - 8.36 (m, 2H), 8.07 (d, *J* = 2.2, 1 H), 7.55 (ddd, *J* = 5.8, 5.2, 2.1, 4H), 7.50 - 7.43 (m, 2H), 7.31 (d, *J* = 0.9, 1 H), 6.46 (d, *J* = 3.4, 1 H), 3.20 (s, breit, 1 H + Wasser), 3.18 (s, 3H), 2.82 (s, 8H);
¹³C NMR (75 MHz, DMSO-d₆) δ [ppm] 152.78, 151.47, 149.63, 148.80, 148.47, 146.31, 141.15, 132.86, 132.25, 127.56, 126.00, 125.73, 125.35, 124.76, 121.80, 121.67, 119.54, 118.81, 117.48, 115.76, 100.21, 54.39, 45.86, 30.26.

### 2-Methyl-2-{4-[3-methyl-2-oxo-5-(4-thiophen-3-yl-pyridin-2-yl)-2,3-dihydro-imidazol-1-yl]-phenyl}-propionitril ("A26")

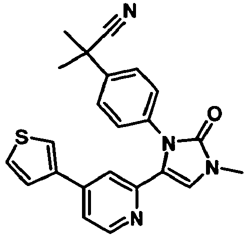

Analytische Daten "A26": DC: R_{f} = 0.24 (Si60, CH₂Cl₂/5% MeOH/1% NH₃); Smp.: 209°C; LC-MS: t_{R} = 2.018 min (UV = 220 nm), t_{R} = 2.0039 min. (TIC, mit [MH]⁺ = 401); ¹H NMR (300 MHz, DMSO-d₆) δ [ppm] 8.41 (dd, *J* = 5.2, 0.6, 1 H), 7.91 (dd, *J* = 2.9, 1.3, 1 H), 7.70 (dd, *J* = 5.0, 2.9, 1 H), 7.66 - 7.61 (m, 2H), 7.53 - 7.49 (m, 1 H), 7.45 (s, 1 H), 7.38 - 7.29 (m, 3H), 7.21 (d, *J* = 0.9, 1 H), 3.36 (s, 3H), 1.77 (s, 6H);
¹³C NMR (75 MHz, DMSO-d₆) δ [ppm] 152.78, 149.84, 148.74, 141.82, 140.23, 138.54, 135.94, 127.86, 127.81, 127.37, 125.68, 125.37, 124.47, 124.05, 121.71, 118.12, 116.64, 115.53, 36.43, 30.24, 28.21.

### 2-Methyl-2-(4-{3-methyl-2-oxo-5-[4-(2-trifluormethyl-1H-pyrrolo[2,3-b]pyridin-5-yl)-pyridin-2-yl]-2,3-dihydro-imidazol-1-yl}-phenyl)-propionitril ("A27")

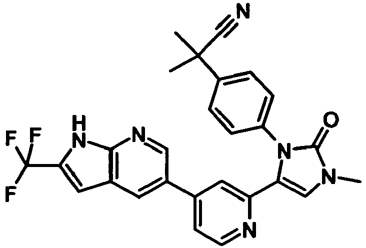

Analytische Daten "A27": DC: R_{f} = 0.23 (Si60, CH₂Cl₂/5% MeOH/1% NH₃); LC-MS: t_{R} = 2.090 min (UV = 220 nm), t_{R} = 2.087 min. (TIC, mit [MH]⁺ = 503); ¹H NMR (300 MHz, DMSO-d₆) δ [ppm] 13.15 (s, 1H), 8.47 - 8.43 (m, 2H), 8.28 (d, *J* = 2.1, 1 H), 7.65 - 7.60 (m, 2H), 7.54 (dd, *J* = 5.2, 1.6, 1 H), 7.46 (s, 1H), 7.33 (d, *J* = 8.5, 2H), 7.24 (s, 1 H), 7.09 (d, *J* = 1.0, 1 H), 3.33 (s, 3H), 1.74 (s, 6H);
¹³C NMR (75 MHz, DMSO-d₆) δ [ppm] 152.80, 149.84, 148.90, 148.70, 145.40, 144.75, 140.30, 135.89, 128.59, 127.76, 126.86, 126.41, 125.61, 124.47, 122.73, 121.73, 119.18, 118.93, 117.99, 117.53, 115.79, 102.31, 36.53, 30.25, 28.30.

### 2-Methyl-2-(4-{2-oxo-5-[4-(1H-pyrrolo[2,3-b]pyridin-5-yl)-pyridin-2-yl]-2,3-dihydro-imidazol-1-yl}-phenyl)-propionitril ("A28")

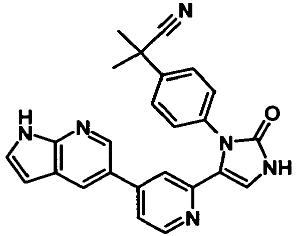

Analytische Daten "A28": DC: R_{f} = 0.23 (Si60, CH₂Cl₂/8% MeOH/1% NH₃); LC-MS: t_{R} = 1.604 min (UV = 220 nm), t_{R} = 1.599 min. (TIC, mit [MH]⁺ = 421); ¹H NMR (300 MHz, DMSO-d₆) δ [ppm] 11.85 (s, 1H), 10.78 (s, 1 H), 8.41 (d, *J* = 5.2, 1 H), 8.29 (d, J = 2.1, 1 H), 8.07 (d, *J* = 2.1, 1 H), 7.61 (d, *J* = 8.6, 2H), 7.56 - 7.48 (m, 2H), 7.32 (d, *J* = 8.8, 3H), 7.26 (s, 1 H), 6.50 (dd, J = 3.3, 1.5, 1H), 1.74 (s, 6H);
¹³C NMR (75 MHz, DMSO-d₆) δ [ppm] 153.60, 149.66, 148.89, 148.76, 146.22, 141.16, 140.01, 135.90, 127.73, 127.52, 126.08, 125.49, 124.84, 124.49, 123.37, 119.54, 118.76, 117.47, 111.77, 100.33, 36.53, 28.36.

### 5-(2-{3-[4-(Cyan-dimethyl-methyl)-phenyl]-1-methyl-2-oxo-2,3-dihydro-1H-imidazol-4-yl}-pyridin-4-yl)-1H-pyrrolo[2,3-b]pyridin-3-carbonsäuremethylester ("A29")

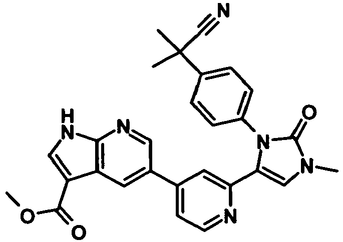

Analytische Daten "A29": DC: R_{f} = 0.15 (Si60, CH₂Cl₂/5% MeOH/1% NH₃); LC-MS: t_{R} = 1.811 min (UV = 220 nm), t_{R} = 1.814 min. (TIC, mit [MH]⁺ = 493); ¹H NMR (300 MHz, DMSO-d₆) δ [ppm] 12.71 (s, 1 H), 8.48 - 8.43 (m, 2H), 8.30 (dd, J = 7.2, 2.6, 2H), 7.63 - 7.57 (m, 2H), 7.53 (dd, J = 5.2, 1.7, 1 H), 7.47 (s, 1H), 7.36 - 7.31 (m, 2H), 7.24 (d, J = 0.9, 1H), 3.87 (s, 3H), 3.32 (s, 3H), 1.71 (s, 6H);
¹³C NMR (75 MHz, DMSO-d₆) δ [ppm] 163.95, 152.82, 149.90, 148.91, 148.73, 145.70, 142.56, 140.28, 135.88, 134.12, 127.71, 127.08, 126.61, 125.61, 124.40, 121.74, 119.08, 117.94, 117.62, 115.81, 105.70, 50.99, 36.53, 30.25, 28.27.

### 3-[4-(2-Amino-1,1-dimethyl-ethyl)-phenyl]-1-methyl-4-(4-chinolin-3-yl-pyridin-2-yl)-1,3-dihydro-imidazol-2-on ("A30")

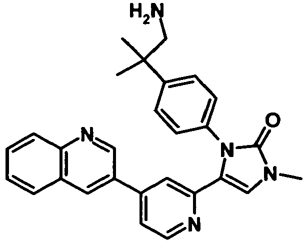

Analytische Daten "A30": DC: R_{f} = 0.21 (Si60, CH₂Cl₂/10% MeOH); Smp.: 132°C; LC-MS: t_{R} = 1.522 min (UV = 220 nm), t_{R} = 1.515 min. (TIC, mit [MH]⁺ = 450); ¹H NMR (300 MHz, DMSO-d₆) δ 8.84 (d, J = 2.3 Hz, 1H), 8.62 (d, J = 2.2 Hz, 1 H), 8.52 (d, J = 5.1 Hz, 1 H), 8.28 (s, 2H), 8.10 - 7.99 (m, 2H), 7.86 - 7.78 (m, 1H), 7.73 - 7.63 (m, 2H), 7.53 - 7.42 (m, 3H), 7.34 (d, J = 0.9 Hz, 1 H), 7.26 (d, J = 8.6 Hz, 2H), 3.33 (s, 3H), 2.98 (s, 2H), 1.34 (s, 6H).

### (4-{2-Oxo-5-[4-(1H-pyrrolo[2,3-b]pyridin-5-yl)-pyridin-2-yl]-2,3-dihydro-imidazol-1-yl}-phenyl)-acetonitril ("A31")

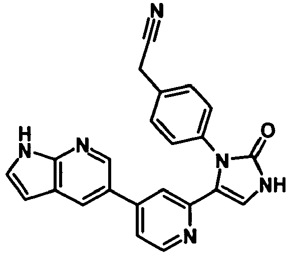

Analytische Daten "A31": DC: R_{f} = 0.31 (Si60, CH₂Cl₂/10% MeOH/1% NH₃); Smp.: >410°C (Zersetzung); LC-MS: t_{R} = 1.510 min (UV = 220 nm), t_{R} = 1.504 min. (TIC, mit [MH]⁺ = 393); ¹H NMR (500 MHz, DMSO-d₆) δ 11.83 (s, 1H), 10.74 (s, 1H), 8.50 (d, J = 2.1 Hz, 1H), 8.39 - 8.33 (m, 1 H), 8.14 (d, J = 2.0 Hz, 1 H), 7.58 - 7.48 (m, 3H), 7.39 (d, J = 8.4 Hz, 2H), 7.32 (s, 1 H), 7.25 (d, J = 8.4 Hz, 2H), 6.54 (dd, J = 3.3, 1.7 Hz, 1 H), 4.09 (s, 2H).

### (4-{3-Methyl-2-oxo-5-[4-(1H-pyrrolo[2,3-b]pyridin-5-yl)-pyridin-2-yl]-2,3-dihydro-imidazol-1-yl}-phenyl)-acetonitril ("A32")

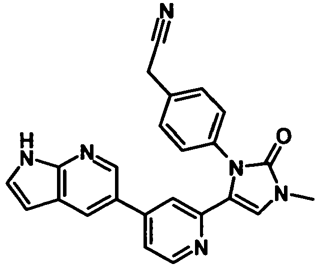

Analytische Daten "A32": DC: R_{f} = 0.18 (Si60, CH₂Cl₂/5% MeOH/1% NH₃); Smp.: 252°C; LC-MS: t_{R} = 1.600 min (UV = 220 nm), t_{R} = 1.615 min. (TIC, mit [MH]⁺ = 407); ¹H NMR (300 MHz, DMSO-d₆) δ 11.84 (s, 1H), 8.48 (d, J = 2.2 Hz, 1 H), 8.40 - 8.32 (m, 1 H), 8.09 (d, J = 2.2 Hz, 1 H), 7.54 (ddd, J = 7.0, 4.2, 1.8 Hz, 2H), 7.47 - 7.38 (m, 4H), 7.32 - 7.21 (m, 2H), 6.54 (d, J = 2.9 Hz, 1 H), 4.11 (s, 2H), 3.32 (s, 3H); ¹³C NMR (75 MHz, DMSO) δ 152.85, 149.59, 148.83, 148.78, 146.40, 141.27, 136.00, 129.83, 128.35, 127.67, 127.49, 126.16, 124.80, 121.85, 119.61, 119.08, 118.81, 117.61, 115.66, 100.47, 30.24, 22.00.

### 2-Methyl-2-(4-{3-methyl-2-oxo-5-[4-(1H-pyrrolo[2,3-b]pyridin-5-yl)-pyridin-2-yl]-2,3-dihydro-imidazol-1-yl}-phenyl)-propionsäure ("A33")

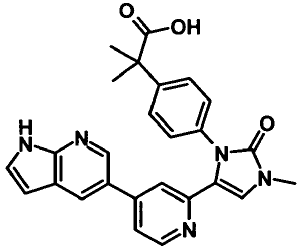

Analytische Daten "A33": LC-MS: t_{R} = 1.654 min (UV = 220 nm), t_{R} = 1.649 min. (TIC, mit [MH]⁺ = 454); ¹H NMR (500 MHz, DMSO-d₆) δ 12.46 (s, 1H), 11.84 (bs, 1H), 8.45 (d, J = 5.2 Hz, 1H), 8.25 (d, J = 2.1 Hz, 1H), 7.98 (d, J = 2.0 Hz, 1H), 7.58 - 7.51 (m, 2H), 7.46 (d, J = 8.5 Hz, 2H), 7.41 (s, 1H), 7.25 (d, J = 8.5 Hz, 2H), 7.12 (s, 1H), 6.50 (dd, J = 3.3, 1.7 Hz, 1H), 3.32 (s, 3H), 1.55 (s, 6H).

### 2-(4-{3-Methyl-2-oxo-5-[4-(1H-pyrrolo[2,3-b]pyridin-5-yl)-pyridin-2-yl]-2,3-dihydro-imidazol-1-yl}-phenyl)-isobutyramid ("A34")

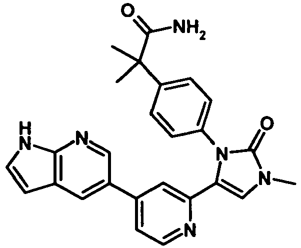

Analytische Daten "A34": DC: R_{f} = 0.46 (Si60, CH₂Cl₂/10% MeOH); Smp.: 299°C; LC-MS: t_{R} = 1.549 min (UV = 220 nm), t_{R} = 1.544 min. (TIC, mit [MH]⁺ = 453); ¹H NMR (400 MHz, DMSO-d₆) δ 11.84 (s, 1 H), 8.44 (d, J = 5.2 Hz, 1 H), 8.17 (d, J = 2.2 Hz, 1 H), 8.00 (d, J = 2.1 Hz, 1 H), 7.56 - 7.49 (m, 2H), 7.47 - 7.43 (m, 2H), 7.40 (s, 1 H), 7.26 - 7.21 (m, 2H), 7.07 (d, J = 0.9 Hz, 1 H), 7.02 (s, 1 H), 6.94 (s, 1 H), 6.50 (dd, J = 3.4, 1.8 Hz, 1 H), 3.31 (s, 3H), 1.51 (s, 6H); ¹³C NMR (101 MHz, DMSO) δ 176.98, 152.39, 149.33, 148.23, 148.12, 145.67, 145.01, 140.58, 133.92, 126.98, 126.64, 125.74, 125.56, 124.32, 121.49, 119.08, 118.29, 116.76, 114.90, 99.92, 45.38, 29.74, 26.37.

### 3-[4-(2-Amino-1,1-dimethyl-ethyl)-phenyl]-1-methyl-4-[4-(1H-pyrrolo[2,3-b]pyridin-5-yl)-pyridin-2-yl]-1,3-dihydro-imidazol-2-on ("A35")

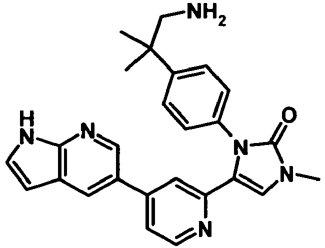

Analytische Daten "A35": DC: R_{f} = 0.10 (Si60, CH₂Cl₂/10% MeOH); Smp.: 91 °C; LC-MS: t_{R} = 1.349 min (UV = 220 nm), t_{R} = 1.351 min. (TIC, mit [MH]⁺ = 439); ¹H NMR (500 MHz, DMSO-d₆) δ 11.86 (s, 1H), 8.44 (d, J = 5.1 Hz, 1 H), 8.26 (s, 1 H), 8.14 (d, J = 2.2 Hz, 1 H), 7.99 (d, J = 2.2 Hz, 1 H), 7.58 - 7.46 (m, 4H), 7.39 (d, J = 3.9 Hz, 1 H), 7.25 (d, J = 8.6 Hz, 2H), 7.03 (d, J = 0.9 Hz, 1 H), 6.46 (s, 1H), 3.62 (bs, 2H), 3.31 (s, 3H), 2.90 (s, 2H), 1.34 (s, 6H).

### 2-Methyl-2-{4-[3-methyl-2-oxo-5-(4-quinolin-3-yl-pyridin-2-yl)-2,3-dihydro-imidazol-1-yl]-phenyl}-propionsäureethylester ("A36")

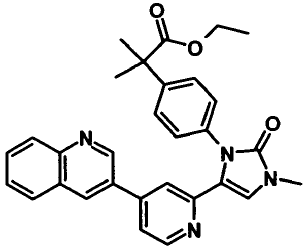

Analytische Daten "A36": DC: R_{f} = 0.15 (Si60, EtOAc/7% MeOH); Smp.: 158°C; LC-MS: t_{R} = 2.235 min (UV = 220 nm), t_{R} = 2.285 min. (TIC, mit [MH]⁺ = 493); ¹H NMR (400 MHz, DMSO-d₆) δ 8.95 (d, J = 2.3 Hz, 1 H), 8.59 (d, J = 2.1 Hz, 1 H), 8.50 (dd, J = 5.2, 0.6 Hz, 1 H), 8.05 (t, J = 9.2 Hz, 2H), 7.83 (s, 1 H), 7.71 - 7.66 (m, 2H), 7.47 (s, 1 H), 7.43 - 7.35 (m, 3H), 7.28 - 7.21 (m, 2H), 3.98 (q, J = 7.1 Hz, 2H), 3.32 (s, 3H), 1.52 (s, 6H), 1.00 (t, J = 7.1 Hz, 3H); ¹³C NMR (101 MHz, DMSO) δ 175.05, 152.41, 149.48, 148.53, 148.00, 147.04, 143.86, 143.06, 134.30, 133.25, 129.96, 129.27, 128.22, 128.06, 126.84, 126.72, 125.48, 121.28, 118.62, 117.25, 115.34, 59.85, 45.42, 29.78, 25.84, 13.26.

### 6-Amino-2'-{3-[4-(cyano-dimethyl-methyl)-phenyl]-1-methyl-2-oxo-2,3-dihydro-1H-imidazol-4-yl}-[3,4']bipyridinyl-5-carbonitril ("A37")

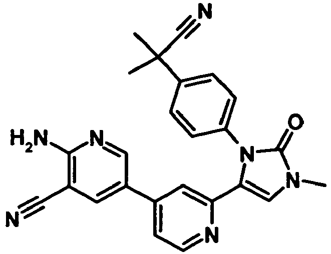

Analytische Daten "A37": DC: R_{f} = 0.10 (Si60, CH₂Cl₂/5% MeOH/10% NH₃); LC-MS: t_{R} = 1.627 min (UV = 220 nm), t_{R} = 1.625 min. (TIC, mit [MH]⁺ = 436); ¹H NMR (500 MHz, DMSO-d₆) δ 8.39 (d, J = 2.5 Hz, 1H), 8.31 (s, 1H), 8.18 (d, J = 2.5 Hz, 1 H), 7.55 (d, J = 8.6 Hz, 2H), 7.43 (d, J = 13.8 Hz, 2H), 7.33 (s, 1H), 7.26 (dd, J = 5.8, 2.7 Hz, 4H), 3.31 (s, 3H), 1.71 (s, 6H).

### 2-(4-{5-[4-(3H-Imidazo[4,5-b]pyridin-6-yl)-pyridin-2-yl]-3-methyl-2-oxo-2,3-dihydro-imidazol-1-yl}-phenyl)-2-methyl-propionitril ("A38")

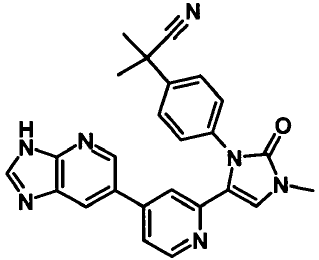

Analytische Daten "A38": DC: R_{f} = 0.17 (Si60, CH₂Cl₂/8% MeOH/1% NH₃); LC-MS: t_{R} = 1.494 min (UV = 220 nm), t_{R} = 1.491 min. (TIC, mit [MH]⁺ = 436); ¹H NMR (400 MHz, DMSO-d₆) δ 13.14 (s, 1 H), 8.52 (s, 1 H), 8.44 (d, J = 5.2 Hz, 1 H), 8.38 (d, J = 2.0 Hz, 1 H), 8.18 (d, J = 2.0 Hz, 1 H), 7.64 - 7.55 (m, 3H), 7.48 (s, 1 H), 7.33 (d, J = 8.5 Hz, 2H), 7.27 (s, 1 H), 3.32 (s, 3H), 1.75 (s, 6H).

### 2-{4-[5-(6-Hydroxy-[3,4']bipyridinyl-2'-yl)-3-methyl-2-oxo-2,3-dihydro-imidazol-1-yl]-phenyl}-2-methyl-propionitril ("A39")

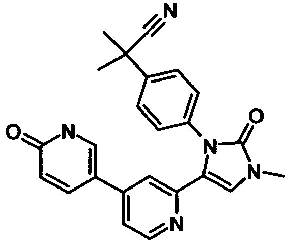

Analytische Daten "A39": LC-MS: t_{R} = 1.427 min (UV = 220 nm), t_{R} = 1.418 min. (TIC, mit [MH]⁺ = 412); ¹H NMR (500 MHz, DMSO-d₆) δ 12.04 (s, 1H), 8.29 (d, J = 5.3 Hz, 1 H), 7.82 (s, 1 H), 7.58 - 7.52 (m, 3H), 7.40 (s, 1 H), 7.36 (dd, J = 5.3, 1.8 Hz, 1 H), 7.28 - 7.24 (m, 2H), 7.19 (d, J = 1.1 Hz, 1 H), 6.39 (d, J = 9.6 Hz, 1H), 3.30 (s, 3H), 1.71 (s, 6H).

### 2-{4-[5-(6-Amino-5-trifluoromethyl-[3,4']bipyridinyl-2'-yl)-3-methyl-2-oxo-2,3-dihydro-imidazol-1-yl]-phenyl}-2-methyl-propionitril ("A40")

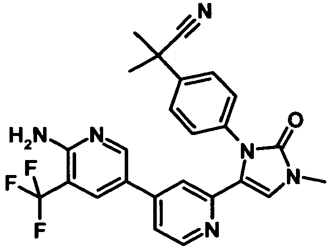

Analytische Daten "A40": DC: R_{f} = 0.33 (Si60, CH₂Cl₂/6% MeOH/1% NH₃); Smp.: 195°C; LC-MS: t_{R} = 1.900 min (UV = 220 nm), t_{R} = 1.908 min. (TIC, mit [MH]⁺ = 479); ¹H NMR (400 MHz, DMSO-d₆) δ 8.39 (d, J = 2.1 Hz, 1H), 8.34 (dd, J = 5.3, 0.5 Hz, 1 H), 7.91 (d, J = 2.1 Hz, 1 H), 7.58 - 7.53 (m, 2H), 7.48 (dd, J = 5.3, 1.8 Hz, 1 H), 7.42 (s, 1 H), 7.27 (dd, J = 6.6, 2.0 Hz, 3H), 6.82 (s, 2H), 3.30 (s, 3H), 1.70 (s, 6H).

### 2-Methyl-2-(4-{3-methyl-2-oxo-5-[4-(1H-pyrazolo[3,4-b]pyridin-5-yl)-pyridin-2-yl]-2,3-dihydro-imidazol-1-yl}-phenyl)-propionitril ("A41")

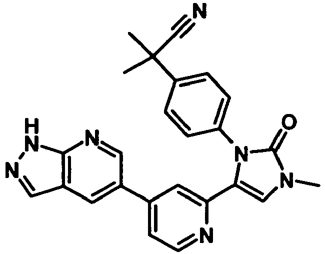

Analytische Daten "A41": DC: R_{f} = 0.23 (Si60, CH₂Cl₂/6% MeOH/1% NH₃); Smp.: 294°C; LC-MS: t_{R} = 1.655 min (UV = 220 nm), t_{R} = 1.649 min. (TIC, mit [MH]⁺ = 436); ¹H NMR (400 MHz, DMSO-d₆) δ 13.84 (d, J = 14.8 Hz, 1H), 8.55 (d, J = 2.2 Hz, 1 H), 8.45 (d, J = 5.2 Hz, 1 H), 8.40 (s, 1 H), 8.21 (s, 1 H), 7.59 (ddd, J = 6.9, 5.9, 1.8 Hz, 3H), 7.48 (s, 1 H), 7.34 - 7.30 (m, 2H), 7.28 (s, 1 H), 3.32 (s, 3H), 1.74 (s, 6H).

### 2-Methyl-2-{4-[3-methyl-5-(6-methylamino-[3,4']bipyridinyl-2'-yl)-2-oxo-2,3-dihydro-imidazol-1-yl]-phenyl}-propionitril ("A42")

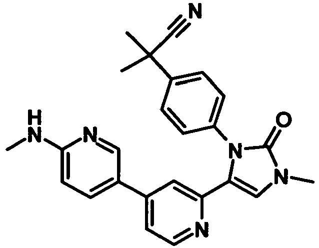

Analytische Daten "A42": DC: R_{f} = 0.24 (Si60, CH₂Cl₂/6% MeOH/1% NH₃); Smp.: 191°C; LC-MS: t_{R} = 1.362 min (UV = 220 nm), t_{R} = 1.359 min. (TIC, mit [MH]⁺ = 425); ¹H NMR (300 MHz, DMSO-d₆) δ 8.34 (d, J = 5.3 Hz, 1H), 8.05 (d, J = 2.4 Hz, 1H), 7.61 (d, J = 8.5 Hz, 2H), 7.45 (dd, J = 8.8, 2.4 Hz, 1H), 7.38 - 7.28 (m, 4H), 6.96 (s, 1 H), 6.49 (d, J = 8.8 Hz, 1 H), 3.31 (s, 3H), 2.80 (s, 3H), 1.75 (s, 6H); ¹³C NMR (75 MHz, DMSO) δ 159.50, 152.79, 149.69, 148.29, 145.74, 144.97, 140.51, 135.84, 134.39, 127.91, 125.73, 124.46, 121.87, 119.65, 117.06, 115.55, 115.36, 107.94, 36.53, 30.23, 28.28, 27.75.

### 2-(4-{5-[4-(2,3-Dihydro-1H-pyrrolo[2,3-b]pyridin-5-yl)-pyridin-2-yl]-3-methyl-2-oxo-2,3-dihydro-imidazol-1-yl}-phenyl)-2-methyl-propionitril ("A43")

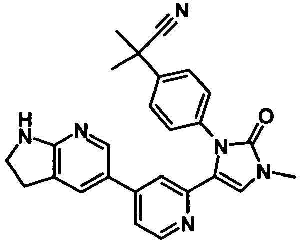

Analytische Daten "A43": DC: R_{f} = 0.12 (Si60, CH₂Cl₂/5% MeOH/1% NH₃); Smp.: 247°C; LC-MS: t_{R} = 1.388 min (UV = 220 nm), t_{R} = 1.386 min. (TIC, mit [MH]⁺ = 437); ¹H NMR (400 MHz, DMSO-d₆) δ 8.31 (d, *J* = 5.3, 1 H), 7.77 (d, *J* = 2.2, 1H), 7.62 - 7.57 (m, 2H), 7.39 (s, 1 H), 7.31 (ddd, *J* = 8.6, 5.9, 1.9, 4H), 6.98 (d, *J* = 1.0, 1 H), 6.85 (s, 1 H), 3.52 (t, *J* = 8.5, 2H), 3.30 (s, 3H), 2.99 (t, *J* = 8.4, 2H), 1.74 (d, *J* = 6.5, 6H); ¹³C NMR (101 MHz, DMSO) δ 164.97, 152.28, 149.14, 147.92, 145.07, 144.28, 139.79, 135.39, 127.84, 127.25, 125.11, 123.95, 122.12, 121.40, 120.25, 116.77, 115.26, 114.95, 42.97, 36.04, 29.73, 27.85, 26.13.

### 2-Methyl-2-(4-{3-methyl-2-oxo-5-[4-(2-oxo-2,3-dihydro-1H-pyrrolo[2,3-b]pyridin-5-yl)-pyridin-2-yl]-2,3-dihydro-imidazol-1-yl}-phenyl)-propionitril ("A44")

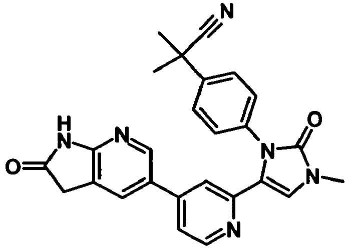

Analytische Daten "A44": DC: R_{f} = 0.28 (Si60, CH₂Cl₂/6% MeOH/1% NH₃); Smp.: 263°C; LC-MS: t_{R} = 1.563 min (UV = 220 nm), t_{R} = 1.574 min. (TIC, mit [MH]⁺ = 451); ¹H NMR (300 MHz, DMSO-d₆) δ 11.24 (s, 1 H), 8.44 (d, *J =* 5.2, 1H), 8.17 (d, *J =* 2.0, 1H), 7.74 (d, *J =* 1.8, 1H), 7.64 (d, *J =* 8.6, 2H), 7.48 (dd, *J* = 4.6, 2.0, 2H), 7.35 (d, *J* = 8.6, 2H), 7.20 (s, 1 H), 3.64 (s, 2H), 3.36 (s, 3H), 1.78 (s, 6H); ¹³C NMR (75 MHz, DMSO) δ 175.68, 159.16, 152.79, 149.81, 148.69, 144.79, 144.60, 140.23, 135.84, 129.64, 127.67, 126.58, 125.58, 124.47, 121.69, 120.95, 118.24, 116.78, 115.79, 36.49, 35.19, 30.25, 28.30.

### 2-Methyl-2-(4-{3-methyl-2-oxo-5-[4-(7-oxo-5,6,7,8-tetrahydro-[1,8]naphthyridin-3-yl)-pyridin-2-yl]-2,3-dihydro-imidazol-1-yl}-phenyl)-propionitril ("A45")

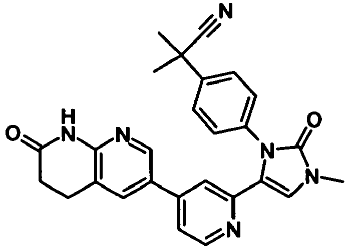

Analytische Daten "A45": DC: R_{f} = 0.21 (Si60, CH₂Cl₂/5% MeOH/1% NH₃); Smp.: 284°C; LC-MS: t_{R} = 1.630 min (UV = 220 nm), t_{R} = 1.620 min. (TIC, mit [MH]⁺ = 465); ¹H NMR (300 MHz, DMSO-d₆) δ 10.62 (s, 1H), 8.39 (d, *J =* 5.1, 1 H), 8.23 (s, 1 H), 7.82 (s, 1 H), 7.57 (d, *J =* 8.4, 2H), 7.47 (d, *J =* 5.1, 1 H), 7.42 (s, 1 H), 7.28 (d, *J* = 7.8, 3H), 3.31 (s, 3H), 2.93 (t, *J* = 7.4, 2H), 2.54 (t, *J* = 7.4, 2H), 1.71 (s, 6H).

### 1-Methyl-3-(4-piperazin-1-yl-3-trifluoromethyl-phenyl)-4-[4-(1H-pyrazolo[3,4-b]pyridin-5-yl)-pyridin-2-yl]-1,3-dihydro-imidazol-2-on ("A46")

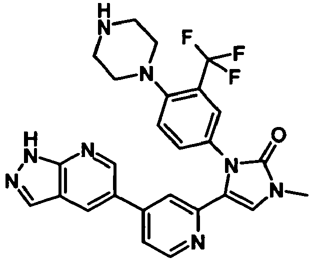

Analytische Daten "A46": DC: R_{f} = 0.10 (Si60, CH₂Cl₂/8% MeOH/1% NH₃); LC-MS: t_{R} = 1.370 min (UV = 220 nm), t_{R} = 1.387 min. (TIC, mit [MH]⁺ = 521); ¹H NMR (300 MHz, DMSO-d₆) δ 8.70 (d, *J* = 2.1, 1H), 8.46 (d, *J* = 2.1, 1H), 8.41 (d, *J* = 5.2, 1 H), 8.20 (s, 1 H), 7.60 - 7.51 (m, 4H), 7.46 (dd, *J* = 8.3, 2.4, 2H), 3.33 (s, 3H), 2.81 (s, 8H); ¹³C NMR (75 MHz, DMSO) δ 152.77, 151.81, 151.32, 149.64, 148.67, 147.55, 145.32, 133.68, 132.87, 132.15, 128.09, 126.04, 125.85, 125.41, 125.21, 124.65, 121.79, 121.53, 119.00, 117.80, 115.97, 114.35, 54.32, 45.82, 30.28.

### 1-Methyl-3-phenyl-4-[4-(1H-pyrrolo[2,3-b]pyridin-5-yl)-pyridin-2-yl]-1,3-dihydroimidazol-2-on ("A47")

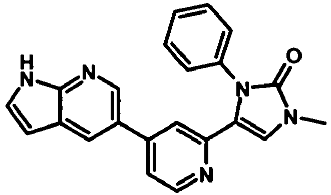

Analytische Daten "A47": DC: R_{f} = 0.38 (Si60, CH₂Cl₂/5% MeOH); Smp.: 264°C; LC-MS: t_{R} = 1.632 min (UV = 220 nm), t_{R} = 1.610 min. (TIC, mit [MH]⁺ = 368); ¹H NMR (400 MHz, DMSO-d₆) δ 11.84 (s, 1 H), 8.40 (d, *J* = 5.2, 1 H), 8.33 (d, *J* = 1.9, 1 H), 8.06 (d, *J* = 2.0, 1 H), 7.58 - 7.54 (m, 1 H), 7.51 (dd, *J* = 5.2, 1.7, 1 H), 7.49 - 7.38 (m, 4H), 7.29 - 7.23 (m, 3H), 6.51 (dd, *J* = 3.4, 1.8, 1H), 3.32 (s, 3H); ¹³C NMR (101 MHz, DMSO) δ 152.38, 149.21, 148.23, 145.70, 140.61, 136.04, 128.29, 127.02, 126.97, 126.59, 125.67, 124.28, 121.45, 119.08, 118.21, 116.91, 114.97, 99.89, 29.74.

### 4-{3-Methyl-2-oxo-5-[4-(1H-pyrrolo[2,3-b]pyridin-5-yl)-pyridin-2-yl]-2,3-dihydro-imidazol-1-yl}-benzonitril ("A48")

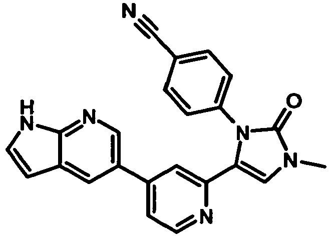

Analytische Daten "A48": DC: R_{f} = 0.20 (Si60, CH₂Cl₂/5% MeOH/; LC-MS: t_{R} = 1.647 min (UV = 220 nm), t_{R} = 1.644 min. (TIC, mit [MH]⁺ = 393); ¹H NMR (400 MHz, DMSO-d₆) δ 11.88 (s, 1 H), 8.58 (d, *J* = 2.2, 1 H), 8.34 - 8.31 (m, 1 H), 8.29 (d, *J* = 2.2, 1 H), 7.90 - 7.86 (m, 2H), 7.72 (d, *J* = 1.0, 1 H), 7.60 - 7.55 (m, 3H), 7.43 - 7.39 (m, 2H), 6.54 (d, J = 3.4, 1 H), 3.32 (s, 3H); ¹³C NMR (101 MHz, DMSO) δ 151.92, 148.87, 148.34, 148.15, 146.18, 140.87, 140.32, 132.00, 127.06, 126.95, 125.88, 124.24, 120.79, 119.13, 118.45, 118.08, 117.25, 116.31, 108.20, 99.92, 29.82.

### 1-Methyl-3-[4-(4-methyl-piperazin-1-yl)-3-trifluoromethyl-phenyl]-4-[4-(1 H-pyrrolo[2,3-b]pyridin-5-yl)-pyridin-2-yl]-1,3-dihydro-imidazol-2-on ("A49")

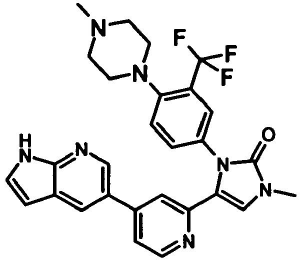

Analytische Daten "A49": DC: R_{f} = 0.19 (Si60, CH₂Cl₂/8% MeOH/1% NH₃); Smp.: 241 °C; LC-MS: t_{R} = 1.425 min (UV = 220 nm), t_{R} = 1.425 min. (TIC, mit [MH]⁺ = 534); ¹H NMR (500 MHz, DMSO-d₆) δ 11.84 (s, 1 H), 8.42 - 8.37 (m, 2H), 8.08 (d, *J* = 2.0, 1H), 7.63 - 7.52 (m, 4H), 7.51 - 7.43 (m, 2H), 7.32 (s, 1 H), 6.46 (dd, *J* = 3.4, 1.8, 1 H), 3.32 (s, 3H), 2.94 - 2.85 (m, 4H), 2.41 - 2.49 (m, 4H), 2.24 (s, 3H).

### 1-Methyl-3-[4-(4-methyl-piperazin-1-yl)-phenyl]-4-[4-(1 H-pyrrolo[2,3-b]pyridin-5-yl)-pyridin-2-yl]-1,3-dihydro-imidazol-2-on ("A50")

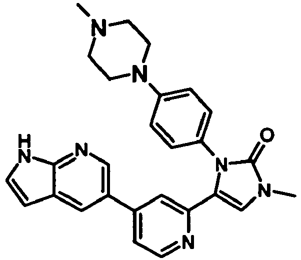

Analytische Daten "A50": DC: R_{f} = 0.15 (Si60, CH₂Cl₂/8% MeOH/1% NH₃); LC-MS: t_{R} = 1.316 min (UV = 220 nm), t_{R} = 1.303 min. (TIC, mit [MH]⁺ = 466); ¹H NMR (400 MHz, DMSO-d₆) δ 11.82 (s, 1 H), 8.45 (d, *J* = 5.2, 1 H), 8.29 (d, *J* = 2.2, 1 H), 7.88 (d, *J* = 2.1, 1 H), 7.56 - 7.53 (m, 1 H), 7.50 (dd, *J* = 5.2, 1.7, 1 H), 7.35 (s, 1 H), 7.15 - 7.11 (m, 2H), 7.03 (dd, *J* = 5.1, 4.0, 3H), 6.48 (dd, *J* = 3.4, 1.8, 1 H), 3.30 (s, 3H), 3.24 - 3.19 (m, 4H), 2.50 - 2.44 (m, 4H), 2.23 (s, 3H).

### 3-(4-Fluoro-3-trifluoromethyl-phenyl)-1-methyl-4-[4-(1 H-pyrrolo[2,3-b]pyridin-5-yl)-pyridin-2-yl]-1,3-dihydro-imidazol-2-on ("A51")

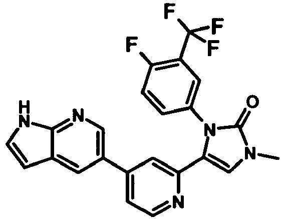

Analytische Daten "A51": DC: R_{f} = 0.55 (Si60, CH₂Cl₂/5% MeOH/1% NH₃); LC-MS: t_{R} = 1.982 min (UV = 220 nm), t_{R} = 1.981 min. (TIC, mit [MH]⁺ = 454); ¹H NMR (400 MHz, DMSO-d₆) δ 11.88 (s, 1 H), 8.59 (d, *J* = 2.2, 1 H), 8.30 (dd, *J* = 5.8, 3.7, 2H), 7.72 (dd, *J* = 14.2, 4.0, 2H), 7.62 - 7.53 (m, 5H), 6.53 (dd, *J* = 3.4, 1.8, 1 H), 3.33 (s, 3H); ¹³C NMR (101 MHz, DMSO) δ 162.57, 148.75, 140.82, 133.39, 133.30, 127.05, 125.82, 125.33, 118.34, 117.10, 116.92, 116.70, 115.55, 99.90, 29.80.

### 2-(4-{5-[4-(1H-Indazol-5-yl)-pyridin-2-yl]-3-methyl-2-oxo-2,3-dihydro-imidazol-1-yl}-phenyl)-2-methyl-propionitrile ("A52")

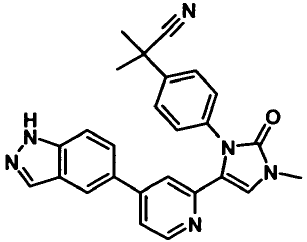

Analytische Daten "A52": DC: R_{f} = 0.26 (Si60, CH₂Cl₂/5% MeOH/1% NH₃); Smp.: 165°C; LC-MS: t_{R} = 1.728 min (UV = 220 nm), t_{R} = 1.750 min. (TIC, mit [MH]⁺ = 435); ¹H NMR (300 MHz, DMSO-d₆) δ 13.26 (s, 1H), 8.48 - 8.43 (m, 1 H), 8.16 (s, 1 H), 7.94 (d, *J* = 0.8, 1 H), 7.69 - 7.59 (m, 3H), 7.53 (dd, *J* = 5.2, 1.7, 1 H), 7.46 (s, 1 H), 7.42 - 7.32 (m, 3H), 7.18 (d, *J* = 1.0, 1 H), 3.36 (s, 3H), 1.77 (s, 6H).

### 2-(4-{5-[4-(1H-Indol-5-yl)-pyridin-2-yl]-3-methyl-2-oxo-2,3-dihydro-imidazol-1-yl}-phenyl)-2-methyl-propionitrile ("A53")

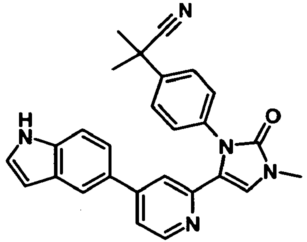

Analytische Daten "A53": DC: R_{f} = 0.26 (Si60, CH₂Cl₂/5% MeOH/1% NH₃); Smp.: 238°C; LC-MS: t_{R} = 1.871 min (UV = 220 nm), t_{R} = 1.898 min. (TIC, mit [MH]⁺ = 434); ¹H NMR (400 MHz, DMSO-d₆) δ 11.27 (s, 1 H), 8.43 - 8.38 (m, 1 H), 7.68 - 7.62 (m, 3H), 7.47 - 7.33 (m, 6H), 7.08 (dd, *J* = 8.5, 1.7, 2H), 6.48 (dd, *J* = 2.5, 1.6, 1 H), 3.33 (d, *J* = 6.8, 3H), 1.75 (s, 6H); ¹³C NMR (101 MHz, DMSO) δ 152.33, 149.21, 148.17, 147.83, 139.92, 135.88, 135.41, 127.57, 127.38, 127.25, 126.17, 125.28, 124.04, 121.49, 119.03, 118.26, 117.85, 116.79, 114.91, 111.45, 101.19, 36.02, 29.75, 27.79.

### 2-Methyl-2-(4-{3-methyl-5-[4-(1-methyl-1H-pyrrolo[2,3-b]pyridin-5-yl)-pyridin-2-yl]-2-oxo-2,3-dihydro-imidazol-1-yl}-phenyl)-propionitrile ("A54")

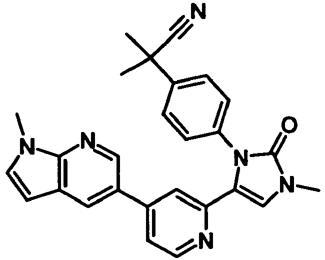

Analytische Daten "A54": DC: R_{f} = 0.21 (Si60, CH₂Cl₂/4% MeOH/1% NH₃); LC-MS: t_{R} = 1.942 min (UV = 220 nm), t_{R} = 1.945 min. (TIC, mit [MH]⁺ = 449); ¹H NMR (500 MHz, DMSO-d₆) δ 8.48 (d, *J* = 5.2, 1 H), 8.37 (d, *J* = 2.1, 1 H), 8.10 (d, *J =* 2.1, 1 H), 7.70 - 7.64 (m, 3H), 7.59 (dd, *J* = 5.2, 1.7, 1 H), 7.50 (d, *J =* 3.7, 1 H), 7.39 (d, *J =* 8.5, 2H), 7.25 (d, *J =* 0.8, 1 H), 6.57 (d, *J* = 3.4, 1 H), 3.90 (d, *J =* 8.4, 3H), 3.38 (s, 3H), 1.81 (s, 6H).

### 3-{3-Methyl-2-oxo-5-[4-(1H-pyrrolo[2,3-b]pyridin-5-yl)-pyridin-2-yl]-2,3-dihydro-imidazol-1-yl}-benzonitrile ("A55")

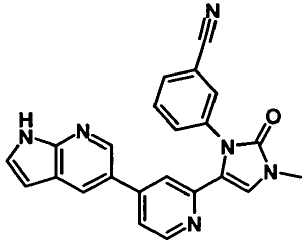

Analytische Daten "A55": DC: R_{f} = 0.47 (Si60, CH₂Cl₂/5% MeOH); Smp.: 265°C; LC-MS: t_{R} = 1.672 min (UV = 220 nm), t_{R} = 1.683 min. (TIC, mit [MH]⁺ = 393); ¹H NMR (300 MHz, DMSO-d₆) δ 11.89 (s, 1 H), 8.61 (d, *J =* 2.2, 1 H), 8.35 (dd, *J* = 4.8, 1.1, 2H), 7.88 - 7.81 (m, 2H), 7.76 (d, *J* = 0.9, 1 H), 7.68 - 7.52 (m, 5H), 6.59 (d, *J =* 3.4, 1H), 3.37 (s, 3H); ¹³C NMR (75 MHz, DMSO) δ 152.63, 149.27, 148.83, 148.63, 146.61, 141.33, 137.56, 132.02, 130.43, 130.32, 129.72, 127.51, 126.34, 124.73, 121.42, 119.63, 118.87, 118.20, 117.66, 116.25, 111.26, 100.44, 30.29.

### 4-(6-Amino-5-trifluoromethyl-[3,4']bipyridinyl-2'-yl)-1-methyl-3-(4-piperazin-1-yl-3-trifluoromethyl-phenyl)-1,3-dihydro-imidazol-2-one ("A56")

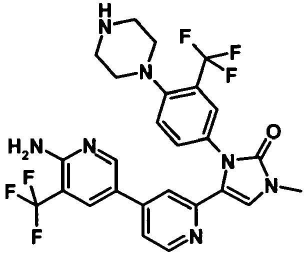

Analytische Daten "A56": DC: R_{f} = 0.17 (Si60, CH₂Cl₂/8% MeOH/1% NH₃); LC-MS: t_{R} = 1.519 min (UV = 220 nm), t_{R} = 1.515 min. (TIC, mit [MH]⁺ = 564); ¹H NMR (400 MHz, DMSO-d₆) δ 8.49 (d, *J =* 2.0, 1 H), 8.31 (d, *J =* 5.3, 1 H), 7.98 (d, *J* = 1.9, 1H), 7.53 - 7.46 (m, 4H), 7.41 (dd, *J* = 8.2, 2.5, 2H), 6.87 (s, 2H), 3.31 (s, 3H), 2.80 (d, *J* = 8.7, 8H).

### 4-[4-(1H-Indol-5-yl)-pyridin-2-yl]-1-methyl-3-(4-piperazin-1-yl-3-trifluoromethyl-phenyl)-1,3-dihydro-imidazol-2-one ("A57")

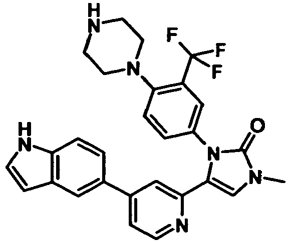

Analytische Daten "A57": DC: R_{f} = 0.20 (Si60, CH₂Cl₂/10% MeOH/1% NH₃); LC-MS: t_{R} = 1.474 min (UV = 220 nm), t_{R} = 1.510 min. (TIC, mit [MH]⁺ = 519); ¹H NMR (300 MHz, DMSO-d₆) δ 11.26 (s, 1 H), 8.37 (d, *J* = 5.2, 1 H), 7.67 - 7.54 (m, 3H), 7.51 - 7.38 (m, 5H), 7.24 - 7.15 (m, 2H), 6.46 - 6.41 (m, 1 H), 3.32 (s, 3H), 3.25 (s, breit, 4H), 2.83 (s, breit, 4H).

### 3-(4-Chloro-3-methoxy-phenyl)-1-methyl-4-[4-(1 H-pyrrolo[2,3-b]pyridin-5-yl)-pyridin-2-yl]-1,3-dihydro-imidazol-2-one ("A58")

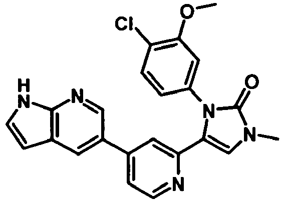

Analytische Daten "A58": DC: R_{f} = 0.18 (Si60, CH₂Cl₂/5% MeOH/1% NH₃); Smp.: 223°C; LC-MS: t_{R} = 1.787 min (UV = 220 nm), t_{R} = 1.815 min. (TIC, mit [MH]⁺ = 432); ¹H NMR (400 MHz, DMSO-d₆) δ 11.92 (s, 1 H), 8.51 (d, *J =* 2.2, 1 H), 8.45 (d, *J* = 5.3, 1 H), 8.15 (d, *J* = 2.0, 1 H), 7.63 - 7.57 (m, 2H), 7.52 (t, *J* = 4.2, 2H), 7.48 (d, *J =* 0.9, 1 H), 7.15 (d, *J* = 2.3, 1 H), 6.86 (dd, *J* = 8.4, 2.3, 1 H), 6.55 (dd, *J* = 3.4, 1.8, 1 H), 3.79 (s, 3H), 3.36 (s, 3H); ¹³C NMR (101 MHz, DMSO) δ 153.92, 152.16, 149.12, 148.29, 148.18, 145.81, 140.70, 136.05, 128.99, 127.07, 125.70, 124.28, 121.37, 119.87, 119.12, 119.05, 118.27, 117.06, 115.07, 111.92, 99.89, 55.76, 29.75.

### 4-(6-Amino-[3,4']bipyridinyl-2'-yl)-1-methyl-3-(4-piperazin-1-yl-3-trifluoromethyl-phenyl)-1,3-dihydro-imidazol-2-one ("A59")

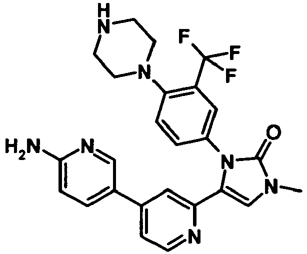

Analytische Daten "A59": DC: R_{f} = 0.10 (Si60, CH₂Cl₂/8% MeOH/1% NH₃); LC-MS: t_{R} = 1.128 min (UV = 220 nm), t_{R} = 1.164 min. (TIC, mit [MH]⁺ = 496); ¹H NMR (300 MHz, DMSO-d₆) δ 8.31 - 8.27 (m, 1 H), 8.17 (d, *J* = 2.2, 1 H), 7.57 - 7.49 (m, 3H), 7.47 - 7.41 (m, 2H), 7.37 (dd, *J* = 5.3, 1.7, 1 H), 7.17 *(d, J =* 1.0, 1 H), 6.46 (d, *J =* 8.7, 1 H), 6.34 (s, 2H), 3.30 (s, 3H), 2.82 (d, *J =* 3.7, 8H); ¹³C NMR (75 MHz, DMSO) δ 160.42, 152.75, 151.39, 149.50, 148.39, 146.43, 145.21, 134.76, 132.88, 132.17, 125.78, 125.26, 124.71, 121.72, 120.06, 117.11, 115.71, 115.54, 107.75, 54.41, 45.88, 30.24.

### 4-[4-(4-Amino-phenyl)-pyridin-2-yl]-1-methyl-3-(4-piperazin-1-yl-3-trifluoromethyl-phenyl)-1,3-dihydro-imidazol-2-one ("A60")

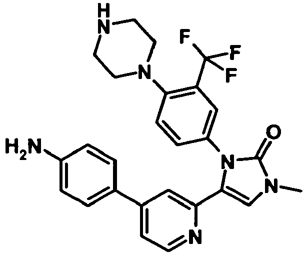

Analytische Daten "A60": DC: R_{f} = 0.12 (Si60, CH₂Cl₂/8% MeOH/1% NH₃); LC-MS: t_{R} = 1.329 min (UV = 220 nm), t_{R} = 1.333 min. (TIC, mit [MH]⁺ = 495); ¹H NMR (300 MHz, DMSO-d₆) δ 8.28 (d, *J* = 5.3, 1H), 7.60 - 7.53 (m, 2H), 7.45 (dd, *J*= 8.6, 2.4, 1H), 7.39 (s, 1H), 7.31 (dd, *J* = 5.3, 1.7, 1H), 7.17 (d, *J =* 8.6, 2H), 7.01 (d, *J =* 1.0, 1 H), 6.56 (d, *J =* 8.6, 2H), 5.52 (s, 2H), 3.30 (s, 3H), 2.84 (d, *J* = 4.6, 8H); ¹³C NMR (75 MHz, DMSO) δ 152.76, 151.47, 150.32, 149.50, 148.14, 147.33, 132.92, 132.35, 127.15, 125.95, 125.40, 124.88, 123.13, 121.89, 117.21, 115.75, 115.30, 113.80, 54.33, 45.82, 30.22.

### N-{2'-[1-Methyl-2-oxo-3-(4-piperazin-1-yl-3-trifluoromethyl-phenyl)-2,3-dihydro-1H-imidazol-4-yl]-[3,4']bipyridinyl-6-yl}-methanesulfonamide ("A61")

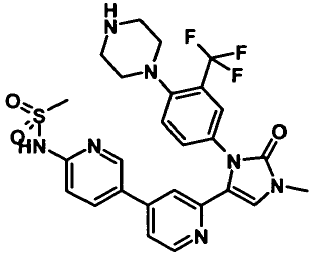

Analytische Daten "A61": DC: R_{f} = 0.08 (Si60, CH₂Cl₂/15% MeOH/1% NH₃); LC-MS: t_{R} = 1.358 min (UV = 220 nm), t_{R} = 1.387 min. (TIC, mit [MH]⁺ = 574); ¹H NMR (400 MHz, DMSO-d₆) δ 8.38 (d, *J* = 5.3, 1 H), 8.31 (d, *J* = 2.3, 1 H), 7.91 (dd, *J* = 8.7, 2.5, 1 H), 7.58 (dd, *J* = 7.5, 5.6, 2H), 7.51 - 7.44 (m, 3H), 7.26 (s, 1H), 6.94 (d, *J* = 8.7, 1H), 3.31 (s, 3H), 3.27 (s, 3H), 2.98 (d, *J* = 5.3, 4H), 2.91 (t, *J* = 8.3, 4H).

### N-(4-{2-[1-Methyl-2-oxo-3-(4-piperazin-1-yl-3-trifluoromethyl-phenyl)-2,3-dihydro-1H-imidazol-4-yl]-pyridin-4-yl}-phenyl)-methanesulfonamide ("A62")

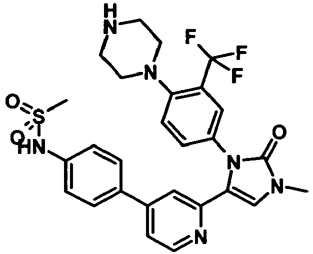

Analytische Daten "A62": DC: R_{f} = 0.20 (Si60, CH₂Cl₂/12% MeOH/1% NH₃); LC-MS: t_{R} = 1.433 min (UV = 220 nm), t_{R} = 1.499 min. (TIC, mit [MH]⁺ = 573); ¹H NMR (400 MHz, DMSO-d₆) δ 8.39 (dd, J = 5.2, 0.5, 1 H), 7.60 - 7.54 (m, 2H), 7.50 - 7.41 (m, 5H), 7.27 - 7.22 (m, 2H), 7.15 (d, *J* = 0.8, 1 H), 3.31 (s, 3H), 3.02 (s, 3H), 2.86 (s, breit, 4H), 2.84 (s, breit, 4H).

### 3-{3-Methyl-2-oxo-5-[4-(1H-pyrazolo[3,4-b]pyridin-5-yl)-pyridin-2-yl]-2,3-dihydro-imidazol-1-yl}-benzonitrile ("A63")

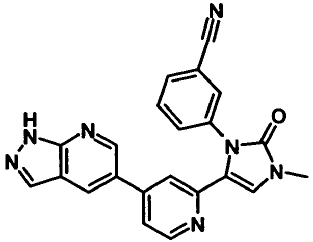

Analytische Daten "A63": DC: R_{f} = 0.13 (Si60, CH₂Cl₂/5% MeOH/10% NH₃); LC-MS: t_{R} = 1.624 min (UV = 220 nm), t_{R} = 1.669 min. (TIC, mit [MH]⁺ = 394); ¹H NMR (400 MHz, DMSO-d₆) δ 13.86 (s, 1 H), 8.90 (d, *J =* 2.2, 1 H), 8.63 (d, *J* = 2.1, 1 H), 8.34 - 8.31 (m, 1 H), 8.26 (d, *J* = 1.2, 1 H), 7.84 (d, *J* = 1.0, 1 H), 7.81 - 7.78 (m, 1 H), 7.76 (t, *J* = 1.6, 1 H), 7.61 (s, 1 H), 7.60 - 7.56 (m, 2H), 7.51 (ddd, *J* = 8.1, 2.1, 1.2, 1 H), 3.33 (s, 3H).

### 3-[5-(6-Amino-5-trifluoromethyl-[3,4']bipyridinyl-2'-yl)-3-methyl-2-oxo-2,3-dihydro-imidazol-1-yl]-benzonitrile ("A64")

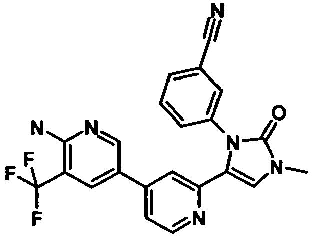

Analytische Daten "A64": DC: R_{f} = 0.22 (Si60, CH₂Cl₂/5% MeOH/10% NH₃); LC-MS: t_{R} = 1.764 min (UV = 220 nm), t_{R} = 1.771 min. (TIC, mit [MH]⁺ = 437); ¹H NMR (400 MHz, DMSO-d₆) δ 8.61 (d, J = 2.1, 1 H), 8.26 (d, *J* = 5.3, 1 H), 8.01 (d, *J* = 2.1, 1 H), 7.81 - 7.77 (m, 1 H), 7.75 (t, *J* = 1.6, 1 H), 7.60 (dd, *J* = 4.4, 3.4, 2H), 7.56 (d, *J* = 5.8, 1 H), 7.49 (ddd, J = 4.8, 2.5, 1.1, 2H), 6.89 (s, 2H), 3.31 (d, *J =* 8.2, 3H).

### 6-Amino-2'-[1-methyl-2-oxo-3-(4-piperazin-1-yl-3-trifluoromethyl-phenyl)-2,3-dihydro-1H-imidazol-4-yl]-[3,4']bipyridinyl-5-carbonitrile ("A65")

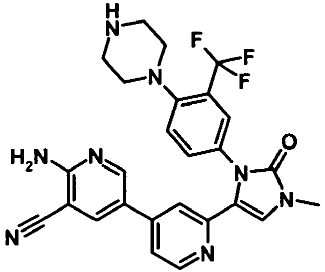

Analytische Daten "A65": DC: R_{f} = 0.12 (Si60, CH₂Cl₂/8% MeOH/1% NH₃); LC-MS: t_{R} = 1.336 min (UV = 220 nm), t_{R} = 1.348 min. (TIC, mit [MH]⁺ = 521); ¹H NMR (300 MHz, DMSO-d₆) δ 8.51 (d, *J* = 2.5, 1H), 8.29 (d, *J* = 5.7, 1H), 8.22 (d, *J* = 2.5, 1 H), 7.45 (qt, *J* = 8.7, 4.4, 6H), 7.28 (s, 2H), 3.31 (s, 3H), 2.81 (s, 8H); ¹³C NMR (75 MHz, DMSO) δ 159.97, 152.75, 151.45, 151.17, 149.44, 148.70, 143.34, 140.06, 132.83, 131.92, 125.55, 125.42, 124.45, 121.49, 120.52, 117.40, 116.49, 116.14, 115.91, 89.42, 54.37, 45.84, 30.26.

### 3-(3-Ethyl-phenyl)-1-methyl-4-[4-(1 H-pyrrolo[2,3-b]pyridin-5-yl)-pyridin-2-yl]-1,3-dihydro-imidazol-2-one ("A66")

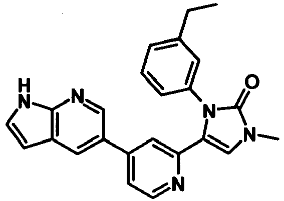

Analytische Daten "A66": LC-MS: t_{R} = 1.748 min (UV = 220 nm), t_{R} = 1.758 min. (TIC, mit [MH]⁺ = 396); ¹H NMR (300 MHz, DMSO-d₆) δ 11.87 (s, 1H), 8.49 (dd, *J* = 5.2, 0.5, 1 H), 8.32 (d, *J* = 2.2, 1 H), 8.01 (d, *J* = 2.2, 1 H), 7.64 - 7.54 (m, 2H), 7.49 - 7.41 (m, 2H), 7.34 (d, *J* = 7.8, 1 H), 7.21 - 7.11 (m, 3H), 6.54 (d, *J* = 3.3, 1 H), 3.37 (s, 3H), 2.67 (q, *J* = 7.5, 2H), 1.16 (t, *J* = 7.6, 3H); ¹³C NMR (75 MHz, DMSO) ö 149.79, 141.04, 128.82, 127.52, 127.06, 126.76, 126.02, 124.93, 118.56, 117.20, 115.26, 100.30, 30.21, 27.88, 15.55.

### 1-Methyl-4-[4-(1H-pyrrolo[2,3-b]pyridin-5-yl)-pyridin-2-yl]-3-(2-trifluoromethyl-phenyl)-1,3-dihydro-imidazol-2-one ("A67")

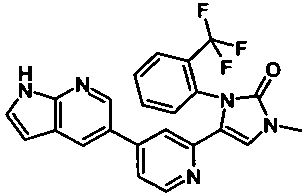

Analytische Daten "A67": LC-MS: t_{R} = 1.779 min (UV = 220 nm), t_{R} = 1.776 min. (TIC, mit [MH]⁺ = 436); ¹H NMR (400 MHz, DMSO-d₆) δ 11.90 (s, 1 H), 8.50 (d, *J* = 2.2, 1 H), 8.26 - 8.21 (m, 2H), 7.94 (d, *J* = 7.8, 1 H), 7.87 (t, *J* = 7.2, 1H), 7.82 - 7.72 (m, 2H), 7.61 (t, *J* = 6.2, 2H), 7.56 (s, 1H), 7.50 (dd, *J* = 5.2, 1.6, 1 H), 6.59 (d, *J* = 2.2, 1 H), 3.38 (s, 3H).

### N-{2'-[3-(3-Cyano-phenyl)-1-methyl-2-oxo-2,3-dihydro-1H-imidazol-4-yl]-5-trifluoromethyl-[3,4']bipyridinyl-6-yl}-acetamide ("A68")

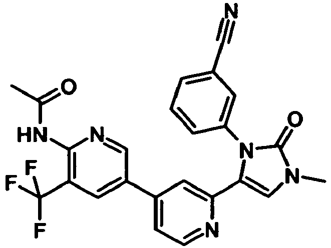

Analytische Daten "A68": DC: R_{f} = 0.29 (Si60, CH₂Cl₂/10% MeOH); LC-MS: t_{R} = 1.560 min (UV = 220 nm), t_{R} = 1.560 min. (TIC, mit [MH]⁺ = 479); ¹H NMR (400 MHz, DMSO-d₆) δ 10.36 (s, 1H), 9.11 (d, *J* = 2.2, 1 H), 8.48 (d, *J* = 2.2, 1 H), 8.37 (d, *J* = 5.2, 1 H), 7.86 (d, J = 0.8, 1 H), 7.81 - 7.78 (m, 1 H), 7.76 (t, *J* = 1.6, 1 H), 7.67 (dd, *J* = 5.2, 1.7, 1 H), 7.62 (d, *J* = 2.8, 1 H), 7.59 (d, *J* = 8.0, 1 H), 7.53 - 7.49 (m, 1H), 3.34 (s, 3H), 2.09 (s, 3H).

### 2-{3-Methyl-2-oxo-5-[4-(1H-pyrrolo[2,3-b]pyridin-5-yl)-pyridin-2-yl]-2,3-dihydro-imidazol-1-yl}-benzonitrile ("A69")

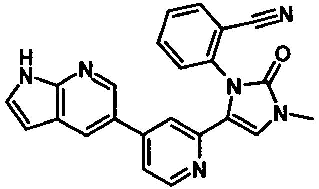

Analytische Daten "A69": LC-MS: t_{R} = 1.652 min (UV = 220 nm), t_{R} = 1.649 min. (TIC, mit [MH]⁺ = 393); ¹H NMR (500 MHz, DMSO-d₆) δ 11.84 (s, 1H), 8.58 (d, *J* = 2.1, 1H), 8.31 (d, *J* = 2.0, 1 H), 8.18 (d, *J* = 5.2, 1 H), 7.95 (dd, *J* = 7.7, 1.2, 1 H), 7.80 (s, 1 H), 7.77 - 7.70 (m, 2H), 7.58 (dt, *J* = 5.8, 5.3, 2H), 7.49 (dd, *J* = 5.2, 1.6, 1 H), 7.42 (d, *J* = 8.0, 1 H), 6.54 (dd, *J* = 3.3, 1.7, 1 H), 3.35 (s, 3H).

### 3-Amino-6-(2-{3-[4-(cyano-dimethyl-methyl)-phenyl]-1-methyl-2-oxo-2,3-dihydro-1H-imidazol-4-yl}-pyridin-4-yl)-pyrazine-2-carbonitrile ("A70°)

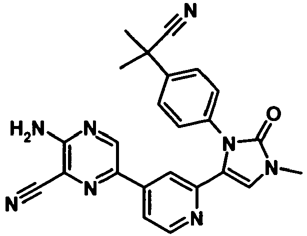

Analytische Daten "A70": DC: R_{f} = 0.24 (Si60, CH₂Cl₂/5% MeOH/10% NH₃); LC-MS: t_{R} = 1.739 min (UV = 220 nm), t_{R} = 1.742 min. (TIC, mit [MH]⁺ = 437); ¹H NMR (300 MHz, DMSO-d₆) δ 8.75 (s, 1H), 8.42 - 8.36 (m, 1H), 7.70 (s, 2H), 7.65 (dd, J = 5.2, 1.6, 1 H), 7.58 - 7.51 (m, 3H), 7.43 (s, 1 H), 7.30 - 7.22 (m, 2H), 3.31 (s, 3H), 1.71 (s, 6H); ¹³C NMR (75 MHz, DMSO) δ 156.51, 152.83, 149.80, 148.94, 145.37, 142.46, 139.96, 137.48, 135.85, 127.46, 125.43, 124.45, 121.65, 116.73, 115.86, 115.54, 115.42, 110.70, 36.41, 30.24, 28.27.

### 1-Methyl-3-(4-morpholin-4-yl-3-trifluoromethyl-phenyl)-4-[4-(1 H-pyrrolo[2,3-b]pyridin-5-yl)-pyridin-2-yl]-1,3-dihydro-imidazol-2-one ("A71")

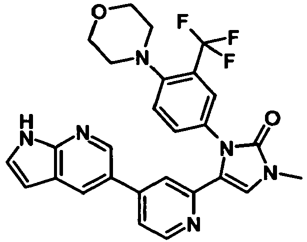

Analytische Daten "A71 ": DC: R_{f} = 0.29 (Si60, CH₂Cl₂/8% MeOH/1% NH₃); LC-MS: t_{R} = 1.891 min (UV = 220 nm), t_{R} = 1.886 min. (TIC, mit [MH]⁺ = 521); ¹H NMR (500 MHz, DMSO-d₆) δ 11.83 (s, 1 H), 8.39 - 8.37 (m, 2H), 8.12 (d, *J* = 2.0, 1 H), 7.63 (d, *J* = 8.6, 1 H), 7.60 (d, *J* = 2.4, 1 H), 7.57 - 7.53 (m, 2H), 7.51 - 7.47 (m, 2H), 7.35 (d, *J* = 0.8, 1 H), 6.48 (dd, *J* = 3.4, 1.8, 1 H), 3.76 - 3.68 (m, 4H), 3.32 (s, 3H), 2.91 - 2.85 (m, 4H).

### 3-[4-(4-Acetyl-piperazin-1-yl)-3-trifluoromethyl-phenyl]-1-methyl-4-[4-(1 H-pyrrolo[2,3-b]pyridin-5-yl)-pyridin-2-yl]-1,3-dihydro-imidazol-2-one ("A72")

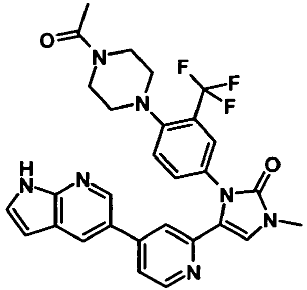

Analytische Daten "A72": DC: R_{f} = 0.19 (Si60, CH₂Cl₂/8% MeOH/1% NH₃); LC-MS: t_{R} = 1.772 min (UV = 220 nm), t_{R} = 1.766 min. (TIC, mit [MH]⁺ = 562).

### 3-(3-Methoxy-phenyl)-1-methyl-4-[4-(1H-pyrrolo[2,3-b]pyridin-5-yl)-pyridin-2-yl]-1,3-dihydro-imidazol-2-one ("A73")

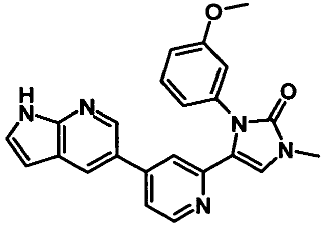

Analytische Daten "A73": DC: R_{f} = 0.16 (Si60, CH₂Cl₂/5% MeOH/10% NH₃); LC-MS: t_{R} = 1.609 min (UV = 220 nm), t_{R} = 1.607 min. (TIC, mit [MH]⁺ = 398); ¹H NMR (400 MHz, DMSO-d₆) δ 11.82 (s, 1 H), 8.42 (d, *J =* 5.2, 1 H), 8.33 (d, *J* = 2.2, 1 H), 8.05 (d, *J* = 2.0, 1 H), 7.56 - 7.50 (m, 2H), 7.42 (s, 1 H), 7.35 (t, *J =* 8.1, 1 H), 7.25 (d, *J* = 1.0, 1 H), 7.02 - 6.98 (m, 1 H), 6.88 (t, *J* = 2.2, 1 H), 6.82 - 6.77 (m, 1 H), 6.50 (dd, *J* = 3.4, 1.8, 1 H), 3.73 (s, 3H), 3.31 (s, 3H).

### 3-[4-(4-Methanesulfonyl-piperazin-1-yl)-3-trifluoromethyl-phenyl]-1-methyl-4-[4-(1H-pyrrolo[2,3-b]pyridin-5-yl)-pyridin-2-yl]-1,3-dihydro-imidazol-2-one ("A74")

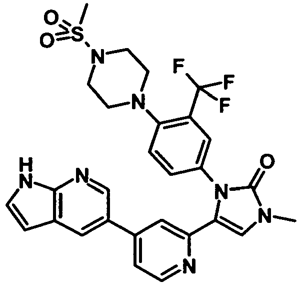

Analytische Daten "A74": DC: R_{f} = 0.17 (Si60, CH₂Cl₂/5% MeOH/1% NH₃); LC-MS: t_{R} = 1.886 min (UV = 220 nm), t_{R} = 1.884 min. (TIC, mit [MH]⁺ = 598); ¹H NMR (500 MHz, DMSO-d₆) δ 11.85 (s, 1 H), 8.39 - 8.36 (m, 2H), 8.15 (d, *J =* 2.0, 1 H), 7.69 (d, *J* = 8.6, 1 H), 7.60 (d, *J* = 2.4, 1 H), 7.57 - 7.53 (m, 2H), 7.52 - 7.47 (m, 2H), 7.39 (s, 1 H), 6.48 (dd, J = 3.3, 1.6, 1 H), 3.32 (s, 3H), 3.27 - 3.24 (m, 4H), 3.02 - 2.98 (m, 4H), 2.96 (s, 3H).

### 3-{3-Difluoromethyl-2-oxo-5-[4-(1H-pyrrolo[2,3-b]pyridin-5-yl)-pyridin-2-yl]-2,3-dihydro-imidazol-1-yl}-benzonitrile ("A75")

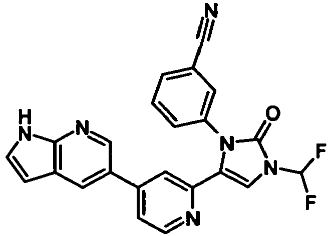

Analytische Daten "A75": DC: R_{f} = 0.32 (Si60, CH₂Cl₂/5% MeOH/10% NH₃); LC-MS: t_{R} = 1.971 min (UV = 220 nm), t_{R} = 1.967 min. (TIC, mit [MH]⁺ = 429).

### 1-Methyl-4-[4-(1H-pyrrolo[2,3-b]pyridin-5-yl)-pyridin-2-yl]-3-(2-trifluoromethoxy-phenyl)-1,3-dihydro-imidazol-2-one ("A76")

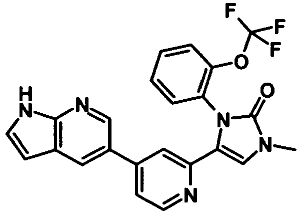

Analytische Daten "A76": LC-MS: t_{R} = 1.823 min (UV = 220 nm), t_{R} = 1.822 min. (TIC, mit [MH]⁺ = 452); ¹H NMR (500 MHz, DMSO-d₆) δ 11.82 (s, 1H), 8.46 (d, J = 2.1 Hz, 1H), 8.24 (d, J = 5.2 Hz, 1H), 8.20 (d, J = 2.0 Hz, 1H), 7.60 (s, 1 H), 7.58 - 7.53 (m, 4H), 7.53 - 7.48 (m, 2H), 7.46 - 7.43 (m, 1 H), 6.53 (dd, J = 3.4, 1.8 Hz, 1 H), 3.32 (s, 3H).

### 3-[4-(4-Ethyl-piperazin-1-yl)-3-trifluoromethyl-phenyl]-1-methyl-4-[4-(1 H-pyrrolo[2,3-b]pyridin-5-yl)-pyridin-2-yl]-1,3-dihydro-imidazol-2-one ("A77")

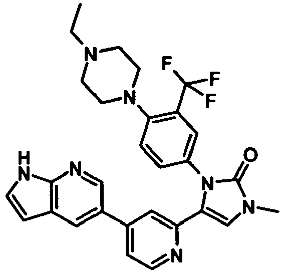

Analytische Daten "A77": DC: R_{f} = 0.33 (Si60, CH₂Cl₂/10% MeOH/1% NH₃); LC-MS: t_{R} = 1.505 min (UV = 220 nm), t_{R} = 1.499 min. (TIC, mit [MH]⁺ = 548); ¹H NMR (400 MHz, DMSO-d₆) δ 11.93 (s, 1H), 8.46 (d, J = 2.2 Hz, 1H), 8.43 (d, J = 5.2 Hz, 1 H), 8.18 (d, J = 2.0 Hz, 1 H), 7.65 (dd, J = 5.5, 3.0 Hz, 2H), 7.62 - 7.51 (m, 4H), 7.45 (s, 1 H), 6.53 (dd, J = 3.4, 1.8 Hz, 1 H), 3.57 - 3.37 (m, breit, 4H), 3.37 (s, 3H), 3.07 (s, breit, 4H), 2.81 (m, breit, 2H), 1.18 (t, J = 7.0 Hz, 3H).

### 2-Methyl-2-(4-{3-methyl-2-oxo-5-[4-(5H-pyrrolo[2,3-b]pyrazin-2-yl)-pyridin-2-yl]-2,3-dihydro-imidazol-1-yl}-phenyl)-propionitrile ("A78")

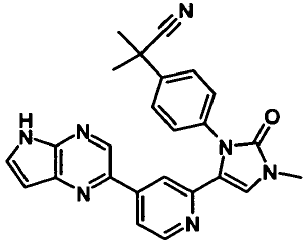

Analytische Daten "A78": LC-MS: t_{R} = 1.690 min (UV = 220 nm), t_{R} = 1.687 min. (TIC, mit [MH]⁺ = 436); ¹H NMR (300 MHz, DMSO-d₆) δ 12.23 (s, 1H), 8.57 (s, 1H), 8.46 (dd, J = 5.2, 0.6 Hz, 1H), 7.98 - 7.94 (m, 1H), 7.86 (dd, J = 5.2, 1.6 Hz, 1 H), 7.67 (d, J = 0.7 Hz, 1 H), 7.61 - 7.56 (m, 2H), 7.47 (s, 1 H), 7.34 - 7.29 (m, 2H), 6.67 (dd, J = 3.6, 1.7 Hz, 1 H), 3.32 (s, 3H), 1.71 (s, 7H); ¹³C NMR (DEPT, 75 MHz, DMSO) δ 148.68, 133.60, 131.84, 126.56, 124.43, 117.03, 115.64, 114.62, 99.69, 29.15, 27.24.

### 1-Methyl-4-[4-(1H-pyrrolo[2,3-b]pyridin-5-yl)-pyridin-2-yl]-3-m-tolyl-1,3-dihydro-imidazol-2-one ("A79")

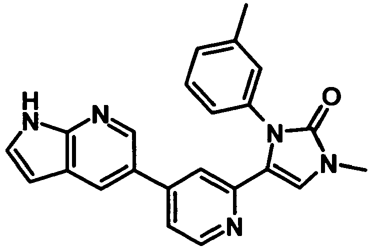

Analytische Daten "A79": LC-MS: t_{R} = 1.663 min (UV = 220 nm), t_{R} = 1.658 min. (TIC, mit [MH]⁺ = 382); ¹H NMR (400 MHz, DMSO-d₆) δ 11.84 (s, 1 H), 8.42 (dd, J = 5.2, 0.6 Hz, 1 H), 8.31 (d, J = 2.2 Hz, 1 H), 8.02 (d, J = 2.2 Hz, 1 H), 7.56 (dd, J = 3.1, 1.7 Hz, 1 H), 7.52 (dd, J = 5.2, 1.7 Hz, 1 H), 7.43 (s, 1 H), 7.34 (t, J = 7.7 Hz, 1 H), 7.25 (d, J = 7.6 Hz, 1 H), 7.22 - 7.15 (m, 2H), 7.01 (d, J = 7.9 Hz, 1 H), 6.50 (d, J = 2.9 Hz, 1 H), 3.31 (s, 3H), 2.34 (s, 3H).

### 3-(3-lsopropyl-phenyl)-1-methyl-4-[4-(1H-pyrrolo[2,3-b]pyridin-5-yl)-pyridin-2-yl]-1,3-dihydro-imidazol-2-one ("A80")

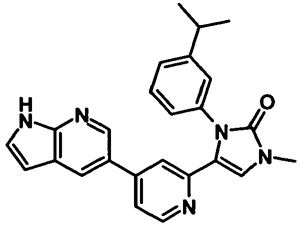

Analytische Daten "A80": LC-MS: t_{R} = 1.847 min (UV = 220 nm), t_{R} = 1.844 min. (TIC, mit [MH]⁺ = 410); ¹H NMR (400 MHz, DMSO-d₆) δ 11.85 (s, 1 H), 8.45 (dd, J = 5.2, 0.6 Hz, 1 H), 8.23 (d, J = 2.2 Hz, 1 H), 7.92 (d, J = 2.2 Hz, 1 H), 7.57 - 7.51 (m, 2H), 7.43 - 7.29 (m, 3H), 7.15 - 7.10 (m, 2H), 7.02 (d, J = 1.0 Hz, 1H), 6.48 (dd, J = 3.4, 1.2 Hz, 1 H), 3.32 (s, 3H), 2.88 (dq, J = 13.7, 6.9 Hz, 1 H), 1.13 (s, 3H), 1.11 (s, 3H).

### 2-Methyl-2-{4-[3-methyl-2-oxo-5-(4-pyrido[2,3-b]pyrazin-7-yl-pyridin-2-yl)-2,3-dihydro-imidazol-1-yl]-phenyl}-propionitrile ("A81")

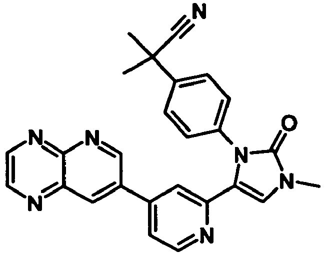

Analytische Daten "A81": LC-MS: t_{R} = 1.652 min (UV = 220 nm), t_{R} = 1.652 min. (TIC, mit [MH]⁺ = 448); ¹H NMR (400 MHz, DMSO-d₆) δ 9.32 (d, J = 2.5 Hz, 1H), 9.17 (d, J = 1.8 Hz, 1H), 9.13 (d, J = 1.8 Hz, 1H), 8.71 (d, J = 2.5 Hz, 1 H), 8.55 - 8.48 (m, 1 H), 7.79 (dd, J = 5.2, 1.7 Hz, 1 H), 7.63 - 7.57 (m, 2H), 7.57 - 7.54 (m, 2H), 7.34 - 7.28 (m, 2H), 3.33 (s, 3H), 1.74 (s, 6H).

### 5-[5-(6-Amino-5-trifluoromethyl-[3,4']bipyridinyl-2'-yl)-3-methyl-2-oxo-2,3-dihydro-imidazol-1-yl]-2-(4-methyl-piperazin-1-yl)-benzonitrile (A82")

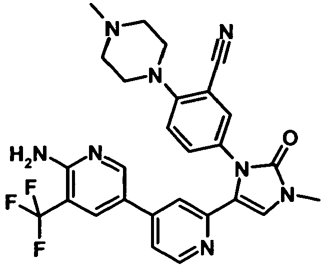

Analytische Daten "A82": DC: R_{f} = 0.18 (Si60, CH₂Cl₂/8% MeOH/1% NH₃); LC-MS: t_{R} = 1.400 min (UV = 220 nm), t_{R} = 1.388 min. (TIC, mit [MH]⁺ = 535); ¹H NMR (400 MHz, DMSO-d₆) δ 8.56 (d, J = 2.1 Hz, 1 H), 8.32 - 8.29 (m, 1 H), 8.14 (s, 1 H), 7.98 (d, J = 2.1 Hz, 1 H), 7.58 (d, J = 2.5 Hz, 1 H), 7.50 - 7.46 (m, 2H), 7.37 (dd, J = 8.8, 2.6 Hz, 1H), 7.15 (d, J = 8.9 Hz, 1H), 6.85 (s, 2H), 3.30 (s, 3H), 3.21 - 3.15 (m, 4H), 2.57 - 2.52 (m, 4H), 2.27 (s, 3H).

### 5-{3-Methyl-2-oxo-5-[4-(1H-pyrrolo[2,3-b]pyridin-5-yl)-pyridin-2-yl]-2,3-dihydro-imidazol-1-yl}-2-(4-methyl-piperazin-1-yl)-benzonitrile ("A83")

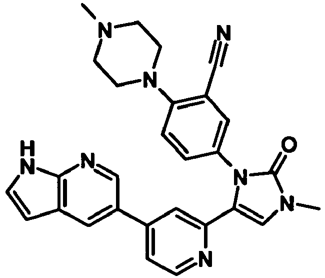

Analytische Daten "A83": DC: R_{f} = 0.16 (Si60, CH₂Cl₂/8% MeOH/1% NH₃); LC-MS: t_{R} = 1.365 min (UV = 220 nm), t_{R} = 1.351 min. (TIC, mit [MH]⁺ = 491); ¹H NMR (400 MHz, DMSO-d₆) δ 11.88 (s, 1H), 8.49 (d, J = 2.2 Hz, 1H), 8.37 (d, J = 5.3 Hz, 1 H), 8.20 (d, J = 2.0 Hz, 1 H), 7.66 (d, J = 2.5 Hz, 1 H), 7.58 - 7.50 (m, 4H), 7.44 - 7.40 (m, 1 H), 7.19 (dd, J = 11.0, 5.6 Hz, 1 H), 6.52 (dd, J = 3.4, 1.8 Hz, 1 H), 3.31 (s, 3H), 3.21 (s, 4H), 2.59 (s, breit, 4H), 2.34 - 2.27 (s, 3H).

### 2-{4-[5-(6-Amino-5-phenyl-[3,4']bipyridinyl-2'-yl)-3-methyl-2-oxo-2,3-dihydro-imidazol-1-yl]-phenyl]-2-methyl-propionitrile ("A84")

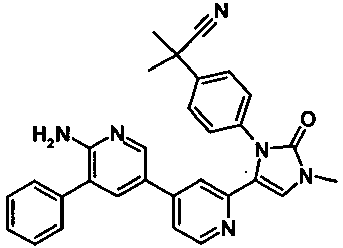

Analytische Daten "A84": DC: R_{f} = 0.21 (Si60, CH₂Cl₂/10% EtOH); LC-MS: t_{R} = 1.611 min (UV = 220 nm), t_{R} = 1.606 min. (TIC, mit [MH]⁺ = 487); ¹H NMR (400 MHz, DMSO-d₆) δ 8.31 (d, J = 5.2 Hz, 1 H), 8.16 (d, J = 2.3 Hz, 1 H), 7.54 - 7.39 (m, 11 H), 7.32 - 7.24 (m, 4H), 3.29 (s, 3H), 1.63 (s, 6H).

### 2-Methyl-2-(4-{3-methyl-5-[4-(2-methyl-1H-pyrrolo[2,3-b]pyridin-5-yl)-pyridin-2-yl]-2-oxo-2,3-dihydro-imidazol-1-yl}-phenyl)-propionitrile ("A85")

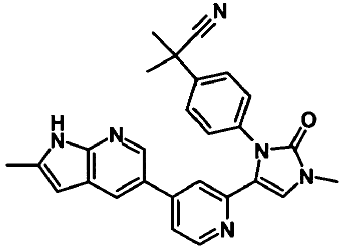

Analytische Daten "A85": DC: R_{f} = 0.35 (Si60, CH₂Cl₂/10% EtOH); LC-MS: t_{R} = 1.795 min (UV = 220 nm), t_{R} = 1.791 min. (TIC, mit [MH]⁺ = 449); ¹H NMR (400 MHz, DMSO-d₆) δ 11.67 (s, 1 H), 8.42 - 8.38 (m, 1 H), 8.10 (d, J = 2.2 Hz, 1 H), 7.89 (d, J = 2.0 Hz, 1 H), 7.64 - 7.61 (m, 2H), 7.50 (dd, J = 5.2, 1.7 Hz, 1 H), 7.44 (s, 1H), 7.35 - 7.29 (m, 2H), 7.15 (d, J = 1.0 Hz, 1H), 6.18 (dd, J = 1.9, 1.1 Hz, 1H), 3.31 (s, 3H), 2.40 (s, 3H), 1.74 (s, 6H).

### 4-[4-(2-tert.-Butyl-1 H-pyrrolo[2,3-b]pyridin-5-yl)-pyridin-2-yl]-1-methyl-3-[4-(4-methyl-piperazin-1-yl)-3-trifluoromethyl-phenyl]-1,3-dihydro-imidazol-2-one ("A86")

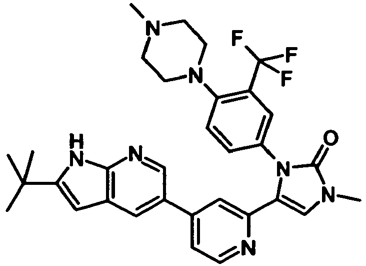

Analytische Daten "A86": DC: R_{f} = 0.34 (Si60, CH₂Cl₂/10% MeOH/1% NH₃); LC-MS: t_{R} = 1.762 min (UV = 220 nm), t_{R} = 1.755 min. (TIC, mit [MH]⁺ = 590); ¹H NMR (400 MHz, DMSO-d₆) δ 11.72 (s, 1 H), 8.40 (d, J = 5.3 Hz, 1 H), 8.29 (d, J = 2.2 Hz, 1 H), 7.85 (d, J = 2.1 Hz, 1 H), 7.63 - 7.58 (m, 2H), 7.53 - 7.44 (m, 3H), 7.19 (d, J = 0.9 Hz, 1H), 6.14 (d, J = 2.1 Hz, 1H), 3.30 (s, 3H), 2.90 (t, J = 4.6 Hz, 4H), 2.47 (s, breit, 4H), 2.24 (s, 3H), 1.37 (s, 9H).

### 1-Methyl-3-[4-(4-methyl-piperazin-1-yl)-3-trifluoromethyl-phenyl]-4-[4-(2-trifluoromethyl-1H-pyrrolo[2,3-b]pyridin-5-yl)-pyridin-2-yl]-1,3-dihydro-imidazol-2-one ("A87")

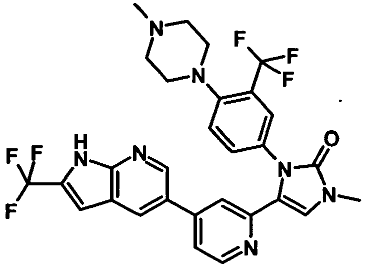

Analytische Daten "A87": DC: R_{f} = 0.27 (Si60, CH₂Cl₂/8% MeOH/1% NH₃); LC-MS: t_{R} = 1.673 min (UV = 220 nm), t_{R} = 1.668 min. (TIC, mit [MH]⁺ = 602); ¹H NMR (400 MHz, DMSO-d₆) δ 8.61 (d, J = 2.2 Hz, 1 H), 8.42 (dd, J = 5.2, 0.5 Hz, 1 H), 8.35 (d, J = 2.2 Hz, 1 H), 8.18 (s, 1 H), 7.61 - 7.55 (m, 3H), 7.53 (s, 1H), 7.47 (dd, J = 8.5, 2.5 Hz, 1H), 7.40 (d, J = 0.9 Hz, 1H), 7.07 (d, J = 1.2 Hz, 1 H), 3.32 (s, 3H), 2.89 (t, J = 4.6 Hz, 4H), 2.48 (s, breit, 4H), 2.25 (s, 3H).

### 1-Methyl-3-[4-(4-methyl-piperazin-1-yl)-3-trifluoromethyl-phenyl]-4-[4-(2-methyl-1H-pyrrolo[2,3-b]pyridin-5-yl)-pyridin-2-yl]-1,3-dihydro-imidazol-2-one ("A88")

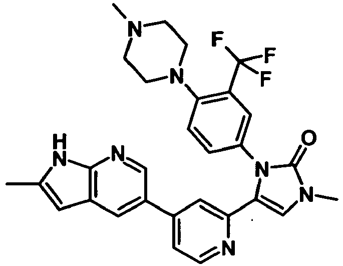

Analytische Daten "A88": DC: R_{f} = 0.10 (Si60, CH₂Cl₂/8% MeOH/1% NH₃); LC-MS: t_{R} = 1.509 min (UV = 220 nm), t_{R} = 1.503 min. (TIC, mit [MH]⁺ = 548); ¹H NMR (400 MHz, DMSO-d₆) δ 11.70 (s, 1 H), 8.39 - 8.30 (m, 1 H), 8.28 (d, J = 2.2 Hz, 1 H), 7.95 (d, J = 2.0 Hz, 1 H), 7.62 - 7.56 (m, 2H), 7.55 - 7.49 (m, 2H), 7.46 (dd, J = 8.7, 2.3 Hz, 1H), 7.35 (s, 1H), 6.17 (dd, J = 1.8, 1.0 Hz, 1H), 3.33 (s, 3H), 2.96 (s, breit, 4H), 2.67 (s, breit, 4H), 2.42 (s, 6H).

### 3-[4-(4-Acetyl-piperazin-1-yl)-3-trifluoromethyl-phenyl]-4-[4-(1H-indol-5-yl)-pyridin-2-yl]-1-methyl-1,3-dihydro-imidazol-2-one ("A89")

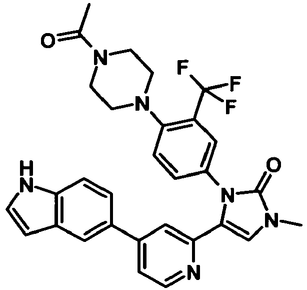

Analytische Daten "A89": DC: R_{f} = 0.38 (Si60, CH₂Cl₂/10% MeOH/1% NH₃); LC-MS: t_{R} = 1.873 min (UV = 220 nm), t_{R} = 1.889 min. (TIC, mit [MH]⁺ = 541); ¹H NMR (400 MHz, DMSO-d₆) δ 11.25 (s, breit, 1 H), 8.36 (d, J = 5.3, 1 H), 7.70 (s, 1 H), 7.62 (d, J = 2.1, 2H), 7.51 - 7.45 (m, 3H), 7.42 (s, 2H), 7.25 (d, J = 1.6, 2H), 6.47 - 6.42 (m, 1H), 3.57 (m, 4H), 3.31 (s, 3H), 2.95 - 2.79 (m, 4H), 2.04 (s, 3H).

### 1-Methyl-3-(3-phenoxy-phenyl)-4-[4-(1 H-pyrrolo[2,3-b]pyridin-5-yl)-pyridin-2-yl]-1,3-dihydro-imidazol-2-one ("A90")

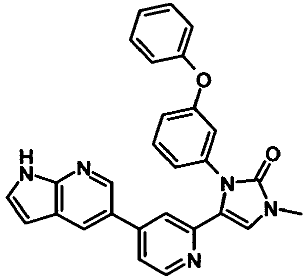

Analytische Daten "A90": DC: R_{f} = 0.35 (Si60, EtOH/EtOAc 1:1); LC-MS: t_{R} = 1.955 min (UV = 220 nm), t_{R} = 1.955 min. (TIC, mit [MH]⁺ = 460); ¹H NMR (500 MHz, DMSO-d₆) δ 11.88 (s, 1 H), 8.45 (dd, *J* = 4.8, 4.0, 2H), 8.20 (d, *J* = 2.0, 1H), 7.61 - 7.56 (m, 2H), 7.47 (dd, *J =* 18.3, 10.2, 2H), 7.38 (d, *J =* 0.9, 1 H), 7.30 - 7.24 (m, 2H), 7.12 - 7.01 (m, 3H), 6.89 (dd, *J* = 8.6, 0.9, 2H), 6.79 (t, *J* = 2.1, 1 H), 6.53 (dd, *J* = 3.4, 1.8, 1 H), 3.30 (s, 3H).

### 2-Methyl-2-{4-[3-methyl-2-oxo-5-(4-quinazolin-6-yl-pyridin-2-yl)-2,3-dihydroimidazol-1-yl]-phenyl}-propionitrile ("A91")

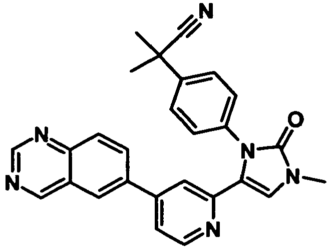

Analytische Daten "A91": DC: R_{f} = 0.60 (Si60, CH₂Cl₂/15% NH₃); LC-MS: t_{R} = 1.763 min (UV = 220 nm), t_{R} = 1.749 min. (TIC, mit [MH]⁺ = 447); ¹H NMR (400 MHz, DMSO-d₆) δ 9.64 (s, 1H), 9.35 (s, 1H), 8.47 (s, 2H), 8.08 (s, 2H), 7.60 (d, *J* = 8.6, 3H), 7.49 (s, 1 H), 7.48 - 7.44 (m, 1 H), 7.32 (d, *J* = 8.6, 2H), 3.33 (s, 3H), 1.71 (s, 6H).

### 2-(4-{5-[4-(2-tert-Butyl-1H-pyrrolo[2,3-b]pyridin-5-yl)-pyridin-2-yl]-3-methyl-2-oxo-2,3-dihydro-imidazol-1-yl}-phenyl)-2-methyl-propionitrile ("A92")

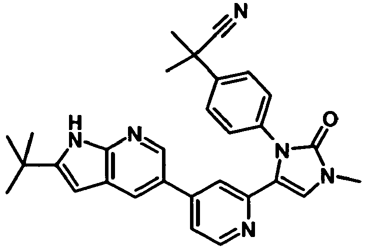

Analytische Daten "A92": LC-MS: t_{R} = 2.161 min (UV = 220 nm), t_{R} = 2.172 min. (TIC, mit [MH]⁺ = 491); ¹H NMR (400 MHz, DMSO-d₆) δ 11.71 (s, 1H), 8.43 (d, *J =* 5.7, 1H), 8.12 (d, *J* = 2.2, 1 H), 7.85 (d, *J* = 2.1, 1 H), 7.68 - 7.60 (m, 2H), 7.49 (dd, *J* = 5.2, 1.7, 1 H), 7.43 (s, 1 H), 7.38 - 7.29 (m, 2H), 7.09 (d, *J* = 0.9, 1 H), 6.18 (d, *J =* 2.1, 1 H), 3.32 (s, 3H), 1.75 (s, 6H), 1.36 (s, 9H).

### 2-(4-{5-[4-(4-Amino-3-trifluoromethyl-phenyl)-pyridin-2-yl]-3-methyl-2-oxo-2,3-dihydro-imidazol-1-yl}-phenyl)-2-methyl-propionitrile ("A93")

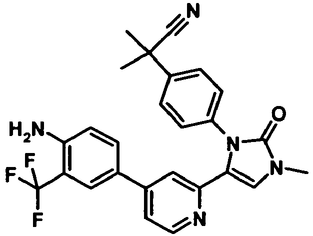

Analytische Daten "A93": DC: R_{f} = 0.24 (Si60, CH₂Cl₂/5% MeOH/10% NH₃); LC-MS: t_{R} = 2.000 min (UV = 220 nm), t_{R} = 1.994 min. (TIC, mit [MH]⁺ = 478); ¹H NMR (400 MHz, DMSO-d₆) δ 8.35 (d, *J =* 5.3, 1H), 7.62 - 7.56 (m, 2H), 7.50 (d, *J* = 1.9, 1 H), 7.39 (dd, *J* = 7.7, 6.0, 3H), 7.34 - 7.27 (m, 2H), 7.10 (s, 1 H), 6.88 (d, *J* = 8.7, 1 H), 5.99 (s, 2H), 3.36 (s, 3H), 1.72 (s, 6H).

### 2-(4-{5-[4-(4-Amino-3-trifluoromethyl-phenyl)-pyridin-2-yl]-3-methyl-2-oxo-2,3-dihydro-imidazol-1-yl}-phenyl)-2-methyl-propionitrile ("A94")

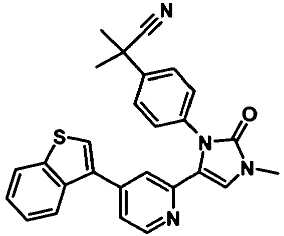

Analytische Daten "A94": DC: R_{f} = 0.58 (Si60, CH₂Cl₂/10% MeOH/10% NH₃); LC-MS: t_{R} = 2.338 min (UV = 220 nm), t_{R} = 2.362 min. (TIC, mit [MH]⁺ = 451); ¹H NMR (400 MHz, DMSO-d₆) δ 8.51 (dd, *J =* 5.1, 0.7, 1 H), 8.12 - 8.05 (m, 1H), 7.83 (s, 1H), 7.65 - 7.56 (m, 3H), 7.49 - 7.39 (m, 4H), 7.37 - 7.32 (m, 2H), 7.15 (d, *J =* 0.7, 1 H), 3.32 (s, 3H), 1.65 (s, 6H).

### 2-Methyl-2-{4-[3-methyl-5-(6-methylaminomethyl-[3,4']bipyridinyl-2'-yl)-2-oxo-2,3-dihydro-imidazol-1-yl]-phenyl}-propionitrile ("A95")

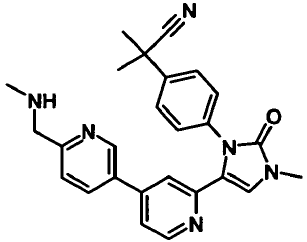

Analytische Daten "A95": LC-MS: t_{R} = 1.284 min (UV = 220 nm), t_{R} = 1.285 min. (TIC, mit [MH]⁺ = 439); ¹H NMR (400 MHz, DMSO-d₆) δ 8.58 (d, *J* = 1.9, 1 H), 8.45 (d, *J* = 5.3, 1 H), 7.85 (dd, *J* = 8.1, 2.4, 1 H), 7.63 - 7.58 (m, 2H), 7.52 - 7.45 (m, 3H), 7.33 - 7.29 (m, 2H), 7.19 - 7.23 (m, 1 H), 3.79 (s, 2H), 3.32 (s, 3H), 2.30 (s, 3H), 1.73 (s, 6H).

### 3-Fluoro-5-{3-methyl-2-oxo-5-[4-(1H-pyrrolo[2,3-b]pyridin-5-yl)-pyridin-2-yl]-2,3-dihydro-imidazol-1-yl}-benzonitrile ("A96")

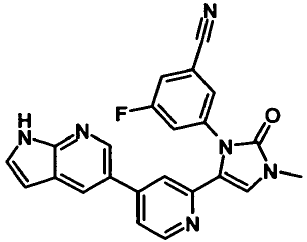

Analytische Daten "A96": DC: R_{f} = 0.15 (Si60, CH₂Cl₂/5% MeOH/1% NH₃); LC-MS: t_{R} = 1.710 min (UV = 220 nm), t_{R} = 1.765 min. (TIC, mit [MH]⁺ = 411); ¹H NMR (400 MHz, DMSO-d₆) δ 11.84 (s, 1 H), 8.64 (d, *J* = 2.2, 1 H), 8.38 (d, *J* = 2.1, 1 H), 8.31 (d, *J* = 5.3, 1 H), 7.88 - 7.80 (m, 2H), 7.65 - 7.61 (m, 2H), 7.57 (dd, *J* = 5.3, 1.8, 2H), 7.54 - 7.50 (m, 1 H), 6.57 (dd, *J* = 3.4, 1.8, 1 H), 3.35 - 3.28 (m, 3H).

### 4-(6-Amino-5-trifluoromethyl-[3,4']bipyridinyl-2'-yl)-3-(4-fluoro-3-trifluoromethyl-phenyl)-1-methyl-1,3-dihydro-imidazol-2-one ("A97")

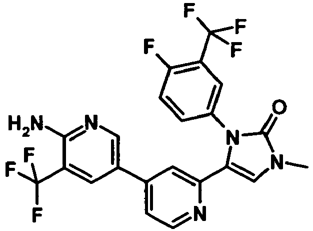

Analytische Daten "A97": DC: R_{f} = 0.37 (Si60, CH₂Cl₂/8% MeOH/1% NH₃); LC-MS: t_{R} = 2.075 min (UV = 220 nm), t_{R} = 2.072 min. (TIC, mit [MH]⁺ = 498); ¹H NMR (400 MHz, DMSO-d₆) δ 8.63 (d, *J* = 2.1, 1 H), 8.30 - 8.27 (m, 1 H), 7.98 (d, *J =* 2.1, 1H), 7.66 (d, *J =* 7.2, 1H), 7.61 - 7.48 (m, 5H), 6.86 (s, 2H), 3.29 (s, 3H).

### 3-{3-Methyl-2-oxo-5-[4-(1H-pyrrolo[2,3-b]pyridin-5-yl)-pyridin-2-yl]-2,3-dihydro-imidazol-1-yl}-5-(4-methyl-piperazin-1-yl)-benzonitrile ("A98")

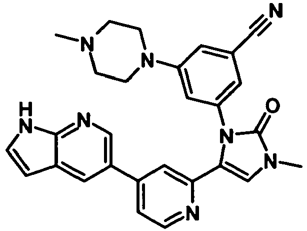

Analytische Daten "A98": DC: R_{f} = 0.36 (Si60, CH₂Cl₂/8% MeOH/1% NH₃); LC-MS: t_{R} = 1.382 min (UV = 220 nm), t_{R} = 1.376 min. (TIC, mit [MH]⁺ = 491); ¹H NMR (400 MHz, DMSO-d₆) δ 11.86 (s, 1 H), 8.49 (d, *J* = 2.2, 1 H), 8.40 (d, *J* = 5.2, 1H), 8.24 (s, 1 H), 8.18 (d, *J =* 2.1, 1 H), 7.59 - 7.55 (m, 1 H), 7.50 (d, *J* = 1.7, 2H), 7.37 (dd, *J* = 2.3, 1.3, 1 H), 7.09 (dt, *J* = 2.9, 1.7, 2H), 6.54 (dd, *J* = 3.4, 1.8, 1 H), 3.32 (s, 3H), 3.20 - 3.13 (m, 4H), 2.38 - 2.31 (m, 4H), 2.16 (s, 3H).

### 2-[4-(5-{4-[3-(2-Amino-6-ethyl-pyrimidin-4-yl)-1H-pyrrolo[2,3-b]pyridin-5-yl]-pyridin-2-yl}-3-methyl-2-oxo-2,3-dihydro-imidazol-1-yl)-phenyl]-2-methyl-propionitrile ("A99")

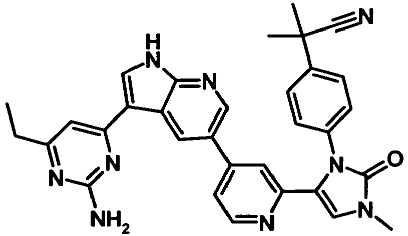

Analytische Daten "A99": LC-MS: t_{R} = 1.413 min (UV = 220 nm), t_{R} = 1.418 min. (TIC, mit [MH]⁺ = 556); ¹H NMR (500 MHz, DMSO-d₆) δ 12.35 (s, 1H), 9.13 (d, *J* = 2.3, 1 H), 8.44 (d, *J* = 5.1, 1 H), 8.39 (s, 1 H), 8.24 (d, *J* = 2.2, 1 H), 7.62 (dd, *J* = 5.2, 1.7, 1 H), 7.59 - 7.56 (m, 2H), 7.48 (s, 1 H), 7.36 - 7.33 (m, 2H), 7.29 (s, 1 H), 7.00 (s, 1 H), 6.53 (s, 2H), 3.32 (s, 3H), 2.56 - 2.51 (q, *J =* 7.6, 2H), 1.66 (s, 6H), 1.23 (t, *J =* 7.6, 3H).

### 2-Methyl-2-(4-{3-methyl-2-oxo-5-[4-(1H-pyrrolo[2,3-b]pyridin-3-yl)-pyridin-2-yl]-2,3-dihydro-imidazol-1-yl}-phenyl)-propionitrile ("A100")

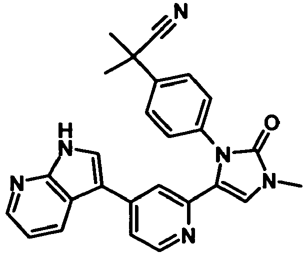

Analytische Daten "A100": LC-MS: t_{R} = 1.475 min (UV = 220 nm), t_{R} = 1.469 min. (TIC, mit [MH]⁺ = 435); ¹H NMR (400 MHz, DMSO-d₆) δ 8.38 - 8.34 (m, 1 H), 8.27 (dd, *J* = 4.6, 1.5, 1 H), 7.96 (s, 1 H), 7.68 (dd, *J* = 8.0, 1.5, 1 H), 7.63 - 7.58 (m, 2H), 7.51 (dd, *J* = 5.3, 1.7, 1 H), 7.38 - 7.31 (m, 3H), 7.21 (d, *J* = 0.9, 1 H), 7.10 (dd, *J =* 8.0, 4.7, 1 H), 3.31 (s, 3H), 1.64 (s, 6H).

### 2-(4-{5-[4-(1-Amino-isoquinolin-4-yl)-pyridin-2-yl]-3-methyl-2-oxo-2,3-dihydro-imidazol-1-yl}-phenyl)-2-methyl-propionitrile ("A101")

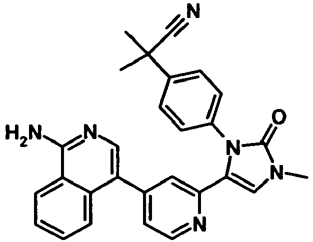

Analytische Daten "A101": LC-MS: t_{R} = 1.431 min (UV = 220 nm), t_{R} = 1.428 min. (TIC, mit [MH]⁺ = 461); ¹H NMR (500 MHz, DMSO-d₆) δ 8.46 (d, *J =* 5.0, 1 H), 8.26 (d, *J* = 8.3, 1 H), 7.65 - 7.51 (m, 6H), 7.40 (s, 1 H), 7.34 - 7.30 (m, 2H), 7.24 (dd, *J =* 5.0, 1.6, 1 H), 7.08 (s, 2H), 6.91 (s, 1 H), 3.30 (s, 4H), 1.65 (s, 6H).

### 4-[4-(1H-Indol-5-yl)-pyridin-2-yl]-1-methyl-3-[4-(4-methyl-piperazin-1-yl)-3-trifluoromethyl-phenyl]-1,3-dihydro-imidazol-2-one ("A102")

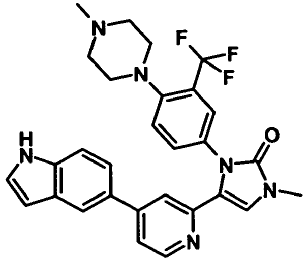

Analytische Daten "A102": DC: R_{f} = 0.42 (Si60, CH₂Cl₂/8% MeOH/1% NH₃); LC-MS: t_{R} = 1.472 min (UV = 220 nm), t_{R} = 1.467 min. (TIC, mit [MH]⁺ = 533); ¹H NMR (500 MHz, DMSO-d₆) δ 11.29 (s, 1 H), 8.37 (d, *J =* 5.2, 1 H), 7.67 (d, *J* = 1.1, 1 H), 7.64 - 7.60 (m, 2H), 7.51 - 7.46 (m, 3H), 7.43 (dd, *J* = 5.4, 2.7, 2H), 7.25 - 7.20 (m, 2H), 6.46 - 6.42 (m, 1 H), 3.32 (s, 4H), 2.94 (t, *J* = 4.6, 4H), 2.65 - 2.54 (m, 3H), 2.32 (s, 4H), 2.28 - 2.25 (m, 1 H), 2.21 (d, *J* = 1.8, 2H).

### 4-(6-Amino-5-trifluoromethyl-[3,4']bipyridinyl-2'-yl)-1-methyl-3-[4-(4-methylpiperazin-1-yl)-3-trifluoromethyl-phenyl]-1,3-dihydro-imidazol-2-one ("A103")

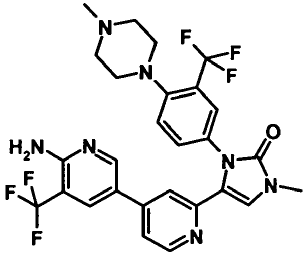

Analytische Daten "A103": DC: R_{f} = 0.21 (Si60, CH₂Cl₂/8% MeOH/1% NH₃); LC-MS: t_{R} = 1.494 min (UV = 220 nm), t_{R} = 1.492 min. (TIC, mit [MH]⁺ = 578).

### 1-Methyl-4-[4-(2-methyl-1H-pyrrolo[2,3-b]pyridin-5-yl)-pyridin-2-yl]-3-(3-phenoxy-phenyl)-1,3-dihydro-imidazol-2-one ("A104")

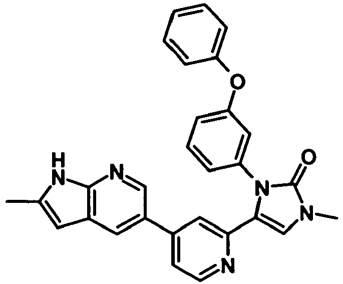

Analytische Daten "A104": LC-MS: t_{R} = 1.956 min (UV = 220 nm), t_{R} = 1.956 min. (TIC, mit [MH]⁺ = 474); ¹H NMR (500 MHz, DMSO-d₆) δ 11.69 (s, 1 H), 8.43 (d, *J* = 5.1, 1 H), 8.30 (d, *J* = 2.2, 1 H), 8.01 (d, *J* = 2.1, 1 H), 7.57 (dd, *J =* 5.2, 1.7, 1 H), 7.48 (t, *J =* 8.1, 1H), 7.40 (s, 1 H), 7.32 (d, *J =* 1.0, 1 H), 7.30 - 7.24 (m, 2H), 7.06 (dddd, *J =* 15.5, 8.3, 2.1, 0.8, 3H), 6.91 - 6.85 (m, 2H), 6.76 (t, *J* = 2.2, 1 H), 6.25 - 6.20 (m, 1 H), 3.30 (s, 3H), 2.43 (s, 3H).

### 4-[4-(3-Fluoro-1 H-pyrrolo[2,3-b]pyridin-5-yl)-pyridin-2-yl]-3-(4-fluoro-3-trifluoromethyl-phenyl)-1-methyl-1,3-dihydro-imidazol-2-one ("A105")

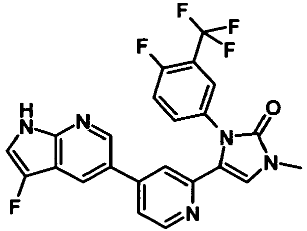

Analytische Daten "A105": DC: R_{f} = 0.15 (Si60, CH₂Cl₂/8% MeOH/1% NH₃); Smp.: 268 °C; LC-MS: t_{R} = 2.051 min (UV = 220 nm), t_{R} = 2.074 min. (TIC, mit [MH]⁺ = 472); ¹H NMR (500 MHz, DMSO-d₆) δ 11.71 (s, 1 H), 8.66 (d, *J* = 2.2, 1 H), 8.36 (d, *J* = 2.1, 1 H), 8.32 (d, *J* = 5.3, 1 H), 7.79 (d, *J* = 0.9, 1 H), 7.69 (d, *J* = 6.9, 1 H), 7.62 (s, 1 H), 7.60 (dd, *J* = 5.2, 1.7, 1 H), 7.57 - 7.54 (m, 3H), 3.34 (s, 3H).

### 1-Methyl-3-[4-(4-pyridin-3-ylmethyl-piperazin-1-yl)-3-trifluoromethyl-phenyl]-4-[4-(1 H-pyrrolo[2,3-b]pyridin-5-yl)-pyridin-2-yl]-1,3-dihydro-imidazol-2-one ("A106")

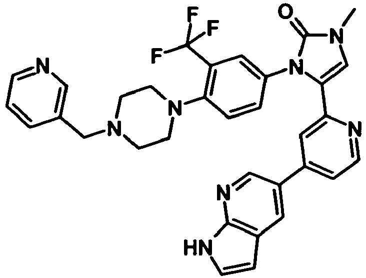

Analytische Daten "A106": DC: R_{f} = 0.28 (Si60, CH₂Cl₂/8% MeOH/1% NH₃); LC-MS: t_{R} = 1.477 min (UV = 220 nm), t_{R} = 1.473 min. (TIC, mit [MH]⁺ = 611); ¹H NMR (500 MHz, DMSO-d₆) δ 11.83 (s, 1 H), 8.54 (t, *J =* 3.1, 1 H), 8.49 (dd, *J* = 4.8, 1.6, 1H), 8.39 (t, *J* = 4.1, 2H), 8.10 (d, *J* = 2.2, 1H), 7.76 (dt, *J* = 7.8, 1.8, 1 H), 7.62 (d, *J* = 8.6, 1 H), 7.58 (d, *J* = 2.5, 1 H), 7.55 (dd, *J* = 5.5, 2.4, 2H), 7.50 - 7.45 (m, 2H), 7.39 (dd, *J* = 7.7, 4.8, 1 H), 7.34 (d, *J* = 0.9, 1 H), 6.47 (d, *J =* 3.4, 1 H), 3.59 (s, 2H), 3.32 (s, 3H), 2.93 - 2.90 (m, 4H), 2.54 (s, breit, 4H).

### 2-(4-{5-[4-(4-Amino-3-fluoro-5-trifluoromethyl-phenyl)-pyridin-2-yl]-3-methyl-2-oxo-2,3-dihydro-imidazol-1-yl}-phenyl)-2-methyl-propionitrile ("A107")

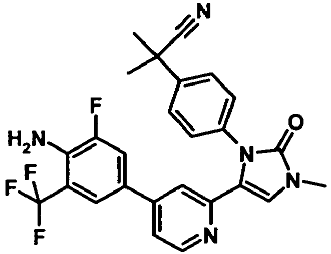

Analytische Daten "A107": LC-MS: t_{R} = 2.117 min (UV = 220 nm), t_{R} = 2.117 min. (TIC, mit [MH]⁺ = 496); ¹H NMR (500 MHz, DMSO-d₆) δ 8.34 (d, *J* = 5.2, 1H), 7.59 - 7.54 (m, 2H), 7.51 - 7.40 (m, 5H), 7.31 - 7.26 (m, 2H), 7.20 (d, *J =* 1.0, 1H), 6.06 (s, 2H), 3.30 (s, 3H), 1.70 (s, 6H).

### 3-(4-Methoxy-benzyl)-1-methyl-4-[4-(1H-pyrrolo[2,3-b]pyridin-5-yl)-pyridin-2-yl]-1,3-dihydro-imidazol-2-one ("A108")

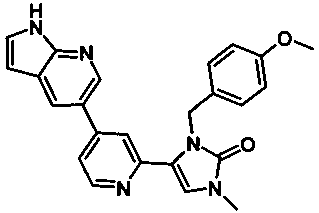

Analytische Daten "A108": DC: R_{f} = 0.33 (Si60, CH₂Cl₂/5% NH₃); LC-MS: t_{R} = 1.933 min (UV = 220 nm), t_{R} = 1.978 min. (TIC, mit [MH]⁺ = 412); ¹H NMR (400 MHz, DMSO-d₆) δ 11.86 (s, 1 H), 8.68 (d, *J =* 2.2, 1 H), 8.56 (d, *J =* 5.3, 1 H), 8.40 (d, *J* = 2.1, 1 H), 7.92 (d, *J* = 0.9, 1 H), 7.60 (dd, *J* = 5.3, 1.7, 1 H), 7.57 - 7.55 (m, 1 H), 7.54 (s, 1 H), 7.11 (d, *J* = 8.7, 2H), 6.82 - 6.75 (m, 2H), 6.54 (dd, *J =* 3.4, 1.8, 1 H), 5.42 (s, 2H), 3.66 (s, 3H), 3.28 (s, 3H); ¹³C NMR (101 MHz, DMSO) δ 157.62, 153.14, 149.54, 148.66, 148.32, 146.20, 140.98, 130.46, 127.92, 126.96, 125.97, 124.22, 120.55, 119.13, 118.19, 116.30, 114.11, 113.09, 99.96, 54.42, 43.87, 29.63.

### 3-(4-Methoxy-benzyl)-1-methyl-4-[4-(1H-pyrazolo[3,4-b]pyridin-5-yl)-pyridin-2-yl]-1,3-dihydro-imidazol-2-one ("A109")

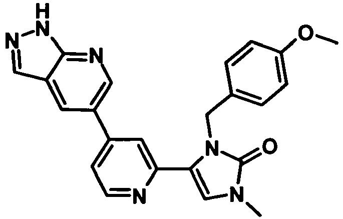

Analytische Daten "A109": DC: R_{f} = 0.15 (Si60, CH₂Cl₂/5% MeOH/10% NH₃); LC-MS: t_{R} = 1.682 min (UV = 220 nm), t_{R} = 1.704 min. (TIC, mit [MH]⁺ = 413); ¹H NMR (300 MHz, DMSO-d₆) δ 13.84 (s, 1H), 8.99 (d, *J =* 2.2, 1H), 8.67 (d, *J* = 2.0, 1 H), 8.59 (d, *J* = 5.3, 1 H), 8.25 (d, *J* = 1.2, 1 H), 7.97 (d, *J* = 0.9, 1 H), 7.62 (dd, *J* = 5.3, 1.7, 1 H), 7.54 (s, 1 H), 7.12 (d, *J* = 8.7, 2H), 6.84 - 6.73 (m, 2H), 5.42 (s, 2H), 3.66 (s, 3H), 3.29 (s, 3H); ¹³C NMR (75 MHz, DMSO) δ 158.12, 153.64, 151.80, 150.14, 149.24, 147.88, 145.63, 133.94, 130.95, 128.43, 126.04, 120.94, 118.80, 117.06, 114.78, 114.45, 113.58, 54.91, 44.40, 30.13.

### 4-(6-Amino-5-trifluoromethyl-[3,4']bipyridinyl-2'-yl)-3-(4-methoxy-benzyl)-1-methyl-1,3-dihydro-imidazol-2-one ("A110")

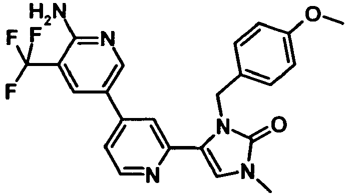

Analytische Daten "A110": LC-MS: t_{R} = 1.973 min (UV = 220 nm), t_{R} = 2.027 min. (TIC, mit [MH]⁺ = 456); ¹H NMR (300 MHz, DMSO-d₆) δ 8.71 (d, *J* = 2.0, 1 H), 8.51 (d, *J =* 5.4, 1 H), 8.16 (d, *J* = 2.0, 1 H), 7.83 (d, *J* = 0.9, 1 H), 7.53 (dd, *J* = 5.3, 1.7, 1 H), 7.49 (s, 1 H), 7.08 (d, *J* = 8.7, 2H), 6.84 (s, 2H), 6.78 (d, *J =* 2.1, 1 H), 6.75 (d, *J =* 2.0, 1 H), 5.39 (s, 2H), 3.65 (s, 3H), 3.27 (s, 3H).

### N-{2'-[3-(4-Methoxy-benzyl)-1-methyl-2-oxo-2,3-dihydro-1H-imidazol-4-yl]-5-trifluoromethyl-[3,4']bipyridinyl-6-yl}-acetamide ("A111")

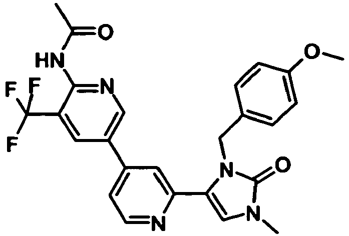

Analytische Daten "A111": DC: R_{f} = 0.26 (Si60, EtOAc/10% MeOH/5% NH₃); LC-MS: t_{R} = 1.795 min (UV = 220 nm), t_{R} = 1.799 min. (TIC, mit [MH]⁺ = 498); ¹H NMR (400 MHz, DMSO-d₆) δ 10.35 (s, 1H), 9.18 (d, *J =* 2.1, 1H), 8.63 (d, *J* = 5.3, 1 H), 8.60 (d, *J =* 2.2, 1H), 8.03 (d, *J =* 0.8, 1 H), 7.69 (dd, *J =* 5.3, 1.7, 1 H), 7.56 (s, 1 H), 7.10 (d, *J =* 8.7, 2H), 6.80 - 6.75 (m, 2H), 5.42 (s, 2H), 3.65 (s, 3H), 3.29 (s, 3H), 2.09 (s, 3H).

### 4-[4-(2-tert-Butyl-1H-pyrrolo[2,3-b]pyridin-5-yl)-pyridin-2-yl]-3-[3-(1,1-difluoro-ethyl)-4-fluoro-phenyl]-1-methyl-1,3-dihydro-imidazol-2-one ("A112")

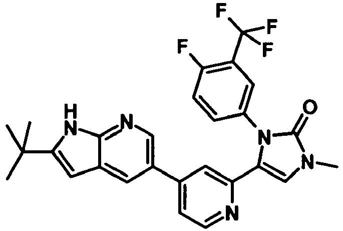

Analytische Daten "A112": DC: R_{f} = 0.35 (Si60, CH₂Cl₂/5% EtO); LC-MS: t_{R} = 2.378 min (UV = 220 nm), t_{R} = 2.363 min. (TIC, mit [MH]⁺ = 510); ¹H NMR (400 MHz, DMSO-d₆) δ 11.75 (s, 1 H), 8.48 (d, J = 2.2 Hz, 1 H), 8.31 (d, J = 5.2 Hz, 1 H), 8.14 (d, J = 2.1 Hz, 1 H), 7.73 - 7.66 (m, 2H), 7.54 (ddd, J = 7.0, 5.9, 1.5 Hz, 4H), 6.20 (d, J = 2.1 Hz, 1 H), 3.33 (s, 3H), 1.38 (s, 9H).

### N-(2-Methyl-2-{4-[3-methyl-2-oxo-5-(4-quinolin-3-yl-pyridin-2-yl)-2,3-dihydro-imidazol-1-yl]-phenyl}-propyl)-acetamid ("A113")

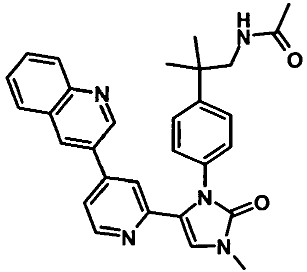

### 2'-[1-Methyl-2-oxo-3-(4-piperazin-1-yl-3-trifluoromethyl-phenyl)-2,3-dihydro-1H-imidazol-4-yl]-1 H-[3,4']bipyridinyl-6-on ("A114")

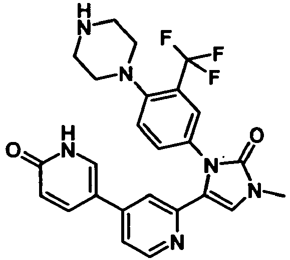

### N-(2'-{3-[4-(Cyano-dimethyl-methyl)-phenyl]-1-methyl-2-oxo-2,3-dihydro-1H-imidazol-4-yl}-[3,4']bipyridinyl-6-yl)-acetamid ("A115")

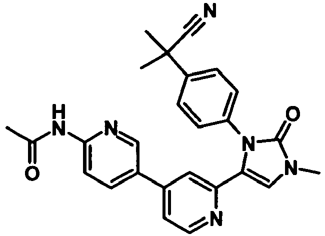

### 2-Methyl-2-(4-{3-methyl-2-oxo-5-[5-(1H-pyrrolo[2,3-b]pyridin-5-yl)-pyridin-3-yl]-2,3-dihydro-imidazol-1-yl}-phenyl)-propionitril ("A116")

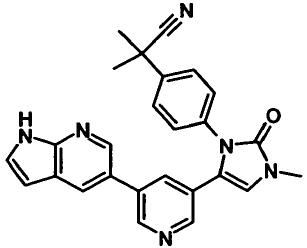

### 2-(4-{3-Methyl-2-oxo-5-[4-(1H-pyrrolo[2,3-b]pyridin-5-yl)-pyridin-2-yl]-2,3-dihydro-imidazol-1-yl}-phenyl)-propionitril ("A117")

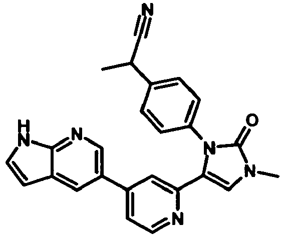

### 1-Methyl-3-(4-piperazin-1-yl-3-trifluoromethyl-phenyl)-4-[4-(3-trifluoromethyl-1 H-pyrrolo[2,3-b]pyridin-5-yl)-pyridin-2-yl]-1 ,3-dihydro-imidazol-2-on ("A118")

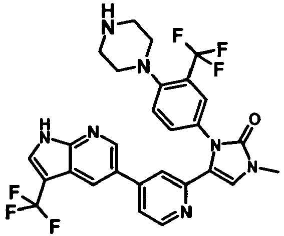

### 1-Methyl-3-(4-piperazin-1-yl-phenyl)-4-[4-(1 H-pyrrolo[2,3-b]pyridin-5-yl)-pyridin-2-yl]-1,3-dihydro-imidazol-2-on ("A119")

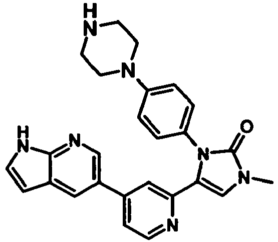

### 1-Methyl-3-(4-piperidin-4-yl-phenyl)-4-[4-(1H-pyrrolo[2,3-b]pyridin-5-yl)-pyridin-2-yl]-1,3-dihydro-imidazol-2-on ("A120")

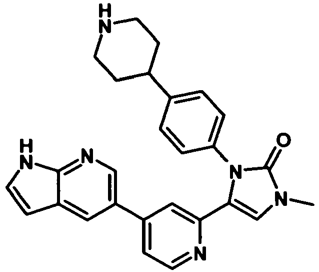

### 3-Benzyl-1-methyl-4-[4-(1H-pyrrolo[2,3-b]pyridin-5-yl)-pyridin-2-yl]-1,3-dihydro-imidazol-2-on ("A121 ")

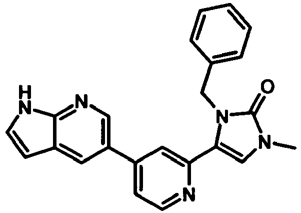

### 3-(4-tert-Butyl-phenyl)-1-methyl-4-[4-(1H-pyrrolo[2,3-b]pyridin-5-yl)-pyridin-2-yl]-1,3-dihydro-imidazol-2-on (A122")

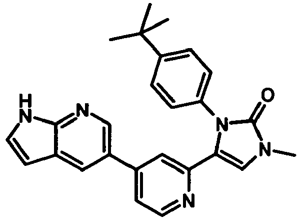

### 1-Methyl-3-{4-[methyl-(2-methylamino-ethyl)-amino]-3-trifluoromethyl-phenyl}-4-[4-(1 H-pyrrolo[2,3-b]pyridin-5-yl)-pyridin-2-yl]-1,3-dihydro-imidazol-2-on ("A123")

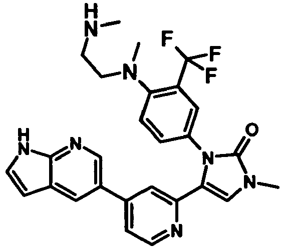

### 3-[4-(2-Dimethylamino-ethylamino)-3-trifluoromethyl-phenyl]-1-methyl-4-[4-(1 H-pyrrolo[2,3-b]pyridin-5-yl)-pyridin-2-yl]-1,3-dihydro-imidazol-2-on ("A124")

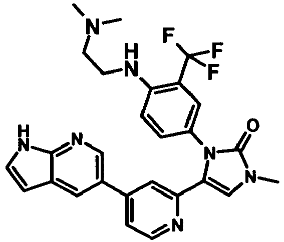

### 3-[4-(4-Amino-piperidin-1-yl)-3-trifluoromethyl-phenyl]-1-methyl-4-[4-(1H-pyrrolo[2,3-b]pyridin-5-yl)-pyridin-2-yl]-1,3-dihydro-imidazol-2-on ("A125")

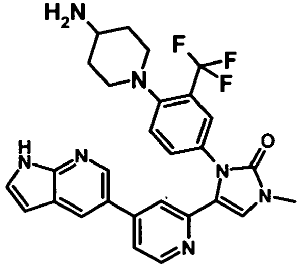

### 1-Methyl-3-[4-(methyl-piperidin-4-yl-amino)-3-trifluoromethyl-phenyl]-4-[4-(1H-pyrrolo[2,3-b]pyridin-5-yl)-pyridin-2-yl]-1,3-dihydro-imidazol-2-on ("A126")

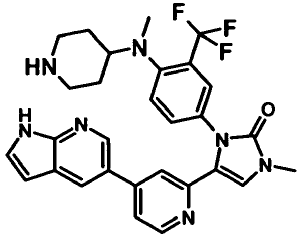

### 1-Methyl-3-[4-(piperidin-4-ylamino)-3-trifluoromethyl-phenyl]-4-[4-(1H-pyrrolo[2,3-b]pyridin-5-yl)-pyridin-2-yl]-1,3-dihydro-imidazol-2-on ("A127")

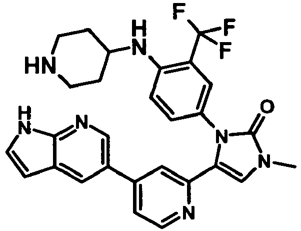

Zum Nachweis der PI3K Inhibitoraktivität der Pyridinylimidazolon Strukturklasse kann ein biochemischer PI3K Assay, ein BIACORE PI3K Bindungsassay und ein zellulärer PI3K Assay verwendet werden.

### PI3K Assay

Die Wirksamkeit der erfindungsgemäßen Verbindungen bei der Inhibierung der PI3K-induzierten Lipidphosphorylierung kann im folgenden Bindungsassay untersucht werden. Der Assay kombiniert die Szintillationsproximitätsassay(SPA)-Technologie (Amersham) mit der Fähigkeit von Neomycin (einem polykationischen Antibiotikum), Phospholipide mit hoher Affinität und Spezifizität zu binden. Der Szintillationsproximitätsassay basiert auf den Eigenschaften von schwach emittierenden Isotopen (wie z. B. ³H, ²⁵I, ³³P). Das Beschichten von SPA-Kugeln mit Neomycin erlaubt den Nachweis von phosphorylierten Lipidsubstraten nach Inkubation mit rekombinantem PI3K und radioaktivem ATP im gleichen Well durch Einfang der radioaktiven Phospholipide durch die SPA-Kugel aufgrund ihrer spezifischen Bindung an Neomycin. Zu einer 96-Well-MTP, die 10 µl der Testverbindung der Formel (I) enthält (gelöst in 10 % DMSO, um eine Konzentration von 100, 25, 5,0, 1,25, 0,312, 0,078, 0,0195, 0,00488, 0,00122 bzw. 0,0003 µM der Testverbindung zu ergeben) wurden die folgenden Assay-Bestandteile gegeben: 1)10 µL Lipidmizellen 2) 20 mL Kinasepuffer ([³³P]γATP162 µM/300 nCi, MgCl₂ 2,5 mM, DTT 2,5 mM, Na₃VO₄ 25 µM in Hepes 40 mM, pH 7,4) und 3) 10 µL (100 ng) humanes, rekombinantes GST-PI3K (in Hepes 40 mM, pH 7,4, Ethylenglykol 4 %). Nach 120minütiger Inkubation bei Raumtemperatur unter leichtem Rühren wurde die Reaktion durch Zugabe von 200 µL einer Lösung gestoppt, die 250 µg mit Neomycin beschichteter PVT-SPA-Kugeln, ATP 60 mM und EDTA 6,2 mM in PBS enthielt. Der Assay wird weitere 60 Minuten bei Raumtemperatur unter leichtem Rühren inkubiert, um eine Bindung der Phospholipide an die Neomycin-SPA-Kugeln zu ermöglichen. Nach dem Ausfällen der mit Neomycin beschichteten PVT-SPA-Kugeln über 5 Minuten bei 1500 x g wird radioaktives Ptdlns(3)P durch Szintillationszählung in einem Wallac MicroBeta™ Plattenzähler quantifiziert.

**Ergebnisse biochemischer PI3Kalpha und PI3Kbeta Assay**

| Beispiel | | IC50 PI3Kalpha [µM] | IC50 PI3Kbeta [µM] |
|---|---|---|---|
| A1 | | B | B |
| A3 | | B | C |
| A4 | | B | B |
| A5 | | B | B |
| A6 | | B | B |
| A7 | | A | B |
| A8 | | A | B |
| A9 | | B | B |
| A10 | | B | B |
| A11 | | B | C |
| A12 | | A | B |
| A13 | | C | C |
| A14 | | B | B |
| A15 | | B | B |
| A16 | | B | B |
| A17 | | B | B |
| A18 | | B | B |
| A19 | | B | B |
| A20 | | B | B |
| A21 | | B | B |
| A22 | | B | C |
| A23 | | B | C |
| A24 | | B | B |
| A25 | | B | A |
| A26 | | B | C |
| A27 | | B | C |
| A28 | | A | B |
| A29 | | A | B |
| A30 | | B | C |
| A31 | | B | B |
| A32 | | A | B |
| A33 | | B | B |
| A34 | | A | B |
| A35 | | B | B |
| A36 | | B | C |
| A37 | | B | B |
| A38 | | A | B |
| A40 | | A | A |
| A41 | | A | B |
| A42 | | B | B |
| A43 | | A | B |
| A44 | | B | B |
| A45 | | A | B |
| A46 | | A | B |
| A47 | | B | B |
| A48 | | B | B |
| A49 | | B | B |
| A50 | | B | B |
| A51 | | B | C |
| A52 | | B | B |
| A53 | | B | C |
| A54 | | B | B |
| A55 | | B | B |
| A56 | | B | B |
| A57 | | B | B |
| A58 | | B | B |
| A59 | | B | B |
| A60 | | B | C |
| A61 | | B | C |
| A62 | | C | C |
| A63 | | A | B |
| A64 | | B | B |
| A65 | | B | B |
| A66 | | C | C |
| A67 | | B | B |
| A68 | | B | C |
| A69 | | C | C |
| A70 | | B | B |
| A71 | | B | B |
| A72 | | B | B |
| A73 | | B | B |
| A74 | | B | B |
| A75 | | B | C |
| A76 | | C | C |
| A77 | | B | B |
| A78 | | B | B |
| A79 | | B | B |
| A80 | | B | B |
| A81 | | B | B |
| A82 | | B | B |
| A83 | | B | B |
| A84 | | A | B |
| A85 | | B | B |
| A86 | | B | B |
| A87 | | B | B |
| A88 | | B | B |
| A89 | | B | C |
| A90 | | B | B |
| A91 | | B | C |
| A92 | | B | B |
| A93 | | B | B |
| A94 | | C | C |
| A95 | | C | C |
| A96 | | B | B |
| A97 | | B | B |
| A98 | | B | B |
| A99 | | B | B |
| A100 | | B | B |
| A101 | | B | B |
| A102 | | B | |
| A103 | | B | B |
| A104 | | B | |
| A108 | | C | C |
| A109 | | B | B |
| A110 | | B | B |
| A111 | | C | C |

| | | | |
|---|---|---|---|
| IC₅₀: 0,1 nM - 0,1 µM = A 0,1 µM - 10µM = B > 10 µM = C | | | |

### BIACORE PI3K Bindungsassay

SPR Messungen der Bindungsaffinitäten und Bindungskinetiken auf PI3Kalpha Oberflächen mit Hilfe von Biacore Systemen. Die Experimente werden auf einen Biacore S51 "Compound Characterization" oder einen Biacore T100 Gerät durchgeführt unter Nutzung der Default Application "Kinetics/Affinity" Versuchsbedingungen: Recombinantes PI3Kalpha (verkürzte Form der katalytischen Unterheit, fehlender N-Terminus von ca. 100 bis 150 aa, M_{w} ca. 110 kDa, hergestellt von Proteros Biostructures GmbH) wird auf Biacore CM5 Chips immobilisiert (25°C, Flussrate: 10 µL/min, unter Benutzung von Aminkupplung bei pH 7.40 - Biacore Standard Protokoll). PI3Kalpha wird in Konzentrationen zwischen 10 und 25 µg/mL eingesetzt. Abhängig von der Dauer der Injektionszeit werden Immobilizationsspiegel von 1700 bis 11000 RU erreicht._Die zu vermessenden Substanzen werden in Form einer Titrationserie zugeführt. Die Substanzkonzentration wird bei jeder nachfolgenden Injektion verdoppelt. Üblicherweise werden 10 unterschiedliche Substanzkonzentrationen injeziert, die einen 500-fachen Verdünnungsbereich umfassen. Vor und nach jeder Titrationserie wird die Bindungsfähigkeit der Oberfläche kontrolliertg durch Injektion einer Positivkontrolle (Substanz MSC2036175A), bei einer fixierten Konzentration von 125 nM. Kinetische Titrationsexperimente werden bei 25°C mit einer Flussrate von 30 µL/min durchgeführt, die Probenkontaktzeit beträgt 180 s und die Dissoziationszeit 240 s in einem laufenden Puffer (20 mM HEPES pH7.40, 150 mM NaCl, 5 mM MgCl₂, 1 mM DTT, 0.1 mM EDTA, 0.05% Tween 20), der 2% DMSO enthält. Pufferinjektionen (als doppelte Referenzen) identisch zu den Probenijektionen werden jeweils am Beginn einer fortlaufenden Serie durchgeführt. Lösungsmittelkorrektionzyklen (acht Korrekturpunkte, 1.4% - 2.8% DMSO) werden in gleichen abständen durchgeführt. Um die Oberfläche zu konditionieren werden mindestens fünf Start-up Zyklen (Pufferinjektionen) durchgeführt. Datapunkte werden mit einer Samplerate von 10 Hz gesammelt. Eine Oberflächenregenerierung wird nach jedem Messzyklus durchgeführt, ausser nach den Lösungsmittelkorrekturläufen. Zu diesem Zeck werden zweimal 100 mM NaHCO₃ pH 8.60 für 30 sec injeziert._Datensets werden mit der Software Biacore S51 Evaluation, Version 1.2.1 oder Biacore T100 Evaluation, Version 2.0 prozessiert und analysiert. Lösungsmittelkorregierte und doppelt-referenzierte Assoziations- und Dissoziationsphasendaten werden zu einem einfachen 1:1 Interaktion Modell mit Massentransportlimitationen angepasst.

**Ergebnisse PI3Kalpha Biacore-Bindungsassay:**

| Beispiel | KD (Bindung) |
|---|---|
| A1 | B |
| A2 | |
| A3 | B |
| A4 | B |
| A5 | B |
| A6 | B |
| A7 | A |
| A8 | A |
| A9 | B |
| A10 | B |
| A11 | |
| A12 | A |
| A13 | B |
| A14 | B |
| A15 | B |
| A16 | B |
| A17 | B |
| A18 | A |
| A19 | B |
| A20 | B |
| A21 | A |
| A22 | B |
| A23 | B |
| A24 | A |
| A25 | A |
| A26 | B |
| A27 | B |
| A28 | A |
| A29 | A |
| A30 | |
| A31 | A |
| A32 | A |
| A33 | A |
| A36 | B |
| A37 | A |
| A38 | A |
| A40 | A |
| A41 | A |
| A42 | A |
| A43 | A |
| A44 | A |
| A46 | A |
| A48 | B |
| A49 | A |
| A50 | A |
| A52 | A |
| A53 | B |
| A55 | B |
| A56 | A |
| A57 | B |
| A58 | B |
| A59 | A |
| A60 | B |
| A62 | C |
| A63 | A |
| A64 | A |
| A65 | A |
| A66 | C |
| A67 | B |
| A69 | B |
| A70 | |
| A71 | A |
| A72 | A |
| A73 | A |
| A83 | A |
| A84 | A |
| A85 | A |
| A86 | B |
| A87 | B |
| A88 | B |
| A89 | B |
| A90 | A |
| A91 | B |
| A92 | B |
| A93 | A |
| A94 | B |
| A95 | |
| A96 | B |
| A97 | B |
| A98 | B |
| A99 | B |
| A108 | B |
| A109 | B |
| A110 | B |

| | |
|---|---|
| KD: 0,1 nM - 0,1 µM = A 0,1 µM - 10 µM = B > 10 µM =C | |

### Zellulärer PI3K Assay

**Versuchsbedingungen:** Als Maß für die zelluläre PI3K Aktivität wird die PI3K-abhängige Phosphorylierung von PKB an Serin 473 verwendet. Der zelluläre Assay zur Bestimmung der P-S473-PKB Level wird als Luminex-Assay im 96-well Format in PC3 Zellen durchgeführt. PC3 Zellen weisen aufgrund einer PTEN Mutation eine konstitutive Phosphorylierung von PKB auf. PC3 Zellen werden mit 20.000 Zellen pro well in 100 µl Medium (45% RPMI1460 / 45% Ham's F12 / 10% FCS) ausgesät und am folgenden Tag für 30 min mit einer seriellen Verdünnung der Prüfsubstanz (7 Konzentrationen) unter serumfreien Bedingungen inkubiert. Im Anschluss werden die Zellen mit 90 µl Lysepuffer (20mM Tris/HCl pH 8,0, 150mM NaCl, 1% NP40, 10% Glycerol, 1% Phosphatase-Inhibitor I, 1% Phosphatase-Inhibitor II, 0,1% Protease-Inhibitor Cocktail III, 0,01 % Benzonase) pro well lysiert, und die Lysate werden mittels Zentrifugation durch eine 96-well Filterplatte (0,65 µm) von unlöslichen Zellbestandteilen abgetrennt. Die Lysate werden üN bei 4°C mit Luminex-Beads, an die ein anti-total PKB Antikörper gekoppelt ist, unter Schütteln inkubiert. Am folgenden Tag erfolgt die Detektion durch Zugabe eines P-S473-PKB Antikörpers sowie eines speziesspezifischen PEmarkierten Sekundärantikörpers. Der Nachweis von P-S473-PKB erfolgt durch Messung im Luminex100 Gerät durch Bestimmung von 100 Ereignissen pro Kavität in 60 sec Messzeit. Als pharmakologischer Blank werden die erhaltenen Signale von Zellen, die mit 3 µM Wortmannin behandelt wurden, von allen anderen Ansätzen abgezogen. Als Kontrollwert der maximalen Phosphorylierung von PKB an S473 werden die Signale von Zellen, die nur mit dem Lösungsmittel (0,3% DMSO) behandelt wurden, verwendet. Die Werte der mit Prüfsubstanz behandelten Ansätze werden hiervon als Prozent von Kontrolle berechnet und IC50 Werte werden mittels RS1 ermittelt.

**Ergebnisse PI3K-Zell Inhibierung**

| Beispiel | IC50 (Zelle) |
|---|---|
| A1 | B |
| A2 | |
| A3 | C |
| A4 | B |
| A5 | B |
| A6 | C |
| A7 | B |
| A8 | B |
| A9 | B |
| A10 | C |
| A11 | C |
| A12 | A |
| A13 | C |
| A14 | B |
| A15 | B |
| A16 | C |
| A17 | B |
| A18 | A |
| A19 | B |
| A20 | B |
| A21 | C |
| A22 | C |
| A23 | B |
| A24 | B |
| A25 | B |
| A26 | C |
| A27 | B |
| A28 | B |
| A29 | B |
| A30 | C |
| A31 | B |
| A32 | B |
| A33 | C |
| A34 | B |
| A35 | B |
| A36 | C |
| A37 | B |
| A38 | B |
| A39 | C |
| A40 | A |
| A41 | B |
| A42 | B |
| A43 | B |
| A44 | B |
| A45 | B |
| A46 | B |
| A47 | B |
| A48 | B |
| A49 | B |
| A50 | B |
| A51 | B |
| A52 | B |
| A53 | B |
| A54 | B |
| A55 | B |
| A56 | B |
| A57 | B |
| A58 | B |
| A59 | B |
| A60 | B |
| A61 | C |
| A62 | C |
| A63 | B |
| A64 | B |
| A65 | B |
| A66 | C |
| A67 | B |
| A68 | B |
| A69 | C |
| A70 | B |
| A71 | A |
| A72 | A |
| A73 | B |
| A74 | A |
| A75 | B |
| A76 | C |
| A77 | A |
| A78 | B |
| A79 | B |
| A80 | B |
| A81 | C |
| A82 | B |
| A83 | B |
| A84 | B |
| A85 | B |
| A86 | B |
| A87 | B |
| A88 | B |
| A89 | B |
| A90 | A |
| A91 | C |
| A92 | C |
| A93 | B |
| A94 | B |
| A95 | C |
| A96 | B |
| A97 | B |
| A98 | C |
| A99 | B |
| A100 | B |
| A101 | B |
| A102 | B |
| A103 | B |
| A104 | B |
| A105 | B |
| A106 | A |
| A107 | B |
| A108 | B |
| A109 | B |
| A110 | B |
| A111 | C |

| | |
|---|---|
| IC₅₀: 0,1 nM - 0,1 µM = A 0,1 µM - 10 µM = B > 10 µM = C | |

### DNA-PK-inhibitorische Aktivität

### DNA-PK Assay:

Der Kinaseassay wird in 348-Well Mikrotiter FlashPlates®, beschichtet mit Streptavidin, durchgeführt. In einem Well, das 500 ng DNA aus Kalbsthymus, 0.1 µCi 33P-ATP und 1.8 % DMSO enthält, werden 1,5 µg des DNA-PK Proteinkomplexes und 100 mg biotinyliertes Substrat, z.B. PESQEAFADLWKK Biotin-NH2 ("biotin-DNA-PK-peptide") in einem Gesamtvolumen von 36,5 µL (34.25 mM Hepes/KOH, 7.85 mM Tris-HCl, 68.5 mM KCl, 5 µM ATP, 6.85 mM MgCl2, 0.5 mM EDTA, 0.14 mM EGTA, 0.69 mM DTT, pH 7.4) mit oder ohne Testsubstanz für 90 Minuten bei Raumtemperatur inkubiert. Die Reaktion wird durch Zugabe von 50 µL/Well 200 mM EDTA gestoppt. Nach 30 Minuten Inkubation werden die Flüssigkeiten bei Raumtemperatur entfernt. Jedes Well wird dreimal mit 100 µL 0.9 % NaCl-Lösung gewaschen. Nicht-spezifische Reaktion (Blank) wird mit einem proprietären Kinaseinhibitor (10 µM) bestimmt. Die Radioaktitvität wird mittels einem Topcount gemessen. IC50-Werte in RS1 kalkuliert. Literatur: Molecular Cancer Therapeutics 2003, 1257-1264; DNA-dependent protein kinase inhibitors as drug candidates for the treatment of cancer; A. Kashishian, H. Douangpanya, D. Clark, S. T. Schlachter, C. Todd Eary, J. G. Schiro, H. Huang, L. E. Burgess, E. A. Kesicki, and J. Halbrook.

**Ergebnisse biochemischer DNA-PK Assay**

| Beispiel | IC50 DNA-PK |
|---|---|
| A1 | A |
| A2 | B |
| A3 | A |
| A4 | B |
| A5 | B |
| A6 | B |
| A7 | B |
| A8 | B |
| A9 | A |
| A10 | A |
| A11 | A |
| A12 | A |
| A13 | A |
| A14 | B |
| A15 | B |
| A16 | B |
| A17 | B |
| A18 | B |
| A19 | B |
| A20 | A |
| A21 | A |
| A22 | B |
| A23 | A |
| A24 | A |
| A25 | B |
| A26 | A |
| A27 | A |
| A28 | A |
| A29 | A |
| A30 | B |
| A31 | A |
| A32 | A |
| A33 | A |
| A34 | A |
| A35 | A |
| A36 | A |
| A37 | A |
| A38 | B |
| A39 | A |
| A40 | A |
| A41 | A |
| A42 | A |
| A43 | A |
| A44 | A |
| A45 | A |
| A46 | A |
| A47 | A |
| A48 | A |
| A49 | A |
| A50 | A |
| A51 | A |
| A52 | A |
| A53 | A |
| A54 | A |
| A55 | A |
| A56 | B |
| A57 | B |
| A58 | B |
| A59 | A |
| A60 | A |
| A61 | A |
| A62 | A |
| A63 | A |
| A64 | A |
| A65 | A |
| A66 | C |
| A67 | A |
| A68 | B |
| A69 | C |
| A70 | B |
| A71 | A |
| A72 | A |
| A73 | A |
| A74 | A |
| A75 | A |
| A76 | A |
| A77 | A |
| A78 | B |
| A79 | A |
| A80 | A |
| A81 | A |
| A82 | A |
| A83 | B |
| A84 | A |
| A85 | A |
| A86 | A |
| A87 | A |
| A88 | B |
| A89 | B |
| A90 | A |
| A91 | A |
| A92 | A |
| A93 | A |
| A94 | B |
| A95 | B |
| A96 | A |
| A97 | B |
| A98 | B |
| A99 | A |
| A100 | B |
| A101 | B |
| A102 | B |
| A103 | B |
| A104 | A |
| A105 | B |
| A106 | B |
| A107 | A |
| A108 | A |
| A109 | A |
| A110 | B |
| A111 | C |

| | |
|---|---|
| IC₅₀: 0,1 nM - 0,1 µM = A 0,1 µM - 10 µM = B > 10 µM = C | |

### mTOR inhibitorische Aktivität

Der biochemische mTOR Assay wurde nach dem im Millipore mTOR Assay Kit (Katalognummer 14-770) beschriebenen Verfahren durchgeführt.

| Beispiel | IC50.mTOR |
|---|---|
| A1 | A |
| A3 | C |
| A9 | B |
| A12 | B |
| A13 | B |
| A20 | B |
| A21 | B |
| A22 | B |
| A23 | B |
| A24 | A |
| A26 | B |
| A27 | A |
| A31 | B |
| A32 | A |
| A34 | B |
| A35 | B |
| A36 | B |
| A39 | B |
| A41 | B |
| A43 | B |
| A44 | B |
| A46 | B |
| A47 | B |
| A49 | B |
| A50 | B |
| A51 | B |
| A52 | B |
| A55 | B |
| A60 | C |
| A61 | B |
| A63 | A |
| A72 | B |
| A73 | A |
| A74 | A |
| A75 | B |
| A76 | A |
| A77 | C |
| A79 | B |
| A80 | C |
| A85 | B |
| A86 | B |
| A87 | B |
| A91 | B |
| A92 | B |
| A93 | B |
| A96 | B |

| | |
|---|---|
| IC₅₀: 0,1 nM - 0,1 µM = A 0,1 µM - 10 µM = B > 10 µM = C | |

### Die nachfolgenden Beispiele betreffen Arzneimittel:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ • 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 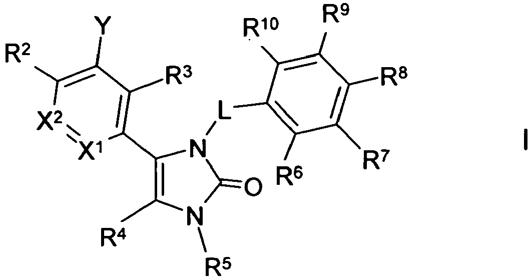 worin
X¹ CR¹ oder N,
X² CR¹ oder N,
wobei X¹ oder X² N
und das jeweils andere CR¹ bedeutet,
Y Het oder Ar,
L [C(R^{a})₂]ₘ,
R¹ R², R³, R⁴ H,
R⁵ H oder A',
R⁶, R⁹, R¹⁰ H,
R⁷ H oder A"',
R⁸ H, A", Het¹, CN, Hal, NHHet¹ oder NA'Het¹,
A unverzweigtes oder verzweigtes Alkyl mit 1-10 C-Atomen, worin 1-7 H-Atome durch F und/oder Cl ersetzt sein können, oder
cyclisches Alkyl mit 3-7 C-Atomen,
A' unverzweigtes oder verzweigtes Alkyl mit 1-6 C-Atomen, worin 1-5 H-Atome durch F und/oder Cl ersetzt sein können,
A" unverzweigtes oder verzweigtes Alkyl mit 1-10 C-Atomen, worin 1-7 H-Atome durch OH, CN, NH₂, CONH₂, COOH, COOA, NHCOA, -C=H, F, Cl und/oder Br ersetzt sein können, und/oder worin eine oder zwei nicht-benachbarte CH- und/oder CH₂-Gruppen durch O, NH, NA', S, SO, SO₂ und/oder CH=CH-Gruppen ersetzt sein können,
A'" unverzweigtes oder verzweigtes Alkyl mit 1-10 C-Atomen, worin 1-7 H-Atome durch F und/oder Cl ersetzt sein können, und/oder worin eine oder zwei nicht-benachbarte CH- und/oder CH₂-Gruppen durch O, NH und/oder NA' ersetzt sein können,
R^{a} H oder A',
Het Pyridinyl, Pyrimidinyl, Furyl, Thienyl, Oxazolyl, Oxadiazolyl, Imidazolyl, Pyrrolyl, Isoxazolyl, Benzodioxolyl, Dihydrobenzofuranyl, Dihydrobenzodioxinyl, Benzofuranyl, Benzodioxanyl, Indolyl, Chinolinyl, Isochinolinyl, Benzimidazolyl, Benzothiadiazolyl, Indazolyl, Dihydrobenzimidazolyl, Dihydroindolyl, Imidazo-pyridinyl, Pyrrolo-pyridinyl, Pyrazolo-pyridinyl, 2,3-Dihydro-pyrrolopyridinyl oder Tetrahydro-naphthyridinyl, wobei die Reste auch ein-, zwei- oder dreifach durch A, (CH₂)ₙNH₂, (CH₂)ₙNHA, (CH₂)ₙNA₂, NHCOA, (CH₂)ₙCONH₂, (CH₂)ₙCONHA, (CH₂)ₙCONA₂, CN, =O, Hal, (CH₂)ₙOH, (CH₂)ₙOA, COOH und/oder COOA substituiert sein können,
Ar unsubstituiertes oder ein-, zwei- oder dreifach durch (CH₂)ₙOH und/oder (CH₂)ₙOA substituiertes Phenyl,
Het¹ Piperidinyl, Pyrrolidinyl, Morpholinyl, Piperazinyl, Oxazolidinyl, Tetrahydrothienyl, Tetrahydropyranyl oder Thiomorpholinyl,
wobei die Reste auch ein- oder zweifach durch =O, NH₂, Pyridylmethyl und/oder A substituiert sein können,
Hal F, Cl, Br oder I,
m 0 oder 1,
n 0, 1, 2, 3 oder 4,
bedeuten, sowie ihre pharmazeutisch verwendbaren Salze, Tautomere und Stereoisomere, einschließlich deren Mischungen in allen Verhältnissen.

2. Verbindungen nach Anspruch 1, ausgewählt aus der Gruppe
2-Methyl-2-{4-[3-methyl-2-oxo-5-(4-chinolin-3-yl-pyridin-2-yl)-2,3-dihydro-imidazol-1-yl]-phenyl}-propionitril ("A1")
2-Methyl-2-{4-[3-methyl-2-oxo-5-(5-chinolin-3-yl-pyridin-3-yl)-2,3-dihydro-imidazol-1-yl]-phenyl}-propionitril ("A2")
2-(4-{5-[4-(3-Methoxy-phenyl)-pyridin-2-yl]-3-methyl-2-oxo-2,3-dihydro-imidazol-1-yl}-phenyl)-2-methyl-propionitril ("A3")
5-(6-Methoxy-[3,4']bipyridinyl-2'-yl)-1-(4-piperazin-1-yl-3-trifluormethyl-phenyl)-1,3-dihydro-imidazol-2-on ("A4")
1-(4-Piperazin-1-yl-3-trifluormethyl-phenyl)-5-(4-chinolin-3-yl-pyridin-2-yl)-1,3-dihydro-imidazol-2-on ("A5")
2-{4-[5-(2'-Methoxy-[4,4']bipyridinyl-2-yl)-3-methyl-2-oxo-2,3-dihydro-imidazol-1-yl]-phenyl}-2-methyl-propionitril ("A6")
2-Methyl-2-(4-{3-methyl-5-[4-(2-methyl-3H-imidazo[4,5-b]pyridin-6-yl)-pyridin-2-yl]-2-oxo-2,3-dihydro-imidazol-1-yl}-phenyl)-propionitril ("A7")
2-{4-[5-(6-Amino-[3,4']bipyridinyl-2'-yl)-3-methyl-2-oxo-2,3-dihydro-imidazol-1-yl]-phenyl}-2-methyl-propionitril ("A8")
2-{4-[5-(6-Methoxy-[3,4']bipyridinyl-2'-yl)-3-methyl-2-oxo-2,3-dihydro-imidazol-1-yl]-phenyl}-2-methyl-propionitril ("A9")
2-[4-(5-[3,4']Bipyridinyl-2'-yl-3-methyl-2-oxo-2,3-dihydro-imidazol-1-yl)-phenyl]-2-methyl-propionitril ("A10")
2-[4-(5-[4,4']Bipyridinyl-2-yl-3-methyl-2-oxo-2,3-dihydro-imidazol-1-yl)-phenyl]-2-methyl-propionitril ("A11")
2-Methyl-2-(4-{3-methyl-2-oxo-5-[4-(1H-pyrrolo[2,3-b]pyridin-5-yl)-pyridin-2-yl]-2,3-dihydro-imidazol-1-yl}-phenyl)-propionitril ("A12")
2-(4-{5-[4-(4-Methoxy-phenyl)-pyridin-2-yl]-3-methyl-2-oxo-2,3-dihydro-imidazol-1-yl}-phenyl)-2-methyl-propionitril ("A13")
2-(4-{5-[5-(4-Methoxy-phenyl)-pyridin-3-yl]-3-methyl-2-oxo-2,3-dihydro-imidazol-1-yl}-phenyl)-2-methyl-propionitril ("A14")
2-{4-[5-(6-Methoxy-[3,4']bipyridinyl-2'-yl)-2-oxo-2,3-dihydro-imidazol-1-yl]-phenyl}-2-methyl-propionitril ("A15")
1-[4-(2-Amino-1,1-dimethyl-ethyl)-phenyl]-5-(4-chinolin-3-yl-pyridin-2-yl)-1,3-dihydro-imidazol-2-on ("A16")
2-Methyl-2-{4-[2-oxo-5-(4-chinolin-3-yl-pyridin-2-yl)-2,3-dihydro-imidazol-1-yl]-phenyl}-propionitril ("A17")
2-Methyl-2-(4-{3-methyl-2-oxo-5-[4-(3-trifluormethyl-1H-pyrrolo[2,3-b]pyridin-5-yl)-pyridin-2-yl]-2,3-dihydro-imidazol-1-yl}-phenyl)-propionitril ("A18")
1-Methyl-3-(4-piperazin-1-yl-3-trifluormethyl-phenyl)-4-(4-chinolin-3-yl-pyridin-2-yl)-1,3-dihydro-imidazol-2-on ("A19")
4-(6-Methoxy-[3,4']bipyridinyl-2'-yl)-1-methyl-3-(4-piperazin-1-yl-3-trifluormethyl-phenyl)-1,3-dihydro-imidazol-2-on ("A20")
2-{4-[3-Methyl-2-oxo-5-(4-chinolin-3-yl-pyridin-2-yl)-2,3-dihydro-imidazol-1-yl]-phenyl}-isobutyramid ("A21")
2-Methyl-2-{4-[3-methyl-2-oxo-5-(4-chinolin-3-yl-pyridin-2-yl)-2,3-dihydro-imidazol-1-yl]-phenyl}-propionsäure ("A22")
2-Methyl-2-(4-{3-methyl-2-oxo-5-[4-(1H-pyrrolo[2,3-b]pyridin-4-yl)-pyridin-2-yl]-2,3-dihydro-imidazol-1-yl}phenyl)-propionitril ("A23")
2-(4-{3-Ethyl-2-oxo-5-[4-(1H-pyrrolo[2,3-b]pyridin-5-yl)-pyridin-2-yl]-2,3-dihydro-imidazol-1-yl}-phenyl)-2-methyl-propionitril ("A24")
1-Methyl-3-(4-piperazin-1-yl-3-trifluormethyl-phenyl)-4-[4-(1H-pyrrolo[2,3-b]pyridin-5-yl)-pyridin-2-yl]-1,3-dihydro-imidazol-2-on ("A25")
2-Methyl-2-{4-[3-methyl-2-oxo-5-(4-thiophen-3-yl-pyridin-2-yl)-2,3-dihydro-imidazol-1-yl]-phenyl}-propionitril ("A26")
2-Methyl-2-(4-{3-methyl-2-oxo-5-[4-(2-trifluormethyl-1H-pyrrolo[2,3-b]pyridin-5-yl)-pyridin-2-yl]-2,3-dihydro-imidazol-1-yl}-phenyl)-propionitril ("A27")
2-Methyl-2-(4-{2-oxo-5-[4-(1H-pyrrolo[2,3-b]pyridin-5-yl)-pyridin-2-yl]-2,3-dihydro-imidazol-1-yl}-phenyl)-propionitril ("A28")
5-(2-{3-[4-(Cyan-dimethyl-methyl)-phenyl]-1-methyl-2-oxo-2,3-dihydro-1H-imidazol-4-yl}-pyridin-4-yl)-1H-pyrrolo[2,3-b]pyridin-3-carbonsäuremethylester ("A29")
3-[4-(2-Amino-1,1-dimethyl-ethyl)-phenyl]-1-methyl-4-(4-chinolin-3-yl-pyridin-2-yl)-1,3-dihydro-imidazol-2-on ("A30")
(4-{2-Oxo-5-[4-(1H-pyrrolo[2,3-b]pyridin-5-yl)-pyridin-2-yl]-2,3-dihydro-imidazol-1-yl}-phenyl)-acetonitril ("A31")
(4-{3-Methyl-2-oxo-5-[4-(1H-pyrrolo[2,3-b]pyridin-5-yl)-pyridin-2-yl]-2,3-dihydro-imidazol-1-yl}-phenyl)-acetonitril ("A32")
2-Methyl-2-(4-{3-methyl-2-oxo-5-[4-(1H-pyrrolo[2,3-b]pyridin-5-yl)-pyridin-2-yl]-2,3-dihydro-imidazol-1-yl}-phenyl)-propionsäure ("A33")
2-(4-{3-Methyl-2-oxo-5-[4-(1H-pyrrolo[2,3-b]pyridin-5-yl)-pyridin-2-yl]-2,3-dihydro-imidazol-1-yl}-phenyl)-isobutyramid ("A34")
3-[4-(2-Amino-1,1-dimethyl-ethyl)-phenyl]-1-methyl-4-[4-(1H-pyrrolo[2,3-b]pyridin-5-yl)-pyridin-2-yl]-1,3-dihydro-imidazol-2-on ("A35")
2-Methyl-2-{4-[3-methyl-2-oxo-5-(4-quinolin-3-yl-pyridin-2-yl)-2,3-dihydro-imidazol-1-yl]-phenyl}-propionsäureethylester ("A36")
6-Amino-2'-{3-[4-(cyano-dimethyl-methyl)-phenyl]-1-methyl-2-oxo-2,3-dihydro-1H-imidazol-4-yl}-[3,4']bipyridinyl-5-carbonitril ("A37")
2-(4-{5-[4-(3H-Imidazo[4,5-b]pyridin-6-yl)-pyridin-2-yl]-3-methyl-2-oxo-2,3-dihydro-imidazol-1-yl}-phenyl)-2-methyl-propionitril ("A38")
2-{4-[5-(6-Hydroxy-[3,4']bipyridinyl-2'-yl)-3-methyl-2-oxo-2,3-dihydro-imidazol-1-yl]-phenyl}-2-methyl-propionitril ("A39")
2-{4-[5-(6-Amino-5-trifluoromethyl-[3,4']bipyridinyl-2'-yl)-3-methyl-2-oxo-2,3-dihydro-imidazol-1-yl]-phenyl}-2-methyl-propionitril ("A40")
2-Methyl-2-(4-{3-methyl-2-oxo-5-[4-(1H-pyrazolo[3,4-b]pyridin-5-yl)-pyridin-2-yl]-2,3-dihydro-imidazol-1-yl}-phenyl)-propionitril ("A41")
2-Methyl-2-{4-[3-methyl-5-(6-methylamino-[3,4']bipyridinyl-2'-yl)-2-oxo-2,3-dihydro-imidazol-1-yl]-phenyl}-propionitril ("A42")
2-(4-{5-[4-(2,3-Dihydro-1H-pyrrolo[2,3-b]pyridin-5-yl)-pyridin-2-yl]-3-methyl-2-oxo-2,3-dihydro-imidazol-1-yl}-phenyl)-2-methyl-propionitril ("A43")
2-Methyl-2-(4-{3-methyl-2-oxo-5-[4-(2-oxo-2,3-dihydro-1H-pyrrolo[2,3-b]pyridin-5-yl)-pyridin-2-yl]-2,3-dihydro-imidazol-1-yl}-phenyl)-propionitril ("A44")
2-Methyl-2-(4-{3-methyl-2-oxo-5-[4-(7-oxo-5,6,7,8-tetrahydro-[1,8]naphthyridin-3-yl)-pyridin-2-yl]-2,3-dihydro-imidazol-1-yl}-phenyl)-propionitril ("A45")
1-Methyl-3-(4-piperazin-1-yl-3-trifluoromethyl-phenyl)-4-[4-(1H-pyrazolo[3,4-b]pyridin-5-yl)-pyridin-2-yl]-1,3-dihydro-imidazol-2-on ("A46")
1-Methyl-3-phenyl-4-[4-(1H-pyrrolo[2,3-b]pyridin-5-yl)-pyridin-2-yl]-1,3-dihydro-imidazol-2-on ("A47")
4-{3-Methyl-2-oxo-5-[4-(1H-pyrrolo[2,3-b]pyridin-5-yl)-pyridin-2-yl]-2,3-dihydro-imidazol-1-yl}-benzonitril ("A48")
1-Methyl-3-[4-(4-methyl-piperazin-1-yl)-3-trifluoromethyl-phenyl]-4-[4-(1H-pyrrolo[2,3-b]pyridin-5-yl)-pyridin-2-yl]-1,3-dihydro-imidazol-2-on ("A49")
1-Methyl-3-[4-(4-methyl-piperazin-1-yl)-phenyl]-4-[4-(1H-pyrrolo[2,3-b]pyridin-5-yl)-pyridin-2-yl]-1,3-dihydro-imidazol-2-on ("A50")
3-(4-Fluoro-3-trifluoromethyl-phenyl)-1-methyl-4-[4-(1 H-pyrrolo[2,3-b]pyridin-5-yl)-pyridin-2-yl]-1,3-dihydro-imidazol-2-on ("A51")
2-(4-{5-[4-(1H-Indazol-5-yl)-pyridin-2-yl]-3-methyl-2-oxo-2,3-dihydro-imidazol-1-yl}-phenyl)-2-methyl-propionitrile ("A52")
2-(4-{5-[4-(1H-Indol-5-yl)-pyridin-2-yl]-3-methyl-2-oxo-2,3-dihydroimidazol-1-yl}-phenyl)-2-methyl-propionitrile ("A53")
2-Methyl-2-(4-{3-methyl-5-[4-(1-methyl-1H-pyrrolo[2,3-b]pyridin-5-yl)-pyridin-2-yl]-2-oxo-2,3-dihydro-imidazol-1-yl}-phenyl)-propionitrile ("A54")
3-{3-Methyl-2-oxo-5-[4-(1H-pyrrolo[2,3-b]pyridin-5-yl)-pyridin-2-yl]-2,3-dihydro-imidazol-1-yl}-benzonitrile ("A55")
4-(6-Amino-5-trifluoromethyl-[3,4']bipyridinyl-2'-yl)-1-methyl-3-(4-piperazin-1-yl-3-trifluoromethyl-phenyl)-1,3-dihydro-imidazol-2-one ("A56")
4-[4-(1H-Indol-5-yl)-pyridin-2-yl]-1-methyl-3-(4-piperazin-1-yl-3-trifluoromethyl-phenyl)-1,3-dihydro-imidazol-2-one ("A57")
3-(4-Chloro-3-methoxy-phenyl)-1-methyl-4-[4-(1H-pyrrolo[2,3-b]pyridin-5-yl)-pyridin-2-yl]-1,3-dihydro-imidazol-2-one ("A58")
4-(6-Amino-[3,4']bipyridinyl-2'-yl)-1-methyl-3-(4-piperazin-1-yl-3-trifluoromethyl-phenyl)-1,3-dihydro-imidazol-2-one ("A59")
4-[4-(4-Amino-phenyl)-pyridin-2-yl]-1-methyl-3-(4-piperazin-1-yl-3-trifluoromethyl-phenyl)-1,3-dihydro-imidazol-2-one ("A60")
N-{2'-[1-Methyl-2-oxo-3-(4-piperazin-1-yl-3-trifluoromethyl-phenyl)-2,3-dihydro-1H-imidazol-4-yl]-[3,4']bipyridinyl-6-yl}-methanesulfonamide ("A61 ")
N-(4-{2-[1-Methyl-2-oxo-3-(4-piperazin-1-yl-3-trifluoromethyl-phenyl)-2,3-dihydro-1H-imidazol-4-yl]-pyridin-4-yl}-phenyl)-methanesulfonamide ("A62")
3-{3-Methy)-2-oxo-5-[4-(1H-pyrazolo[3,4-b]pyridin-5-yl)-pyridin-2-yl]-2,3-dihydro-imidazol-1-yl}-benzonitrile ("A63")
3-[5-(6-Amino-5-trifluoromethyl-[3,4']bipyridinyl-2'-yl)-3-methyl-2-oxo-2,3-dihydro-imidazol-1-yl]-benzonitrile ("A64")
6-Amino-2'-[1-methyl-2-oxo-3-(4-piperazin-1-yl-3-trifluoromethyl-phenyl)-2,3-dihydro-1H-imidazol-4-yl]-[3,4']bipyridinyl-5-carbonitrile ("A65")
3-(3-Ethyl-phenyl)-1-methyl-4-[4-(1 H-pyrrolo[2,3-b]pyridin-5-yl)-pyridin-2-yl]-1,3-dihydro-imidazol-2-one ("A66")
1-Methyl-4-[4-(1H-pyrrolo[2,3-b]pyridin-5-yl)-pyridin-2-yl]-3-(2-trifluoromethyl-phenyl)-1,3-dihydro-imidazol-2-one ("A67")
N-{2'-[3-(3-Cyano-phenyl)-1-methyl-2-oxo-2,3-dihydro-1H-imidazol-4-yl]-5-trifluoromethyl-[3,4']bipyridinyl-6-yl}-acetamide ("A68")
2-{3-Methyl-2-oxo-5-[4-(1H-pyrrolo[2,3-b]pyridin-5-yl)-pyridin-2-yl]-2,3-dihydro-imidazol-1-yl}-benzonitrile ("A69")
3-Amino-6-(2-{3-[4-(cyano-dimethyl-methyl)-phenyl]-1-methyl-2-oxo-2,3-dihydro-1H-imidazol-4-yl}-pyridin-4-yl)-pyrazine-2-carbonitrile ("A70")
1-Methyl-3-(4-morpholin-4-yl-3-trifluoromethyl-phenyl)-4-[4-(1H-pyrrolo[2,3-b]pyridin-5-yl)-pyridin-2-yl]-1,3-dihydro-imidazol-2-one ("A71")
3-[4-(4-Acetyl-piperazin-1-yl)-3-trifluoromethyl-phenyl]-1-methyl-4-[4-(1H-pyrrolo[2,3-b]pyridin-5-yl)-pyridin-2-yl]-1,3-dihydro-imidazol-2-one ("A72")
3-(3-Methoxy-phenyl)-1-methyl-4-[4-(1H-pyrrolo[2,3-b]pyridin-5-yl)-pyridin-2-yl]-1,3-dihydro-imidazol-2-one ("A73")
3-[4-(4-Methanesulfonyl-piperazin-1-yl)-3-trifluoromethyl-phenyl]-1-methyl-4-[4-(1H-pyrrolo[2,3-b]pyridin-5-yl)-pyridin-2-yl]-1,3-dihydro-imidazol-2-one ("A74")
3-{3-Difluoromethyl-2-oxo-5-[4-(1H-pyrrolo[2,3-b]pyridin-5-yl)-pyridin-2-yl]-2,3-dihydro-imidazol-1-yl}-benzonitrile ("A75")
1-Methyl-4-[4-(1H-pyrrolo[2,3-b]pyridin-5-yl)-pyridin-2-yl]-3-(2-trifluoromethoxy-phenyl)-1,3-dihydro-imidazol-2-one ("A76")
3-[4-(4-Ethyl-piperazin-1-yl)-3-trifluoromethyl-phenyl]-1-methyl-4-[4-(1 H-pyrrolo[2,3-b]pyridin-5-yl)-pyridin-2-yl]-1,3-dihydro-imidazol-2-one ("A77")
2-Methyl-2-(4-{3-methyl-2-oxo-5-[4-(5H-pyrrolo[2,3-b]pyrazin-2-yl)-pyridin-2-yl]-2,3-dihydro-imidazol-1-yl}-phenyl)-propionitrile ("A78")
1-Methyl-4-[4-(1H-pyrrolo[2,3-b]pyridin-5-yl)-pyridin-2-yl]-3-m-tolyl-1,3-dihydro-imidazol-2-one ("A79")
3-(3-Isopropyl-phenyl)-1-methyl-4-[4-(1H-pyrrolo[2,3-b]pyridin-5-yl)-pyridin-2-yl]-1,3-dihydro-imidazol-2-one ("A80")
2-Methyl-2-{4-[3-methyl-2-oxo-5-(4-pyrido[2,3-b]pyrazin-7-yl-pyridin-2-yl)-2,3-dihydro-imidazol-1-yl]-phenyl}-propionitrile ("A81")
5-[5-(6-Amino-5-trifluoromethyl-[3,4']bipyridinyl-2'-yl)-3-methyl-2-oxo-2,3-dihydro-imidazol-1-yl]-2-(4-methyl-piperazin-1-yl)-benzonitrile ("A82")
5-{3-Methyl-2-oxo-5-[4-(1H-pyrrolo[2,3-b]pyridin-5-yl)-pyridin-2-yl]-2,3-dihydro-imidazol-1-yl}-2-(4-methyl-piperazin-1-yl)-benzonitrile ("A83")
2-{4-[5-(6-Amino-5-phenyl-[3,4']bipyridinyl-2'-yl)-3-methyl-2-oxo-2,3-dihydro-imidazol-1-yl]-phenyl}-2-methyl-propionitrile ("A84")
2-Methyl-2-(4-{3-methyl-5-[4-(2-methyl-1H-pyrrolo[2,3-b]pyridin-5-yl)-pyridin-2-yl]-2-oxo-2,3-dihydro-imidazol-1-yl}-phenyl)-propionitrile ("A85")
4-[4-(2-tert.-Butyl-1H-pyrrolo[2,3-b]pyridin-5-yl)-pyridin-2-yl]-1-methyl-3-[4-(4-methyl-piperazin-1-yl)-3-trifluoromethyl-phenyl]-1,3-dihydro-imidazol-2-one ("A86")
1-Methyl-3-[4-(4-methyl-piperazin-1-yl)-3-trifluoromethyl-phenyl]-4-[4-(2-trifluoromethyl-1H-pyrrolo[2,3-b]pyridin-5-yl)-pyridin-2-yl]-1,3-dihydro-imidazol-2-one ("A87")
1-Methyl-3-[4-(4-methyl-piperazin-1-yl)-3-trifluoromethyl-phenyl]-4-[4-(2-methyl-1 H-pyrrolo[2,3-b]pyridin-5-yl)-pyridin-2-yl]-1,3-dihydro-imidazol-2-one ("A88")
3-[4-(4-Acetyl-piperazin-1-yl)-3-trifluoromethyl-phenyl]-4-[4-(1H-indol-5-yl)-pyridin-2-yl]-1-methyl-1,3-dihydro-imidazol-2-one ("A89")
1-Methyl-3-(3-phenoxy-phenyl)-4-[4-(1H-pyrrolo[2,3-b]pyridin-5-yl)-pyridin-2-yl]-1,3-dihydro-imidazol-2-one ("A90")
2-Methyl-2-{4-[3-methyl-2-oxo-5-(4-quinazolin-6-yl-pyridin-2-yl)-2,3-dihydro-imidazol-1-yl]-phenyl}-propionitrile ("A91")
2-(4-{5-[4-(2-tert-Butyl-1H-pyrrolo[2,3-b]pyridin-5-yl)-pyridin-2-yl]-3-methyl-2-oxo-2,3-dihydro-imidazol-1-yl}-phenyl)-2-methyl-propionitrile ("A92")
2-(4-{5-[4-(4-Amino-3-trifluoromethyl-phenyl)-pyridin-2-yl]-3-methyl-2-oxo-2,3-dihydro-imidazol-1-yl}-phenyl)-2-methyl-propionitrile ("A93")
2-(4-{5-[4-(4-Amino-3-trifluoromethyl-phenyl)-pyridin-2-yl]-3-methyl-2-oxo-2,3-dihydro-imidazol-1-yl}-phenyl)-2-methyl-propionitrile ("A94")
2-Methyl-2-{4-[3-methyl-5-(6-methylaminomethyl-[3,4']bipyridinyl-2'-yl)-2-oxo-2,3-dihydro-imidazol-1-yl]-phenyl}-propionitrile ("A95")
3-Fluoro-5-{3-methyl-2-oxo-5-[4-(1H-pyrrolo[2,3-b]pyridin-5-yl)-pyridin-2-yl]-2,3-dihydro-imidazol-1-yl}-benzonitrile ("A96")
4-(6-Amino-5-trifluoromethyl-[3,4']bipyridinyl-2'-yl)-3-(4-fluoro-3-trifluoromethyl-phenyl)-1-methyl-1,3-dihydro-imidazol-2-one ("A97")
3-{3-Methyl-2-oxo-5-[4-(1H-pyrrolo[2,3-b]pyridin-5-yl)-pyridin-2-yl]-2,3-dihydro-imidazol-1-yl}-5-(4-methyl-piperazin-1-yl)-benzonitrile ("A98")
2-[4-(5-{4-[3-(2-Amino-6-ethyl-pyrimidin-4-yl)-1H-pyrrolo[2,3-b]pyridin-5-yl]-pyridin-2-yl}-3-methyl-2-oxo-2,3-dihydro-imidazol-1-yl)-phenyl]-2-methyl-propionitrile ("A99")
2-Methyl-2-(4-{3-methyl-2-oxo-5-[4-(1H-pyrrolo[2,3-b]pyridin-3-yl)-pyridin-2-yl]-2,3-dihydro-imidazol-1-yl}-phenyl)-propionitrile ("A100")
2-(4-{5-[4-(1-Amino-isoquinolin-4-yl)-pyridin-2-yl]-3-methyl-2-oxo-2,3-dihydro-imidazol-1-yl}-phenyl)-2-methyl-propionitrile ("A101")
4-[4-(1H-lndol-5-yl)-pyridin-2-yl]-1-methyl-3-[4-(4-methyl-piperazin-1-yl)-3-trifluoromethyl-phenyl]-1,3-dihydro-imidazol-2-one ("A102")
4-(6-Amino-5-trifluoromethyl-[3,4']bipyridinyl-2'-yl)-1-methyl-3-[4-(4-methyl-piperazin-1-yl)-3-trifluoromethyl-phenyl]-1,3-dihydro-imidazol-2-one ("A103")
1-Methyl-4-[4-(2-methyl-1H-pyrrolo[2,3-b]pyridin-5-yl)-pyridin-2-yl]-3-(3-phenoxy-phenyl)-1,3-dihydro-imidazol-2-one ("A104")
4-[4-(3-Fluoro-1 H-pyrrolo[2,3-b]pyridin-5-yl)-pyridin-2-yl]-3-(4-fluoro-3-trifluoromethyl-phenyl)-1-methyl-1,3-dihydro-imidazol-2-one ("A105")
1-Methyl-3-[4-(4-pyridin-3-ylmethyl-piperazin-1-yl)-3-trifluoromethyl-phenyl]-4-[4-(1H-pyrrolo[2,3-b]pyridin-5-yl)-pyridin-2-yl]-1,3-dihydroimidazol-2-one ("A106")
2-(4-{5-[4-(4-Amino-3-fluoro-5-trifluoromethyl-phenyl)-pyridin-2-yl]-3-methyl-2-oxo-2,3-dihydro-imidazol-1-yl}-phenyl)-2-methyl-propionitrile ("A107")
3-(4-Methoxy-benzyl)-1-methyl-4-[4-(1H-pyrrolo[2,3-b]pyridin-5-yl)-pyridin-2-yl]-1,3-dihydro-imidazol-2-one ("A108")
3-(4-Methoxy-benzyl)-1-methyl-4-[4-(1H-pyrazolo[3,4-b]pyridin-5-yl)-pyridin-2-yl]-1,3-dihydro-imidazol-2-one ("A109")
4-(6-Amino-5-trifluoromethyl-[3,4']bipyridinyl-2'-yl)-3-(4-methoxy-benzyl)-1-methyl-1,3-dihydro-imidazol-2-one ("A110")
N-{2'-[3-(4-Methoxy-benzyl)-1-methyl-2-oxo-2,3-dihydro-1H-imidazol-4-yl]-5-trifluoromethyl-[3,4']bipyridinyl-6-yl}-acetamide ("A111 ")
4-[4-(2-tert-Butyl-1H-pyrrolo[2,3-b]pyridin-5-yl)-pyridin-2-yl]-3-[3-(1,1-difluoro-ethyl)-4-fluoro-phenyl]-1-methyl-1,3-dihydro-imidazol-2-one ("A112°)
N-(2-Methyl-2-{4-[3-methyl-2-oxo-5-(4-quinolin-3-yl-pyridin-2-yl)-2,3-dihydro-imidazol-1-yl]-phenyl}-propyl)-acetamid ("A113")
2'-[1-Methyl-2-oxo-3-(4-piperazin-1-yl-3-trifluoromethyl-phenyl)-2,3-dihydro-1 H-imidazol-4-yl]-1 H-[3,4']bipyridinyl-6-on ("A114")
N-(2'-{3-[4-(Cyano-dimethyl-methyl)-phenyl]-1-methyl-2-oxo-2,3-dihydro-1H-imidazol-4-yl}-[3,4']bipyridinyl-6-yl)-acetamid ("A115")
2-Methyl-2-(4-{3-methyl-2-oxo-5-[5-(1H-pyrrolo[2,3-b]pyridin-5-yl)-pyridin-3-yl]-2,3-dihydro-imidazol-1-yl}-phenyl)-propionitril ("A116")
2-(4-{3-Methyl-2-oxo-5-[4-(1H-pyrrolo[2,3-b]pyridin-5-yl)-pyridin-2-yl]-2,3-dihydro-imidazol-1-yl}-phenyl)-propionitril ("A117")
1-Methyl-3-(4-piperazin-1-yl-3-trifluoromethyl-phenyl)-4-[4-(3-trifluoromethyl-1H-pyrrolo[2,3-b]pyridin-5-yl)-pyridin-2-yl]-1,3-dihydro-imidazol-2-on ("A118")
1-Methyl-3-(4-piperazin-1-yl-phenyl)-4-[4-(1H-pyrrolo[2,3-b]pyridin-5-yl)-pyridin-2-yl]-1,3-dihydro-imidazol-2-on ("A119")
1-Methyl-3-(4-piperidin-4-yl-phenyl)-4-[4-(1 H-pyrrolo[2,3-b]pyridin-5-yl)-pyridin-2-yl]-1,3-dihydro-imidazol-2-on ("A120")
3-Benzyl-1-methyl-4-[4-(1H-pyrrolo[2,3-b]pyridin-5-yl)-pyridin-2-yl]-1,3-dihydro-imidazol-2-on ("A121")
3-(4-tert-Butyl-phenyl)-1-methyl-4-[4-(1 H-pyrrolo[2,3-b]pyridin-5-yl)-pyridin-2-yl]-1,3-dihydro-imidazol-2-on ("A122")
1-Methyl-3-{4-[methyl-(2-methylamino-ethyl)-amino]-3-trifluoromethylphenyl}-4-[4-(1H-pyrrolo[2,3-b]pyridin-5-yl)-pyridin-2-yl]-1,3-dihydroimidazol-2-on ("A123")
3-[4-(2-Dimethylamino-ethylamino)-3-trifluoromethyl-phenyl]-1-methyl-4-[4-(1 H-pyrrolo[2,3-b]pyridin-5-yl)-pyridin-2-yl]-1,3-dihydro-imidazol-2-on ("A124")
3-[4-(4-Amino-piperidin-1-yl)-3-trifluoromethyl-phenyl]-1-methyl-4-[4-(1H-pyrrolo[2,3-b]pyridin-5-yl)-pyridin-2-yl]-1,3-dihydro-imidazol-2-on ("A125")
1-Methyl-3-[4-(methyl-piperidin-4-yl-amino)-3-trifluoromethyl-phenyl]-4-[4-(1 H-pyrrolo[2,3-b]pyridin-5-yl)-pyridin-2-yl]-1,3-dihydro-imidazol-2-on ("A126")
1-Methyl-3-[4-(piperidin-4-ylamino)-3-trifluoromethyl-phenyl]-4-[4-(1H-pyrrolo[2,3-b]pyridin-5-yl)-pyridin-2-yl]-1,3-dihydro-imidazol-2-on ("A127") sowie ihre pharmazeutisch verwendbaren Salze, Tautomere und Stereoisomere, einschließlich deren Mischungen in allen Verhältnissen.

3. Verfahren zur Herstellung von Verbindungen der Formel I nach den Ansprüchen 1-2 sowie ihrer pharmazeutisch verwendbaren Salze,
Tautomeren und Stereoisomeren,
**dadurch gekennzeichnet, daß** man in einer Suzuki-Reaktion eine Verbindung der Formel I,
worin Y Hal bedeutet,
in eine Verbindung der Formel I umwandelt,
worin Y Het oder Ar bedeutet,
und/oder
eine Base oder Säure der Formel I in eines ihrer Salze umwandelt.

4. Arzneimittel, enthaltend mindestens eine Verbindung der Formel I nach Anspruch 1-2 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

5. Verbindungen der Formel I zur Verwendung bei der Behandlung von Autoimmunerkrankungen, Entzündungserkrankungen, Herz-Kreislauf-Erkrankungen, neurodegenerativen Erkrankungen, Allergie, Asthma, Pankreatitis, Multiorganversagen, Nierenerkrankungen, Blutplättchenaggregation, Krebs, Spermienbeweglichkeit, Transplantationsabstoßung, Transplantat-Abstoßung und Lungenverletzungen.

6. Set (Kit), bestehend aus getrennten Packungen von
(a) einer wirksamen Menge an einer Verbindung der Formel I gemäß einem oder mehreren der Ansprüche 1 bis 2, und/oder ihrer pharmazeutisch verwendbaren Salze und Stereoisomere, einschließlich deren Mischungen in allen Verhältnissen,
und
(b) einer wirksamen Menge eines weiteren Arzneimittelswirkstoffs.

## Claims

1. Compounds of the formula I 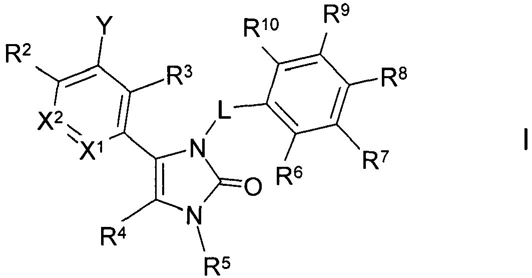 in which
X¹ denotes CR¹ or N,
X² denotes CR¹ or N,
where X¹ or X² denotes N
and the respective other denotes CR¹,
Y denotes Het or Ar,
L denotes [C(R^{a})₂]m,
R¹, R², R³, R⁴ denote H,
R⁵ denotes H or A',
R⁶, R⁹, R¹⁰ denote H,
R⁷ denotes H or A"',
R⁸ denotes H, A", Het¹, CN, Hal, NHHet¹ or NA'Het¹,
A denotes unbranched or branched alkyl having 1-10 C atoms, in which 1-7 H atoms may be replaced by F and/or Cl,
or
cyclic alkyl having 3-7 C atoms,
A' denotes unbranched or branched alkyl having 1-6 C atoms, in which 1-5 H atoms may be replaced by F and/or Cl,
A" denotes unbranched or branched alkyl having 1-10 C atoms, in which 1-7 H atoms may be replaced by OH, CN, NH₂, CONH₂, COOH, COOA, NHCOA, -C=H, F, Cl and/or Br, and/or in which one or two non-adjacent CH and/or CH₂ groups may be replaced by O, NH, NA', S, SO, SO₂ and/or CH=CH groups,
A"' denotes unbranched or branched alkyl having 1-10 C atoms, in which 1-7 H atoms may be replaced by F and/or Cl, and/or in which one or two non-adjacent CH and/or CH₂ groups may be replaced by O, NH and/or NA',
R^{a} denotes H or A',
Het denotes pyridinyl, pyrimidinyl, furyl, thienyl, oxazolyl, oxadiazolyl, imidazolyl, pyrrolyl, isoxazolyl, benzodioxolyl, dihydrobenzofuranyl, dihydrobenzodioxinyl, benzofuranyl, benzodioxanyl, indolyl, quinolinyl, isoquinolinyl, benzimidazolyl, benzothiadiazolyl, indazolyl, dihydrobenzimidazolyl, dihydroindolyl, imidazopyridinyl, pyrrolopyridinyl, pyrazolopyridinyl, 2,3-dihydropyrrolopyridinyl or tetrahydronaphthyridinyl, where the radicals may also be mono-, di- or trisubstituted by A, (CH₂)ₙNH₂, (CH₂)ₙNHA, (CH₂)ₙNA₂, NHCOA, (CH₂)ₙCONH₂, (CH₂)nCONHA, (CH₂)ₙCONA₂, CN, =O, Hal, (CH₂)ₙOH, (CH₂)ₙOA, COOH and/or COOA,
Ar denotes phenyl which is unsubstituted or mono-, di- or trisubstituted by (CH₂)ₙOH and/or (CH₂)ₙOA,
Het¹ denotes piperidinyl, pyrrolidinyl, morpholinyl, piperazinyl, oxazolidinyl, tetrahydrothienyl, tetrahydropyranyl or thiomorpholinyl, where the radicals may also be mono- or disubstituted by =O, NH₂, pyridylmethyl and/or A,
Hal denotes F, Cl, Br or I,
m denotes 0 or 1,
n denotes 0, 1, 2, 3 or 4,
and pharmaceutically usable salts, tautomers and stereoisomers thereof, including mixtures thereof in all ratios.

2. Compounds according to Claim 1, selected from the group
2-methyl-2-{4-[3-methyl-2-oxo-5-(4-quinolin-3-ylpyridin-2-yl)-2,3-dihydroimidazol-1-yl]phenyl}propionitrile ("A1")
2-methyl-2-{4-[3-methyl-2-oxo-5-(5-quinolin-3-ylpyridin-3-yl)-2,3-dihydroimidazol-1-yl]phenyl}propionitrile ("A2")
2-(4-{5-[4-(3-methoxyphenyl)pyridin-2-yl]-3-methyl-2-oxo-2,3-dihydroimidazol-1-yl}phenyl)-2-methylpropionitrile ("A3")
5-(6-methoxy-[3,4']bipyridinyl-2'-yl)-1-(4-piperazin-1-yl-3-trifluoromethylphenyl)-1,3-dihydroimidazol-2-one ("A4")
1-(4-piperazin-1-yl-3-trifluoromethylphenyl)-5-(4-quinolin-3-ylpyridin-2-yl)-1,3-dihydroimidazol-2-one ("A5")
2-{4-[5-(2'-methoxy-[4,4']bipyridinyl-2-yl)-3-methyl-2-oxo-2,3-dihydroimidazol-1-yl]phenyl}-2-methylpropionitrile ("A6")
2-methyl-2-(4-{3-methyl-5-[4-(2-methyl-3H-imidazo[4,5-b]pyridin-6-yl)-pyridin-2-yl]-2-oxo-2,3-dihydroimidazol-1-yl}phenyl)propionitrile ("A7")
2-{4-[5-(6-amino-[3,4']bipyridinyl-2'-yl)-3-methyl-2-oxo-2,3-dihydroimidazol-1-yl]phenyl}-2-methylpropionitrile ("A8")
2-{4-[5-(6-methoxy-[3,4']bipyridinyl-2'-yl)-3-methyl-2-oxo-2,3-dihydroimidazol-1-yl]phenyl}-2-methylpropionitrile ("A9")
2-[4-(5-[3,4']bipyridinyl-2'-yl-3-methyl-2-oxo-2,3-dihydroimidazol-1-yl)-phenyl]-2-methylpropionitrile ("A10")
2-[4-(5-[4,4']bipyridinyl-2-yl-3-methyl-2-oxo-2,3-dihydroimidazol-1-yl)-phenyl]-2-methylpropionitrile ("A11")
2-methyl-2-(4-{3-methyl-2-oxo-5-[4-(1H-pyrrolo[2,3-b]pyridin-5-yl)pyridin-2-yl]-2,3-dihydroimidazol-1-yl}phenyl)propionitrile ("A12")
2-(4-{5-[4-(4-methoxyphenyl)pyridin-2-yl]-3-methyl-2-oxo-2,3-dihydroimidazol-1-yl}phenyl)-2-methylpropionitrile ("A13")
2-(4-{5-[5-(4-methoxyphenyl)pyridin-3-yl]-3-methyl-2-oxo-2,3-dihydroimidazol-1-yl}phenyl)-2-methylpropionitrile ("A14")
2-{4-[5-(6-methoxy-[3,4']bipyridinyl-2'-yl)-2-oxo-2,3-dihydroimidazol-1-yl]-phenyl}-2-methylpropionitrile ("A15")
1-[4-(2-amino-1,1-dimethylethyl)phenyl]-5-(4-quinolin-3-ylpyridin-2-yl)-1,3-dihydroimidazol-2-one ("A16")
2-methyl-2-{4-[2-oxo-5-(4-quinolin-3-ylpyridin-2-yl)-2,3-dihydroimidazol-1-yl]phenyl}propionitrile ("A17")
2-methyl-2-(4-{3-methyl-2-oxo-5-[4-(3-trifluoromethyl-1H-pyrrolo[2,3-b]-pyridin-5-yl)pyridin-2-yl]-2,3-dihydroimidazol-1-yl}phenyl)propionitrile ("A18")
1-methyl-3-(4-piperazin-1-yl-3-trifluoromethylphenyl)-4-(4-q u inolin-3-yl-pyridin-2-yl)-1,3-dihydroimidazol-2-one ("A19")
4-(6-methoxy-[3,4']bipyridinyl-2'-yl)-1-methyl-3-(4-piperazin-1-yl-3-trifluoromethylphenyl)-1,3-dihydroimidazol-2-one ("A20")
2-{4-[3-methyl-2-oxo-5-(4-quinolin-3-ylpyridin-2-yl)-2,3-dihydroimidazol-1-yl]phenyl}isobutyramide ("A21")
2-methyl-2-{4-[3-methyl-2-oxo-5-(4-quinolin-3-ylpyridin-2-yl)-2,3-dihydroimidazol-1-yl]phenyl}propionic acid ("A22")
2-methyl-2-(4-{3-methyl-2-oxo-5-[4-(1H-pyrrolo[2,3-b]pyridin-4-yl)pyridin-2-yl]-2,3-dihydroimidazol-1-yl}phenyl)propionitrile ("A23")
2-(4-{3-ethyl-2-oxo-5-[4-(1H-pyrrolo[2,3-b]pyridin-5-yl)pyridin-2-yl]-2,3-dihydroimidazol-1-yl}phenyl)-2-methylpropionitrile ("A24")
1-methyl-3-(4-piperazin-1-yl-3-trifluoromethylphenyl)-4-[4-(1 H-pyrrolo[2,3-b]pyridin-5-yl)pyridin-2-yl]-1,3-dihydroimidazol-2-one ("A25")
2-methyl-2-{4-[3-methyl-2-oxo-5-(4-thiophen-3-ylpyridin-2-yl)-2,3-dihydroimidazol-1-yl]phenyl}propionitrile ("A26")
2-methyl-2-(4-{3-methyl-2-oxo-5-[4-(2-trifluoromethyl-1H-pyrrolo[2,3-b]-pyridin-5-yl)pyridin-2-yl]-2,3-dihydroimidazol-1-yl}phenyl)propionitrile ("A27")
2-methyl-2-(4-{2-oxo-5-[4-(1H-pyrrolo[2,3-b]pyridin-5-yl)pyridin-2-yl]-2,3-dihydroimidazol-1-yl}phenyl)propionitrile ("A28")
methyl 5-(2-{3-[4-(cyanodimethylmethyl)phenyl]-1-methyl-2-oxo-2,3-dihydro-1H-imidazol-4-yt}pyridin-4-yl)-1H-pyrrolo[2,3-b]pyridine-3-carboxylate ("A29")
3-[4-(2-amino-1,1-dimethylethyl)phenyl]-1-methyl-4-(4-quinolin-3-yl-pyridin-2-yl)-1,3-dihydroimidazol-2-one ("A30")
(4-{2-oxo-5-[4-(1H-pyrrolo[2,3-b]pyridin-5-yl)pyridin-2-yl]-2,3-dihydroimidazol-1-yl}phenyl)acetonitrile ("A31")
(4-{3-methyl-2-oxo-5-[4-(1H-pyrrolo[2,3-b]pyridin-5-yl)pyridin-2-yl]-2,3-dihydroimidazol-1-yl}phenyl)acetonitrile ("A32")
2-methyl-2-(4-{3-methyl-2-oxo-5-[4-(1H-pyrrolo[2,3-b]pyridin-5-yl)pyridin-2-yl]-2,3-dihydroimidazol-1-yl}phenyl)propionic acid ("A33")
2-(4-{3-methyl-2-oxo-5-[4-(1H-pyrrolo[2,3-b]pyridin-5-yl)pyridin-2-yl]-2,3-dihydroimidazol-1-yl}phenyl)isobutyramide ("A34")
3-[4-(2-amino-1,1 1-dimethylethyl)phenyl]-1-methyl-4-[4-(1H-pyrrolo[2,3-b]-pyridin-5-yl)pyridin-2-yl]-1,3-dihydroimidazol-2-one ("A35")
ethyl 2-methyl-2-{4-[3-methyl-2-oxo-5-(4-quinolin-3-ylpyridin-2-yl)-2,3-dihydroimidazol-1-yl]phenyl}propionate ("A36")
6-amino-2'-{3-[4-(cyanodimethylmethyl)phenyl]-1-methyl-2-oxo-2,3-dihydro-1H-imidazol-4-yl}-[3,4']bipyridinyl-5-carbonitrile ("A37")
2-(4-{5-[4-(3H-imidazo[4,5-b]pyridin-6-yl)pyridin-2-yl]-3-methyl-2-oxo-2,3-dihydroimidazol-1-yl}phenyl)-2-methylpropionitrile ("A38")
2-{4-[5-(6-hydroxy-[3,4']bipyridinyl-2'-yl)-3-methyl-2-oxo-2,3-dihydroimidazol-1-yl]phenyl}-2-methylpropionitrile ("A39")
2-{4-[5-(6-amino-5-trifluoromethyl-[3,4']bipyridinyl-2'-yl)-3-methyl-2-oxo-2,3-dihydroimidazol-1-yl]phenyl}-2-methylpropionitrile ("A40")
2-methyl-2-(4-{3-methyl-2-oxo-5-[4-(1H-pyrazolo[3,4-b]pyridin-5-yl)pyridin-2-yl]-2,3-dihydroimidazol-1-yl}phenyl)propionitrile ("A41")
2-methyl-2-{4-[3-methyl-5-(6-methylamino-[3,4']bipyridinyl-2'-yl)-2-oxo-2,3-dihydroimidazol-1-yl]phenyl}propionitrile ("A42")
2-(4-{5-[4-(2,3-dihydro-1H-pyrrolo[2,3-b]pyridin-5-yl)pyridin-2-yl]-3-methyl-2-oxo-2,3-dihydroimidazol-1-yl}phenyl)-2-methylpropionitrile ("A43")
2-methyl-2-(4-{3-methyl-2-oxo-5-[4-(2-oxo-2,3-dihydro-1H-pyrrolo[2,3-b]-pyridin-5-yl)pyridin-2-yl]-2,3-dihydroimidazol-1-yl}phenyl)propionitrile ("A44")
2-methyl-2-(4-{3-methyl-2-oxo-5-[4-(7-oxo-5,6,7,8-tetrahydro-[1,8]naphthyridin-3-yl)pyridin-2-yl]-2,3-dihydroimidazol-1-yl}phenyl)propionitrile ("A45")
1-methyl-3-(4-piperazin-1-yl-3-trifluoromethylphenyl)-4-[4-(1H-pyrazolo-[3,4-b]pyridin-5-yl)pyridin-2-yl]-1,3-dihydroimidazol-2-one ("A46")
1-methyl-3-phenyl-4-[4-(1H-pyrrolo[2,3-b]pyridin-5-yl)pyridin-2-yl]-1,3-dihydroimidazol-2-one ("A47")
4-{3-methyl-2-oxo-5-[4-(1H-pyrrolo[2,3-b]pyridin-5-yl)pyridin-2-yl]-2,3-dihydroimidazol-1-yl}benzonitrile ("A48")
1-methyl-3-[4-(4-methylpiperazin-1-yl)-3-trifluoromethylphenyl]-4-[4-(1H-pyrrolo[2,3-b]pyridin-5-yl)pyridin-2-yl]-1,3-dihydroimidazol-2-one ("A49")
1-methyl-3-[4-(4-methylpiperazin-1-yl)phenyl]-4-[4-(1H-pyrrolo[2,3-b]-pyridin-5-yl)pyridin-2-yl]-1,3-dihydroimidazol-2-one ("A50")
3-(4-fluoro-3-trifluoromethylphenyl)-1-methyl-4-[4-(1 H-pyrrolo[2,3-b]-pyridin-5-yl)pyridin-2-yl]-1,3-dihydroimidazol-2-one ("A51")
2-(4-{5-[4-(1H-indazol-5-yl)pyridin-2-yl]-3-methyl-2-oxo-2,3-dihydroimidazol-1-yl}phenyl)-2-methylpropionitrile ("A52")
2-(4-{5-[4-(1H-indol-5-yl)pyridin-2-yl]-3-methyl-2-oxo-2,3-dihydroimidazol-1-yl}phenyl)-2-methylpropionitrile ("A53")
2-methyl-2-(4-{3-methyl-5-[4-(1-methyl-1H-pyrrolo[2,3-b]pyridin-5-yl)-pyridin-2-yl]-2-oxo-2,3-dihydroimidazol-1-yl}phenyl)propionitrile ("A54")
3-{3-methyl-2-oxo-5-[4-(1H-pyrrolo[2,3-b]pyridin-5-yl)pyridin-2-yl]-2,3-dihydroimidazol-1-yl}benzonitrile ("A55")
4-(6-amino-5-trifluoromethyl-[3,4']bipyridinyl-2'-yl)-1-methyl-3-(4-piperazin-1-yl-3-trifluoromethylphenyl)-1,3-dihydroimidazol-2-one ("A56")
4-[4-(1H-indol-5-yl)pyridin-2-yl]-1-methyl-3-(4-piperazin-1-yl-3-trifluoromethylphenyl)-1,3-dihydroimidazol-2-one ("A57")
3-(4-chloro-3-methoxyphenyl)-1-methyl-4-[4-(1 H-pyrrolo[2,3-b]pyridin-5-yl)pyridin-2-yl]-1,3-dihydroimidazol-2-one ("A58")
4-(6-amino-[3,4']bipyridinyl-2'-yl)-1-methyl-3-(4-piperazin-1-yl-3-trifluoromethylphenyl)-1,3-dihydroimidazol-2-one ("A59")
4-[4-(4-aminophenyl)pyridin-2-yl]-1-methyl-3-(4-piperazin-1-yl-3-trifluoromethylphenyl)-1,3-dihydroimidazol-2-one ("A60")
N-{2'-[1-methyl-2-oxo-3-(4-piperazin-1-yl-3-trifluoromethylphenyl)-2,3-dihydro-1H-imidazol-4-yl]-[3,4']bipyridinyl-6-yl}methanesulfonamide ("A61")
N-(4-{2-[1-methyl-2-oxo-3-(4-piperazin-1-yl-3-trifluoromethylphenyl)-2,3-dihydro-1H-imidazol-4-yl]pyridin-4-yl}phenyl)methanesulfonamide ("A62")
3-{3-methyl-2-oxo-5-[4-(1H-pyrazolo[3,4-b]pyridin-5-yl)pyridin-2-yl]-2,3-dihydroimidazol-1-yl}benzonitrile ("A63")
3-[5-(6-amino-5-trifluoromethyl-[3,4']bipyridinyl-2'-yl)-3-methyl-2-oxo-2,3-dihydroimidazol-1-yl]benzonitrile ("A64")
6-amino-2'-[1-methyl-2-oxo-3-(4-piperazin-1-yl-3-trifluoromethylphenyl)-2,3-dihydro-1H-imidazol-4-yl]-[3,4']bipyridinyl-5-carbonitrile ("A65")
3-(3-ethylphenyl)-1-methyl-4-[4-(1 H-pyrrolo[2,3-b]pyridin-5-yl)pyridin-2-yl]-1,3-dihydroimidazol-2-one ("A66")
1-methyl-4-[4-(1 H-pyrrolo[2,3-b]pyridin-5-yl)pyridin-2-yl]-3-(2-trifluoromethylphenyl)-1,3-dihydroimidazol-2-one ("A67")
N-{2'-[3-(3-cyanophenyl)-1-methyl-2-oxo-2,3-dihydro-1H-imidazol-4-yl]-5-trifluoromethyl-[3,4']bipyridinyl-6-yl}acetamide ("A68")
2-{3-methyl-2-oxo-5-[4-(1H-pyrrolo[2,3-b]pyridin-5-yl)pyridin-2-yl]-2,3-dihydroimidazol-1-yl}benzonitrile ("A69")
3-amino-6-(2-{3-[4-(cyanodimethylmethyl)phenyl]-1-methyl-2-oxo-2,3-dihydro-1H-imidazol-4-yl}pyridin-4-yl)pyrazine-2-carbonitrile ("A70")
1-methyl-3-(4-morpholin-4-yl-3-trifluoromethylphenyl)-4-[4-(1H-pyrrolo-[2,3-b]pyridin-5-yl)pyridin-2-yl]-1,3-dihydroimidazol-2-one ("A71")
3-[4-(4-acetylpiperazin-1-yl)-3-trifluoromethylphenyl]-1-methyl-4-[4-(1H-pyrrolo[2,3-b]pyridin-5-yl)pyridin-2-yl]-1,3-dihydroimidazol-2-one ("A72")
3-(3-methoxyphenyl)-1-methyl-4-[4-(1 H-pyrrolo[2,3-b]pyridin-5-yl)pyridin-2-yl]-1,3-dihydroimidazol-2-one ("A73")
3-[4-(4-methanesulfonylpiperazin-1-yl)-3-trifluoromethylphenyl]-1-methyl-4-[4-(1 H-pyrrolo[2,3-b]pyridin-5-yl)pyridin-2-yl]-1,3-dihydroimidazol-2-one ("A74")
3-{3-difluoromethyl-2-oxo-5-[4-(1H-pyrrolo[2,3-b]pyridin-5-yl)pyridin-2-yl]-2,3-dihydroimidazol-1-yl}benzonitrile ("A75")
1-methyl-4-[4-(1 H-pyrrolo[2,3-b]pyridin-5-yl)pyridin-2-yl]-3-(2-trifluoromethoxyphenyl)-1,3-dihydroimidazol-2-one ("A76")
3-[4-(4-ethylpiperazin-1-yl)-3-trifluoromethylphenyl]-1-methyl-4-[4-(1H-pyrrolo[2,3-b]pyridin-5-yl)pyridin-2-yl]-1,3-dihydroimidazol-2-one ("A77")
2-methyl-2-(4-{3-methyl-2-oxo-5-[4-(5H-pyrrolo[2,3-b]pyrazin-2-yl)pyridin-2-yl]-2,3-dihydroimidazol-1-yl}phenyl)propionitrile ("A78")
1-methyl-4-[4-(1 H-pyrrolo[2,3-b]pyridin-5-yl)pyridin-2-yl]-3-m-tolyl-1,3-dihydroimidazol-2-one ("A79")
3-(3-isopropylphenyl)-1-methyl-4-[4-(1 H-pyrrolo[2,3-b]pyridin-5-yl)pyridin-2-yl]-1,3-dihydroimidazol-2-one ("A80")
2-methyl-2-{4-[3-methyl-2-oxo-5-(4-pyrido[2,3-b]pyrazin-7-ylpyridin-2-yl)-2,3-dihydroimidazol-1-yl]phenyl}propionitrile ("A81")
5-[5-(6-amino-5-trifluoromethyl-[3,4']bipyridinyl-2'-yl)-3-methyl-2-oxo-2,3-dihydroimidazol-1-yl]-2-(4-methylpiperazin-1-yl)benzonitrile ("A82")
5-{3-methyl-2-oxo-5-[4-(1H-pyrrolo[2,3-b]pyridin-5-yl)pyridin-2-yl]-2,3-dihydroimidazol-1-yl}-2-(4-methylpiperazin-1-yl)benzonitrile ("A83")
2-{4-[5-(6-amino-5-phenyl-[3,4']bipyridinyl-2'-yl)-3-methyl-2-oxo-2,3-dihydroimidazol-1-yl]phenyl}-2-methylpropionitrile ("A84")
2-methyl-2-(4-{3-methyl-5-[4-(2-methyl-1H-pyrrolo[2,3-b]pyridin-5-yl)-pyridin-2-yl]-2-oxo-2,3-dihydroimidazol-1-yl}phenyl)propionitrile ("A85")
4-[4-(2-tert-butyl-1 H-pyrrolo[2,3-b]pyridin-5-yl)pyridin-2-yl]-1-methyl-3-[4-(4-methylpiperazin-1-yl)-3-trifluoromethylphenyl]-1,3-dihydroimidazol-2-one ("A86")
1-methyl-3-[4-(4-methylpiperazin-1-yl)-3-trifluoromethylphenyl]-4-[4-(2-trifluoromethyl-1H-pyrrolo[2,3-b]pyridin-5-yl)pyridin-2-yl]-1,3-dihydroimida-zol-2-one ("A87")
1-methyl-3-[4-(4-methylpiperazin-1-yl)-3-trifluoromethylphenyl]-4-[4-(2-methyl-1H-pyrrolo[2,3-b]pyridin-5-yl)pyridin-2-yl]-1,3-dihydroimidazol-2-one ("A88")
3-[4-(4-acetylpiperazin-1-yl)-3-trifluoromethylphenyl]-4-[4-(1H-indol-5-yl)-pyridin-2-yl]-1-methyl-1,3-dihydroimidazol-2-one ("A89")
1-methyl-3-(3-phenoxyphenyl)-4-[4-(1H-pyrrolo[2,3-b]pyridin-5-yl)pyridin-2-yl]-1,3-dihydroimidazol-2-one ("A90")
2-methyl-2-{4-[3-methyl-2-oxo-5-(4-quinazolin-6-ylpyridin-2-yl)-2,3-dihydroimidazol-1-yl]phenyl}propionitrile ("A91")
2-(4-{5-[4-(2-tert-butyl-1H-pyrrolo[2,3-b]pyridin-5-yl)pyridin-2-yl]-3-methyl-2-oxo-2,3-dihydroimidazol-1-yl}phenyl)-2-methylpropionitrile ("A92")
2-(4-{5-[4-(4-amino-3-trifluoromethylphenyl)pyridin-2-yl]-3-methyl-2-oxo-2,3-dihydroimidazol-1-yl}phenyl)-2-methylpropionitrile ("A93")
2-(4-{5-[4-(4-amino-3-trifluoromethylphenyl)pyridin-2-yl]-3-methyl-2-oxo-2,3-dihydroimidazol-1-yl}phenyl)-2-methylpropionitrile ("A94")
2-methyl-2-{4-[3-methyl-5-(6-methylaminomethyl-[3,4']bipyridinyl-2'-yl)-2-oxo-2,3-dihydroimidazol-1-yl]phenyl}propionitrile ("A95")
3-fluoro-5-{3-methyl-2-oxo-5-[4-(1H-pyrrolo[2,3-b]pyridin-5-yl)pyridin-2-yl]-2,3-dihydroimidazol-1-yl}benzonitrile ("A96")
4-(6-amino-5-trifluoromethyl-[3,4']bipyridinyl-2'-yl)-3-(4-fluoro-3-trifluoromethylphenyl)-1-methyl-1,3-dihydroimidazol-2-one ("A97")
3-{3-methyl-2-oxo-5-[4-(1H-pyrrolo[2,3-b]pyridin-5-yl)pyridin-2-yl]-2,3-dihydroimidazol-1-yl}-5-(4-methylpiperazin-1-yl)benzonitrile ("A98")
2-[4-(5-{4-[3-(2-amino-6-ethylpyrimidin-4-yl)-1H-pyrrolo[2,3-b]pyridin-5-yl]-pyridin-2-yl}-3-methyl-2-oxo-2,3-dihydroimidazol-1-yl)phenyl]-2-methyl-propionitrile ("A99")
2-methyl-2-(4-{3-methyl-2-oxo-5-[4-(1H-pyrrolo[2,3-b]pyridin-3-yl)pyridin-2-yl]-2,3-dihydroimidazol-1-yl}phenyl)propionitrile ("A100")
2-(4-{5-[4-(1-aminoisoquinolin-4-yl)pyridin-2-yl]-3-methyl-2-oxo-2,3-dihydroimidazol-1-yl}phenyl)-2-methylpropionitrile ("A101")
4-[4-(1H-indol-5-yl)pyridin-2-yl]-1-methyl-3-[4-(4-methylpiperazin-1-yl)-3-trifluoromethylphenyl]-1,3-dihydroimidazol-2-one ("A102")
4-(6-amino-5-trifluoromethyl-[3,4']bipyridinyl-2'-yl)-1-methyl-3-[4-(4-methylpiperazin-1-yl)-3-trifluoromethylphenyl]-1,3-dihydroimidazol-2-one ("A103")
1-methyl-4-[4-(2-methyl-1H-pyrrolo[2,3-b]pyridin-5-yl)pyridin-2-yl]-3-(3-phenoxyphenyl)-1,3-dihydroimidazol-2-one ("A104")
4-[4-(3-fluoro-1 H-pyrrolo[2,3-b]pyridin-5-yl)pyridin-2-yl]-3-(4-fluoro-3-trifluoromethylphenyl)-1-methyl-1,3-dihydroimidazol-2-one ("A105")
1-methyl-3-[4-(4-pyridin-3-ylmethylpiperazin-1-yl)-3-trifluoromethylphenyl]-4-[4-(1H-pyrrolo[2,3-b]pyridin-5-yl)pyridin-2-yl]-1,3-dihydroimidazol-2-one ("A106")
2-(4-{5-[4-(4-amino-3-fluoro-5-trifluoromethylphenyl)pyridin-2-yl]-3-methyl-2-oxo-2,3-dihydroimidazol-1-yl}phenyl)-2-methylpropionitrile ("A107")
3-(4-methoxybenzyl)-1-methyl-4-[4-(1 H-pyrrolo[2,3-b]pyridin-5-yl)pyridin-2-yl]-1,3-dihydroimidazol-2-one ("A108")
3-(4-methoxybenzyl)-1-methyl-4-[4-(1 H-pyrazolo[3,4-b]pyridin-5-yl)pyridin-2-yl]-1,3-dihydroimidazol-2-one ("A109")
4-(6-amino-5-trifluoromethyl-[3,4']bipyridinyl-2'-yl)-3-(4-methoxybenzyl)-1-methyl-1,3-dihydroimidazol-2-one ("A110")
N-{2'-[3-(4-methoxybenzyl)-1-methyl-2-oxo-2,3-dihydro-1H-imidazol-4-yl]-5-trifluoromethyl-[3,4']bipyridinyl-6-yl}acetamide ("A111")
4-[4-(2-tert-butyl-1H-pyrrolo[2,3-b]pyridin-5-yl)pyridin-2-yl]-3-[3-(1,1-difluoroethyl)-4-fluorophenyl]-1-methyl-1,3-dihydroimidazol-2-one ("A112")
N-(2-methyl-2-{4-[3-methyl-2-oxo-5-(4-quinolin-3-ylpyridin-2-yl)-2,3-dihydroimidazol-1-yl]phenyl}propyl)acetamide ("A113")
2'-[1-methyl-2-oxo-3-(4-piperazin-1-yl-3-trifluoromethylphenyl)-2,3-dihydro-1 H-imidazol-4-yl]-1 H-[3,4']bipyridinyl-6-one ("A114")
N-(2'-{3-[4-(cyanodimethylmethyl)phenyl]-1-methyl-2-oxo-2,3-dihydro-1H-imidazol-4-yl}-[3,4']bipyridinyl-6-yl)acetamide ("A115")
2-methyl-2-(4-{3-methyl-2-oxo-5-[5-(1H-pyrrolo[2,3-b]pyridin-5-yl)pyridin-3-yl]-2,3-dihydroimidazol-1-yl}phenyl)propionitrile ("A116")
2-(4-{3-methyl-2-oxo-5-[4-(1H-pyrrolo[2,3-b]pyridin-5-yl)pyridin-2-yl]-2,3-dihydroimidazol-1-yl}phenyl)propionitrile ("A117")
1-methyl-3-(4-piperazin-1-yl-3-trifluoromethylphenyl)-4-[4-(3-trifluoromethyl-1H-pyrrolo[2,3-b]pyridin-5-yl)pyridin-2-yl]-1,3-dihydroimidazol-2-one ("A118")
1-methyl-3-(4-piperazin-1-ylphenyl)-4-[4-(1H-pyrrolo[2,3-b]pyridin-5-yl)-pyridin-2-yl]-1,3-dihydroimidazol-2-one ("A119")
1-methyl-3-(4-piperidin-4-ylphenyl)-4-[4-(1 H-pyrrolo[2,3-b]pyridin-5-yl)-pyridin-2-yl]-1,3-dihydroimidazol-2-one ("A120")
3-benzyl-1-methyl-4-[4-(1H-pyrrolo[2,3-b]pyridin-5-yl)pyridin-2-yl]-1,3-dihydroimidazol-2-one ("A121")
3-(4-tert-butylphenyl)-1-methyl-4-[4-(1 H-pyrrolo[2,3-b]pyridin-5-yl)pyridin-2-yl]-1,3-dihydroimidazol-2-one ("A122")
1-methyl-3-{4-[methyl-(2-methylaminoethyl)amino]-3-trifluoromethylphenyl}-4-[4-(1H-pyrrolo[2,3-b]pyridin-5-yl)pyridin-2-yl]-1,3-dihydroimidazol-2-one ("A123")
3-[4-(2-dimethylaminoethylamino)-3-trifluoromethylphenyl]-1-methyl-4-[4-(1H-pyrrolo[2,3-b]pyridin-5-yl)pyridin-2-yl]-1,3-dihydroimidazol-2-one ("A124")
3-[4-(4-aminopiperidin-1-yl)-3-trifluoromethylphenyl]-1-methyl-4-[4-(1H-pyrrolo[2,3-b]pyridin-5-yl)pyridin-2-yl]-1,3-dihydroimidazol-2-one ("A125")
1-methyl-3-[4-(methylpiperidin-4-ylamino)-3-trifluoromethylphenyl]-4-[4-(1H-pyrrolo[2,3-b]pyridin-5-yl)pyridin-2-yl]-1,3-dihydroimidazol-2-one ("A126")
1-methyl-3-[4-(piperidin-4-ylamino)-3-trifluoromethylphenyl]-4-[4-(1H-pyrrolo[2,3-b]pyridin-5-yl)pyridin-2-yl]-1,3-dihydroimidazol-2-one ("A127") and pharmaceutically usable salts, tautomers and stereoisomers thereof, including mixtures thereof in all ratios.

3. Process for the preparation of compounds of the formula I according to Claims 1-2 and pharmaceutically usable salts, tautomers and stereoisomers thereof,
**characterised in that** a compound of the formula I
in which Y denotes Hal
is converted into a compound of the formula I
in which Y denotes Het or Ar
in a Suzuki reaction,
and/or
a base or acid of the formula I is converted into one of its salts.

4. Medicaments comprising at least one compound of the formula I according to Claims 1-2 and/or pharmaceutically usable salts, tautomers and stereoisomers thereof, including mixtures thereof in all ratios, and optionally excipients and/or adjuvants.

5. Compounds of the formula I for use in the treatment of autoimmune diseases, inflammatory diseases, cardiovascular diseases, neurodegenerative diseases, allergy, asthma, pancreatitis, multiorgan failure, kidney diseases, blood platelet aggregation, cancer, sperm motility, transplant rejection, graft rejection and lung injuries.

6. Set (kit) consisting of separate packs of
(a) an effective amount of a compound of the formula I according to one or more of Claims 1 to 2, and/or pharmaceutically usable salts and stereoisomers thereof, including mixtures thereof in all ratios, and
(b) an effective amount of a further medicament active compound.

## Revendications

1. Composés de formule I 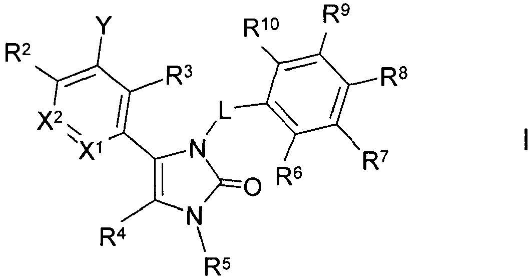 dans lesquels
X¹ désigne CR¹ ou N,
X² désigne CR¹ ou N,
où X¹ ou X² désigne N
et l'autre respectif désigne CR¹,
Y désigne Hét ou Ar,
L désigne [C(R^{a})₂]ₘ,
R¹, R², R³, R⁴ désignent H,
R⁵ désigne H ou A',
R⁶, R⁹, R¹⁰ désignent H,
R⁷ désigne H ou A"',
R⁸ désigne H, A", Hét¹, CN, Hal, NHHét¹ ou NA'Hét¹,
A désigne alkyle non ramifié ou ramifié ayant 1-10 atomes de C, où 1-7 atomes de H peuvent être remplacés par F et/ou Cl,
ou
alkyle cyclique ayant 3-7 atomes de C,
A' désigne alkyle non ramifié ou ramifié ayant 1-6 atomes de C, où 1-5 atomes de H peuvent être remplacés par F et/ou Cl,
A" désigne alkyle non ramifié ou ramifié ayant 1-10 atomes de C, où 1-7 atomes de H peuvent être remplacés par OH, CN, NH₂, CONH₂, COOH, COOA, NHCOA, -C=H, F, Cl et/ou Br, et/ou où un ou deux groupements CH et/ou CH₂ non adjacents peuvent être remplacés par O, NH, NA', S, SO, SO₂ et/ou des groupements CH=CH,
A"' désigne alkyle non ramifié ou ramifié ayant 1-10 atomes de C, où 1-7 atomes de H peuvent être remplacés par F et/ou Cl, et/ou où un ou deux groupements CH et/ou CH₂ non adjacents peuvent être remplacés par O, NH et/ou NA',
R^{a} désigne H ou A',
Hét désigne pyridinyle, pyrimidinyle, furyle, thiényle, oxazolyle, oxadiazolyle, imidazolyle, pyrrolyle, isoxazolyle, benzodioxolyle, dihydrobenzofuranyle, dihydrobenzodioxinyle, benzofuranyle, benzodioxanyle, indolyle, quinoléinyle, isoquinoléinyle, benzimidazolyle, benzothiadiazolyle, indazolyle, dihydrobenzimidazolyle, dihydroindolyle, imidazopyridinyle, pyrrolopyridinyle, pyrazolopyridinyle, 2,3-dihydropyrrolopyridinyle ou tétrahydronaphtyridinyle, où les radicaux peuvent également être mono-, di- ou trisubstitués par A, (CH₂)ₙNH₂, (CH₂)ₙNHA, (CH₂)ₙNA₂, NHCOA, (CH₂)ₙCONH₂, (CH₂)ₙCONHA, (CH₂)ₙCONA₂, CN, =O, Hal, (CH₂)ₙOH, (CH₂)ₙOA, COOH et/ou COOA,
Ar désigne phényle qui est non substitué ou mono-, di- ou trisubstitué par (CH₂)ₙOH et/ou (CH₂)ₙOA,
Hét¹ désigne pipéridinyle, pyrrolidinyle, morpholinyle, pipérazinyle, oxazolidinyle, tétrahydrothiényle, tétrahydropyranyle ou thiomorpholinyle, où les radicaux peuvent également être monoou disubstitués par =O, NH₂, pyridylméthyle et/ou A,
Hal désigne F, Cl, Br ou I,
m désigne 0 ou 1,
n désigne 0, 1, 2, 3 ou 4,
ainsi que les sels, tautomères et stéréoisomères pharmaceutiquement utilisables de ceux-ci, y compris des mélanges de ceux-ci en toutes proportions.

2. Composés selon la revendication 1, choisis dans le groupe constitué par
le 2-méthyl-2-{4-[3-méthyl-2-oxo-5-(4-quinoléin-3-ylpyridin-2-yl)-2,3-dihydroimidazol-1-yl]phényl}propionitrile (« A1 »)
le 2-méthyl-2-{4-[3-méthyl-2-oxo-5-(5-quinoléin-3-ylpyridin-3-yl)-2,3-dihydroimidazol-1-yl]phényl}propionitrile (« A2 »)
le 2-(4-{5-[4-(3-méthoxyphényl)pyridin-2-yl]-3-méthyl-2-oxo-2,3-dihydroimidazol-1-yl}phényl)-2-méthylpropionitrile (« A3 »)
la 5-(6-méthoxy-(3,4']bipyridinyl-2'-yl)-1-(4-pipérazin-1-yl-3-trifluorométhylphényl)-1,3-dihydroimidazol-2-one (« A4 »)
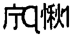 -(4-pipérazin-1-yl-3-trifluorométhylphényl)-5-(4-quinoléin-3-yl)-1,3-dihydroimidazol-2-one (« A5 »)
le 2-{4-[5-(2'-méthoxy-[4,4']bipyridinyl-2-yl)-3-méthyl-2-oxo-2,3-dihydroimidazol-1-yl]phényl}-2-méthylpropionitrile (« A6 »)
le 2-méthyl-2-(4-{3-méthyl-5-[4-(2-méthyl-3H-imidazo[4,5-b]pyridin-6-yl)pyridin-2-yl]-2-oxo-2,3-dihydroimidazol-1-yl}phényl)propionitrile (« A7 »)
le 2-{4-[5-(6-amino-[3,4']bipyridinyl-2'-yl)-3-méthyl-2-oxo-2,3-dihydroimidazol-1-yl]phényl}-2-méthylpropionitrile (« A8 »)
le 2-{4-[5-(6-méthoxy-[3,4']bipyridinyl-2'-yl)-3-méthyl-2-oxo-2,3-dihydroimidazol-1-yl]phényl}-2-méthylpropionitrile (« A9 »)
le 2-[4-(5-[3,4']bipyridinyl-2'-yl-3-méthyl-2-oxo-2,3-dihydroimidazol-1-yl)-phényl]-2-méthylpropionitrile (« A10 »)
le 2-[4-(5-[4,4']bipyridinyl-2-yl-3-méthyl-2-oxo-2,3-dihydroimidazol-1-yl)-phényl]-2-méthylpropionitrile (« A11 »)
le 2-méthyl-2-(4-{3-méthyl-2-oxo-5-[4-(1H-pyrrolo[2,3-b]pyridin-5-yl)-pyridin-2-yl]-2,3-dihydroimidazol-1-yl}phényl)propionitrile (« A12 »)
le 2-(4-{5-[4-(4-méthoxyphényl)pyridin-2-yl]-3-méthyl-2-oxo-2,3-dihydroimidazol-1-yl}phényl)-2-méthylpropionitrile (« A13 »)
le 2-(4-{5-[5-(4-méthoxyphényl)pyridin-3-yl]-3-méthyl-2-oxo-2,3-dihydroimidazol-1-yl}phényl)-2-méthylpropionitrile (« A14 »)
le 2-{4-[5-(6-méthoxy-[3,4']bipyridinyl-2'-yl)-2-oxo-2,3-dihydroimidazol-1-yl]phényl}-2-méthylpropionitrile (« A15 »)
la 1-[4-(2-amino-1,1-diméthyléthyl)phényl]-5-(4-quinoléin-3-ylpyridin-2-yl)-1,3-dihydroimidazol-2-one (« A16 »)
le 2-méthyl-2-{4-[2-oxo-5-(4-quinoléin-3-ylpyridin-2-yl)-2,3-dihydroimidazol-1-yl]phényl}propionitrile (« A17 »)
le 2-méthyl-2-(4-{3-méthyl-2-oxo-5-[4-(3-trifluorométhyl-1H-pyrrolo[2,3-b]pyridin-5-yl)pyridin-2-yl]-2,3-dihydroimidazol-1-yl}phényl)propionitrile (« A18 »)
la 1-méthyl-3-(4-p ipérazin-1-yl-3-trifluorométhylphényl)-4-(4-quinoléin-3-ylpyridin-2-yl)-1,3-dihydroimidazol-2-one (« A19 »)
la 4-(6-méthoxy-[3,4']bipyridinyl-2'-yl)-1-méthyl-3-(4-pipérazin-1-yl-3-trifluorométhylphényl)-1,3-dihydroimidazol-2-one (« A20 »)
le 2-{4-[3-méthyl-2-oxo-5-(4-quinoléin-3-ylpyridin-2-yl)-2,3-dihydroimidazol-1-yl]phényl}isobutyramide (« A21 »)
l'acide 2-méthyl-2-{4-[3-méthyl-2-oxo-5-(4-quinoléin-3-ylpyridin-2-yl)-2,3-dihydroimidazol-1-yl]phényl}propionique (« A22 »)
le 2-méthyl-2-(4-{3-méthyl-2-oxo-5-[4-(1H-pyrrolo[2,3-b]pyridin-4-yl)-pyridin-2-yl]-2,3-dihydroimidazol-1-yl}phényl)propionitrile (« A23 »)
le 2-(4-{3-éthyl-2-oxo-5[4-(1H-pyrrolo[2,3-b]pyridin-5-yl)pyridin-2-yl]-2,3-dihydroimidazol-1-yl}phényl)-2-méthylpropionitrile (« A24 »)
la 1-méthyl-3-(4-pipérazin-1-yl-3-trifluorométhylphényl)-4-[4-(1H-pyrrolo-[2,3-b]pyridin-5-yl)pyridin-2-yl]-1,3-dihydroimidazol-2-one (« A25 »)
le 2-méthyl-2-{4-[3-méthyl-2-oxo-5-(4-thiophén-3-ylpyridin-2-yl)-2,3-dihydroimidazol-1-yl]phényl}propionitrile (« A26 »)
le 2-méthyl-2-(4-{3-méthyl-2-oxo-5-[4-(2-trifluorométhyl-1H-pyrrolo[2,3-b]-pyridin-5-yl)pyridin-2-yl]-2,3-dihydroimidazol-1-yl}phényl)propionitrile (« A27 »)
le 2-méthyl-2-(4-{2-oxo-5-[4-(1H-pyrrolo[2,3-b]pyridin-5-yl)pyridin-2-yl]-2,3-dihydroimidazol-1-yl}phényl)propionitrile (« A28 »)
le 5-(2-{3-[4-(cyanodiméthylméthyl)phényl]-1-méthyl-2-oxo-2,3-dihydro-1H-imidazol-4-yl}pyridin-4-yl)-1H-pyrrolo[2,3-b]pyridine-3-carboxylate de méthyle (« A29 »)
la 3-[4-(2-amino-1,1-diméthyléthyl)phényl]-1-méthyl-4-(4-quinoléin-3-yl-pyridin-2-yl)-1,3-dihydroimidazol-2-one (« A30 »)
le (4-{2-oxo-5-[4-(1H-pyrrolo[2,3-b]pyridin-5-yl)pyridin-2-yl]-2,3-dihydroimidazol-1-yl}phényl)acétonitrile (« A31 »)
le (4-{3-méthyl-2-oxo-5-[4-(1H-pyrrolo[2,3-b]pyridin-5-yl)pyridin-2-yl]-2,3-dihydroimidazol-1-yl}phényl)acétonitrile (« A32 »)
l'acide 2-méthyl-2-(4-{3-méthyl-2-oxo-5-[4-(1H-pyrrolo[2,3-b]pyridin-5-yl)pyridin-2-yl]-2,3-dihydroimidazol-1-yl}phényl)propionique (« A33 »)
le 2-(4-{3-méthyl-2-oxo-5-[4-(1H-pyrrolo[2,3-b]pyridin-5-yl)pyridin-2-yl]-2,3-dihydroimidazol-1-yl}phényl)isobutyramide (« A34 »)
la 3-[4-(2-amino-1,1-diméthytéthyl)phényl]-1-méthyl-4-[4-(1H-pyrrolo-[2,3-b]pyridin-5-yl)pyridin-2-yl]-1,3-dihydroimidazol-2-one (« A35 »)
le 2-méthyl-2-{4-[3-méthyl-2-oxo-5-(4-quinoléin-3-ylpyridin-2-yl)-2,3-dihydroimidazol-1-yl]phényl}propionate d'éthyle (« A36 »)
le 6-amino-2'-{3-[4-(cyanodiméthylméthyl)phényl]-1-méthyl-2-oxo-2,3-dihydro-1H-imidazol-4-yl}-[3,4']bipyridinyl-5-carbonitrile (« A37 »)
le 2-(4-{5-[4-(3H-imidazo[4,5-b]pyridin-6-yl)pyridin-2-yl]-3-méthyl-2-oxo-2,3-dihydroimidazol-1-yl}phényl)-2-méthylpropionitrile (« A38 »)
le 2-{4-[5-(6-hydroxy-[3,4']bipyridinyl-2'-yl)-3-méthyl-2-oxo-2,3-dihydroimidazol-1-yl]phényl}-2-méthylpropionitrile (« A39 »)
le 2-{4-[5-(6-amino-5-trifluorométhyl-[3,4']bipyridinyl-2'-yl)-3-méthyl-2-oxo-2,3-dihydroimidazol-1-yl]phényl}-2-méthylpropionitrile (« A40 »)
le 2-méthyl-2-(4-{3-méthyl-2-oxo-5-[4-(1H-pyrazolo[3,4-b]pyridin-5-yl)-pyridin-2-yl]-2,3-dihydroimidazol-1-yl}phényl)propionitrile (« A41 »)
le 2-méthyl-2-{4-[3-méthyl-5-(6-méthylamino-[3,4']bipyridinyl-2'-yl)-2-oxo-2,3-dihydroimidazol-1-yl]phényl}propionitrile (« A42 »)
le 2-(4-{5-[4-(2,3-dihydro-1H-pyrrolo[2,3-b]pyridin-5-yl)pyridin-2-yl]-3-méthyl-2-oxo-2,3-dihydroimidazol-1-yl}phényl)-2-méthylpropionitrile (« A43 »)
le 2-méthyl-2-(4-{3-méthyl-2-oxo-5-[4-(2-oxo-2,3-dihydro-1H-pyrrolo[2,3-b]pyridin-5-yl)pyridin-2-yl]-2,3-dihydroimidazol-1-yl}phényl)propionitrile (« A44 »)
le 2-méthyl-2-(4-{3-méthyl-2-oxo-5-[4-(7-oxo-5,6,7,8-tétrahydro-[1,8]-naphtyridin-3-yl)pyridin-2-yl]-2,3-dihydroimidazol-1-yl}phényl)propionitrile (« A45 »)
la 1-méthyl-3-(4-pipérazin-1-yl-3-trifluorométhylphényl)-4-[4-(1H-pyrazolo-[3,4-b]pyridin-5-yl)pyridin-2-yl]-1,3-dihydroimidazol-2-one (« A46 »)
la 1-méthyl-3-phényl-4-[4-(1H-pyrrolo[2,3-b]pyridin-5-yl)pyridin-2-yl]-1,3-dihydroimidazol-2-one (« A47 »)
le 4-{3-méthyl-2-oxo-5-[4-(1H-pyrrolo[2,3-b]pyridin-5-yl)pyridin-2-yl]-2,3-dihydroimidazol-1-yl}benzonitrile (« A48 »)
la 1-méthyl-3-[4-(4-méthylpipérazin-1-yl)-3-trifluorométhylphényl]-4-[4-(1 H-pyrrolo[2,3-b]pyridin-5-yl)pyridin-2-yl]-1,3-dihydroimidazol-2-one (« A49 »)
la 1-méthyl-3-[4-(4-méthylpipérazin-1-yl)phényl]-4-[4-(1 H-pyrrolo[2,3-b]-pyridin-5-yl)pyridin-2-yl]-1,3-dihydroimidazol-2-one (« A50 »)
la 3-(4-fluoro-3-trifluorométhylphényl)-1-méthyl-4-[4-(1H-pyrrolo[2,3-b]-pyridin-5-yl)pyridin-2-yl]-1,3-dihydroimidazol-2-one (« A51 »)
le 2-(4-{5-[4-(1H-indazol-5-yl)pyridin-2-yl]-3-méthyl-2-oxo-2,3-dihydroimidazol-1-yl}phényl)-2-méthylpropionitrile (« A52 »)
le 2-(4-{5-[4-(1H-indol-5-yl)pyridin-2-yl]-3-méthyl-2-oxo-2,3-dihydroimidazol-1-yl}phényl)-2-méthylpropionitrile (« A53 »)
le 2-méthyl-2-(4-{3-méthyl-5-[4-(1-méthyl-1H-pyrrolo[2,3-b]pyridin-5-yl)-pyridin-2-yl]-2-oxo-2,3-dihydroimidazol-1-yl}phényl)propionitrile (« A54 »)
le 3-{3-méthyl-2-oxo-5-[4-(1H-pyrrolo[2,3-b]pyridin-5-yl)pyridin-2-yl]-2,3-dihydroimidazol-1-yl}benzonitrile (« A55 »)
la 4-(6-amino-5-trifluorométhyl-[3,4']bipyridinyl-2'-yl)-1-méthyl-3-(4-pipérazin-1-yl-3-trifluorométhylphényl)-1,3-dihydroimidazol-2-one (« A56 »)
la 4-[4-(1 H-indol-5-yl)pyridin-2-yl]-1-méthyl-3-(4-pipérazin-1-yl-3-trifluorométhylphényl)-1,3-dihydroimidazol-2-one (« A57 »)
la 3-(4-chloro-3-méthoxyphényl)-1-méthyl-4-[4-(1 H-pyrrolo[2,3-b]pyridin-5-yl)pyridin-2-yl]-1,3-dihydroimidazol-2-one (« A58 »)
la 4-(6-amino-[3,4']bipyridinyl-2'-yl)-1-méthyl-3-(4-pipérazin-1-yl-3-trifluorométhylphényl)-1,3-dihydroimidazol-2-one (« A59 »)
la 4-[4-(4-aminophényl)pyridin-2-yl]-1-méthyl-3-(4-pipérazin-1-yl-3-trifluorométhylphényl)-1,3-dihydroimidazol-2-one (« A60 »)
le N-{2'-[1-méthyl-2-oxo-3-(4-pipérazin-1-yl-3-trifluorométhylphényl)-2,3-dihydro-1H-imidazol-4-yl]-[3,4']bipyridinyl-6-yl}méthanesulfonamide (« A61 »)
le N-(4-{2-[1-méthyl-2-oxo-3-(4-pipérazin-1-yl-3-trifluorométhylphényl)-2,3-dihydro-1H-imidazol-4-yl]pyridin-4-yl}phényl)méthanesulfonamide (« A62 »)
le 3-{3-méthyl-2-oxo-5-[4-(1H-pyrazolo[3,4-b]pyridin-5-yl)pyridin-2-yl]-2,3-dihydroimidazol-1-yl}benzonitrile (« A63 »)
le 3-[5-(6-amino-5-trifluorométhyl-[3,4']bipyridinyl-2'-yl)-3-méthyl-2-oxo-2,3-dihydroimidazol-1-yl]benzonitrile (« A64 »)
le 6-amino-2'-[1-méthyl-2-oxo-3-(4-pipérazin-1-yl-3-trifluorométhylphényl)-2,3-dihydro-1H-imidazol-4-yl]-[3,4']bipyridinyl-5-carbonitrile (« A65 »)
la 3-(3-éthylphényl)-1-méthyl-4-[4-(1 H-pyrrolo[2,3-b]pyridin-5-yl)pyridin-2-yl]-1,3-dihydroimidazol-2-one (« A66 »)
la 1-méthyl-4-[4-(1H-pyrrolo[2,3-b]pyridin-5-yl)pyridin-2-yl]-3-(2-trifluorométhylphényl)-1,3-dihydroimidazol-2-one (« A67 »)
le N-{2'-[3-(3-cyanophényl)-1-méthyl-2-oxo-2,3-dihydro-1H-imidazol-4-yl]-5-trifluorométhyl-[3,4']bipyridinyl-6-yl}acétamide (« A68 »)
le 2-{3-méthyl-2-oxo-5-[4-(1H-pyrrolo[2,3-b]pyridin-5-yl)pyridin-2-yl]-2,3-dihydroimidazol-1-yl}benzonitrile (« A69 »)
le 3-amino-6-(2-{3-[4-(cyanodiméthylméthyl)phényl]-1-méthyl-2-oxo-2,3-dihydro-1H-imidazol-4-yl}pyridin-4-yl)pyrazine-2-carbonitrile (« A70 »)
la 1-méthyl-3-(4-morpholin-4-yl-3-trifluorométhylphényl)-4-[4-(1H-pyrrolo-[2,3-b]pyridin-5-yl)pyridin-2-yl]-1,3-dihydroimidazol-2-one (« A71 »)
la 3-[4-(4-acétylpipérazin-1-yl)-3-trifluorométhylphényl]-1-méthyl-4-[4-(1H-pyrrolo[2,3-b]pyridin-5-yl)pyridin-2-yl]-1,3-dihydroimidazol-2-one (« A72 »)
la 3-(3-méthoxyphényl)-1-méthyl-4-[4-(1H-pyrrolo[2,3-b]pyridin-5-yl)-pyridin-2-yl]-1,3-dihydroimidazol-2-one (« A73 »)
la 3-[4-(4-méthanesulfonylpipérazin-1-yl)-3-trifluorométhylphényl]-1-méthyl-4-[4-(1H-pyrrolo[2,3-b]pyridin-5-yl)pyridin-2-yl]-1,3-dihydroimidazol-2-one (« A74 »)
le 3-{3-difluorométhyl-2-oxo-5-[4-(1H-pyrrolo[2,3-b]pyridin-5-yl)pyridin-2-yl]-2,3-dihydroimidazol-1-yl}benzonitrile (« A75 »)
la 1-méthyl-4-[4-(1H-pyrrolo[2,3-b]pyridin-5-yl)pyridin-2-yl]-3-(2-trifluorométhoxyphényl)-1,3-dihydroimidazol-2-one (« A76 »)
la 3-[4-(4-éthylpipérazin-1-yl)-3-trifluorométhylphényl]-1-méthyl-4-[4-(1H-pyrrolo[2,3-b]pyridin-5-yl)pyridin-2-yl]-1,3-dihydroimidazol-2-one (« A77 »)
le 2-méthyl-2-(4-{3-méthyl-2-oxo-5-[4-(5H-pyrrolo[2,3-b]pyrazin-2-yl)-pyridin-2-yl]-2,3-dihydroimidazol-1-yl}phényl)propionitrile (« A78 »)
la 1-méthyl-4-[4-(1H-pyrrolo[2,3-b]pyridin-5-yl)pyridin-2-yl]-3-m-tolyl-1,3-dihydroimidazol-2-one (« A79 »)
la 3-(3-isopropylphényl)-1-méthyl-4-[4-(1H-pyrrolo[2,3-b]pyridin-5-yl)-pyridin-2-yl]-1,3-dihydroimidazol-2-one (« A80 »)
le 2-méthyl-2-{4-[3-méthyl-2-oxo-5-(4-pyrido[2,3-b]pyrazin-7-ylpyridin-2-yl)-2,3-dihydroimidazol-1-yl]phényl}propionitrile (« A81 »)
le 5-[5-(6-amino-5-trifluorométhyl-[3,4']bipyridinyl-2'-yl)-3-méthyl-2-oxo-2,3-dihydroimidazol-1-yl]-2-(4-méthylpipérazin-1-yl)benzonitrile (« A82 »)
le 5-{3-méthyl-2-oxo-5-[4-(1H-pyrrolo[2,3-b]pyridin-5-yl)pyridin-2-yl]-2,3-dihydroimidazol-1-yl}-2-(4-méthylpipérazin-1-yl)benzonitrile (« A83 »)
le 2-{4-[5-(6-amino-5-phényl-[3,4']bipyridinyl-2'-yl)-3-méthyl-2-oxo-2,3-dihydroimidazol-1-yl]phényl}-2-méthylpropionitrile (« A84 »)
le 2-méthyl-2-(4-{3-méthyl-5-[4-(2-méthyl-1H-pyrrolo[2,3-b]pyridin-5-yl)-pyridin-2-yl]-2-oxo-2,3-dihydroimidazol-1-yl}phényl)propionitrile (« A85 »)
la 4-[4-(2-tertio-butyl-1H-pyrrolo[2,3-b]pyridin-5-yl)pyridin-2-yl]-1-méthyl-3-[4-(4-méthylpipérazin-1-yl)-3-trifluorométhylphényl]-1,3-dihydroimidazol-2-one (« A86 »)
la 1-méthyl-3-[4-(4-méthylpipérazin-1-yl)-3-trifluorométhylphényl]-4-[4-(2-trifluorométhyl-1H-pyrrolo[2,3-b]pyridin-5-yl)pyridin-2-yl]-1,3-dihydroimidazol-2-one (« A87 »)
la 1-méthyl-3-[4-(4-méthylpipérazin-1-yl)-3-trifluorométhylphényl]-4-[4-(2-méthyl-1H-pyrrolo[2,3-b]pyridin-5-yl)pyridin-2-yl]-1,3-dihydroimidazol-2-one (« A88 »)
la 3-[4-(4-acétylpipérazin-1-yl)-3-trifluorométhylphényl]-4-[4-(1H-indol-5-yl)pyridin-2-yl]-1-méthyl-1,3-dihydroimidazol-2-one (« A89 »)
la 1-méthyl-3-(3-phénoxyphényl)-4-[4-(1H-pyrrolo[2,3-b]pyridin-5-yl)-pyridin-2-yl]-1,3-dihydroimidazol-2-one (« A90 »)
le 2-méthyl-2-{4-[3-méthyl-2-oxo-5-(4-quinazolin-6-ylpyridin-2-yl)-2,3-dihydroimidazol-1-yl]phényl}propionitrile (« A91 »)
le 2-(4-{5-[4-(2-tertio-butyl-1H-pyrrolo[2,3-b]pyridin-5-yl)pyridin-2-yl]-3-méthyl-2-oxo-2,3-dihydroimidazol-1-yl}phényl)-2-méthylpropionitrile (« A92 »)
le 2-(4-{5-[4-(4-amino-3-trifluorométhylphényl)pyridin-2-yl]-3-méthyl-2-oxo-2,3-dihydroimidazol-1-yl}phényl)-2-méthylpropionitrile (« A93 »)
le 2-(4-{5-[4-(4-amino-3-trifluorométhylphényl)pyridin-2-yl]-3-méthyl-2-oxo-2,3-dihydroimidazol-1-yl}phényl)-2-méthylpropionitrile (« A94 »)
le 2-méthyl-2-{4-[3-méthyl-5-(6-méthylaminométhyl-[3,4']bipyridinyl-2'-yl)-2-oxo-2,3-dihydroimidazol-1-yl]phényl}propionitrile (« A95 »)
le 3-fluoro-5-{3-méthyl-2-oxo-5-[4-(1H-pyrrolo[2,3-b]pyridin-5-yl)pyridin-2-yl]-2,3-dihydroimidazol-1-yl}benzonitrile (« A96 »)
la 4-(6-amino-5-trifluorométhyl-[3,4']bipyridinyl-2'-yl)-3-(4-fluoro-3-trifluorométhylphényl)-1-méthyl-1,3-dihydroimidazol-2-one (« A97 »)
le 3-{3-méthyl-2-oxo-5-[4-(1H-pyrrolo[2,3-b]pyridin-5-yl)pyridin-2-yl]-2,3-dihydroimidazol-1-yl}-5-(4-méthylpipérazin-1-yl)benzonitrile (« A98 »)
le 2-[4-(5-{4-[3-(2-amino-6-éthylpyrimidin-4-yl)-1H-pyrrolo[2,3-b]pyridin-5-yl]pyridin-2-yl}-3-méthyl-2-oxo-2,3-dihydroimidazol-1-yl)phényl]-2-méthylpropionitrile (« A99 »)
le 2-méthyl-2-(4-{3-méthyl-2-oxo-5-[4-(1H-pyrrolo[2,3-b]pyridin-3-yl)-pyridin-2-yl]-2,3-dihydroimidazol-1-yl}phényl)propionitrile (« A100 »)
le 2-(4-{5-[4-(1-aminoisoquinoléin-4-yl)pyridin-2-yl]-3-méthyl-2-oxo-2,3-dihydroimidazol-1-yl}phényl)-2-méthylpropionitrile (« A101 »)
la 4-[4-(1 H-indol-5-yl)pyridin-2-yl]-1-méthyl-3-[4-(4-méthylpipérazin-1-yl)-3-trifluorométhylphényl]-1,3-dihydroimidazol-2-one (« A102 »)
la 4-(6-amino-5-trifluorométhyl-[3,4']bipyridinyl-2'-yl)-1-méthyl-3-[4-(4-méthylpipérazin-1-yl)-3-trifluorométhylphényl]-1,3-dihydroimidazol-2-one (« A103 »)
la 1-méthyl-4-[4-(2-méthyl-1H-pyrrolo[2,3-b]pyridin-5-yl)pyridin-2-yl]-3-(3-phénoxyphényl)-1,3-dihydroimidazol-2-one (« A104 »)
la 4-[4-(3-fluoro-1 H-pyrrolo[2,3-b]pyridin-5-yl)pyridin-2-yl]-3-(4-fluoro-3-trifluorométhylphényl)-1-méthyl-1,3-dihydroimidazol-2-one (« A105 »)
la 1-méthyl-3-[4-(4-pyridin-3-ylméthylpipérazin-1-yl)-3-trifluorométhylphényl]-4-[4-(1H-pyrrolo[2,3-b]pyridin-5-yl)pyridin-2-yl]-1,3-dihydroimidazol-2-one (« A106 »)
le 2-(4-{5-[4-(4-amino-3-fluoro-5-trifluorométhylphényl)pyridin-2-yl]-3-méthyl-2-oxo-2,3-dihydroimidazol-1-yl}phényl)-2-méthylpropionitrile (« A107 »)
la 3-(4-méthoxybenzyl)-1-méthyl-4-[4-(1 H-pyrrolo[2,3-b]pyridin-5-yl)-pyridin-2-yl]-1,3-dihydroimidazol-2-one (« A108 »)
la 3-(4-méthoxybenzyl)-1-méthyl-4-[4-(1H-pyrazolo[3,4-b]pyridin-5-yl)-pyridin-2-yl]-1,3-dihydroimidazol-2-one (« A109 »)
la 4-(6-amino-5-trifluorométhyl-[3,4']bipyridinyl-2'-yl)-3-(4-méthoxybenzyl)-1-méthyl-1,3-dihydroimidazol-2-one (« A110 »)
le N-{2'-[3-(4-méthoxybenzyl)-1-méthyl-2-oxo-2,3-dihydro-1H-imidazol-4-yl]-5-trifluorométhyl-[3,4']bipyridinyl-6-yl}acétamide (« A111 »)
la 4-[4-(2-tertio-butyl-1H-pyrrolo[2,3-b]pyridin-5-yl)pyridin-2-yl]-3-[3-(1,1-difluoroéthyl)-4-fluorophényl]-1-méthyl-1,3-dihydroimidazol-2-one (« A112 »)
le N-(2-méthyl-2-{4-[3-méthyl-2-oxo-5-(4-quinoléin-3-ylpyridin-2-yl)-2,3-dihydroimidazol-1-yl]phényl}propyl)acétamide (« A113 »)
la 2'-[1-méthyl-2-oxo-3-(4-pipérazin-1-yl-3-trifluorométhylphényl)-2,3-dihydro-1H-imidazol-4-yl]-1H-[3,4']bipyridinyl-6-one (« A114 »)
le N-(2'-{3-[4-(cyanodiméthylméthyl)phényl]-1-méthyl-2-oxo-2,3-dihydro-1H-imidazol-4-yl}-[3,4']bipyridinyl-6-yl)acétamide (« A115 »)
le 2-méthyl-2-(4-{3-méthyl-2-oxo-5-[5-(1H-pyrrolo[2,3-b]pyridin-5-yl)-pyridin-3-yl]-2,3-dihydroimidazol-1-yl}phényl)propionitrile (« A116 »)
le 2-(4-{3-méthyl-2-oxo-5-[4-(1H-pyrrolo[2,3-b]pyridin-5-yl)pyridin-2-yl]-2,3-dihydroimidazol-1-yl}phényl)propionitrile (« A117 »)
la 1-méthyl-3-(4-pipérazin-1-yl-3-trifluorométhylphényl)-4-[4-(3-trifluorométhyl-1 H-pyrrolo[2,3-b]pyridin-5-yl)pyridin-2-yl]-1,3-dihydroimidazol-2-one (« A118 »)
la 1-méthyl-3-(4-pipérazin-1-ylphényl)-4-[4-(1H-pyrrolo[2,3-b]pyridin-5-yl)pyridin-2-yl]-1,3-dihydroimidazol-2-one (« A119 »)
la 1-méthyl-3-(4-pipéridin-4-ylphényl)-4-[4-(1H-pyrrolo[2,3-b]pyridin-5-yl)pyridin-2-yl]-1,3-dihydroimidazol-2-one (« A120 »)
la 3-benzyl-1-méthyl-4-[4-(1H-pyrrolo[2,3-b]pyridin-5-yl)pyridin-2-yl]-1,3-dihydroimidazol-2-one (« A121 »)
la 3-(4-tertio-butylphényl)-1-méthyl-4-[4-(1H-pyrrolo[2,3-b]pyridin-5-yl)-pyridin-2-yl]-1,3-dihydroimidazol-2-one (« A122 »)
la 1-méthyl-3-{4-[méthyl-(2-méthylaminoéthyl)amino]-3-trifluorométhylphényl}-4-[4-(1H-pyrrolo[2,3-b]pyridin-5-yl)pyridin-2-yl]-1,3-dihydroimidazol-2-one (« A123 »)
la 3-[4-(2-diméthylaminoéthylamino)-3-trifluorométhylphényl]-1-méthyl-4-[4-(1 H-pyrrolo[2,3-b]pyridin-5-yl)pyridin-2-yl]-1,3-dihydroimidazol-2-one (« A124 »)
la 3-[4-(4-aminopipéridin-1-yl)-3-trifluorométhylphényl]-1-méthyl-4-[4-(1H-pyrrolo[2,3-b]pyridin-5-yl)pyridin-2-yl]-1,3-dihydroimidazol-2-one (« A125 »)
la 1-méthyl-3-[4-(méthylpipéridin-4-ylamino)-3-trifluorométhylphényl]-4-[4-(1 H-pyrrolo[2,3-b]pyridin-5-yl)pyridin-2-yl]-1,3-dihydroimidazol-2-one (« A126 »)
la 1-méthyl-3-[4-(pipéridin-4-ylamino)-3-trifluorométhylphényl]-4-[4-(1H-pyrrolo[2,3-b]pyridin-5-yl)pyridin-2-yl]-1,3-dihydroimidazol-2-one (« A127 »)
ainsi que les sels, tautomères et stéréoisomères pharmaceutiquement utilisables de ceux-ci, y compris des mélanges de ceux-ci en toutes proportions.

3. Procédé de préparation de composés de formule I selon les revendications 1-2 et de sels, tautomères et stéréoisomères pharmaceutiquement utilisables de ceux-ci,
**caractérisé en ce qu'**un composé de formule I
dans lequel Y désigne Hal
est converti en un composé de formule I
dans lequel Y désigne Hét ou Ar
dans une réaction de Suzuki,
et/ou
une base ou un acide de formule I est converti(e) en l'un de ses sels.

4. Médicaments comprenant au moins un composé de formule I selon les revendications 1-2 et/ou des sels, tautomères et stéréoisomères pharmaceutiquement utilisables de celui-ci, y compris des mélanges de ceux-ci en toutes proportions, et éventuellement des excipients et/ou des adjuvants.

5. Composés de formule I pour une utilisation dans le traitement de maladies auto-immunes, de maladies inflammatoires, de maladies cardiovasculaires, de maladies neurodégénératives, des allergies, de l'asthme, de la pancréatite, du syndrome d'insuffisance multi-viscérale, de maladies rénales, de l'agrégation plaquettaire, du cancer, de la motilité du sperme, du rejet de transplant, du rejet de greffe et des lésions pulmonaires.

6. Ensemble (kit) constitué de conditionnements séparés
(a) d'une quantité efficace d'un composé de formule I selon l'une ou plusieurs parmi les revendications 1 à 2, et/ou de sels et stéréoisomères pharmaceutiquement utilisables de celui-ci, y compris des mélanges de ceux-ci en toutes proportions,
et
(b) d'une quantité efficace d'un autre composé actif médicamenteux.
